(19) Europäisches Patentamt
European Patent Office
Office européen des brevets

(11) EP 2 578 084 A1

(12) **EUROPEAN PATENT APPLICATION**
published in accordance with Art. 153(4) EPC

(43) Date of publication:
**10.04.2013 Bulletin 2013/15**

(21) Application number: **11786585.7**

(22) Date of filing: **23.05.2011**

(51) Int Cl.:
***A01N 43/90*** *(2006.01)* ***A01N 47/18*** *(2006.01)*
***A01N 55/00*** *(2006.01)* ***A01P 7/04*** *(2006.01)*
***C07D 491/052*** *(2006.01)* ***C07D 493/04*** *(2006.01)*
***C07F 7/18*** *(2006.01)*

(86) International application number:
**PCT/JP2011/061730**

(87) International publication number:
**WO 2011/148886 (01.12.2011 Gazette 2011/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **24.05.2010 JP 2010118397**

(71) Applicant: **Meiji Seika Pharma Co., Ltd.**
**Tokyo 104-8002 (JP)**

(72) Inventors:
- **GOTO Kimihiko**
**Yokohama-shi**
**Kanagawa 222-8567 (JP)**
- **HORIKOSHI Ryo**
**Yokohama-shi**
**Kanagawa 222-8567 (JP)**
- **OYAMA Kazuhiko**
**Yokohama-shi**
**Kanagawa 222-8567 (JP)**
- **FUKUDA Yoshimasa**
**Yokohama-shi**
**Kanagawa 222-8567 (JP)**
- **NAKANISHI Nozomu**
**Yokohama-shi**
**Kanagawa 222-8567 (JP)**
- **TANI Masato**
**Odawara-shi**
**Kanagawa 250-0852 (JP)**
- **MINOWA Nobuto**
**Yokohama-shi**
**Kanagawa 222-8567 (JP)**
- **MITOMI Masaaki**
**Yokohama-shi**
**Kanagawa 222-8567 (JP)**
- **OMURA Satoshi**
**Tokyo 157-0076 (JP)**
- **SUNAZUKA Toshiaki**
**Funabashi-shi**
**Chiba 273-0043 (JP)**
- **TOMODA Hiroshi**
**Chofu-shi**
**Tokyo 182-0035 (JP)**

(74) Representative: **Regimbeau**
**139, rue Vendôme**
**69477 Lyon Cedex 06 (FR)**

# (54) NOXIOUS ORGANISM CONTROL AGENT

(57) The present invention provides a composition for use as a harmful organism control agent comprising as an active ingredient one or more of compounds represented by formula (I) or salts thereof and an agriculturally or zootechnically acceptable carrier.

EP 2 578 084 A1

[Chemical formula 1]

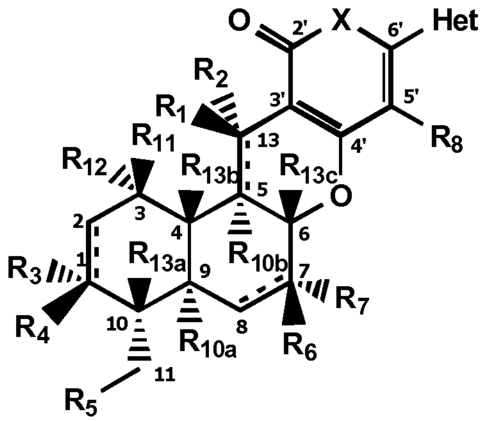

(I)

wherein Het represents pyridyl; X represents an oxygen atom; $R_1$, $R_2$, $R_3$, $R_7$, $R_{10a}$, $R_{10b}$, $R_{11}$, and $R_{12}$ represent a hydrogen atom; $R_4$, $R_5$, and $R_6$ represent a hydrogen atom, hydroxyl, optionally substituted $C_{1-18}$ alkylcarbonyloxy, optionally substituted $C_{1-18}$ alkylsulfonyloxy, optionally substituted arylcarbonyloxy, $C_{1-6}$ alkyloxy-$C_{1-6}$ alkyloxy, $C_{1-6}$ alkyloxy-$C_{1-6}$ alkyloxy-$C_{1-6}$ alkyloxy; $R_8$ represents a hydrogen atom; and $R_{13a}$, $R_{13b}$, and $R_{13c}$ represent methyl.

## Description

CROSS-REFERENCE TO RELATED APPLICATIONS

This application claims the benefit of priority on the basis of the prior Japanese Patent Application No. 2010-118397 (filed on date: May 24, 2010), and the entire disclosure of which whole description in the Japanese patent application is incorporated herein by reference.

BACKGROUND OF THE INVENTION

Technical Field

The present invention relates to a harmful organism control agent comprising as an active ingredient a pyripyropene analogue.

Background Art

As have hitherto been described in Japanese Patent Application Laid-Open No. 360895/1992 (patent document 1) and Journal of Antibiotics (1993), 46(7), 1168-9 (non-patent document 1), pyripyropene A has ACAT (acyl-CoA: cholesterol acyltransferase) inhibitory activity and is expected to be applied, for example, to treatment of diseases induced by cholesterol accumulation.
Further, pyripyropene analogues and derivatives and ACAT inhibitory activity thereof are described in Journal of Society of Synthetic Organic Chemistry, Japan (1998), Vol. 56, No. 6, pp. 478-488 (non-patent document 2), WO 94/09417 (patent document 2), Japanese Patent Application Laid-Open No. 259569/1996 (patent document 3), Japanese Patent Application Laid-Open No. 269062/1996 (patent document 4), and WO 2009/081957 (patent document 5).
Furthermore, Applied and Environmental Microbiology (1995), 61(12), 4429-35 (non-patent document 3) describes that pyripyropene A has insecticidal activity against larvae of Helicoverpa zea. Furthermore, WO 2004/060065 (patent document 6) describes that pyripyropene A has insecticidal activity against Plutella xylostella L larvae and Tenebrio molitor L. In these documents, however, there is no specific description on insecticidal activity of pyripyropene A against other pests. However, none of the above documents describes details of insecticidal activity of pyripyropene analogues and derivatives.
WO 2006/129714 (patent document 7), WO 2008/066153 (patent document 8), and WO 2009/081851 (patent document 9) describe that derivatives having an ester group at the 1-, 7-, or 11-position of the pyripyropene derivative have insecticidal activity.
Up to now, many harmful organism control agents having insecticidal activity have been reported. However, insect species, which are resistant to or can be hardly controlled by these harmful organism control agents have been found and further have a problem with safety against human and animals. Accordingly, the development of a novel harmful organism control agent having potent insecticidal activity has still been desired.

PRIOR ART DOCUMENTS

Patent documents

Patent document 1: Japanese Patent Application Laid-Open No. 360895/1992
Patent document 2: WO 94/09417
Patent document 3: Japanese Patent Application Laid-Open No. 259569/1996
Patent document 4: Japanese Patent Application Laid-Open No. 269062/1996
Patent document 5: WO 2009/081957
Patent document 6: WO 2004/060065
Patent document 7: WO 2006/129714
Patent document 8: WO 2008/066153
Patent document 9: W02009/081851

Non-patent documents

Non-patent document 1: Journal of Antibiotics (1993), 46(7), 1168-9

Non-patent document 2: Journal of Society of Synthetic Organic Chemistry, Japan (1998), Vol. 56, No. 6, pp. 478-488
Non-patent document 3: Applied and Environmental Microbiology (1995), 61(12), 4429-35

SUMMARY OF THE INVENTION

Problems to be Solved by the Invention

The present inventors have now newly found that pyripyropene analogue has a significant harmful organism control effect. The present invention has been made based on such finding.

Accordingly, an object of the present invention is to provide a composition as a harmful organism control agent, the composition comprising a pyripyropene analogue.

Means for Solving the Problems

According to the present invention, there is provided a composition for use as a harmful organism control agent, the composition comprising as an active ingredient at least one of compounds represented by formula (I) or salts thereof and an agriculturally or zootechnically acceptable carrier:

[Chemical formula 1]

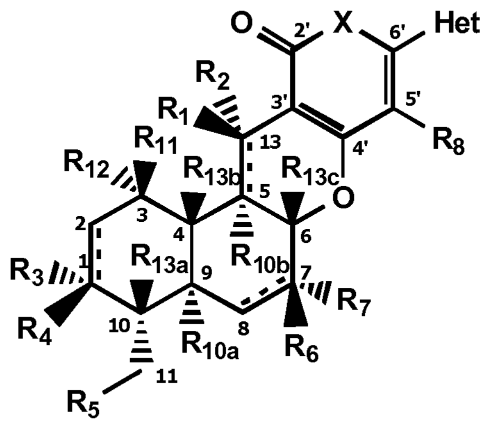

(I)

wherein

Het represents optionally substituted heterocyclic group, optionally substituted phenyl,
optionally substituted $C_{1-18}$ alkyl, or
optionally substituted $C_{2-18}$ alkenyl,
X represents an oxygen atom or $NR_9$ wherein $R_9$ represents a hydrogen atom, $C_{1-6}$ alkyl, or aryl $C_{1-6}$ alkyl,
$R_1$, $R_2$, $R_3$, $R_4$, $R_5$, $R_6$, $R_7$, $R_{10a}$, $R_{10b}$, $R_{11}$, and $R_{12}$, which may be the same or different, each independently represent a hydrogen atom,
hydroxyl, optionally substituted $C_{1-18}$ alkylaminocarbonyloxy,
optionally substituted $C_{1-18}$ alkylcarbonyloxy, adamantylcarbonyloxy,
optionally substituted aryl $C_{1-6}$ alkylcarbonyloxy,
optionally substituted $C_{2-6}$ alkenylcarbonyloxy,
optionally substituted $C_{2-6}$ alkynylcarbonyloxy,
optionally substituted saturated or unsaturated heterocyclic $C_{1-6}$ alkylcarbonyloxy,
optionally substituted saturated or unsaturated heterocyclic $C_{2-6}$ alkenylcarbonyloxy,
optionally substituted arylcarbonyloxy,
optionally substituted carbamoyloxy,
optionally substituted carbamoyl,
optionally substituted $C_{1-6}$ alkylsulfonyloxy,
optionally substituted $C_{1-6}$ alkylsufonyl,
optionally substituted arylsufonyloxy,

optionally substituted aryl $C_{1-6}$ alkyloxy,
optionally substituted aryloxycarbonyloxy,
optionally substituted arylaminocarbonyloxy, optionally substituted arylsulfonyl,
optionally substituted arylsulfanyl,
optionally substituted saturated or unsaturated heterocyclic sulfanyl,
optionally substituted $C_{1-6}$ alkyloxy,
optionally substituted $C_{2-6}$ alkenyloxy,
optionally substituted $C_{2-6}$ alkynyloxy,
optionally substituted aryloxy,
$C_{1-6}$ alkyloxy-$C_{1-6}$alkyloxy,
$C_{1-6}$ alkylthio-$C_{1-6}$alkyloxy,
$C_{1-6}$ alkyloxy-$C_{1-6}$ alkyloxy-$C_{1-6}$alkyloxy,
optionally substituted $C_{1-6}$ alkyloxycarbonyloxy,
optionally substituted saturated or unsaturated heterocyclic oxy,
optionally substituted saturated or unsaturated heterocyclic thio,
optionally substituted saturated or unsaturated heterocyclic carbonyloxy,
optionally substituted saturated or unsaturated heterocyclic thiocarbonyloxy,
optionally substituted phosphate group,
optionally substituted $C_{1-6}$ alkyl,
tri-$C_{1-6}$ alkylsilyloxy,
optionally substituted saturated or unsaturated heterocyclic group,
azide,
optionally substituted imino,
optionally substituted amino,
optionally substituted hydrazino,
cyano,
a halogen atom,
-O-N=C-Y1
wherein Y1 represents a hydrogen atom, optionally substituted $C_{1-6}$ alkyl, optionally substituted $C_{3-7}$ cycloalkyl, optionally substituted $C_{2-6}$ alkenyl, optionally substituted $C_{2-6}$ alkynyl, optionally substituted $C_{1-6}$ alkoxy, optionally substituted phenyl, or optionally substituted heterocyclic group or
either $R_1$ and $R_2$, $R_3$ and $R_4$, $R_6$ and $R_7$, and $R_{11}$ and $R_{12}$ each independently together, or one of hydrogen atoms substituted at the carbon atom of the 11-position and $R_5$ together represent
oxo,
=C-Y2 wherein Y2 represents nitro, cyano, optionally substituted imino, hydroxymethyl, hydroxycarbonyl, optionally substituted $C_{1-6}$ alkoxycarbonyl, optionally substituted phenyl, optionally substituted benzyl, optionally substituted phenoxymethyl, optionally substituted aryl oxymethyl, optionally substituted pyridyloxymethyl, optionally substituted pyrimidinyloxymethyl, optionally substituted $C_{1-6}$ alkylcarbonyl, optionally substituted $C_{1-6}$ alkyloxy carbonyl, optionally substituted $C_{1-4}$ alkylaminocarbonyl, optionally substituted phenylaminocarbonyloxy, optionally substituted benzylaminocarbonyloxy, or optionally substituted heterocyclic aminocarbonyloxy, or
=N-Q-Y3 wherein Y3 represents $R_1$', -Z-$R_1$', -Z-O -$R_1$', or -Z-N($R_1$') ($R_1$"), Z represents a bond, -C(=O)-, -C(=S)-, -C(=O)-N-, -C(=S)-N-, or -$SO_2$-, Q represents O or -N-$R_5$', $R_1$' and $R_1$," which may be the same or different, each independently represent a hydrogen atom, optionally substituted $C_1$-$C_{12}$ alkyl, optionally substituted $C_2$-$C_{12}$ alkenyl, optionally substituted $C_2$-$C_{12}$ alkynyl, optionally substituted $C_3$-$C_{12}$-cycloalkyl, optionally substituted $C_5$-$C_{12}$-cycloalkenyl, optionally substituted aryl, or optionally substituted heterocyclic group, or $R_1$' and $R_1$" together may form an optionally substituted three- to seven-membered saturated or unsaturated cycloalkyl, or a three- to seven-membered heterocyclic group comprising one or two atoms or groups selected from oxygen, nitrogen, and sulfur atoms and sulfoxide and sulfone groups, the carbon and nitrogen atoms optionally comprised in the ring are optionally substituted with $C_{1-8}$ alkyl, hydroxy-$C_{1-8}$ alkyl, $C_{3-8}$ cycloalkyl, $C_{2-8}$ alkenyl, $C_{2-8}$ alkynyl, phenyl, benzyl, $C_{1-6}$ alkyl carbonyl, $C_{2-6}$ alkenyl carbonyl, $C_{1-6}$ alkyl carbonylmethyl, or $C_{2-6}$ alkenyl carbonylmethyl, $R_5$' represents a hydrogen atom, $C_{1-8}$ alkyl, hydroxy-$C_{1-8}$ alkyl, $C_{3-8}$ cycloalkyl, $C_{2-8}$ alkenyl, $C_{2-8}$ alkynyl, phenyl, benzyl, $C_{1-6}$ alkylcarbonyl, $C_{2-6}$ alkenylcarbonyl, $C_{1-6}$ alkylcarbonylmethyl, or $C_{2-6}$ alkenylcarbonylmethyl,
when Y3 represents $R_1$' while Q represents -N-$R_5$', $R_1$' and $R_5$' together may form an optionally substituted three- to seven-membered saturated or unsaturated cycloalkyl, or a three- to seven-membered heterocyclic group comprising one or two atoms or groups selected from oxygen, nitrogen, and sulfur atoms, sulfoxide and sulfone groups, the carbon and nitrogen atoms comprised in the ring being optionally substituted by a group selected from the group consisting of $C_{1-8}$ alkyl, hydroxy-$C_{1-8}$ alkyl, $C_{3-8}$ cycloalkyl, $C_{2-8}$ alkenyl, $C_{2-8}$ alkynyl, phenyl, benzyl, $C_{1-6}$ alkyl carbonyl, $C_{2-6}$ alkenyl carbonyl, $C_{1-6}$ alkyl carbonylmethyl, or $C_{2-6}$ alkenyl carbonylmethyl, wherein the substituent

optionally substituted at each of $R_1$', $R_1$," and $R_5$' represents a group selected from the group consisting of halogens, cyano, nitro, hydroxyl, $C_{1-4}$ alkyl optionally substituted by a halogen, $C_{1-4}$ alkyloxy optionally substituted by a halogen, $C_{1-4}$ alkylthio optionally substituted by a halogen, $C_{1-4}$ alkylsulfinyl optionally substituted by a halogen, $C_{1-4}$ alkylsufonyl optionally substituted by a halogen, $C_{1-4}$ alkyl carbonyl optionally substituted by a halogen, $C_{1-4}$ alkoxycarbonyl optionally substituted by a halogen, and $C_{3-6}$ trialkylsilyl, or

one of hydrogen atoms substituted at the carbon atom of the 11-position and $R_5$ together may further represent formyl, carboxyl,

aryl, or $C_{1-6}$ alkyloxy carbonyl optionally substituted by a saturated or unsaturated heterocyclic group,

aryl $C_{1-6}$ alkylaminocarbonyl optionally substituted by $C_{1-6}$ alkyloxy,

$C_{1-6}$ alkylaminocarbonyl,

saturated or unsaturated heterocyclic aminocarbonyl, hydroxy $C_{1-6}$ alkylaminocarbonyl or

$C_{1-6}$ alkylaminocarbonyl optionally substituted by $C_{1-6}$ alkyloxycarbonyl and/or aryl, or

$R_1$ and $R_2$, $R_3$ and $R_4$, $R_6$ and $R_7$, and $R_{11}$ and $R_{12}$ each independently together represent

optionally substituted three- to seven-membered saturated or unsaturated cycloalkyl,

or may form a three- to seven-membered heterocyclic group comprising one or two atoms or groups selected from oxygen, nitrogen, sulfur atoms and sulfoxide and sulfone groups, the carbon and nitrogen atoms comprised in the ring being optionally substituted by $C_{1-8}$ alkyl, hydroxy-$C_{1-8}$ alkyl, $C_{3-8}$cycloalkyl, $C_{2-8}$ alkenyl, $C_{2-8}$ alkynyl, phenyl, benzyl, $C_{1-6}$ alkyl carbonyl, $C_{2-6}$ alkenyl carbonyl, $C_{1-6}$ alkyl carbonylmethyl, or $C_2$-$_6$ alkenyl carbonylmethyl, or

$R_1$ or $R_2$ is absent, and a hydrogen atom substituted at the carbon atom of the 5-position is lost to represent a double bond between the 5-position and the 13-position, or

$R_6$ or $R_7$ is absent, and one of hydrogen atoms substituted at the carbon atom of the 8-position is lost to represent a double bond between the 7-position and the 8-position, or

$R_3$ or $R_4$ is absent, and a hydrogen atom substituted at the carbon atom of the 2-position is lost to represent a double bond between the 1-position and the 2-position, or

$R_3$ or $R_4$ and $R_5$ together represent -O-CR$_3$'(R$_4$')-O- wherein $R_3$' and $R_4$', which may be the same or different, represent a hydrogen atom, $C_{1-6}$ alkyl, $C_{1-6}$alkyloxy, $C_{2-6}$ alkenyl, optionally substituted aryl, or optionally substituted aryl $C_{1-6}$ alkyl, or $R_3$' and $R_4$' together represent oxo, thioxo, or $C_{2-6}$ alkylene; or -O-SiR$_3$'(R$_4$')-O- wherein $R_3$' and $R_4$' are as defined above,

$R_8$ represents a hydrogen atom, cyano, a halogen atom, or benzyl,

$R_{13a}$, $R_{13b}$, and $R_{13c}$, which may be the same or different, each independently represents

$C_{1-6}$ alkyl optionally substituted by a group selected from the group consisting of hydroxyl, halogen atoms, and cyano or

$C_{2-6}$ alkenyl optionally substituted by a group selected from the group consisting of hydroxyl, halogen atoms, and cyano.

According to the present invention, there is provided a compund represented by formula (I-a) or a salt thereof usable as an active ingredient of harmful organism control agents:

[Chemical formula 2]

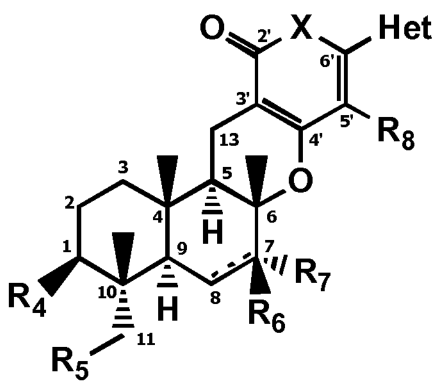

(I-a)

wherein Het, X, $R_3$, $R_4$, $R_5$, $R_6$, $R_7$, and $R_8$ are as defined above.

According to the present invention, there is provided a compound represented by formula (I-a') or a salt thereof usable as an active ingredient of harmful organism control agents:

[Chemical formula 3]

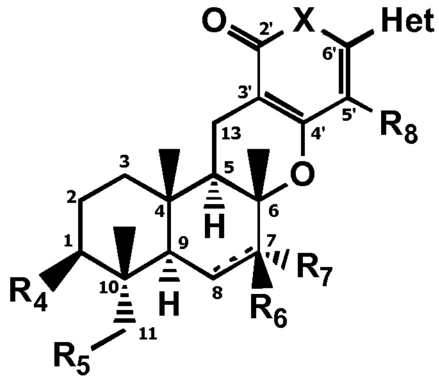

(I-a')

wherein

Het represents optionally substituted pyridyl,
X represents an oxygen atom,
$R_4$ represents hydroxyl or optionally substituted $C_{1-18}$ alkylcarbonyloxy,
$R_5$ represents a hydrogen atom, hydroxyl, or optionally substituted $C_{1-18}$ alkylcarbonyloxy or
$R_4$ and $R_5$ together represent -O-CR$_3$'(R$_4$')-O- wherein $R_3$' and $R_4$', which may be the same or different, represent a hydrogen atom or $C_{1-6}$ alkyl, or $R_3$' and $R_4$' together represent thioxo,
$R_6$ represents a hydrogen atom, hydroxyl, or optionally substituted $C_{1-18}$ alkylcarbonyloxy, or
$R_7$ and $R_8$ represent a hydrogen atom,
$R_6$ or $R_7$ is absent and one of hydrogen atoms substituted at the carbon atom of the 8-position is lost to form a double bond between the 7-position and the 8-position, provided that
the following compounds are excluded:

the compound wherein $R_5$, $R_6$, and $R_7$ simultaneously represent hydrogen atoms, and $R_4$ represents hydroxyl, acetyloxy, or propionyloxy,
the compound wherein $R_6$ and $R_7$ represent a hydrogen atom, and $R_4$ represents acetyloxy and $R_5$ represents propionyloxy,
the compound wherein $R_6$ and $R_7$ represent a hydrogen atom, and $R_4$ and $R_5$ represents acetyloxy,
the compound wherein $R_6$ and $R_7$ represent a hydrogen atom, and $R_4$ represents propionyloxy and $R_5$ represents acetyloxy, and
the compound wherein $R_4$ and $R_5$ represent acetyloxy, $R_6$ represents propionyloxy and $R_7$ represents a hydrogen atom.

According to the present invention, there is provided a compound represented by formula (I-b) or a salt thereof usable as an active ingredient of harmful organism control agents:

[Chemical formula 4]

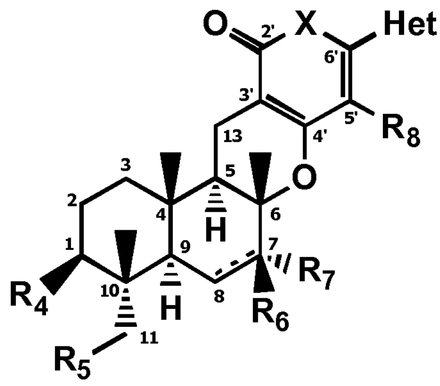

(I-b)

wherein

Het represents optionally substituted pyridyl,
X represents an oxygen atom,
$R_4$ represents hydroxyl or optionally substituted $C_{3-6}$ cycloalkylcarbonyloxy,
$R_5$ represents a hydrogen atom, hydroxyl, or optionally substituted $C_{3-6}$ cycloalkylcarbonyloxy,
$R_6$ represents a hydrogen atom, hydroxyl, or optionally substituted $C_{3-6}$ cycloalkylcarbonyloxy,
$R_7$ represent a hydrogen atom, or
$R_6$ or $R_7$ is absent and one of hydrogen atoms substituted at the carbon atom of the 8-position is lost to form a double bond between the 7-position and the 8-position,
$R_8$ represent a hydrogen atom, provided that, when $R_5$ and $R_6$ simulatneously represent a hydrogen atom, $R_4$ does not represent hydroxyl.

According to the present invention, there is provided a compound represented by formula (I-c) or a salt thereof usable as an active ingredient of harmful organism control agents:

[Chemical formula 5]

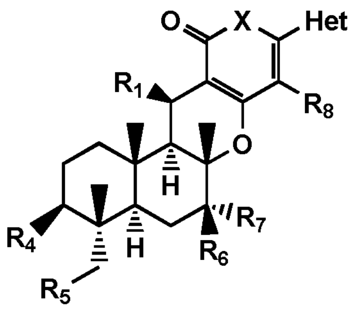

(I-c)

wherein

Het represents 3-pyridyl,
X represents an oxygen atom,
$R_1$ represents liner, branched or cyclic $C_{1-6}$ alkylcarbonyloxy, or $C_{1-6}$ alkyloxy
$R_4$ represents $C_{3-6}$ cycloalkylcarbonyloxy,
$R_5$ represents $C_{3-6}$ cycloalkylcarbonyloxy,
$R_6$ represent hydroxyl, acetyloxy or r $C_{3-6}$ cycloalkylcarbonyloxy,
$R_7$ or $R_8$ is a hydrogen atom.

According to the present invention, there is provided a composition for use as a harmful organism control agent, the composition comprising as an active ingredient a compound represented by formula (I-a), (1-a'), (I-b) or (I-c) or a salt thereof, and an agriculturally or zootechnically acceptable carrier.

Compounds represented by formula (I), (I-a), (I-a'), (I-b) or (I-c) have potent control effect against agricultural and horticultural insect pests, sanitary insect pests, zooparasites, stored grain insect pests, clothing insect pests, and house insect pests, and compositions comprising the compounds as an active ingredient can be advantageously utilized as harmful organism control agents. Further, according to one embodiment, said compositions are preferably used as a pharmaceutical aget for use in controlling harmful organisms, i.e.,an anti-harmful organism agent.

DETAILED DESCRIPTION OF THE INVENTION

The term "halogen" as used herein means fluorine, chlorine, bromine, or iodine, preferably fluorine, chlorine, or bromine.
The terms "alkyl," "alkenyl," and "alkynyl" as used herein as a group or a part of a group respectively mean alkyl, alkenyl, and alkynyl that the group is of a straight chain, branched chain, or cyclic type or a type of a combination thereof unless otherwise specified. Further, for example, "$C_{1-6}$" in "$C_{1-6}$ alkyl" as a group or a part of a group means that the number of carbon atoms in the alkyl group is 1 to 6. Further, in the case of cycloalkyl, "$C_{1-6}$" means that the number of carbon atoms is at least three.
The term "aryl" as used herein as a group or a part of a group means phenyl or naphthyl.
The term "heterocyclic ring" as used herein means a five- to seven-membered saturated, unsaturated or aromatic heterocyclic ring containing one or more, preferably one to four, heteroatoms, which may be the same or different, selected from the group consisting of nitrogen, oxygen, and sulfur atoms, or a five- to seven-membered saturated, unsaturated or aromatic heterocyclic group containing one or more, preferably one to four, heteroatoms, selected from the group consisting of nitrogen, oxygen, and sulfur atoms, and a heterocyclic group obtained by condensing 2 to 4 rings selected from five- to seven-membered saturated, unsaturated or aromatic hydrocarbocylic rings.
Further, the expression "optionally substituted" alkyl as used herein means that one or more hydrogen atoms on the alkyl group may be substituted by one or more substituents which may be the same or different. It will be apparent to a person having ordinary skill in the art that the maximum number of substituents may be determined depending upon the number of substitutable hydrogen atoms on the alkyl group. This is true of functional groups other than the alkyl group.

"Heterocyclic group" and "phenyl" indicated by Het is optionally substituted, and such substituents include halogen atoms, $C_{1-4}$ alkyl, $C_{1-4}$ alkyloxy, nitro, cyano, formyl, trifluoromethyl, trifluoromethoxy, trifluoromethylthio, trifluoromethylsulfinyl, triluoromethylsulfonyl, acetyl, or acetyloxy. Preferred are halogen atoms and trifluoromethyl. A chlorine atom or trifluoromethyl is more preferred.
"$C_{1-18}$ alkyl" indicated by Het is preferably $C_{1-6}$ alkyl, and "$C_{2-18}$ alkenyl" indicated by Het is preferably $C_{2-6}$ alkenyl.
"$C_{1-18}$ alkyl" and "$C_{2-18}$ alkenyl" indiated by Het are optionally substituted, and such substituents include halogen atoms, $C_{1-4}$alkyloxy optionally substituted by a halogen, cyano, phenyl, a heterocyclic group, phenyloxy, and heterocyclic oxy, wherein phenyl, heterocyclic group, phenyloxy, and heterocyclic oxy are optionally substituted by a group selected from the group consisting of halogen atoms, $C_{1-4}$ alkyl, $C_{1-4}$ alkyloxy, nitro, cyano, formyl, trifluoromethyl, trifluoromethoxy, trifluoromethylthio, trifluoromethylsulfinyl, triluoromethylsulfonyl, acetyl, and acetyloxy.
Heterocyclic group, phenyl, $C_{1-18}$ alkyl, or $C_{2-18}$ alkenyl indicated by Het is preferably a heterocyclic group or phenyl, more preferably pyridyl, particularly preferably 3-pyridyl.

The oxygen atom or $NR_9$ wherein $R_9$ represents a hydrogen atom, $C_{1-6}$ alkyl, or aryl $C_{1-6}$ alkyl indicated by X is preferably an oxygen atom.

"$C_{1-18}$ alkylaminocarbonyloxy" indicated by $R_1$, $R_2$, $R_3$, $R_4$, $R_5$, $R_6$, $R_7$, $R_{10a}$, $R_{10b}$, $R_{11}$, and $R_{12}$ is preferably $C_{1-6}$ alkylaminocarbonyloxy, more preferably propionyloxy or $C_{1-3}$ lkylaminocarbonyloxy,
The $C_{1-18}$ alkylaminocarbonyloxy group is optionally substituted, and such substituents include halogen atoms, cyano, phenyl, trifluoromethoxy or trifluoromethoxythio.

"$C_{1-18}$ alkylcarbonyloxy" indicated by $R_1$ and $R_2$ is preferably $C_{1-6}$ alkylcarbonyloxy and is optionally substituted, and such substituents include halogen atoms, cyano, phenyl, trifluoromethoxy, or trifluoromethylthio.

"$C_{1-18}$ alkylcarbonyloxy" indicated by $R_3$, $R_4$, $R_5$, $R_6$, $R_7$, $R_{10a}$, $R_{10b}$, $R_{11}$, and $R_{12}$ is preferably $C_{1-6}$ alkylcarbonyloxy, more preferably propionyloxy or $C_{3-6}$ cycloalkylcarbonyloxy. The $C_{1-18}$ alkylcarbonyloxy group is optionally substituted, and such substituents include halogen atoms, cyano, $C_{3-6}$ cycloalkyl, $C_{1-6}$ alkyloxy-$C_{1-6}$alkyloxy, phenyl, $C_{1-6}$alkyloxy optionally substituted by a halogen, $C_{1-6}$ alkylthio optionally substituted by a halogen, oxime optionally substituted by $C_{1-6}$ alkyl, pyridyl, or pyridylthio. A halogen atom, $C_{3-6}$cycloalkyl, or pyridyl is preferred.

"$C_{2-6}$ alkenylcarbonyloxy" indicated by $R_1$, $R_2$, $R_3$, $R_4$, $R_5$, $R_6$, $R_7$, $R_{10a}$, $R_{10b}$, $R_{11}$, and $R_{12}$ is optionally substituted, and such substituents include halogen atoms, cyano, phenyl, trifluoromethoxy, or trifluoromethylthio.

$C_{2-6}$ alkynylcarbonyloxy indicated by $R_1$, $R_2$, $R_3$, $R_4$, $R_5$, $R_6$, $R_7$, $R_{10a}$, $R_{10b}$, $R_{11}$, and $R_{12}$ is optionally substituted, and such substituents include halogen atoms, cyano, phenyl, trifluoromethoxy, or trifluoromethylthio.

"$C_{1-6}$ alkyloxy" and "$C_{1-6}$ alkyl" indicated by $R_1$, $R_2$, $R_3$, $R_4$, $R_5$, $R_6$, $R_7$, $R_{10a}$, $R_{10b}$, $R_{11}$, and $R_{12}$ are optionally substituted, and such substituents include halogen atoms, cyano, aryl optionally substituted by $C_{1-6}$ alkyloxy, trifluoromethoxy, trifluoromethylthio, $C_{1-6}$ alkyl carbonyl optionally substituted by a halogen atom, $C_{1-6}$ alkyloxy, $C_{1-6}$ alkyloxy-$C_{1-6}$ alkyloxy, or $C_{1-6}$ alkylcarbonyloxy optionally substituted by a halogen atom.

"$C_{2-6}$ alkenyloxy" indicated by $R_1$, $R_2$, $R_3$, $R_4$, $R_5$, $R_6$, $R_7$, $R_{10a}$, $R_{10b}$, $R_{11}$, and $R_{12}$ is optionally substituted, and such substituents include halogen atoms, cyano, phenyl, trifluoromethoxy, trifluoromethylthio, $C_{1-6}$ alkyl carbonyl optionally substituted by a halogen atom, and $C_{1-6}$ alkylcarbonyloxy optionally substituted by a halogen atom.

"$C_{2-6}$ alkynyloxy" indicated by $R_1$, $R_2$, $R_3$, $R_4$, $R_5$, $R_6$, $R_7$, $R_{10a}$, $R_{10b}$, $R_{11}$, and $R_{12}$ is optionally substituted, and such substituents include halogen atoms, cyano, phenyl, trifluoromethoxy, trifluoromethylthio, $C_{1-6}$ alkyl carbonyl optionally substituted by a halogen atom, or $C_{1-6}$ alkylcarbonyloxy optionally substituted by a halogen atom.

Phenyl in "aryloxy" and "aryl $C_{1-6}$alkyloxy" indicated by $R_1$, $R_2$, $R_3$, $R_4$, $R_5$, $R_6$, $R_7$, $R_{10a}$, $R_{10b}$, $R_{11}$, and $R_{12}$ is optionally substituted, and such substituents include halogen atoms, $C_{1-6}$ alkyloxy optionally substituted by a halogen atom, $C_{1-6}$ alkyl optionally substituted by a halogen atom, $C_{1-6}$ alkyl carbonyl optionally substituted by a halogen atom, $C_{1-6}$ alkylcarbonyloxy optionally substituted by a halogen atom, $C_{1-6}$ alkylcarbonyl amino optionally substituted by a halogen atom, $C_{1-6}$ alkylaminocarbonyloxy optionally substituted by a halogen atom, $C_{1-6}$ alkylaminocarbonyl optionally substituted by a halogen atom, $C_{1-6}$ alkylsulfonyloxy optionally substituted by a halogen atom, $C_{1-6}$ alkylthio optionally substituted by a halogen atom, $C_{1-6}$ alkylsulfinyl optionally substituted by a halogen atom, $C_{1-6}$ alkylsufonyl optionally substituted by a halogen atom, cyano, formyl, azide, guanidyl, group $-C(=NH)-NH_2$, or group $-CH=N-O-CH_3$.

Phenyl and alkyl in "arylcarbonyloxy," "aryl $C_{1-6}$ alkylcarbonyloxy," "aryloxycarbonyloxy," and "aryl aminocarbonyloxy" indicated by $R_1$, $R_2$, $R_3$, $R_4$, $R_5$, $R_6$, $R_7$, $R_{10a}$, $R_{10b}$, $R_{11}$, and $R_{12}$ are optionally substituted, and such substituents include halogen atoms, $C_{1-6}$ alkyloxy optionally substituted by a halogen atom, $C_{1-6}$ alkyl optionally substituted by a halogen atom, $C_{2-6}$ alkenyl, $C_{1-6}$ alkyl carbonyl optionally substituted by a halogen atom, $C_{1-6}$ alkylcarbonyloxy optionally substituted by a halogen atom, $C_{1-6}$ alkylcarbonylamino optionally substituted by a halogen atom, $C_{1-6}$ alkylaminocarbonyloxy optionally substituted by a halogen atom, $C_{1-6}$ alkylaminocarbonyl optionally substituted by a halogen atom, $C_{1-6}$ alkylsulfonyloxy optionally substituted by a halogen atom, $C_{1-6}$ alkylthio optionally substituted by a halogen atom, $C_{1-6}$ alkylsulfinyl optionally substituted by a halogen atom, $C_{1-6}$ alkylsufonyl optionally substituted by a halogen atom, cyano, nitro, formyl, carbamoyl, amino, trialkylsilyloxy, hydroxy, phenyl, azide, guanidyl, oxime optionally substituted by $C_{1-6}$ alkyl, $-OCF_2O-$, $OCH_2O-$, $-C(=NH)-NH_2$, or group $-CH=N-O-CH_3$. A halogen atom, $C_{1-6}$ alkyl optionally substituted by a halogen atom, cyano, or nitro is preferred.

Phenyl in "aryl sufonyloxy," "arylsulfonyl," and "arylsulfanyl" indicated by $R_1$, $R_2$, $R_3$, $R_4$, $R_5$, $R_6$, $R_7$, $R_{10a}$, $R_{10b}$, $R_{11}$, and $R_{12}$ is optionally substitued, and such substituents include halogen atoms, $C_{1-6}$ alkyloxy optionally substituted by a halogen atom, $C_{1-6}$ alkyl optionally substituted by a halogen atom, $C_{1-6}$ alkyl carbonyl optionally substituted by a halogen atom, $C_{1-6}$ alkylcarbonyloxy optionally substituted by a halogen atom, $C_{1-6}$ alkylcarbonylamino optionally substituted by a halogen atom, $C_{1-6}$ alkylaminocarbonyloxy optionally substituted by a halogen atom, $C_{1-6}$ alkylaminocarbonyl optionally substituted by a halogen atom, $C_{1-6}$ alkylsulfonyloxy optionally substituted by a halogen atom, $C_{1-6}$ alkylthio optionally substituted by a halogen atom, $C_{1-6}$ alkylsulfinyl optionally substituted by a halogen atom, $C_{1-6}$ alkylsufonyl optionally substituted by a halogen atom, cyano, formyl, azide, guanidyl, or group $-C(=NH)-NH_2$, or group $-CH=N-O-CH_3$.

"$C_{1-6}$ alkylsulfonyloxy" and "$C_{1-6}$ alkylsufonyl" indicated by $R_1$, $R_2$, $R_3$, $R_4$, $R_5$, $R_6$, $R_7$, $R_{10a}$, $R_{10b}$, $R_{11}$, and $R_{12}$ are preferably "$C_{1-3}$ alkylsulfonyloxy" and "$C_{1-3}$ alkylsufonyl" respectively.

"$C_{1-6}$ alkylsulfonyloxy" and "$C_{1-6}$ alkylsufonyl" indicated by $R_1$, $R_2$, $R_3$, $R_4$, $R_5$, $R_6$, $R_7$, $R_{10a}$, $R_{10b}$, $R_{11}$, and $R_{12}$ are optionally substitued, and such substituents include halogen atoms, cyano, phenyl, trifluoromethoxy, or trifluoromethylthio.

"Phosphate group" indicated by $R_1$, $R_2$, $R_3$, $R_4$, $R_5$, $R_6$, $R_7$, $R_{10a}$, $R_{10b}$, $R_{11}$, and $R_{12}$ is optionally substituted, and such substituents include $C_{1-6}$ alkyl or $C_{1-6}$ alkyl, and phenyl optionally substituted by a halogen atom.

"$C_{1-6}$ alkyloxycarbonyloxy" indicated by $R_1$, $R_2$, $R_3$, $R_4$, $R_5$, $R_6$, $R_7$, $R_{10a}$, $R_{10b}$, $R_{11}$, and $R_{12}$ is optionally substituted, and such substituents include halogen atoms, cyano, phenyl, $C_{1-6}$alkyloxy optionally substituted by a halogen, or $C_{1-6}$ alkylthio optionally substituted by a halogen.

"Carbamoyloxy," "carbamoyl," "amino," "imino," and "hydrazino" indicated by $R_1$, $R_2$, $R_3$, $R_4$, $R_5$, $R_6$, $R_7$, $R_{10a}$, $R_{10b}$, $R_{11}$, and $R_{12}$ are optionally substituted, and such substituents include halogen atoms, cyano, phenyl, trifluoromethoxy, or $C_{1-6}$ alkyl optionally substituted by trifluoromethylthio.

Phenyl in "aryl" and "aryl $C_{1-6}$ alkyl" indicated by $R_3'$ and $R_4'$ is optionally substituted, and such substituents include halogen atoms, $C_{1-4}$ alkyl, $C_{1-4}$ alkyloxy, nitro, cyano, formyl, trifluoromethoxy, acetyl, acetyloxy, or di-$C_{1-4}$ alkyl amino.

In "saturated or unsaturated heterocyclic group," "saturated or unsaturated heterocyclic oxy," "saturated or unsaturated heterocyclic carbonyloxy," "saturated or unsaturated heterocyclic $C_{1-6}$ alkylcarbonyloxy," "saturated or unsaturated heterocyclic $C_{2-6}$ alkenylcarbonyloxy," "saturated or unsaturated heterocyclic thiocarbonyloxy," "saturated or unsaturated heterocyclic thio," or "saturated or unsaturated heterocyclic sulfanyl" indicated by $R_1$, $R_2$, $R_3$, $R_4$, $R_5$, $R_6$, $R_7$, $R_{10a}$, $R_{10b}$, $R_{11}$, and $R_{12}$, the "saturated or unsaturated heterocyclic group" is preferably a saturated or unsaturated

five- or six-membered heterocyclic ring containing 1 to 3 hetero atoms selected from the group consisting of nitrogen, oxygen, and sulfur, more preferably a saturated or unsaturated five- or six-membered heterocyclic ring containing one or two hetero atoms selected from the group consisting of nitrogen, oxygen, and sulfur, still more preferably a saturated or unsaturated five-or six-membered heterocyclic ring containing one or two nitrogen atoms, a saturated or unsaturated five- or six-membered heterocyclic ring containing one or two oxygen atoms, a saturated or unsaturated five- or six-membered heterocyclic ring containing one or two sulfur atoms, a saturated or unsaturated five- or six-membered heterocyclic ring containing one nitrogen atom and one oxygen atom, or a saturated or unsaturated five- or six-membered heterocyclic ring containing one nitrogen atom and one sulfur atom.

More specific examples of the "saturated or unsaturated heterocyclic group" include thienyl, furyl, pyrrolyl, imidazolyl, pyrazolyl, isothiazolyl, isoxazolyl, thiazolyl, oxazolyl, pyridyl, pyrimidinyl, pyrazinyl, pyranyl, pyridazinyl, tetrahydropyranyl, piperidinyl, piperazinyl, morpholinyl, tetrazolyl, tetrabenzylmannosyl, mannosyl, benzo[b]thienyl, 2,2-difluorobenzo [d][1,3]dioxole, indolyl or thienopyridyl, more preferably pyridyl, furanyl, thiazolyl, imidazolyl, tetrahydropyranyl, or mannosyl. Still more specific examples thereof include (2- or 3-)thienyl, (2- or 3-)furyl, (1-, 2- or 3-)pyrrolyl, (1-, 2-, 4- or 5-) imidazolyl, (1-, 3-, 4- or 5-)pyrazolyl, (3-, 4- or 5-)isothiazolyl, (3-, 4- or 5-)isoxazolyl, (2-, 4- or 5-)thiazolyl, (2-, 4- or 5-) oxazolyl, (2-, 3- or 4-)pyridyl or (2-, 4-, 5- or 6-)pyrimidinyl, (2- or 3-)pyrazinyl, (3- or 4-)pyridazinyl, (2-, 3- or 4-)tetrahydropyranyl, (1-, 2-, 3- or 4-)piperidinyl, (1-, 2- or 3-)piperazinyl, and (2-, 3- or 4-)morpholinyl, preferably 3-pyridyl, 2-furanyl, 5-thiazolyl, 1-imidazolyl, 5-imidazolyl or 2-tetrahydropyranyl, preferably 2-tetrahydropyranyl, 2-pyrazinyl or 3-pyridyl, more preferably 3-pyridyl.

The heterocyclic ring in the "saturated or unsaturated heterocyclic carbonyloxy," "saturated or unsaturated heterocyclic thiocarbonyloxy," "saturated or unsaturated heterocyclic sulfanyl," "saturated or unsaturated heterocyclic $C_{1-6}$ alkylcarbonyloxy," and "saturated or unsaturated heterocyclic $C_{2-6}$ alkenylcarbonyloxy" is optionally substitued, and such substituents include halogen atoms, $C_{1-4}$ alkyl, $C_{1-4}$ alkyloxy, phenyl optionally substituted by $C_{1-4}$ alkyloxy, $C_{1-4}$ alkylthio, nitro, cyano, formyl, trifluoromethoxy, trifluoromethyl, trifluoromethylthio, trifluoromethylsulfinyl, triluoromethylsulfonyl, acetyl, acetyloxy, benzoyl, $C_{1-4}$ alkyloxy carbonyl, oxo, preferably a halogen atom, $C_{1-4}$ alkyl, $C_{1-4}$ alkyloxy, or trifluoromethyl.

The heterocyclic ring in the "saturated or unsaturated heterocyclic oxy," "saturated or unsaturated heterocyclic group," and "saturated or unsaturated heterocyclic thio" is optionally substituted, and such substituents include hydroxyl, benzyloxy, halogen atoms, $C_{1-4}$ alkyl, $C_{1-4}$ alkyloxy, phenyl optionally substituted by $C_{1-4}$ alkyloxy, nitro, cyano, formyl, trifluoromethoxy, trifluoromethyl, trifluoromethylthio, trifluoromethylsulfinyl, triluoromethylsulfonyl, acetyl, or acetyloxy, preferably hydroxyl or benzyloxy.

"$C_{1-6}$ alkyl," "$C_{3-7}$cycloalkyl," "$C_{2-6}$ alkenyl," "$C_{2-6}$ alkynyl," "$C_{1-6}$ alkoxy," "phenyl," and "heterocyclic group" indicated by Y1 in -O-N=C-Y1 represented by $R_1$, $R_2$, $R_3$, $R_4$, $R_5$, $R_6$, $R_7$, $R_{10a}$, $R_{10b}$, $R_{11}$, and $R_{12}$, "imino," "$C_{1-6}$ alkoxycarbonyl," "phenyl," " benzyl," "phenoxymethyl," "aryl oxymethyl," "pyridyloxymethyl," "pyrimidinyloxymethyl," "$C_{1-6}$ alkylcarbonyl," "$C_{1-6}$ alkyloxy carbonyl," "$C_{1-4}$ alkylaminocarbonyl," "phenylaminocarbonyloxy," "benzylaminocarbonyloxy," or "heterocyclic aminocarbonyloxy" indicated by Y2 in =C-Y2 represented by each independently combining $R_1$ and $R_2$, $R_3$ and $R_4$, $R_6$ and $R_7$, or $R_{11}$ and $R_{12}$, or by combining one of hydrogen atoms substituted at the carbon atom of the 11-position and $R_5$, and the "$C_{1-12}$ alkyl," "$C_{2-12}$ alkenyl," "$C_{2-12}$ alkynyl," "$C_{3-12}$ cycloalkyl," "$C_{5-12}$ cycloalkenyl," "aryl," and "heterocyclic group" indicated by $R_1$' and $R_1$" in Y3 in =N-Q-Y3 are optionally substituted, and such substituents are selected from the group consisting of halogens, cyano, nitro, hydroxyl, $C_{1-4}$ alkyl optionally substituted by a halogen, $C_{1-4}$ alkyloxy optionally substituted by a halogen, $C_{1-4}$ alkylthio optionally substituted by a halogen, $C_{1-4}$ alkylsulfinyl optionally substituted by a halogen, $C_{1-4}$ alkylsufonyl optionally substituted by a halogen, $C_{1-4}$ alkylcarbonyl optionally substituted by a halogen, $C_{1-4}$ alkoxycarbonyl optionally substituted by a halogen, and $C_{3-6}$ trialkylsilyl.

The carbon atom or the nitrogen atom constituting the "three- to seven-membered saturated or unsaturated cycloalkyl" and "heterocyclic ring" represented by combining $R_1$' and $R_1$" together in Y3 in =N-Q-Y3 represented by each independently combining $R_1$ and $R_2$, $R_3$ and $R_4$, $R_6$ and $R_7$, or $R_{11}$ and $R_{12}$, or by combining one of hydrogen atoms substituted at the carbon atom of the 11-position and $R_5$ is optionally substituted, and such substituents are selected from the group consisting of $C_{1-8}$ alkyl, hydroxy-$C_{1-8}$ alkyl, $C_{3-8}$cycloalkyl, $C_{2-8}$ alkenyl, $C_{2-8}$ alkynyl, phenyl, benzyl, $C_{1-6}$ alkylcarbonyl, $C_{2-6}$ alkenyl carbonyl, $C_{1-6}$ alkylcarbonylmethyl, and $C_{2-6}$ alkenylcarbonylmethyl.

The carbon atom or the nitrogen atom constituting the "three- to seven-membered saturated or unsaturated cycloalkyl" and "heterocyclic ring" representing by combining $R_1$' and $R_5$' together in =N-Q-Y3, wherein Y3 represents $R_1$' and Q represents -N-$R_5$', represented by each independently combining $R_1$ and $R_2$, $R_3$ and $R_4$, $R_6$ and $R_7$, or $R_{11}$ and $R_{12}$, or by combining one of hydrogen atoms substituted at the carbon atom of the 11-position and $R_5$ is optionally substituted, and such substituents are selected from the group consisting of $C_{1-8}$ alkyl, hydroxy-$C_{1-8}$ alkyl, $C_{3-8}$ cycloalkyl, $C_{2-8}$ alkenyl, $C_{2-8}$ alkynyl, phenyl, benzyl, $C_{1-6}$ alkylcarbonyl, $C_{2-6}$ alkenylcarbonyl, $C_{1-6}$ alkylcarbonylmethyl, or $C_{2-6}$ alkenylcarbonylmethyl.

The carbon atom or the nitrogen atom constituting the "three- to seven-membered saturated or unsaturated cycloalkyl" and "heterocyclic ring" represented by each independently combining $R_1$ and $R_2$, $R_3$ and $R_4$, $R_6$ and $R_7$, or $R_{11}$ and $R_{12}$ is optionally substituted, and such substituents are selected from the group consisting of $C_{1-8}$ alkyl, hydroxy-

$C_{1-8}$ alkyl, $C_{3-8}$cycloalkyl, $C_{2-8}$ alkenyl, $C_{2-8}$ alkynyl, phenyl, benzyl, $C_{1-6}$ alkylcarbonyl, $C_{2-6}$ alkenyl carbonyl, $C_{1-6}$ alkylcarbonylmethyl, and $C_{2-6}$ alkenylcarbonylmethyl.

"$C_{1-6}$ alkyl" and "$C_{2-6}$ alkenyl" represented by $R_{13a}$, $R_{13b}$, and $R_{13c}$ are optionally substituted, and such constituents include hydroxyl, a halogen atom, or cyano.

<u>The compound represened by formula (I) (I-a), (I-b) or (I-c), and the composition for use as harmful organism control agent compring the compound</u>

In a preferred embodiment of the present invention, in compounds represented by formula (I) or (I-a), $Het_1$ represents optionally substituted pyridyl or phenyl, more preferably pyridyl, particularly preferably 3-pyridyl. In a preferred embodiment of the present invention, in compounds represented by formula (I) or (I-a), X represents an oxygen atom.

In a preferred embodiment of the present invention, in compouns represnted by formula (I), $R_1$ represents a hydrogen atom, hydroxyl, optionally substituted $C_{1-18}$ alkylcarbonyloxy, optionally substituted carbamoyloxy, $C_{1-6}$ alkyloxy-$C_{1-6}$alkyloxy, or optionally substituted saturated or unsaturated heterocyclic oxy, more preferably a hydrogen atom.

In a preferred embodiment of the present invention, in compounds represented by formula (I), $R_2$, $R_3$, $R_{10a}$, $R_{10b}$, $R_{11}$, and $R_{12}$ reresent a hydrogen atom.

In a preferred embodiment of the present invention, in compounds represented by formula (I) or (I-a), $R_4$ represents hydroxyl, optionally substituted $C_{1-18}$ alkylcarbonyloxy, optionally substituted arylcarbonyloxy, optionally substituted $C_{1-18}$ alkylsulfonyloxy, optionally substituted arylsufonyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, or optionally substituted saturated or unsaturated heterocyclic oxy, more preferably hydroxyl, optionally substituted $C_{1-18}$ alkylcarbonyloxy, optionally substituted arylcarbonyloxy, optionally substituted arylsufonyloxy, $C_{1-6}$alkyloxy-$C_{1-6}$alkyloxy, $C_{1-6}$ alkyloxy-$C_{1-6}$ alkyloxy-$C_{1-6}$ alkyloxy, or optionally substituted saturated or unsaturated heterocyclic oxy, more preferably hydroxyl, optionally substituted $C_{1-18}$ alkylcarbonyloxy, still more preferably hydroxyl, or optionally substituted $C_{3-6}$ cycloalkylcarbonyloxy, particularly preferably hydroxyl or $C_{3-6}$ cycloalkylcarbonyloxy.

In a preferred embodiment of the present invention, in compounds represented by formula (I) or (I-a), $R_5$ represents a hydrogen atom, hydroxyl, optionally substituted $C_{1-18}$ alkylcarbonyloxy, optionally substituted arylcarbonyloxy, optionally substituted $C_{1-18}$ alkylsulfonyloxy, optionally substituted arylsufonyloxy, optionally substituted aryl $C_{1-6}$ alkyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, or optionally substituted phosphate group, more preferably a hydrogen atom, hydroxyl, optionally substituted $C_{1-18}$ alkylcarbonyloxy, optionally substituted arylsufonyloxy, optionally substituted aryl $C_{1-6}$ alkyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, or optionally substituted phosphate group, still more preferably a hydrogen atom, hydroxyl, optionally substituted $C_{1-18}$ alkylcarbonyloxy, further preferably a hydrogen atom, optionally substituted $C_{3-6}$ cycloalkylcarbonyloxy, particularly preferably a hydrogen atom or $C_{3-6}$ cycloalkylcarbonyloxy.

In a preferred embodiment of the present invention, in compounds represented by formula (I) or (I-a), $R_6$ represents a hydrogen atom, hydroxyl, optionally substituted $C_{1-18}$ alkylcarbonyloxy, optionally substituted arylcarbonyloxy, optionally substituted aryl thiocarbonyloxy, optionally substituted $C_{1-18}$ alkylsulfonyloxy, optionally substituted carbamoyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, optionally substituted saturated or unsaturated heterocyclic oxy or optionally substituted saturated or unsaturated heterocyclic thiocarbonyloxy, more preferably a hydrogen atom, hydroxyl, optionally substituted $C_{1-18}$ alkylcarbonyloxy, optionally substituted carbamoyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, optionally substituted saturated or unsaturated heterocyclic oxy or optionally substituted saturated or unsaturated heterocyclic thiocarbonyloxy, still more preferably a hydrogen atom, hydroxyl, optionally substituted $C_{1-18}$ alkylcarbonyloxy, further more preferably a hydrogen atom, hydroxyl, or optionally substituted $C_{3-6}$ cycloalkylcarbonyloxy, particularly preferably a hydrogen atom or hydroxyl.

In a preferred embodiment of the present invention, in compounds represented by formula (I) or (I-a), $R_7$ represents a hydrogen atom, hydroxyl, optionally substituted $C_{1-18}$ alkylcarbonyloxy, or a halogen atom, more preferably a hydrogen atom.

In a preferred embodiment of the present invention, in compounds represented by formula (I), $R_1$ and $R_2$ each indenpendently together represent oxo, or $R_1$ or $R_2$ is absent and a hydrogen atom substituted at the carbon atom of the 5-position is lost to form a double bond between the 5-position and the 13-position, or $R_6$ or $R_7$ is absent and one of hydrogen atoms substituted at the carbon atom of the 8-position is lost to form a double bond between the 7-position and th 8-position, or $R_4$ and $R_5$ together represent -O-$CR_3$'($R_4$')-O-, wherein $R_3$' and $R_4$', which may be the same or different, represent a hydrogen atom or $C_{1-6}$ alkyl, or $R_3$' and $R_4$' together represent thioxo, or $R_6$ and $R_7$ each independently together represent oxo.

In a preferred embodiment of the present invention, in compounds represented by formula (I) or (I-a), $R_8$ represents a hydrogen atom or a halogen atom, more preferably a hydrogen atom.

In a preferred embodiment of the present invention, in compounds represented by formula (I), $R_{13a}$, $R_{13b}$, and $R_{13c}$ represent methyl.

In another preferred embodiment of the present invention, in compounds represented by formula (I),

Het represents optionally substituted pyridyl or phenyl, X represents an oxygen atom or $NR_9$ wherein $R_9$ represents a hydrogen atom, $C_{1-6}$ alkyl or aryl $C_{1-6}$ alkyl,
$R_2$, $R_3$, $R_{10a}$, $R_{10b}$, $R_{11}$, and $R_{12}$ represent a hydrogen atom,
$R_1$ represents a hydrogen atom, hydroxyl, optionally substituted $C_{1-18}$ alkylcarbonyloxy, optionally substituted carbamoyloxy, $C_{1-6}$ alkyloxy-$C_{1-6}$ alkyloxy or optionally substituted saturated or unsaturated heterocyclic oxy,
$R_4$ represents hydroxyl, optionally substituted $C_{1-18}$ alkylcarbonyloxy, optionally substituted arylcarbonyloxy, optionally substituted $C_{1-18}$ alkylsulfonyloxy, optionally substituted arylsufonyloxy, $C_{1-6}$ alkyloxy-$C_{1-6}$ alkyloxy, $C_{1-6}$ alkyloxy-$C_{1-6}$ alkyloxy-$C_{1-6}$ alkyloxy or optionally substituted saturated or unsaturated heterocyclic oxy,
$R_5$ represents a hydrogen atom, hydroxyl, optionally substituted $C_{1-18}$ alkylcarbonyloxy, optionally substituted arylcarbonyloxy, optionally substituted $C_{1-18}$ alkylsulfonyloxy, optionally substituted arylsufonyloxy, optionally substituted aryl $C_{1-6}$ alkyloxy, $C_{1-6}$ alkyloxy-$C_{1-6}$ alkyloxy, $C_{1-6}$ alkyloxy-$C_{1-6}$ alkyloxy-$C_{1-6}$ alkyloxy or optionally substituted phosphate group,
$R_6$ represents a hydrogen atom, hydroxyl, optionally substituted $C_{1-18}$ alkylcarbonyloxy, optionally substituted arylcarbonyloxy, optionally substituted aryl thiocarbonyloxy, optionally substituted $C_{1-18}$ alkylsulfonyloxy, optionally substituted carbamoyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, optionally substituted saturated or unsaturated heterocyclic oxy, or optionally substituted saturated or unsaturated heterocyclic thiocarbonyloxy,
$R_7$ represents a hydrogen atom, hydroxyl, optionally substituted $C_{1-18}$ alkylcarbonyloxy, or a halogen atom, or
$R_1$ and $R_2$ each independently together represent oxo, or
$R_1$ or $R_2$ is absent and a hydrogen atom substituted at the carbon atom of the 5-position is lost to form a double bond between the 5-position and the 13-position, or $R_6$ or $R_7$ is absent and one of hydrogen atoms substituted at the carbon atom of the 8-position is lost to form a double bond between the 7-position and the 8-position, or
$R_4$ and $R_5$ together represent -O-$CR_3$'($R_4$')-O-, wherein $R_3$' and $R_4$', which may be the same or different, represent a hydrogen atom or $C_{1-6}$ alkyl, or $R_3$' and $R_4$' together represent thioxo,
or $R_6$ and $R_7$ each independently together represent oxo,
$R_8$ represents a hydrogen atom or a halogen atom, and
$R_{13a}$, $R_{13b}$, and $R_{13c}$ represnt methyl.

Further, in another preferred embodiment of the present invention, in compounds represented by formula (I), $R_1$, $R_2$, $R_3$, $R_7$, $R_{10a}$, $R_{10b}$, $R_{11}$, and $R_{12}$ represent a hydrogen atom, and
$R_{13a}$, $R_{13b}$, and $R_{13c}$ represenet methyl.

In still another preferred embodiment of the present invention, in compounds represented by formula (I), Het represents optionally substituted pyridyl,
X represents an oxygen atom or $NR_9$, wherein $R_9$ represents a hydrogen atom, $C_{1-6}$ alkyl or aryl $C_{1-6}$ alkyl,
$R_1$, $R_2$, $R_3$, $R_7$, $R_{10a}$, $R_{10b}$, $R_{11}$, and $R_{12}$ represent a hydrogen atom,
$R_4$ represents hydroxyl, optionally substituted $C_{1-18}$ alkylcarbonyloxy, optionally substituted arylsufonyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, or optionally substituted saturated or unsaturated heterocyclic oxy,
$R_5$ represents a hydrogen atom, hydroxyl, optionally substituted $C_{1-18}$ alkylcarbonyloxy, optionally substituted arylsufonyloxy, optionally substituted aryl $C_{1-6}$ alkyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, or optionally substituted phosphate group, or
$R_4$ and $R_5$ together represent -O-$CR_3$'($R_4$')-O-, wherein $R_3$' and $R_4$', which may be the same or different, represent a hydrogen atom or $C_{1-6}$ alkyl, or $R_3$' and $R_4$' together represent thioxo,
$R_6$ represents a hydrogen atom, hydroxyl, optionally substituted $C_{1-18}$ alkylcarbonyloxy, optionally substituted carbamoyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, optionally substituted saturated or unsaturated heterocyclic oxy, or optionally substituted saturated or unsaturated heterocyclic thiocarbonyloxy, or
$R_6$ or $R_7$ is absent and one of hydrogen atoms substituted at the carbon atom of the 8-position is lost to form a double bond between the 7-position and the 8-position,
$R_8$ represents a hydrogen atom or a halogen atom, and
$R_{13a}$, $R_{13b}$, and $R_{13c}$ represent methyl.

In another preferred embodiment of the present invention, in compounds represented by formula (I), Het represents optionally substituted pyridyl,
X represents an oxygen atom,
$R_1$, $R_2$, $R_3$, $R_7$, $R_8$, $R_{10a}$, $R_{10b}$, $R_{11}$, and $R_{12}$ represent a hydrogen atom,
$R_4$ represents hydroxyl or optionally substituted $C_{3-6}$ cycloalkylcarbonyloxy,
$R_5$ represents a hydrogen atom or optionally substituted $C_{3-6}$ cycloalkylcarbonyloxy,
$R_6$ represents a hydrogen atom, hydroxyl or optionally substituted $C_{3-6}$ cycloalkylcarbonyloxy, or
$R_6$ or $R_7$ is absent and one of hydrogen atoms substituted at the carbon atom of the 8-position is lost to form a double bond between the 7-position and the 8-position, and
$R_{13a}$, $R_{13b}$, and $R_{13c}$ represent methyl.

In one embodiment of the present invention, in compounds represented by formula (I),

Het represents optionally substituted pyridyl or phenyl,
X represents an oxygen atom or $NR_9$ wherein $R_9$ represents a hydrogen atom, $C_{1-6}$ alkyl, or aryl $C_{1-6}$ alkyl,
$R_1$ represents a hydrogen atom, hydroxyl, optionally substituted $C_{1-18}$ alkylcarbonyloxy, optionally substituted carbamoyloxy, $C_{1-6}$ alkyloxy-$C_{1-6}$ alkyloxy, or optionally substituted saturated or unsaturated heterocyclic oxy,
$R_2$ represents a hydrogen atom, or
$R_1$ and $R_2$ each independently together represent oxo, or
$R_1$ or $R_2$ is absent and a hydrogen atom substituted at the carbon atom of the 5-position is lost to form a double bond between the 5-position and the13-position,
$R_4$ represents hydroxyl, optionally substituted $C_{1-18}$ alkylcarbonyloxy, optionally substituted arylcarbonyloxy, optionally substituted $C_{1-18}$ alkylsulfonyloxy, optionally substituted aryl sufonyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, or optionally substituted saturated or unsaturated heterocyclic oxy,
$R_5$ represents a hydrogen atom, hydroxyl, optionally substituted $C_{1-18}$ alkylcarbonyloxy, optionally substituted arylcarbonyloxy, optionally substituted $C_{1-18}$ alkylsulfonyloxy, optionally substituted arylsufonyloxy, optionally substituted aryl $C_{1-6}$ alkyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, or optionally substituted phosphate group, or
$R_4$ and $R_5$ together represent -O-$CR_3$'($R_4$')-O- wherein $R_3$' and $R_4$', which may be the same or different, represent a hydrogen atom or $C_{1-6}$ alkyl; or $R_3$' and $R_4$' together represent thioxo,
$R_6$ and $R_7$ each independently together represent oxo
$R_6$ represents a hydrogen atom, hydroxyl, optionally substituted $C_{1-18}$ alkylcarbonyloxy, optionally substituted arylcarbonyloxy, optionally substituted aryl thiocarbonyloxy, optionally substituted $C_{1-18}$ alkylsulfonyloxy, optionally substituted carbamoyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, optionally substituted saturated or unsaturated heterocyclic oxy, or optionally substituted saturated or unsaturated heterocyclic thiocarbonyloxy,
$R_7$ represents a hydrogen atom, hydroxyl, optionally substituted $C_{1-18}$ alkylcarbonyloxy, or a halogen atom, or
$R_6$ or $R_7$ is absent and one of hydrogen atoms substituted at the carbon atom of the 8-position is lost to form a double bond between the 7-position and the 8-position,
$R_8$ represents a hydrogen atom or a halogen atom,
$R_3$, $R_{10a}$, $R_{10b}$, $R_{11}$, and $R_{12}$ represent a hydrogen atom,
$R_{13a}$, $R_{13b}$, and $R_{13c}$ represent methyl.

In another embodiment of the present invention, in compounds represented by formula (I),
Het represents optionally substituted pyridyl,
X represents an oxygen atom or $NR_9$ wherein $R_9$ represents a hydrogen atom, $C_{1-6}$ alkyl, or aryl $C_{1-6}$ alkyl,
$R_1$, $R_2$, $R_3$, $R_{10a}$, $R_{10b}$, $R_{11}$, and $R_{12}$ represent a hydrogen atom,
$R_4$ represents hydroxyl, optionally substituted $C_{1-18}$ alkylcarbonyloxy, optionally substituted aryl sufonyloxy, $C_{1-6}$ alkyloxy-$C_{1-6}$ alkyloxy, $C_{1-6}$ alkyloxy-$C_{1-6}$ alkyloxy-$C_{1-6}$alkyloxy, or optionally substituted saturated or unsaturated heterocyclic oxy,
$R_5$ represents a hydrogen atom, hydroxyl, optionally substituted $C_{1-18}$ alkylcarbonyloxy, optionally substituted aryl sufonyloxy, optionally substituted aryl $C_{1-6}$ alkyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, or optionally substituted phosphate group, or
$R_4$ and $R_5$ together represent -O-$CR_3$'($R_4$')-O- wherein $R_3$' and $R_4$', which may be the same or different, represent a hydrogen atom or $C_{1-6}$ alkyl, or $R_3$' and $R_4$' together represent thioxo,
$R_6$ represents a hydrogen atom, hydroxyl, optionally substituted $C_{1-18}$ alkylcarbonyloxy, optionally substituted carbamoyloxy, $C_{1-6}$ alkyloxy-$C_{1-6}$alkyloxy, $C_{1-6}$ alkyloxy-$C_{1-6}$ alkyloxy-$C_{1-6}$alkyloxy, optionally substituted saturated or unsaturated heterocyclic oxy, or optionally substituted saturated or unsaturated heterocyclic thiocarbonyloxy,
$R_7$ represents a hydrogen atom, or
$R_6$ or $R_7$ is absent and one of hydrogen atoms substituted at the carbon atom of the 8-position is lost to form a double bond between the 7-position and the 8-position,
$R_8$ represents a hydrogen atom or a halogen atom, and
$R_{13a}$, $R_{13b}$, and $R_{13c}$ represent methyl.

In another embodiment of the present invention, in compounds represented by formula (I),
Het represents optionally substituted pyridyl,
X represents an oxygen atom,
$R_1$, $R_2$, $R_3$, $R_8$, $R_{10a}$, $R_{10b}$, $R_{11}$, and $R_{12}$ represent a hydrogen atom,
$R_4$ represents hydroxyl or optionally substituted $C_{3-6}$ cycloalkylcarbonyloxy,
$R_5$ represents a hydrogen atom or optionally substituted $C_{3-6}$ cycloalkylcarbonyloxy.
$R_6$ represents a hydrogen atom, hydroxyl, or optionally substituted $C_{3-6}$cycloalkylcarbonyloxy,
$R_7$ represents a hydrogen atom, or
$R_6$ or $R_7$ is absent and one of hydrogen atoms substituted at the carbon atom of the 8-position is lost to form a double bond between the 7-position and the 8-position, and
$R_{13a}$, $R_{13b}$, and $R_{13c}$ represent methyl.

In one embodiment, in compounds represented by formula (I),
Het represents optionally substituted pyridyl,
X represents an oxygen atom,
$R_1$ represents a hydrogen atom, hydroxyl, optionally substituted $C_{1-18}$ alkylcarbonyloxy, optionally substituted saturated or unsaturated heterocyclic oxy or optionally substituted $C_{1-18}$ alkylaminocarbonyloxy,
$R_2$ represents a hydrogen atom, or
$R_1$ and $R_2$ together form oxo, or
$R_1$ or $R_2$ is absent and a hydrogen atom substituted at the carbon atom of the 5-position is lost to form a double bond between the 5-position and the13-position,
$R_3$ represents a hydrogen atom,
$R_4$ represents hydroxyl, optionally substituted $C_{1-18}$ alkylcarbonyloxy, optionally substituted aryl carbonyloxy, optionally substituted $C_{1-6}$ alkyl sufonyloxy, or optionally substituted saturated or unsaturated heterocyclic carbonyloxy,
$R_5$ represents a hydrogen atom, hydroxyl, optionally substituted $C_{1-18}$ alkylcarbonyloxy, optionally substituted aryl carbonyloxy, optionally substituted $C_{1-6}$ alkyl sufonyloxy, or optionally substituted saturated or unsaturated heterocyclic carbonyloxy
$R_6$ represents a hydrogen atom, hydroxyl, optionally substituted $C_{1-18}$ alkylcarbonyloxy, optionally substituted arylcarbonyloxy, optionally substituted $C_{1-6}$ alkylsufonyloxy, optionally substituted saturated or unsaturated heterocyclic carbonyloxy, optionally substituted saturated or unsaturated heterocyclic thiocarbonyloxy, $C_{1-6}$ alkylthio-$C_{1-6}$alkyloxy, or optionally substituted $C_{1-6}$ alkylaminocarbonyloxy,
$R_7$ represents a hydrogen atom, hydroxy or a halogen atom, or
$R_6$ and $R_7$ together form oxo,
$R_8$ represents a hydrogen atom,
$R_{10a}$, $R_{10b}$, $R_{11}$, and $R_{12}$ represent hydrogen atoms.
$R_{13a}$, $R_{13b}$, and $R_{13c}$ represent $C_{1-6}$ alkyl optionally substituted by a group selected from the group consisting of hydroxyl, halogen atoms, and cyano.

In another embodiment of the present invention, in compounds represented by formula (I),
Het represents 3-pyridyl,
X represents an oxygen atom,
$R_1$ represents a hydrogen atom, hydroxyl, $C_{1-6}$ alkylcarbonyloxy, $C_{1-6}$ alkylaminocarbonyloxy or five- to seven-membered saturated or unsaturated heterocyclic oxy comprising one to three heteroatoms selected from the group consisting of nitrogen, oxygen, and sulfur atoms wherein the heterocyclic oxy is optionally substituted by trifluoromethyl,
$R_2$ represents a hydrogen atom, or
$R_1$ and $R_2$ together form oxo, or
$R_1$ or $R_2$ is absent and a hydrogen atom substituted at the carbon atom of the 5-position is lost to form a double bond between the 5-position and the13-position,
$R_3$ represents a hydrogen atom,
$R_4$ represents hydroxyl, $C_{1-6}$ alkylcarbonyloxy, phenylcarbonyloxy, $C_{1-3}$ alkyl sufonyloxy, or five- to seven-membered saturated or unsaturated heterocyclic carbonyloxy comprising one to three heteroatoms selected from the group consisting of nitrogen, oxygen, and sulfur atoms wherein the heterocyclic carbonyloxy is optionally substituted by trifluoromethyl,
$R_5$ represents a hydrogen atom, hydroxyl, $C_{1-6}$ alkylcarbonyloxy, phenyl carbonyloxy, $C_{1-3}$ alkylsufonyloxy, $C_{1-6}$ alkylthio-$C_{1-6}$alkyloxy, $C_{1-6}$ alkylaminocarbonyloxy, or five- to seven-membered saturated or unsaturated heterocyclic carbonyloxy comprising one to three heteroatoms selected from the group consisting of nitrogen, oxygen, and sulfur atoms wherein the heterocyclic carbonyloxy is optionally substituted by trifluoromethyl, or five- to seven-membered saturated or unsaturated heterocyclic thiocarbonyloxy comprising one to three heteroatoms selected from the group consisting of nitrogen, oxygen, and sulfur atoms wherein the heterocyclic thiocarbonyloxy is optionally substituted by trifluoromethyl,
$R_6$ represents a hydrogen atom, hydroxyl, optionally substituted $C_{1-6}$ alkylcarbonyloxy, optionally substituted phenylcarbonyloxy, optionally substituted $C_{1-3}$ alkylsufonyloxy, optionally substituted five- to seven-membered saturated, unsaturated heterocyclic carbonyloxy comprising one to three heteroatoms selected from the group consisting of nitrogen, oxygen and sulfur atoms, optionally substituted five- to seven-membered saturated, unsaturated heterocyclic thiocarbonyloxy comprising one to three heteroatoms selected from the group consisting of nitrogen, oxygen and sulfur atoms, $C_{1-3}$ alkylthio-$C_{1-3}$ alkyloxy, or optionally substituted $C_{1-6}$ alkylaminocarbonyloxy,
$R_7$ represents a hydrogen atom, hydroxy or a halogen atom, or
$R_6$ and $R_7$ together form oxo,
$R_8$ represents a hydrogen atom,
$R_{10a}$, $R_{10b}$, $R_{11}$, and $R_{12}$ represent hydrogen atoms.
$R_{13a}$, $R_{13b}$, and $R_{13c}$ represent $C_{1-6}$ alkyl.

In another preferred embodiment of the present invention, in compounds represented by formula (I),

Het represents optionally substituted pyridyl,
X represents an oxygen atom,
$R_1$ represents a hydrogen atom, hydroxyl, optionally substituted $C_{1-6}$ alkylcarbonyloxy, optionally substituted five- to seven-membered saturated, unsaturated heterocyclic oxy comprising one to three heteroatoms selected from the group consisting of nitrogen, oxygen, and sulfur atoms or optionally substituted $C_{1-6}$ alkylaminocarbonyloxy,
$R_2$ represents a hydrogen atom, or
$R_1$ and $R_2$ together form oxo, or
$R_1$ or $R_2$ is absent and a hydrogen atom substituted at the carbon atom of the 5-position is lost to form a double bond between the 5-position and the13-position,
$R_3$ represents a hydrogen atom,
$R_4$ represents hydroxyl, optionally substituted $C_{1-6}$ alkylcarbonyloxy, optionally substituted aryl carbonyloxy, optionally substituted $C_{1-3}$ alkyl sufonyloxy, or optionally substituted five- to seven-membered saturated, unsaturated heterocyclic carbonyloxy comprising one to three heteroatoms selected from the group consisting of nitrogen, oxygen and sulfur atoms,
$R_5$ represents a hydrogen atom, hydroxyl, optionally substituted $C_{1-6}$ alkylcarbonyloxy, optionally substituted phenyl carbonyloxy, optionally substituted $C_{1-3}$ alkylsufonyloxy, or optionally substituted saturated or optionally substituted five- to seven-membered saturated, unsaturated heterocyclic carbonyloxy comprising one to three heteroatoms selected from the group consisting of nitrogen, oxygen and sulfur atoms,
$R_6$ represents a hydrogen atom, hydroxyl, optionally substituted $C_{1-6}$ alkylcarbonyloxy, optionally substituted phenylcarbonyloxy, optionally substituted $C_{1-3}$ alkylsufonyloxy, optionally substituted five- to seven-membered saturated, unsaturated heterocyclic carbonyloxy comprising one to three heteroatoms selected from the group consisting of nitrogen, oxygen and sulfur atoms, optionally substituted five- to seven-membered saturated, unsaturated heterocyclic thiocarbonyloxy comprising one to three heteroatoms selected from the group consisting of nitrogen, oxygen and sulfur atoms, $C_{1-3}$ alkylthio-$C_{1-3}$ alkyloxy, or optionally substituted $C_{1-6}$ alkylaminocarbonyloxy,
$R_7$ represents a hydrogen atom, hydroxy or a halogen atom, or
$R_6$ and $R_7$ together form oxo,
$R_8$ represents a hydrogen atom,
$R_{10a}$, $R_{10b}$, $R_{11}$, and $R_{12}$ represent hydrogen atoms.
$R_{13a}$, $R_{13b}$, and $R_{13c}$ represent $C_{1-6}$ alkyl.

In one embodiment, in compounds represented by formula (I-a'),
Het represents optionally substituted pyridyl,
X represents an oxygen atom,
$R_4$ represents hydroxyl or optionally substituted $C_{1-18}$ alkylcarbonyloxy,
$R_5$ represents a hydrogen atom, hydroxyl, or optionally substituted $C_{1-18}$ alkylcarbonyloxy or
$R_4$ and $R_5$ together represent -O-$CR_3$'($R_4$')-O-wherein $R_3$' and $R_4$', which may be the same or different, represent a hydrogen atom or $C_{1-6}$ alkyl, or $R_3$' and $R_4$' together represent thioxo,
$R_6$ represents a hydrogen atom, hydroxyl, or optionally substituted $C_{1-18}$ alkylcarbonyloxy,
$R_7$ represent a hydrogen atom, or
$R_6$ or $R_7$ is absent and one of hydrogen atoms substituted at the carbon atom of the 8-position is lost to form a double bond between the 7-position and the 8-position,
$R_8$ represent a hydrogen atom, provided that,
when $R_5$, $R_6$, and $R_7$ simultaneously represent a hydrogen atom, $R_4$ does not represent hydroxyl, acetyloxy, or propionyloxy,
when $R_6$ and $R_7$ simultaneously represent a hydrogen atom, neither $R_4$ represents acetyloxy nor $R_5$ represent propionyloxy, or
when $R_6$ and $R_7$ simultaneously represent a hydrogen atom, neither $R_4$ nor $R_5$ represents acetyloxy, or
when $R_6$ and $R_7$ simultaneously represent a hydrogen atom, neither $R_4$ nor represents propionyloxy and $R_5$ does not represent acetyloxy, and
when $R_4$ and $R_5$ represent acetyloxy, neither $R_6$ represents propionyloxy nor $R_7$ represents a hydrogen atom.

In one embodiment of the present invention, in compounds represented by formula (I-a'),
Het represents optionally substituted pyridyl,
X represents an oxygen atom,
$R_4$ represents hydroxyl, or optionally substituted $C_{1-18}$ alkylcarbonyloxy,
$R_5$ represents a hydrogen atom, or, optionally substituted $C_{1-18}$ alkylcarbonyloxy,
$R_6$, $R_7$ and $R_8$ represents a hydrogen atom.

In a preffered embodiment of the present invention, in compounds represented by formula (I-a'),
Het represents optionally substituted pyridyl,
X represents an oxygen atom,
$R_4$ represents hydroxyl, or optionally substituted $C_{1-6}$ alkylcarbonyloxy,

$R_5$ represents a hydrogen atom, or optionally substituted $C_{1-6}$ alkylcarbonyloxy,
$R_6$, $R_7$ and $R_8$ represents a hydrogen atom.

In still another preferred embodiment of the present invention, in compounds represented by formula (I-a'), Het represents 3-pyridyl,
X represents an oxygen atom,
$R_4$ represents hydroxyl, or $C_{1-6}$ alkylcarbonyloxy,
$R_5$ represents a hydrogen atom, or $C_{1-6}$ alkylcarbonyloxy,
$R_6$, $R_7$ and $R_8$ represents a hydrogen atom.

In still another preferred embodiment of the present invention, in compounds represented by formula (I-a'), Het represents 3-pyridyl,
X represents an oxygen atom,
$R_4$ represents hydroxyl or $C_{1-6}$ alkylcarbonyloxy,
$R_5$ represents a hydrogen atom, hydroxyl, or $C_{1-6}$ alkylcarbonyloxy, or $R_4$ and $R_5$ together represent -O-$CR_3$'($R_4$')-O-, wherein $R_3$' and $R_4$', which may be the same or different, represent a hydrogen atom or $C_{1-6}$ alkyl,
$R_6$ represents a hydrogen atom, hydroxyl, or $C_{1-6}$ alkylcarbonyloxy, and
$R_7$ and $R_8$ represent a hydrogen atom.

In one embodiment of the present invention, in compounds represented by formula (I-b),
Het represents optionally substituted pyridyl,
X represents an oxygen atom,
$R_4$ represents hydroxyl or optionally substituted $C_{3-6}$ cycloalkylcarbonyloxy,
$R_5$ represents a hydrogen atom, or optionally substituted $C_{3-6}$ cycloalkylcarbonyloxy,
$R_6$, $R_7$ and $R_8$ represent a hydrogen atom.

In a further preferred embodiment of the present invention, in compounds represented by formula (I-b), Het represents 3-pyridyl,
X represents an oxygen atom,
$R_4$ represents hydroxyl or $C_{3-6}$ cycloalkylcarbonyloxy,
$R_5$ represents a hydrogen atom or $C_{3-6}$ cycloalkylcarbonyloxy,
$R_6$ represents a hydrogen atom or hydroxyl, and
$R_7$ and $R_8$ represent a hydrogen atom.

In another preferred embodiment of the present invention, in compounds represented by formula (I-c), Het represents 3-pyridyl,
X represents an oxygen atom,
$R_1$ represents acetyloxy, $C_{3-6}$ cycloalkylcarbonyloxy or methoxy,
$R_4$ represents $C_{3-6}$cycloalkylcarbonyloxy,
$R_5$ represents $C_{3-6}$cycloalkylcarbonyloxy,
$R_6$ represents hydroxyl, acetyloxy or $C_{3-6}$ cycloalkylcarbonyloxy, and
$R_7$ and $R_8$ represent a hydrogen atom.

In still another preferred embodiment of the present invention, in compounds represented by formula (I-c), Het represents 3-pyridyl,
X represents an oxygen atom,
$R_1$ represents acetyloxy, $C_{3-6}$ cycloalkylcarbonyloxy or methoxy,
$R_4$ and $R_5$ represent cyclopropanecarbonyloxy,
$R_6$ represents hydroxyl, acetyloxy or $C_{3-6}$ cyclopropanecarbonyloxy, and
$R_7$ and $R_8$ represent a hydrogen atom.

Salts of compounds represented by formula (I), (I-a), (I-a'), (I-b), or (I-c) include, for example, agricultural or zootechnically acceptable salts, for example, acid addition salts such as hydrochlorides, nitrates, sulfates, phosphates, or acetates.

Specific examples of compounds represented by formula (I) (I-a), or (I-c) include compounds shown in Tables 1 to 30 below. In the following tables, H(=) means that a hydrogen atom in $R_1$ or $R_2$ and a hydrogen atom at the 5-position, or a hydrogen atom in $R_6$ or $R_7$ and a hydrogen atom at the 8-position, are absent and a portion between the 5-position and the 13-position or a portion between the 7-position and the 8-position is a double bond.

[Table 1]

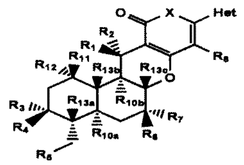

*** This table represents the combination of the groups corresponding to each line in Table A.**

| | Compound No. | $R_1$ | $R_2$ | $R_3$ | X | Het | R10a | R10b | R11 | R12 | R13a | R13b | R13c | |
|---|---|---|---|---|---|---|---|---|---|---|---|---|---|---|
| Table 1 | 1-1~1-1442 | OH | H | H | O | 3-pyridyl | H | H | H | H | CH3 | CH3 | CH3 | * |
| Table 2 | 2-1~2-1442 | OH | H | H | O | 6-Cl-3-pyridyl | H | H | H | H | CH3 | CH3 | CH3 | * |
| Table 3 | 3-1~3-1442 | OH | H | H | O | 4-CF3-3-pyridyl | H | H | H | H | CH3 | CH3 | CH3 | * |
| Table 4 | 4-1~4-1442 | OH | H | H | O | 2-pyridyl | H | H | H | H | CH3 | CH3 | CH3 | * |
| Table 5 | 5-1~5-1442 | OH | H | H | O | 4-pyridyl | H | H | H | H | CH3 | CH3 | CH3 | |
| Table 6 | 6-1~6-1442 | OH | H | H | O | N-oxide 3-pyridyl | H | H | H | H | CH3 | CH3 | CH3 | * |
| Table 7 | 7-1~7-1442 | OH | H | H | O | N-methyl 3-pyridyl | H | H | H | H | CH3 | CH3 | CH3 | * |
| Table 8 | 8-1~8-1442 | OH | H | H | O | Phenyl | H | H | H | H | CH3 | CH3 | CH3 | |
| Table 9 | 9-1~9-1442 | OH | H | H | NH | 3-pyridyl | H | H | H | H | CH3 | CH3 | CH3 | * |
| Table 10 | 10-1~10-1442 | OH | H | H | NH | 6-Cl-3-pyridyl | H | H | H | H | CH3 | CH3 | CH3 | * |
| Table 11 | 11-1~11-1442 | OH | H | H | NH | 4-CF3-3-pyridyl | H | H | H | H | CH3 | CH3 | CH3 | * |
| Table 12 | 12-1~12-1442 | OH | H | H | NH | 2-pyridyl | H | H | H | H | CH3 | CH3 | CH3 | * |
| Table 13 | 13-1~13-1442 | OH | H | H | NH | 4-pyridyl | H | H | H | H | CH3 | CH3 | CH3 | * |
| Table 14 | 14-1~14-1442 | OH | H | H | NCH3 | 3-pyridyl | H | H | H | H | CH3 | CH3 | CH3 | * |
| Table 15 | 15-1~15-1442 | OH | H | H | NCH3 | 6-Cl-3-pyridyl | H | H | H | H | CH3 | CH3 | CH3 | * |
| Table 16 | 16-1~16-1442 | OH | H | H | NCH3 | 4-CF3-3-pyridyl | H | H | H | H | CH3 | CH3 | CH3 | * |
| Table 17 | 17-1~17-1442 | OH | H | H | NCH3 | 2-pyridyl | H | H | H | H | CH3 | CH3 | CH3 | * |
| Table 18 | 18-1~18-1442 | OH | H | H | NCH3 | 4-pyridyl | H | H | H | H | CH3 | CH3 | CH3 | * |
| Table 19 | 19-1~19-1442 | OH | H | H | NCH2C6H5 | 3-pyridyl | H | H | H | H | CH3 | CH3 | CH3 | * |
| Table 20 | 20-1~20-1442 | OH | H | H | NCH2C6H5 | 6-Cl-3-pyridyl | H | H | H | H | CH3 | CH3 | CH3 | * |
| Table 21 | 21-1~21-1442 | OH | H | H | NCH2C6H5 | 4-CF3-3-pyridyl | H | H | H | H | CH3 | CH3 | CH3 | * |
| Table 22 | 22-1~22-1442 | OH | H | F | O | 3-pyridyl | H | H | H | H | CH3 | CH3 | CH3 | * |

(continued)

| 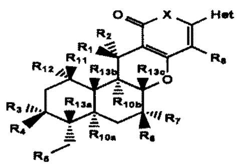 | | | * This table represents the combination of the groups corresponding to each line in Table A. | | | | | | | | | | | |
|---|---|---|---|---|---|---|---|---|---|---|---|---|---|---|
| | Compound No. | $R_1$ | $R_2$ | $R_3$ | X | Het | R10a | R10b | R11 | R12 | R13a | R13b | R13c | |
| Table 23 | 23-1~23-1442 | OH | H | F | O | 6-Cl-3-pyridyl | H | H | H | H | CH3 | CH3 | CH3 | * |

[Table 2]

| | Compound No. | $R_1$ | $R_2$ | $R_8$ | X | Het | R10a | R10b | R11 | R12 | R13a | R13b | R13c | |
|---|---|---|---|---|---|---|---|---|---|---|---|---|---|---|
| Table 24 | 24-1~24-1442 | OH | H | F | NH | 3-pyridyl | H | H | H | H | CH3 | CH3 | CH3 | * |
| Table 25 | 25-1~25-1442 | OH | H | F | NCH3 | 3-pyridyl | H | H | H | H | CH3 | CH3 | CH3 | * |
| Table 26 | 26-1 ~26-1442 | OH | H | Cl | O | 3-pyridyl | H | H | H | H | CH3 | CH3 | CH3 | * |
| Table 27 | 27-1~27-1442 | OH | H | Cl | O | 6-Cl-3-pyridyl | H | H | H | H | CH3 | CH3 | CH3 | * |
| Table 28 | 28-1~28-1442 | OH | H | Cl | NH | 3-pyridyl | H | H | H | H | CH3 | CH3 | CH3 | * |
| Table 29 | 29-1~29-1442 | OH | H | Cl | NH | 4-CF3-3-pyridyl | H | H | H | H | CH3 | CH3 | CH3 | * |
| Table30 | 30-1~30-1442 | OH | H | Cl | NCH3 | 3-pyridyl | H | H | H | H | CH3 | CH3 | CH3 | * |
| Table 31 | 31-1~31-1442 | OH | H | Cl | NCH3 | 6-Cl-3-pyridyl | H | H | H | H | CH3 | CH3 | CH3 | * |
| Table 32 | 32-1~32-1442 | OH | H | Br | O | 3-pyridyl | H | H | H | H | CH3 | CH3 | CH3 | * |
| Table 33 | 33-1~33-1442 | OH | H | Br | O | 4-CF3-3-pyridyl | H | H | H | H | CH3 | CH3 | CH3 | * |
| Table 34 | 34-1~34-1442 | OH | H | Br | NH | 3-pyridyl | H | H | H | H | CH3 | CH3 | CH3 | * |
| Table 35 | 35-1~35-1442 | OH | H | Br | NCH3 | 3-pyridyl | H | H | H | H | CH3 | CH3 | CH3 | * |
| Table 36 | 36-1~36-1442 | OH | H | CN | O | 3-pyridyl | H | H | H | H | CH3 | CH3 | CH3 | * |
| Table 37 | 37-1~37-1442 | OH | H | CN | O | 4-CF3-3-pyridyl | H | H | H | H | CH3 | CH3 | CH3 | * |
| Table 38 | 38-1~38-1442 | OH | H | CN | NH | 3-pyridyl | H | H | H | H | CH3 | CH3 | CH3 | * |
| Table 39 | 39-1~39-1442 | OH | H | CN | NCH3 | 3-pyridyl | H | H | H | H | CH3 | CH3 | CH3 | * |
| Table 40 | 40-1~40-1442 | OH | H | CH2Ph | O | 3-pyridyl | H | H | H | H | CH3 | CH3 | CH3 | * |
| Table 41 | 41-1~41-1442 | OH | H | CH2Ph | O | N-oxide 3-pyridyl | H | H | H | H | CH3 | CH3 | CH3 | * |
| Table 42 | 42-1~42-1442 | OH | H | CH2Ph | NCH3 | 3-pyridyl | H | H | H | H | CH3 | CH3 | CH3 | * |
| Table 43 | 43-1~43-1442 | H | H | H | O | 3-pyridyl | H | H | H | H | CH3 | CH3 | CH3 | * |
| Table 44 | 44-1~44-1442 | H | H | H | O | 6-Cl-3-pyridyl | H | H | H | H | CH3 | CH3 | CH3 | * |
| Table 45 | 45-1~45-1442 | H | H | H | O | 4-CF3-3-pyridyl | H | H | H | H | CH3 | CH3 | CH3 | * |
| Table 46 | 46-1~46-1442 | H | H | H | O | 2-pyridyl | H | H | H | H | CH3 | CH3 | CH3 | * |
| Table 47 | 47-1~47-1442 | H | H | H | O | 4-pyridyl | H | H | H | H | CH3 | CH3 | CH3 | * |
| Table 48 | 48-1~48-1442 | H | H | H | O | Phenyl | H | H | H | H | CH3 | CH3 | CH3 | * |

(continued)

| | Compound No. | $R_1$ | $R_2$ | $R_8$ | X | Het | R10a | R10b | R11 | R12 | R13a | R13b | R13c | |
|---|---|---|---|---|---|---|---|---|---|---|---|---|---|---|
| Table 49 | 49-1~49-1442 | H | H | H | NH | 3-pyridyl | H | H | H | H | CH3 | CH3 | CH3 | * |
| Table 50 | 50-1~50-1442 | H | H | H | NH | 6-Cl-3-pyridyl | H | H | H | H | CH3 | CH3 | CH3 | * |

[Table 3]

| | Compound No. | $R_1$ | $R_2$ | $R_8$ | X | Het | R10a | R10b | R11 | R12 | R13a | R13b | R13c | |
|---|---|---|---|---|---|---|---|---|---|---|---|---|---|---|
| Table 51 | 51-1~51-1442 | H | H | H | NH | 4-pyridyl | H | H | H | H | CH3 | CH3 | CH3 | * |
| Table 52 | 52-1~52-1442 | H | H | H | NCH3 | 3-pyridyl | H | H | H | H | CH3 | CH3 | CH3 | * |
| Table 53 | 53-1~53-1442 | H | H | H | NCH3 | 6-Cl-3-pyridyl | H | H | H | H | CH3 | CH3 | CH3 | * |
| Table 54 | 54-1~54-1442 | H | H | H | NCH3 | 2-pyridyl | H | H | H | H | CH3 | CH3 | CH3 | * |
| Table 55 | 55-1~55-1442 | H | H | H | NCH2C6H5 | 3-pyridyl | H | H | H | H | CH3 | CH3 | CH3 | * |
| Table 56 | 56-1~56-1442 | H | H | F | O | 3-pyridyl | H | H | H | H | CH3 | CH3 | CH3 | * |
| Table 57 | 57-1~57-1442 | H | H | F | NH | 3-pyridyl | H | H | H | H | CH3 | CH3 | CH3 | * |
| Table 58 | 58-1~58-1442 | H | H | F | NCH3 | 3-pyridyl | H | H | H | H | CH3 | CH3 | CH3 | * |
| Table 59 | 59-1~59-1442 | H | H | Cl | O | 3-pyridyl | H | H | H | H | CH3 | CH3 | CH3 | * |
| Table 60 | 6&-1~60-1442 | H | H | Cl | O | 6-Cl-3-pyridyl | H | H | H | H | CH3 | CH3 | CH3 | * |
| Table 61 | 61-1~61-1442 | H | H | Cl | NH | 3-pyridyl | H | H | H | H | CH3 | CH3 | CH3 | * |
| Table 62 | 62-1~62-1442 | H | H | Cl | NCH3 | 3-pyridyl | H | H | H | H | CH3 | CH3 | CH3 | * |
| Table 63 | 63-1~63-1442 | H | H | Br | O | 3-pyridyl | H | H | H | H | CH3 | CH3 | CH3 | * |
| Table 64 | 64-1~64-1442 | H | H | Br | NH | 3-pyridyl | H | H | H | H | CH3 | CH3 | CH3 | * |
| Table 65 | 65-1~65-1442 | H | H | Br | NCH3 | 3-pyridyl | H | H | H | H | CH3 | CH3 | CH3 | * |
| Table 66 | 66-1~66-1442 | H | H | CN | O | 3-pyridyl | H | H | H | H | CH3 | CH3 | CH3 | * |
| Table 67 | 67-1~67-1442 | H | H | CN | O | 4-CF3-3-pyridyl | H | H | H | H | CH3 | CH3 | CH3 | * |
| Table 68 | 68-1~68-1442 | H | H | CN | NH | 3-pyridyl | H | H | H | H | CH3 | CH3 | CH3 | * |
| Table 69 | 69-1~69-1442 | H | H | CN | NCH3 | 3-pyridyl | H | H | H | H | CH3 | CH3 | CH3 | * |
| Table 70 | 70-1~70-1442 | H | H | CH2Ph | O | 3-pyridyl | H | H | H | H | CH3 | CH3 | CH3 | * |
| Table 71 | 71-1~71-1442 | H | H | CH2Ph | NCH3 | 3-pyridyl | H | H | H | H | CH3 | CH3 | CH3 | * |
| Table 72 | 72-1~72-1442 | H(=) | | H | O | 3-pyridyl | H | H | H | H | CH3 | CH3 | CH3 | * |
| Table 73 | 73-1~73-1442 | H(=) | | H | O | 4-CF3-3-pyridyl | H | H | H | H | CH3 | CH3 | CH3 | |
| Table 74 | 74-1~74-1442 | H(=) | | H | O | 2-pyridyl | H | H | H | H | CH3 | CH3 | CH3 | * |
| Table 75 | 75-1~75-1442 | H(=) | | H | O | Phenyl | H | H | H | H | CH3 | CH3 | CH3 | * |

(continued)

| | Compound No. | $R_1$ | $R_2$ | $R_8$ | X | Het | R10a | R10b | R11 | R12 | R13a | R13b | R13c | |
|---|---|---|---|---|---|---|---|---|---|---|---|---|---|---|
| Table 76 | 76-1~76-1442 | H(=) | | H | NH | 3-pyridyl | H | H | H | H | CH3 | CH3 | CH3 | * |
| Table 77 | 77-1~77-1442 | H(=) | | H | NCH3 | 3-pyridyl | H | H | H | H | CH3 | CH3 | CH3 | * |

[Table 4]

| | Compound No. | $R_1$ | $R_2$ | $R_8$ | X | Het | R10a | R10b | R11 | R12 | R13a | R13b | R13c | |
|---|---|---|---|---|---|---|---|---|---|---|---|---|---|---|
| Table 78 | 78-1~78-7442 | H(=) | | H | NCH2C6H5 | 3-pyridyl | H | H | H | H | CH3 | CH3 | CH3 | * |
| Table 79 | 79-1~79-1442 | H(=) | | F | O | 3-pyridyl | H | H | H | H | CH3 | CH3 | CH3 | * |
| Table 80 | 80-1~80-1442 | H(=) | | F | NCH3 | 3-pyridyl | H | H | H | H | CH3 | CH3 | CH3 | * |
| Table 81 | 81-1~-81-1442 | H(=) | | Cl | O | 3-pyridyl | H | H | H | H | CH3 | CH3 | CH3 | * |
| Table 82 | 82-1~82-1442 | H(=) | | Cl | NCH3 | 3-pyridyl | H | H | H | H | CH3 | CH3 | CH3 | * |
| Table 83 | 83-1~83-1442 | H(=) | | Br | O | 3-pyridyl | H | H | H | H | CH3 | CH3 | CH3 | * |
| Table 84 | 84-1~84-1442 | H(=) | | Br | NCH3 | 3-pyridyl | H | H | H | H | CH3 | CH3 | CH3 | * |
| Table 85 | 85-1~85-1442 | H(=) | | CN | O | 3-pyridyl | H | H | H | H | CH3 | CH3 | CH3 | * |
| Table 86 | 86-1~86-1442 | H(=) | | CN | NH | 3-pyridyl | H | H | H | H | CH3 | CH3 | CH3 | * |
| Table 87 | 87-1~87-1442 | H(=) | | CN | NCH3 | 3-pyridyl | H | H | H | H | CH3 | CH3 | CH3 | * |
| Table 88 | 88-1~88-1442 | H(=) | | CH2Ph | O | 3-pyridyl | H | H | H | H | CH3 | CH3 | CH3 | * |
| Table 89 | 89-1~89-1442 | =O | | H | O | 3-pyridyl | H | H | H | H | CH3 | CH3 | CH3 | * |
| Table 90 | 90-1~90-1442 | =O | | H | O | 6-Cl-3-pyridyl | H | H | H | H | CH3 | CH3 | CH3 | * |
| Table 91 | 91-1~91-1442 | =O | | H | O | 4-CF3-3-pyridyl | H | H | H | H | CH3 | CH3 | CH3 | * |
| Table 92 | 92-1~92-1442 | =O | | H | O | 2-pyridyl | H | H | H | H | CH3 | CH3 | CH3 | * |
| Table 93 | 93-1~93-1442 | =O | | H | O | 4-pyridyl | H | H | H | H | CH3 | CH3 | CH3 | * |
| Table 94 | 94-1~94-1442 | =O | | H | O | 6-Cl-3-pyridyl | H | H | H | H | CH3 | CH3 | CH3 | * |
| Table 95 | 95-1~95-1442 | =O | | H | O | N-oxide 3-pyridyl | H | H | H | H | CH3 | CH3 | CH3 | * |
| Table 96 | 96-1~96-1442 | =O | | H | O | Phenyl | H | H | H | H | CH3 | CH3 | CH3 | * |
| Table 97 | 97-1~97-1442 | =O | | H | NH | 3-pyridyl | H | H | H | H | CH3 | CH3 | CH3 | * |
| Table 98 | 98-1~98-1442 | =O | | H | NH | 6-Cl-3-pyridyl | H | H | H | H | CH3 | CH3 | CH3 | * |
| Table 99 | 99-1~99-1442 | =O | | H | NH | 4-pyridyl | H | H | H | H | CH3 | CH3 | CH3 | * |
| Table 100 | 100-1~100-1442 | =O | | H | NCH3 | 3-pyridyl | H | H | H | H | CH3 | CH3 | CH3 | * |
| Table 101 | 101-1~101-1442 | =O | | H | NCH3 | 6-Cl-3-pyridyl | H | H | H | H | CH3 | CH3 | CH3 | * |
| Table 102 | 102-1~102-1442 | =O | | H | NCH3 | 2-pyridyl | H | H | H | H | CH3 | CH3 | CH3 | * |

(continued)

| | Compound No. | $R_1$ | $R_2$ | $R_8$ | X | Het | R10a | R10b | R11 | R12 | R13a | R13b | R13c | |
|---|---|---|---|---|---|---|---|---|---|---|---|---|---|---|
| Table 103 | 103-1~103-1442 | =O | | H | MCH2C6H5 | 3-pyridyl | H | H | H | H | CH3 | CH3 | CH3 | * |
| Table 104 | 104-1~104-1442 | =O | | F | O | 3-pyridyl | H | H | H | H | CH3 | CH3 | CH3 | * |

[Table 5]

| | Compound No. | $R_1$ | $R_2$ | $R_8$ | X | Het | R10a | R10b | R11 | R12 | R13a | R13b | R13c | |
|---|---|---|---|---|---|---|---|---|---|---|---|---|---|---|
| Table 105 | 105-1~105-1442 | =O | | F | NH | 3-pyridyl | H | H | H | H | CH3 | CH3 | CH3 | * |
| Table 106 | 106-1~106-1442 | =O | | F | NCH3 | 3-pyridyl | H | H | H | H | CH3 | CH3 | CH3 | * |
| Table 107 | 107-1~107-1442 | =O | | Cl | O | 3-pyridyl | H | H | H | H | CH3 | CH3 | CH3 | * |
| Tab!e 108 | 108-1~108-1442 | =O | | Cl | NH | 3-pyridyl | H | H | H | H | CH3 | CH3 | CH3 | * |
| Table 109 | 109-1~109-1442 | =O | | Br | O | 3-pyridyl | H | H | H | H | CH3 | CH3 | CH3 | * |
| Table 110 | 110-1~110-1442 | =O | | Br | NCH3 | 3-pyridyl | H | H | H | H | CH3 | CH3 | CH3 | * |
| Table 111 | 111-1~111-1442 | =O | | CN | O | 3-pyridyl | H | H | H | H | CH3 | CH3 | CH3 | * |
| Table 112 | 112-1~112-1442 | =O | | CN | NCH3 | 3-pyridyl | H | H | H | H | CH3 | CH3 | CH3 | * |
| Table 113 | 113-1~113-1442 | =O | | CH2Ph | O | 3-pyridyl | H | H | H | H | CH3 | CH3 | CH3 | * |
| Table 114 | 114-1~14-1442 | OCOC2H5 | H | H | O | 3-pyridyl | H | H | H | H | CH3 | CH3 | CH3 | * |
| Table 115 | 115-1~115-1442 | OCOC6H5 | H | H | O | 3-pyridyl | H | H | H | H | CH3 | CH3 | CH3 | * |
| Table 116 | 116-1~116-1442 | OCOCH2C6H5 | H | H | O | 3-pyridyl | H | H | H | H | CH3 | CH3 | CH3 | * |
| Table 117 | 117-1~117-1442 | OCOCH2-2-pyridyl | H | H | O | 3-pyridyl | H | H | H | H | CH3 | CH3 | CH3 | * |
| Table 118 | 118-1~118-1442 | OCOCHCH2 | H | H | O | 3-pyridyl | H | H | H | H | CH3 | CH3 | CH3 | * |
| Table 119 | 119-1~119-1442 | OCOCCH | H | H | O | 3-pyridyl | H | H | H | H | CH3 | CH3 | CH3 | * |
| Table 120 | 120-1~120-1442 | OCO-3-pyridyl | H | H | O | 3-pyridyl | H | H | H | H | CH3 | CH3 | CH3 | * |
| Table 121 | 121-1~121-1442 | OCH3 | H | H | O | 3-pyridyl | H | H | H | H | CH3 | CH3 | CH3 | * |
| Table 122 | 122-1~122-1442 | OCH3 | H | H | O | N-oxide 3-pyridyl | H | H | H | H | CH3 | CH3 | CH3 | * |
| Table 123 | 123-1~123-1442 | OC2H5 | H | H | O | 3-pyridyl | H | H | H | H | CH3 | CH3 | CH3 | * |
| Table 124 | 124-1~124-1442 | OC4H9 | H | H | O | 3-pyridyl | H | H | H | H | CH3 | CH3 | CH3 | * |
| Table 125 | 125-1~125-1442 | OCH(CH3)2 | H | H | O | 3-pyridyl | H | H | H | H | CH3 | CH3 | CH3 | * |
| Table 126 | 126-1~126-1442 | OC(CH3)3 | H | H | O | 3-pyridyl | H | H | H | H | CH3 | CH3 | CH3 | * |
| Table 127 | 127-1~127-1442 | O-c-C3H5 | H | H | O | 3-pyridyl | H | H | H | H | CH3 | CH3 | CH3 | * |
| Table 128 | 128-1~128-1442 | OCH2-c-C3H5 | H | H | O | 3-pyridyl | H | H | H | H | CH3 | CH3 | CH3 | * |
| Table 129 | 129-1~129-1442 | OCH2C6H5 | H | H | O | 3-pyridyl | H | H | H | H | CH3 | CH3 | CH3 | * |

(continued)

| | Compound No. | $R_1$ | $R_2$ | $R_8$ | X | Het | R10a | R10b | R11 | R12 | R13a | R13b | R13c | |
|---|---|---|---|---|---|---|---|---|---|---|---|---|---|---|
| Table 130 | 130-1~130-1442 | OCOOCH3 | H | H | O | 3-pyridyl | H | H | H | H | CH3 | CH3 | CH3 | * |
| Table 131 | 131-1~131-1442 | OCHCH2 | H | H | O | 3-pyridyl | H | H | H | H | CH3 | CH3 | CH3 | * |

[Table 6]

| | Compound No. | $R_1$ | $R_2$ | $R_8$ | X | Het | R10a | R10b | R11 | R12 | R13a | R13b | R13c | |
|---|---|---|---|---|---|---|---|---|---|---|---|---|---|---|
| Table 132 | 132-1~132-1442 | OCCH | H | H | O | 3-pyridyl | H | H | H | H | CH3 | CH3 | CH3 | * |
| Table 133 | 133-1~133-1442 | OCH2C6H5 | H | H | O | N-oxide 3-pyridyl | H | H | H | H | CH3 | CH3 | CH3 | * |
| Table 134 | 134-1~134-1442 | OCH2OCH3 | H | H | O | 3-pyridyl | H | H | H | H | CH3 | CH3 | CH3 | * |
| Table 135 | 135-1~135-1442 | O-(2-Tetrahydropyranyl) | H | H | O | 3-pyridyl | H | H | H | H | CH3 | CH3 | CH3 | * |
| Table 136 | 136-1~136-1442 | 2-Tetrahydropyranyl | H | H | O | 3-pyridyl | H | H | H | H | CH3 | CH3 | CH3 | * |
| Table 137 | 137-1~137-1442 | O-tetra-O-benzyl-mannose | H | H | O | 3-pyridyl | H | H | H | H | CH3 | CH3 | CH3 | * |
| Table 138 | 138-1~138-1442 | CONHCH3 | H | H | O | 3-pyridyl | H | H | H | H | CH3 | CH3 | CH3 | * |
| Table 139 | 139-1~139-1442 | OCONHCH2CH2CH3 | H | H | O | 3-pyridyl | H | H | H | H | CH3 | CH3 | CH3 | * |
| Table 140 | 140-1~140-1442 | OSO2CH3 | H | H | O | 3-pyridyl | H | H | H | H | CH3 | CH3 | CH3 | * |
| Table 141 | 141-1~141-1442 | OSO2-c-C3H5 | H | H | O | 3-pyridyl | H | H | H | H | CH3 | CH3 | CH3 | * |
| Table 142 | 142-1~142-1442 | SO2C2H5 | H | H | O | 3-pyridyl | H | H | H | H | CH3 | CH3 | CH3 | * |
| Table 143 | 143-1~143-1442 | OSO2C6H5 | H | H | O | 3-pyridyl | H | H | H | H | CH3 | CH3 | CH3 | * |
| Table 144 | 144-1~144-1442 | SO2C6H5 | H | H | O | 3-pyridyl | H | H | H | H | CH3 | CH3 | CH3 | * |
| Table 145 | 145-1~145-1442 | C3H7 | H | H | O | 3-pyridyl | H | H | H | H | CH3 | CH3 | CH3 | * |

(continued)

| | Compound No. | $R_1$ | $R_2$ | $R_8$ | X | Het | R10a | R10b | R11 | R12 | R13a | R13b | R13c | |
|---|---|---|---|---|---|---|---|---|---|---|---|---|---|---|
| Table 146 | 146-1~146-1442 | N3 | H | H | O | 3-pyridyl | H | H | H | H | CH3 | CH3 | CH3 | * |
| Table 147 | 147-1~147-1442 | NH2 | H | H | O | 3-pyridyl | H | H | H | H | CH3 | CH3 | CH3 | * |
| Table 148 | 148-1~148-1442 | NHCH3 | H | H | O | 3-pyridyl | H | H | H | H | CH3 | CH3 | CH3 | * |
| Table 149 | 149-1~149-1442 | N (CH3) 2 | H | H | O | 3-pyridyl | H | H | H | H | CH3 | CH3 | CH3 | * |
| Table 150 | 150-1~150-1442 | CN | H | H | O | 3-pyridyl | H | H | H | H | CH3 | CH3 | CH3 | * |
| Table 151 | 151-1~151-1442 | F | H | H | O | 3-pyridyl | H | H | H | H | CH3 | CH3 | CH3 | * |
| Table 152 | 152-1~152-1442 | Cl | H | H | O | 3-pyridyl | H | H | H | H | CH3 | CH3 | CH3 | * |
| Table 15. | 153-1~153-1442 | Br | H | H | O | 3-pyridyl | H | H | H | H | CH3 | CH3 | CH3 | * |
| Table 154 | 154-1~154-1442 | OH | H | H | O | 3-pyridyl | H | H | H | H | C2H5 | C2H5 | C2H5 | * |
| Table 155 | 155-1~155-1442 | OH | H | H | O | 2-pyridyl | H | H | H | H | C2H5 | C2H5 | C2H5 | * |
| Table 156 | 156-1~156-1442 | OH | H | H | O | 4-pyridyl | H | H | H | H | C2H5 | C2H5 | C2H5 | * |
| Table 157 | 157-1~157-1442 | OH | H | H | NCH3 | 3-pyridyl | H | H | H | H | C2H5 | C2H5 | C2H5 | * |
| Table 158 | 158-1~158-1442 | OH | H | H | NCH2C6H5 | 3-pyridyl | H | H | H | H | C2H5 | C2H5 | C2H5 | * |

[Table 7]

| | Compound No. | $R_1$ | $R_2$ | $R_3$ | X | Het | R10a | R10b | R11 | R12 | R13a | R13b | R13c | |
|---|---|---|---|---|---|---|---|---|---|---|---|---|---|---|
| Table 159 | 159-1~159-1442 | OH | H | Cl | O | 3-pyridyl | H | H | H | H | C2H5 | C2H5 | C2H5 | * |
| Table 160 | 160-1~160-1442 | OH | H | Br | O | 3-pyridyl | H | H | H | H | C2H5 | C2H5 | C2H5 | * |
| Table 161 | 161-1~161-1442 | H | H | H | O | 3-pyridyl | H | H | H | H | C2H5 | C2H5 | C2H5 | * |
| Table 162 | 162-1~162-1442 | H(=) | | H | O | 3-pyridyl | H | H | H | H | C2H5 | C2H5 | C2H5 | * |
| Table 163 | 163-1~163-1442 | H(=) | | H | O | Phenyl | H | H | H | H | C2H5 | C2H5 | C2H5 | * |
| Table 164 | 164-1~164-1442 | =O | | H | O | 3-pyridyl | H | H | H | H | C2H5 | C2H5 | C2H5 | * |
| Table 165 | 165-1~165-1442 | =O | | H | O | 2-pyridyl | H | H | H | H | C2H5 | C2H5 | C2H5 | * |
| Table 166 | 166-1~166-i442 | =O | | H | O | 4-pyridyl | H | H | H | H | C2H5 | C2H5 | C2H5 | * |
| Table 167 | 167-1~167-1442 | =O | | H | O | Phenyl | H | H | H | H | C2H5 | C2H5 | C2H5 | * |
| Table 168 | 168-1~168-1442 | =O | | H | NH | 3-pyridyl | H | H | H | H | C2H5 | C2H5 | C2H5 | * |
| Table 169 | 169-1~169-1442 | =O | | H | NCH2C6H5 | 3-pyridyl | H | H | H | H | C2H5 | C2H5 | C2H5 | * |
| Table 170 | 170-1~170-1442 | =O | | Br | O | 3-pyridyl | H | H | H | H | C2H5 | C2H5 | C2H5 | * |
| Table 171 | 171-1~171-1442 | OCOC2H5 | H | H | O | 3-pyridyl | H | H | H | H | C2H5 | C2H5 | C2H5 | * |
| Table 172 | 172-1~172-1442 | OCH2OCH3 | H | H | O | 3-pyridyl | H | H | H | H | C2H5 | C2H5 | C2H5 | * |
| Table 173 | 173-1~173-1442 | O-(2-Tetrahydropyranyl) | H | H | O | 3-pyridyl | H | H | H | H | C2H5 | C2H5 | C2H5 | * |
| Table 174 | 174-1~174-1442 | OCONHCH2CH2CH3 | H | H | O | 3-pyridyl | H | H | H | H | C2H5 | C2H5 | C2H5 | * |
| Table 175 | 175-1~175-1442 | OH | H | H | O | 3-pyridyl | OH | OH | H | H | CH3 | CH3 | CH3 | * |
| Table 176 | 176-1~176-1442 | OH | H | H | O | 2-pyridyl | OH | OH | H | H | CH3 | CH3 | CH3 | * |
| Table 177 | 177-1~177-1442 | OH | H | H | O | 4-pyridyl | OH | OH | H | H | CH3 | CH3 | CH3 | * |
| Table 178 | 178-1~178-1442 | OH | H | H | NCH3 | 3-pyridyl | OH | OH | H | H | CH3 | CH3 | CH3 | * |
| Table 179 | 179-1~179-1442 | OH | H | H | NCH2C6H5 | 3-pyridyl | OH | OH | H | H | CH3 | CH3 | CH3 | * |
| Table 180 | 180-1~180-1442 | OH | H | Cl | O | 3-pyridyl | OH | OH | H | H | CH3 | CH3 | CH3 | * |
| Table 181 | 181-1~181-1442 | OH | H | Br | O | 3-pyridyl | OH | OH | H | H | CH3 | CH3 | CH3 | * |
| Table 182 | 182-1~182-1442 | H | H | H | O | 3-pyridyl | OH | OH | H | H | CH3 | CH3 | CH3 | * |
| Table 183 | 183-1~183-1442 | H(=) | | H | O | 3-pyridyl | OH | OH | H | H | CH3 | CH3 | CH3 | * |

(continued)

| | Compound No. | $R_1$ | $R_2$ | $R_3$ | X | Het | R10a | R10b | R11 | R12 | R13a | R13b | R13c | |
|---|---|---|---|---|---|---|---|---|---|---|---|---|---|---|
| Table 184 | 184-1~184-1442 | H(=) | | H | O | Phenyl | OH | OH | H | H | CH3 | CH3 | CH3 | * |
| Table 185 | 185-1~185-1442 | =O | | H | O | 3-pyridyl | OH | OH | H | H | CH3 | CH3 | CH3 | * |

[Table 8]

| | Compound No. | $R_1$ | $R_2$ | $R_3$ | X | Het | R10a | R10b | R11 | R12 | R13a | R13b | R13c | |
|---|---|---|---|---|---|---|---|---|---|---|---|---|---|---|
| Table 186 | 186-1~186-1442 | =O | | H | O | 2-pyridyl | OH | OH | H | H | CH3 | CH3 | CH3 | * |
| Table 187 | 187-1~187-1442 | =O | | H | O | 4-pyridyl | OH | OH | H | H | CH3 | CH3 | CH3 | * |
| Table 188 | 188-1~188-1442 | =O | | H | O | Phenyl | OH | OH | H | H | CH3 | CH3 | CH3 | * |
| Table 189 | 189-1~189-1442 | =O | | H | NH | 3-pyridyl | OH | OH | H | H | CH3 | CH3 | CH3 | * |
| Table 190 | 190-1~190-1442 | =O | | H | NCH2C6H5 | 3-pyridyl | OH | OH | H | H | CH3 | CH3 | CH3 | * |
| Table 191 | 191-1~191-1442 | =O | | Br | O | 3-pyridyl | OH | OH | H | H | CH3 | CH3 | CH3 | * |
| Table 192 | 192-1~192-1442 | OCOC2H5 | H | H | O | 3-pyridyl | OH | OH | H | H | CH3 | CH3 | CH3 | * |
| Table 193 | 193-1~193-1442 | OCH2OCH3 | H | H | O | 3-pyridyl | OH | OH | H | H | CH3 | CH3 | CH3 | * |
| Table 194 | 194-1~194-1442 | O-(2-Tetrahydropyranyl) | H | H | O | 3-pyridyl | OH | H | H | H | CH3 | CH3 | CH3 | * |
| Table 195 | 195-1~195-1442 | OCONHCH2CH2CH3 | H | H | O | 3-pyridyl | OH | OH | H | H | CH3 | CH3 | CH3 | * |
| Table 196 | 196-1~196-1442 | OH | H | H | O | 3-pyridyl | OH | OH | =O | | CH3 | CH3 | CH3 | * |
| Table 197 | 197-1~197-1442 | OH | H | H | O | 2-pyridyl | OH | OH | =O | | CH3 | CH3 | CH3 | * |
| Table 198 | 198-1~198-1442 | OH | H | H | O | 4-pyridyl | OH | OH | =O | | CH3 | CH3 | CH3 | * |
| Table 199 | 199-1~199-1442 | OH | H | H | NCH3 | 3-pyridyl | OH | OH | =O | | CH3 | CH3 | CH3 | * |
| Table 200 | 200-1~200-1442 | OH | H | H | NCH2C6H5 | 3-pyridyl | OH | OH | =O | | CH3 | CH3 | CH3 | * |
| Table 201 | 201-1~201-1442 | H | H | Cl | O | 3-pyridyl | OH | OH | =O | | CH3 | CH3 | CH3 | * |
| Table 202 | 202-1~202-1442 | OH | H | Br | O | 3-pyridyl | OH | OH | =O | | CH3 | CH3 | CH3 | * |
| Table 203 | 203-1~203-1442 | H | H | H | O | 3-pyridyl | OH | OH | =O | | CH3 | CH3 | CH3 | * |
| Table 204 | 204-1~204-1442 | H(=) | | H | O | 3-pyridyl | H | OH | =O | | CH3 | CH3 | CH3 | * |
| Table 205 | 205-1~205-1442 | N(=) | | H | O | Phenyl | OH | OH | =O | | CH3 | CH3 | CH3 | * |
| Table 206 | 206-1~206-1442 | =O | | H | O | 3-pyridyl | OH | OH | =O | | CH3 | CH3 | CH3 | * |
| Table 207 | 207-1~207-1442 | =O | | H | O | 2-pyridyl | OH | OH | =O | | CH3 | CH3 | CH3 | * |
| Table 208 | 208-1~208-1442 | =O | | H | O | 4-pyridyl | OH | OH | =O | | CH3 | CH3 | CH3 | * |
| Table 209 | 209-1~209-1442 | =O | | H | O | Phenyl | OH | OH | =O | | CH3 | CH3 | CH3 | * |
| Table 210 | 210-1~210-1442 | =O | | H | NH | 3-pyridyl | OH | OH | =O | | CH3 | CH3 | CH3 | * |

(continued)

| | Compound No. | $R_1$ | $R_2$ | $R_3$ | X | Het | R10a | R10b | R11 | R12 | R13a | R13b | R13c | |
|---|---|---|---|---|---|---|---|---|---|---|---|---|---|---|
| Table 211 | 211-1~211-1442 | =O | | H | NCH2C6H5 | 3-pyridyl | OH | OH | =O | | CH3 | CH3 | CH3 | * |
| Table 212 | 212-1~212-1442 | =O | | Br | O | 3-pyridyl | OH | OH | =O | | CH3 | CH3 | CH3 | * |

[Table 9]

| | Compound No. | $R_1$ | $R_2$ | $R_3$ | X | Het | R10a | R10b | R11 | 12 | R13a | R13b | R13c | |
|---|---|---|---|---|---|---|---|---|---|---|---|---|---|---|
| Table 213 | 213-1~213-1442 | OCOC2H5 | H | H | O | 3-pyridyl | OH | OH | =0 | | CH3 | CH3 | CH3 | * |
| Table 214 | 214-1~214-1442 | OCH2OCH3 | H | H | O | 3-pyridyl | OH | OH | =O | | CH3 | CH3 | CH3 | * |
| Table 215 | 215-1~215-1442 | O-(2-Tetrahydropyranyl) | H | H | O | 3-pyridyl | OH | OH | =O | | CH3 | CH3 | CH3 | * |
| Table 216 | 216-1~216-1442 | OCONHCH2CH2CH3 | H | H | O | 3-pyridyl | OH | OH | =O | | CH3 | CH3 | CH3 | * |
| Table 217 | 217-1~217-1442 | OH | H | H | O | t-Butyl | H | H | H | H | CH3 | CH3 | CH3 | * |
| Table 218 | 218-1~218-1442 | OH | H | H | O | cyclohexyl | H | H | H | H | CH3 | CH3 | CH3 | * |
| Table 219 | 219-1~219-1442 | OH | H | H | O | n-C8H17 | H | H | H | H | CH3 | CH3 | CH3 | * |
| Table 220 | 2201-1~220-1442 | =O-N-O-CH3 | | H | O | 3-pyridyl | H | H | H | H | CH3 | CH3 | CH3 | * |
| Table 221 | 221-1~221-1442 | =C-CN | | H | O | 3-pyridyl | H | H | H | H | CH3 | CH3 | CH3 | * |
| Table 222 | 222-1~222-1442 | =N-NH-Ph | | H | O | 3-pyridyl | H | H | H | H | CH3 | CH3 | CH3 | * |
| Table 223 | 223-1~223-1442 | =N-NH-CH3 | | H | O | 3-pyridyl | H | H | H | H | CH3 | CH3 | CH3 | * |
| Table 224 | 224-1~224-1442 | =N-N-CS-N-CH3 | | H | O | 3-pyridyl | H | H | H | H | CH3 | CH3 | CH3 | * |
| Table 225 | 225-1~225-1442 | =N-N-CO-N-Ph | | H | O | 3-pyridyl | H | H | H | H | CH3 | CH3 | CH3 | * |
| Table 226 | 226-1~226-1442 | OCOCH3 | H | H | O | 3-pyridyl | H | H | H | H | CH3 | CH3 | CH3 | * |

(continued)

| | Compound No. | $R_1$ | $R_2$ | $R_3$ | X | Het | R10a | R10b | R11 | 12 | R13a | R13b | R13c | |
|---|---|---|---|---|---|---|---|---|---|---|---|---|---|---|
| Table 227 | 227-1~227-1442 | OCO-C3H5 | H | H | O | 3-pyridyl | H | H | H | H | CH3 | CH3 | CH3 | * |
| Table 228 | 226-1~226-1442 | OCOCH3 | H | H | O | 2-pyridyl | H | H | H | H | CH3 | CH3 | CH3 | * |
| Table 229 | 229-1~229-1442 | OCO-C3H5 | H | H | O | 4-pyridyl | H | H | H | H | CH3 | CH3 | CH3 | * |
| Table 230 | 230-1~230-1442 | OCOCH3 | H | H | O | 6-Cl-3-pyridyl | H | H | H | H | CH3 | CH3 | CH3 | * |
| Table 231 | 231-1~231-1442 | OCD-C3H5 | H | H | O | 4-CF3-3-pyridyl | H | H | H | H | CH3 | CH3 | CH3 | * |
| Table 232 | 232-1~232-1442 | OCOCH3 | H | H | O | Phenyl | H | H | H | H | CH3 | CH3 | CH3 | * |
| Table 233 | 233-1~233-1442 | OCO-C3H5 | H | H | O | Phenyl | H | H | H | H | CH3 | CH3 | CH3 | * |
| Table 234 | 234-1~234-1442 | OCOCH3 | H | H | NH | 3-pyridyl | H | H | H | H | CH3 | CH3 | CH3 | * |
| Table 235 | 235-1~235-1442 | OCO-C3H5 | H | H | NCH3 | 3-pyridyl | H | H | H | H | CH3 | CH3 | CH3 | * |
| Table 236 | 236-1~236-1442 | OCH3 | H | H | O | Phenyl | H | H | H | H | CH3 | CH3 | CH3 | * |
| Table 237 | 237-1~237-1442 | H3 | H | H | NCH3 | 3-pyridyl | H | H | H | H | CH3 | CH3 | CH3 | * |
| Table 238 | 238-1~238-1442 | OC2H5 | H | H | O | Phenyl | H | H | H | H | CH3 | CH3 | CH3 | * |
| Table 239 | 239-1~239-1442 | OC2H5 | H | H | NCH3 | 3-pyridyl | H | H | H | H | CH3 | CH3 | CH3 | * |

[Table 10]

| Table A | | | | | |
|---|---|---|---|---|---|
| | R3 | R4 | R5 | R6 | R7 |
| 1 | H | OH | OH | OH | H |
| 2 | H | OH | OH | OCH3 | H |
| 3 | H | OH | OH | =O | |
| 4 | H | OH | OH | H | H |
| 5 | H | OH | H | H | H |
| 6 | H | OH | H | OH | H |
| 7 | H | H | OH | OH | H |
| 8 | H | OCOCH3 | OCOCH3 | OCOCH3 | H |
| 9 | H | OCOCH3 | OCOCH3 | H | H |
| 10 | H | OCOCH3 | OCOCH3 | OH | H |
| 11 | H | OCOCH3 | OCOCH3 | =O | |
| 12 | H | OCOCH3 | OCOCH3 | OCOC2H5 | H |
| 13 | H | OCOCH3 | OCOCH3 | OCOC3H7 | H |
| 14 | H | OCOCH3 | OCOCH3 | OCOCH(CH3)2 | H |
| 15 | H | OCOCH3 | OCOCH3 | OCOC(CH3)3 | H |
| 16 | H | OCOCH3 | OCOCH3 | OCO-c-C3H5 | H |
| 17 | H | OCOCH3 | OCOCH3 | OS02CH3 | H |
| 18 | H | OCOCH3 | OCOCH3 | OSO2C2H5 | H |
| 19 | H | OCOCH3 | OCOCH3 | OSO2-c-C3H5 | H |
| 20 | H | OCOCH3 | OCOCH3 | OCOC6H5 | H |
| 21 | H | OCOCH3 | OCOCH3 | OCO-2-pyridyl | H |
| 22 | H | OCOCH3 | OCOCH3 | OCO-3-pyridyl | H |
| 23 | H | OCOCH3 | OCOCH3 | OCO-4-pyridyl | H |
| 24 | H | OCOCH3 | OCOCH3 | OCS-imidazole | H |
| 25 | H | OCOCH3 | OCOCH3 | OCOOCH3 | H |
| 26 | H | OCOCH3 | OCOCH3 | OCOOC2H5 | H |
| 27 | H | OCOCH3 | OCOCH3 | OCOOCH(CH3)2 | H |
| 28 | H | OCOCH3 | OCOCH3 | OCOOC(CH3)3 | H |
| 29 | H | OCOCH3 | OCOCH3 | OCOO-c-C3H5 | H |
| 30 | H | OCOCH3 | OCOCH3 | OCOOC6H5 | H |
| 31 | H | OCOCH3 | OCOCH3 | OCOO-3-pyridyl | H |
| 32 | H | OCOCH3 | OCOCH3 | OCONHCH3 | H |
| 33 | H | OCOCH3 | OCOCH3 | OCONHC2H5 | H |
| 34 | H | OCOCH3 | OCOCH3 | OCONHC3H7 | H |
| 35 | H | OCOCH3 | OCOCH3 | OCON(CH3)2 | H |
| 36 | H | OCOCH3 | OCOCH3 | OCON(C2H5)2 | H |

(continued)

| Table A | | | | | |
|---|---|---|---|---|---|
| | R3 | R4 | R5 | R6 | R7 |
| 37 | H | OCOCH3 | OCOCH3 | OCONH-c-C3H5 | H |
| 38 | H | OCOCH3 | OCOCH3 | OCON(CH3)-(c-C3H5) | H |
| 39 | H | OCOCH3 | OCOCH3 | OCONHC6H5 | H |
| 40 | H | OCOCH3 | OCOCH3 | OCON(CH3)C6H5 | H |
| 4 | H | OCOCH3 | OCOCH3 | OCONH-3-pyridyl | H |
| 42 | H | OCOCH3 | OCOCH3 | OCON(CH3)-(3-pyridyl) | H |
| 43 | H | OCOCH3 | OCOCH3 | OCO-(o-OCH3-C6H4) | H |
| 44 | H | OCOCH3 | OCOCH3 | OCO-(m-OCH3-C8H4) | H |
| 45 | H | OCOCH3 | OCOCH3 | OCO-(p-OCH3-C6H4) | H |
| 46 | H | OCOCH3 | OCOCH3 | OCO-(o-I-C6H4) | H |
| 47 | H | OCOCH3 | OCOCH3 | OCO-(m-I-C6H4) | H |
| 48 | H | OCOCH3 | OCOCH3 | OCO-(p-I-C6H4) | H |
| 49 | H | OCOCH3 | OCOCH3 | OCO-(o-CH3-C6H4) | H |
| 50 | H | OCOCH3 | OCOCH3 | OCO-(m-CH3-C6H4) | H |
| 51 | H | OCOCH3 | OCOCH3 | OCO-(p-CH3-C6H4) | H |
| 52 | H | OCOCH3 | OCOCH3 | OCO-(o-Cl-C6H4) | H |
| 53 | H | OCOCH3 | OCOCH3 | OCO-(m-Cl-C6H4) | H |
| 54 | H | OCOCH3 | OCOCH3 | OCO-(p-Cl-C6H4) | H |
| 55 | H | OCOCH3 | OCOCH3 | OCO-(m-vinyl-C8H4) | H |
| 56 | H | OCOCH3 | OCOCH3 | OCO-(p-vinyl-C6H4) | H |
| 57 | H | OCOCH3 | OCOCH3 | OCO-(o-CN-C6H4) | H |
| 58 | H | OCOCH3 | OCOCH3 | OCO-(m-CN-C6H4) | H |
| 59 | H | OCOCH3 | OCOCH3 | OCO-(p-CN-C6H4) | H |
| 60 | H | OCOCH3 | OCOCH3 | OCO-(p-SCH3-C6H4) | H |
| 61 | H | OCOCH3 | OCOCH3 | OCO-(p-Br-C6H4) | H |
| 62 | H | OCOCH3 | OCOCH3 | OCO-(p-F-C6H4) | H |
| 63 | H | OCOCH3 | OCOCH3 | OCO-c-C6H11 | H |
| 64 | H | OCOCH3 | OCOCH3 | OCO-adamantyl | H |
| 65 | H | OCOCH3 | OCOCH3 | OCO-(m-SCH3-C6H4) | H |
| 66 | H | OCOCH3 | OCOCH3 | OCO-(m-F-C6H4) | H |
| 67 | H | OCOCH3 | OCOCH3 | OCO-(p-C2H5-C6H4) | H |
| 68 | H | OCOCH3 | OCOCH3 | OCO-(m-Br-C6H4) | H |
| 69 | H | OCOCH3 | OCOCH3 | OCO-(o-F-C6H4) | H |
| 70 | H | OCOCH3 | OCOCH3 | OCO-(p-NO2-C6H4) | H |

[Table 11]

| Table A | | | | | | |
|---|---|---|---|---|---|---|
| | R3 | R4 | R5 | R6 | R7 | |
| 71 | H | OCOCH3 | OCOCH3 | OCO-(p-N3-C6H4) | H | |
| 72 | H | OCOCH3 | OCOCH3 | OCO-(p-OH-C6H4) | H | |
| 73 | H | OCOCH3 | OCOCH3 | OCO-(p-NH2-C6H4) | H | |
| 74 | H | OCOCH3 | OCOCH3 | OCO-(4-biphenyl) | H | |
| 75 | H | OCOCH3 | OCOCH3 | OCO-(2-benzothiophene) | H | |
| 76 | H | OCOCH3 | OCOCH3 | OCO-(3.4-O-CF2-O-C5H3) | H | |
| 77 | H | OCOCH3 | OCOCH3 | OCO-(2-naphtyl) | H | |
| 78 | H | OCOCH3 | OCOCH3 | OCO-(1-naphtyl) | H | |
| 79 | H | OCOCH3 | OCOCH3 | H(=) | | |
| 80 | H | OCOCH3 | OCOCH3 | OCO-(c-C5H9) | H | 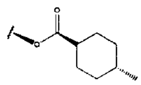 |
| 81 | H | OCOCH3 | OCOCH3 | OCOCH2CH2(p-OCH3-C6H4) | H | (1) |
| 82 | H | OCOCH3 | OCOCH3 | (1) | H | |
| 83 | H | OCOCH3 | OCOCH3 | OCOCH2(p-OCH3-C6H4) | H | |
| 84 | H | OCOCH3 | OCOCH3 | (2) | H | |
| 85 | H | OCOCH3 | OCOCH3 | OCOCH2(1H-tetrazol-1-yl) | H | |
| 86 | H | OCOCH3 | OCOCH3 | OCOCH2CH2CH2CH2OH | H | 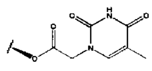 |
| 87 | H | OCOCH3 | OCOCH3 | OCOCH2CH2(piperidin-1-yl) | H | (2) |
| 88 | H | OCOCH3 | OCOCH3 | OCO-(pyrazin-2-yl) | H | |
| 89 | H | OCOCH3 | OCOCH3 | (3) | H | |
| 90 | H | OCOCH3 | OCOCH3 | OCOCH2CH2(C6H5) | | |
| 91 | H | OCOCH3 | OCOCH3 | OCOCH2(1H-tetrazol-5yl) | H | 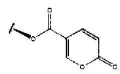 |
| 92 | H | OCOCH3 | OCOCH3 | OCOCH2CH2CH2(p-OCH3-C6H4) | H | (3) |
| 93 | H | OCOCH3 | OCOCH3 | OCOCH2CH2(1-CH3-piperadin-4-yl) | H | |
| 94 | H | OCOCH3 | OCOCH3 | OCO-(m-F-p-Br-C6H3) | H | |
| 95 | H | OCOCH3 | OCOCH3 | OCOCH2OCH2OCH3 | H | |
| 96 | H | OCOCH3 | OCOCH3 | OCOCH2OH | H | |
| 97 | H | OCOCH3 | OCOCH3 | OCOCH2OCH2OCH2CH2OCH3 | H | |
| 98 | H | OCOCH3 | OCOCH3 | OCO-(o-F-p-Br-C6H3) | H | |
| 99 | H | OCOCH3 | OCOCH3 | OCO-(3,4,5-3F-C6H2) | H | |
| 100 | H | OCOCH3 | OCOCH3 | OCO-(p-CONH2-C6H4) | H | |
| 101 | H | OCOCH3 | OCOCH3 | OCO-(m-F-p-CN-C6H3) | H | |
| 102 | H | OCOCH3 | OH | OCOCH3 | H | |
| 103 | H | OH | OCOCH3 | OCOCH3 | H | |
| 104 | H | OCOCH3 | OH | OH | H | |
| 105 | H | OH | OCOCH3 | OH | H | |
| 106 | H | OH | OH | OCOCH3 | H | |
| 107 | H | OCOCH3 | H | OCOCH3 | H | |

(continued)

Table A

| | R3 | R4 | R5 | R6 | R7 |
|---|---|---|---|---|---|
| 108 | H | OCOCH3 | OH | H | H |
| 109 | H | OH | OCOCH3 | H | H |
| 110 | H | OCOCH3 | H | H | H |
| 111 | H | OCOC2H5 | OCOC2H5 | OCOC2H5 | H |
| 112 | H | OCOC2H5 | OCOC2H5 | OH | H |
| 113 | H | OCOC2H5 | OCOC2H5 | =0 | |
| 114 | H | OCOC2H5 | OCOC2H5 | OCOCH3 | H |
| 115 | H | OCOC2H5 | OCOC2H5 | OCOC3H7 | H |
| 116 | H | OCOC2H5 | OCOC2H5 | OCOCH(CH3)2 | H |
| 117 | H | OCOC2H5 | OCOC2H5 | OCOC(CH3)3 | H |
| 118 | H | OCOC2H5 | OCOC2H5 | OCO-c-C3H5 | H |
| 119 | H | OCOC2H5 | OCOC2H5 | OSO2CH3 | H |
| 120 | H | OCOC2H5 | OCOC2H5 | OSO2C2H5 | H |
| 121 | H | OCOC2H5 | OCOC2H5 | OSO2-c-C3H5 | H |
| 122 | H | OCOC2H5 | OCOC2H5 | OCOC6H5 | H |
| 123 | H | OCOC2H5 | OCOC2H5 | OCO-2-pyridyl | H |
| 124 | H | OCOC2H5 | OCOC2H5 | OCO-3-pyridyl | H |
| 125 | H | OCOC2H5 | OCOC2H5 | OCO-4-pyridyl | H |
| 126 | H | OCOC2H5 | OCOC2H5 | OCS-imidazole | H |
| 127 | H | OCOC2H5 | OCOC2H5 | OCOOCH3 | H |
| 128 | H | OCOC2H5 | OCOC2H5 | OCOOC2H5 | H |
| 129 | H | OCOC2H5 | OCOC2H5 | OCOOCH(CH3)2 | H |
| 130 | H | OCOC2H5 | OCOC2H5 | OCOOC(CH3)3 | H |
| 131 | H | OCOC2H5 | OCOC2H5 | OCOO-c-C3H5 | H |
| 132 | H | OCOC2H5 | OCOC2H5 | OCOOC6H5 | H |
| 133 | H | OCOC2H5 | OCOC2H5 | OCOO-3-pyridyl | H |
| 134 | H | OCOC2H5 | OCOC2H5 | OCONHCH3 | H |
| 135 | H | OCOC2H5 | OCOC2H5 | OCONHC2H5 | H |
| 136 | H | OCOC2H5 | OCOC2H5 | OCONHC3H7 | H |
| 137 | H | OCOC2H5 | OCOC2H5 | OCON(CH3)2 | H |
| 138 | H | OCOC2H5 | OCOC2H5 | OCON(C2H5)2 | H |
| 139 | H | OCOC2H5 | OCOC2H5 | OCONH-c-C3H5 | H |
| 140 | H | OCOC2H5 | OCOC2H5 | OCON(CH3)-(c-C3H5) | H |

[Table 12]

| Table A | | | | | |
|---|---|---|---|---|---|
| | R3 | R4 | R5 | R6 | R7 |
| 141 | H | OCOC2H5 | OCOC2H5 | OCONHC6H5 | H |
| 142 | H | OCOC2H5 | OCOC2H5 | OCON(CH3)C6H5 | H |
| 143 | H | OCOC2H5 | OCOC2H5 | OCONH-3-pyridyl | H |
| 144 | H | OCOC2H5 | OCOC2H5 | OCON(CH3)3-pyridyl) | H |
| 145 | H | OCOC2H5 | OCOC2H5 | OCO-(2-benzo[b]thienyl) | H |
| 146 | H | OCOC2H5 | OCOC2H5 | OCO-(3,4-O-CH2-O-C6H3) | H |
| 147 | H | OH | OCOC2H5 | OCOC2H5 | H |
| 148 | H | OCOC2H5 | OH | OCOC2H5 | H |
| 149 | H | OCOC2H5 | OH | OH | H |
| 150 | H | OH | OCOC2H5 | OH | H |
| 151 | H | OH | OH | OCOC2H5 | H |
| 152 | H | OCOC2H5 | OH | H | H |
| 153 | H | OH | OCOC2H5 | H | H |
| 154 | H | OCOC2H5 | H | H | H |
| 155 | H | OCOCH3 | H | OCOC2H5 | H |
| 156 | H | OCOC2H5 | H | OCOC2H5 | H |
| 157 | H | OCOC2H5 | OCOCH3 | OCOC2H5 | H |
| 158 | H | OCOC2H5 | OCOC2H5 | OCOCH3 | H |
| 159 | H | OCOCH3 | OCOC2H5 | OCOC2H5 | H |
| 160 | H | OCOC2H5 | OCOC2H5 | H | H |
| 161 | H | OCOCH3 | OCOC2H5 | H | H |
| 162 | H | OCOC2H5 | OCOCH3 | H | H |
| 163 | H | OCOCH(CH3)2 | OCOCH(CH3)2 | OCOCH(CH3)2 | H |
| 164 | H | OCOCH(CH3)2 | OCOCH(CH3)2 | OH | H |
| 165 | H | OCOCH(CH3)2 | OCOCH(CH3)2 | =O | |
| 166 | H | OCOCH(CH3)2 | OCOCH(CH3)2 | OCH3 | H |
| 167 | H | OCOCH(CH3)2 | OCOCH(CH3)2 | H | H |
| 168 | H | OCOCH(CH3)2 | OCOCH(CH3)2 | OCOCH3 | H |
| 169 | H | OCOCH(CH3)2 | OCOCH(CH3)2 | OCOC2H5 | H |
| 170 | H | OCOCH(CH3)2 | OCOCH(CH3)2 | OCOC3H7 | H |
| 171 | H | OCOCH(CH3)2 | OCOCH(CH3)2 | OCOC(CH3)3 | H |
| 172 | H | OCOCH(CH3)2 | OCOCH(CH3)2 | OCO-c-C3H5 | H |
| 173 | H | OCOCH(CH3)2 | OCOCH(CH3)2 | OS02CH3 | H |
| 174 | H | OCOCH(CH3)2 | OCOCH(CH3)2 | OSO2C2H5 | H |
| 175 | H | OCOCH(CH3)2 | OCOCH(CH3)2 | OSO2-c-C3H5 | H |
| 176 | H | OCOCH(CH3)2 | OCOCH(CH3)2 | OCOC6H5 | H |
| 177 | H | OCOCH(CH3)2 | OCOCH(CH3)2 | OCO-(p-CN-C6H4) | H |

(continued)

| Table A | | | | | |
|---|---|---|---|---|---|
| | R3 | R4 | R5 | R6 | R7 |
| 178 | H | OCOCH(CH3)2 | OCOCH(CH3)2 | OCO-2-pyridyl | H |
| 179 | H | OCOCH(CH3)2 | OCOCH(CH3)2 | OCO-3-pyridyl | H |
| 180 | H | OCOCH(CH3)2 | OCOCH(CH3)2 | OCO-4-pyridyl | H |
| 181 | H | OCOCH(CH3)2 | OCOCH(CH3)2 | OCS-imidazole | H |
| 182 | H | OCOCH(CH3)2 | OCOCH(CH3)2 | OCOOCH3 | H |
| 183 | H | OCOCH(CH3)2 | OCOCH(CH3)2 | OCOOC2H5 | H |
| 184 | H | OCOCH(CH3)2 | OCOCH(CH3)2 | OCOOCH(CH3)2 | H |
| 185 | H | OCOCH(CH3)2 | OCOCH(CH3)2 | OCOOC(CH3)3 | H |
| 186 | H | OCOCH(CH3)2 | OCOCH(CH3)2 | OCOO-c-C3H5 | H |
| 187 | H | OCOCH(CH3)2 | OCOCH(CH3)2 | OCOOC6H5 | H |
| 188 | H | OCOCH(CH3)2 | OCOCH(CH3)2 | OCOO-3-pyridyl | H |
| 189 | H | OCOCH(CH3)2 | OCOCH(CH3)2 | OCONHCH3 | H |
| 190 | H | OCOCH(CH3)2 | OCOCH(CH3)2 | OCONHC2H5 | H |
| 191 | H | OCOCH(CH3)2 | OCOCH(CH3)2 | OCONHC3H7 | H |
| 192 | H | OCOCH(CH3)2 | OCOCH(CH3)2 | OCON(CH3)2 | H |
| 193 | H | OCOCH(CH3)2 | OCOCH(CH3)2 | OCON(C2H5)2 | H |
| 194 | H | OCOCH(CH3)2 | OCOCH(CH3)2 | OCONH-c-C3H5 | H |
| 195 | H | OCOCH(CH3)2 | OCOCH(CH3)2 | OCON(CH3)-(c-C3H5) | H |
| 196 | H | OCOCH(CH3)2 | OCOCH(CH3)2 | OCONHC6H5 | H |
| 197 | H | OCOCH(CH3)2 | OCOCH(CH3)2 | OCON(CH3)C6H5 | H |
| 198 | H | OCOCH(CH3)2 | OCOCH(CH3)2 | OCONH-3-pyridyl | H |
| 199 | H | OCOCH(CH3)2 | OCOCH(CH3)2 | OCON(CH3)(3-pyridyl) | H |
| 200 | H | OCOCH(CH3)2 | OH | OH | H |
| 201 | H | OH | OCOCH(CH3)2 | OH | H |
| 202 | H | OCOCH(CH3)2 | OH | H | H |
| 203 | H | OH | OCOCH(CH3)2 | H | H |
| 204 | H | OCOCH(CH3)2 | H | H | H |
| 205 | H | OCOC(CH3)3 | OCOC(CH3)3 | OCOC(CH3)3 | H |
| 206 | H | OCOC(CH3)3 | OCOC(CH3)3 | OH | H |
| 207 | H | OCOC(CH3)3 | OCOC(CH3)3 | =0 | |
| 208 | H | OCOC(CH3)3 | OCOC(CH3)3 | OCH3 | H |
| 209 | H | OCOC(CH3)3 | OCOC(CH3)3 | OCOC3H7 | H |
| 210 | H | OCOC(CH3)3 | OCOC(CH3)3 | OCOCH(CH3)2 | H |

[Table 13]

| Table A | | | | | |
|---|---|---|---|---|---|
| | R3 | R4 | R5 | R6 | R7 |
| 211 | H | OCOC(CH3)3 | OCOC(CH3)3 | OCO-c-C3H5 | H |
| 212 | H | OCOC(CH3)3 | OCOC(CH3)3 | OSO2CH3 | H |
| 213 | H | OCOC(CH3)3 | OCOC(CH3)3 | OSO2C2H5 | H |
| 214 | H | OCOC(CH3)3 | OCOC(CH3)3 | OSO2-c-C3H5 | H |
| 215 | H | OCOC(CH3)3 | OCOC(CH3)3 | OCOC6H5 | H |
| 216 | H | OCOC(CH3)3 | OCOC(CH3)3 | OCO-2-pyridyl | H |
| 217 | H | OCOC(CH3)3 | OCOC(CH3)3 | OCO-3-pyridyl | H |
| 218 | H | OCOC(CH3)3 | OCOC(CH3)3 | OCO-4-pyridyl | H |
| 219 | H | OCOC(CH3)3 | OCOC(CH3)3 | OCS-imidazole | H |
| 220 | H | OCOC(CH3)3 | OCOC(CH3)3 | OCOOCH3 | H |
| 221 | H | OCOC(CH3)3 | OCOC(CH3)3 | OCOOC2H5 | H |
| 222 | H | OCOC(CH3)3 | OCOC(CH3)3 | OCOOCH(CH3)2 | H |
| 223 | H | OCOC(CH3)3 | OCOC(CH3)3 | OCOOC(CH3)3 | H |
| 224 | H | OCOC(CH3)3 | OCOC(CH3)3 | OCOO-c-C3H5 | H |
| 225 | H | OCOC(CH3)3 | OCOC(CH3)3 | OCOOC6H5 | H |
| 226 | H | OCOC(CH3)3 | OCOC(CH3)3 | OCOO-3-pyridyl | H |
| 227 | H | OCOC(CH3)3 | OCOC(CH3)3 | OCONHCH3 | H |
| 228 | H | OCOC(CH3)3 | OCOC(CH3)3 | OCONHC2H5 | H |
| 229 | H | OCOC(CH3)3 | OCOC(CH3)3 | OCONHC3H7 | H |
| 230 | H | OCOC(CH3)3 | OCOC(CH3)3 | OCON(CH3)2 | H |
| 231 | H | OCOC(CH3)3 | OCOC(CH3)3 | OCON(C2H5)2 | H |
| 232 | H | OCOC(CH3)3 | OCOC(CH3)3 | OCONH-c-C3H5 | H |
| 233 | H | OCOC(CH3)3 | OCOC(CH3)3 | OCON(CH3)-(c-C3H5) | H |
| 234 | H | OCOC(CH3)3 | OCOC(CH3)3 | OCONHC6H5 | H |
| 235 | H | OCOC(CH3)3 | OCOC(CH3)3 | OCON(CH3)C6H5 | H |
| 236 | H | OCOC(CH3)3 | OCOC(CH3)3 | OCONH-3-pyridyl | H |
| 237 | H | OCOC(CH3)3 | OCOC(CH3)3 | OCON(CH3)-(3-pyridyl) | H |
| 238 | H | OCOC(CH3)3 | OH | OH | H |
| 239 | H | OH | OCOC(CH3)3 | OH | H |
| 240 | H | OCOC(CH3)3 | OH | H | H |
| 241 | H | OH | OCOC(CH3)3 | H | H |
| 242 | H | OCOC(CH3)3 | H | H | H |
| 243 | H | OCOC(CH3)3 | OCOC(CH3)3 | H | H |
| 244 | H | OCOCH(CH3)2 | OCOC(CH3)3 | OH | H |
| 245 | H | OCOC(CH3)3 | OCOCH(CH3)2 | OH | H |
| 246 | H | OCOCH(CH3)2 | OCOC(CH3)3 | H | H |

(continued)

| Table A | | | | | |
|---|---|---|---|---|---|
| | R3 | R4 | R5 | R6 | R7 |
| 247 | H | OCOC(CH3)3 | OCOCH(CH3)2 | H | H |
| 248 | H | OCOCH2C6H5 | OCOCH2C6H5 | OCOCH2C6H5 | H |
| 249 | H | OCOCH2C6H5 | OCOCH2C6H5 | OH | H |
| 250 | H | OCOCH2-3-pyridyl | OCOCH2-3-pyridyl | OCOCH2-3-pyridyl | H |
| 251 | H | OCOCH2-3-pyridyl | OCOCH2-3-pyridyl | OH | H |
| 252 | H | OCOCHCH2 | OCOCHCH2 | OCOCHCH2 | H |
| 253 | H | OCOCHCH2 | OCOCHCH2 | OH | H |
| 254 | H | OCOCCH | OCOCCH | OCOCCH | H |
| 255 | H | OCOCCH | OCOCCH | OH | H |
| 256 | H | OCO-adamantyl | OCO-adamantyl | OCO-adamantyl | H |
| 257 | H | OCOCH2CH2(C6H5) | OCOCH2CH2(C6H5) | OCOCH2CH2(C6H5) | H |
| 258 | H | OCOCH2CH2(piperidin-1-yl) | OCOCH2CH2(piperidin-1-yl) | OCOCH2CH2(piperidin-1-yl) | H |
| 259 | H | OCO-c-C3H5 | OCO-c-C3H5 | OCO-c-C3H5 | H |
| 260 | H | OCO-c-C3H5 | OCO-c-C3H5 | OH | H |
| 261 | H | OCO-c-C3H5 | OCO-c-C3H5 | =O | |
| 262 | H | OCO-c-C3H5 | OCO-c-C3H5 | H | H |
| 263 | H | OCO-c-C3H5 | OCO-c-C3H5 | H(=) | |
| 264 | H | OCO-c-C3H5 | OCO-c-C3H5 | OCOCH3 | H |
| 265 | H | OCO-c-C3H5 | OCO-c-C3H5 | OCOC2H5 | H |
| 266 | H | OCO-c-C3H5 | OCO-c-C3H5 | OCOC3H7 | H |
| 267 | H | OCO-c-C3H5 | OCO-c-C3H5 | OCOCH(CH3)2 | H |
| 268 | H | OCO-c-C3H5 | OCO-c-C3H5 | OCOC(CH3)3 | H |
| 269 | H | OCO-c-C3H5 | OCO-c-C3H5 | OSO2CH3 | H |
| 270 | H | OCO-c-C3H5 | OCO-c-C3H5 | OSO2C2H5 | H |
| 271 | H | OCO-c-C3H5 | OCO-c-C3H5 | OSO2-c-C3H5 | H |
| 272 | H | OCO-c-C3H5 | OCO-c-C3H5 | OCOC6H5 | H |
| 273 | H | OCO-c-C3H5 | OCO-c-C3H5 | OCO-2-pyridyl | H |
| 274 | H | OCO-c-C3H5 | OCO-c-C3H5 | OCO-3-pyridyl | H |
| 275 | H | OCO-c-C3H5 | OCO-c-C3H5 | OCO-4-pyridyl | H |
| 276 | H | OCO-c-C3H5 | OCO-c-C3H5 | OCS-imidazole | H |
| 277 | H | OCO-c-C3H5 | OCO-c-C3H5 | OCOOCH3 | H |
| 278 | H | OCO-c-C3H5 | OCO-c-C3H5 | OCOOC2H5 | H |
| 279 | H | OCO-c-C3H5 | OCO-c-C3H5 | OCOOCH(CH3)2 | H |
| 280 | H | OCO-c-C3H5 | OCO-c-C3H5 | OCOOC(CH3)3 | H |

[Table 14]

| Table A | | | | | |
|---|---|---|---|---|---|
| | R3 | R4 | R5 | R6 | R7 |
| 281 | H | OCO-c-C3H5 | OCO-c-C3H5 | OCOO-c-C3H5 | H |
| 282 | H | OCO-c-C3H5 | OCO-c-C3H5 | OCOOC6H5 | H |
| 283 | H | OCO-c-C3H5 | OCO-c-C3H5 | OCOO-3-pyridyl | H |
| 284 | H | OCO-c-C3H5 | OCO-c-C3H5 | OCONHCH3 | H |
| 285 | H | OCO-c-C3H5 | OCO-c-C3H5 | OCONHC2H5 | H |
| 286 | H | OCO-c-C3H5 | OCO-c-C3H5 | OCONHC3H7 | H |
| 287 | H | OCO-c-C3H5 | OCO-c-C3H5 | OCON(CH3)2 | H |
| 288 | H | OCO-c-C3H5 | OCO-c-C3H5 | OCON(C2H5)2 | H |
| 289 | H | OCO-c-C3H5 | OCO-c-C3H5 | OCONH-c-C3H5 | H |
| 290 | H | OCO-c-C3H5 | OCO-c-C3H5 | OCON(CH3)-(c-C3H5) | H |
| 291 | H | OCO-c-C3H5 | OCO-c-C3H5 | OCONHC6H5 | H |
| 292 | H | OCO-c-C3H5 | OCO-c-C3H5 | OCON(CH3)C6H5 | H |
| 293 | H | OCO-c-C3H5 | OCO-c-C3H5 | OCONH-3-pyridyl | H |
| 294 | H | OCO-c-C3H5 | OCO-c-C3H5 | OCON(CH3)-(3-pyridyl) | H |
| 295 | H | OCOC3H7 | OCOC3H7 | OCO-c-C3H5 | H |
| 296 | H | OCOCH(CH3)2 | OCOCH(CH3)2 | OCO-c-C3H5 | H |
| 297 | H | OCOC(CH3)3 | OCOC(CH3)3 | OCO-c-C3H5 | H |
| 298 | H | OS02CH3 | OSO2CH3 | OCO-c-C3H5 | H |
| 299 | H | OSO2C2H5 | OSO2C2H5 | OCO-c-C3H5 | H |
| 300 | H | OSO2-c-C3H5 | OSO2-c-C3H5 | OCO-c-C3H5 | H |
| 301 | H | OCOC6H5 | OCOC6H5 | OCO-c-C3H5 | H |
| 302 | H | OCO-2-pyridyl | OCO-2-pyridyl | OCO-c-C3H5 | H |
| 303 | H | OCO-3-pyridyl | OCO-3-pyridyl | OCO-c-C3H5 | H |
| 304 | H | OCO-4-pyridyl | OCO-4-pyridyl | OCO-c-C3H5 | H |
| 305 | H | OCS-imidazole | OCS-imidazole | OCO-c-C3H5 | H |
| 306 | H | OCOOCH3 | OCOOCH3 | OCO-c-C3H5 | H |
| 307 | H | OCOOC2H5 | OCOOC2H5 | OCO-c-C3H5 | H |
| 308 | H | OCOOCH(CH3)2 | OCOOCH(CH3)2 | OCO-c-C3H5 | H |
| 309 | H | OCOOC(CH3)3 | OCOOC(CH3)3 | OCO-c-C3H5 | H |
| 310 | H | OCOO-c-C3H5 | OCOO-c-C3H5 | OCO-c-C3H5 | H |
| 311 | H | OCOOC6H5 | OCOOC6H5 | OCO-c-C3H5 | H |
| 312 | H | OCOO-3-pyridyl | OCOO-3-pyridyl | OCO-c-C3H5 | H |
| 313 | H | OCONHCH3 | OCONHCH3 | OCO-c-C3H5 | H |
| 314 | H | OCONHC2H5 | OCONHC2H5 | OCO-c-C3H5 | H |
| 315 | H | OCONHC3H7 | OCONHC3H7 | OCO-c-C3H5 | H |
| 316 | H | OCON(CH3)2 | OCON(CH3)2 | OCO-c-C3H5 | H |

(continued)

| Table A | | | | | |
|---|---|---|---|---|---|
| | R3 | R4 | R5 | R6 | R7 |
| 317 | H | OCON(C2H5)2 | OCON(C2H5)2 | OCO-c-C3H5 | H |
| 318 | H | OCONH-c-C3H5 | OCONH-c-C3H5 | OCO-c-C3H5 | H |
| 319 | H | OCON(CH3)-(c-C3H5) | OCON(CH3)-(c-C3H5) | OCO-c-C3H5 | H |
| 320 | H | OCONHC6H5 | OCONHC6H5 | OCO-c-C3H5 | H |
| 321 | H | OCON(CH3)C6H5 | OCON(CH3)C6H5 | OCO-c-C3H5 | H |
| 322 | H | OCONH-3-pyridyl | OCONH-3-pyridyl | OCO-c-C3H5 | H |
| 323 | H | OCON(CH3)-(3-pyridyl) | OCON(CH3)-(3-pyridyl) | OCO-c-C3H5 | H |
| 324 | H | OCO-c-C3H5 | OCOCH3 | OCO-c-C3H5 | H |
| 325 | H | OCO-c-C3H5 | OCOC2H5 | OCO-c-C3H5 | H |
| 326 | H | OCO-c-C3H5 | OCOC3H7 | OCO-c-C3H5 | H |
| 327 | H | OCO-c-C3H5 | OCOCH(CH3)2 | OCO-c-C3H5 | H |
| 328 | H | OCO-c-C3H5 | OCOC(CH3)3 | OCO-c-C3H5 | H |
| 329 | H | OCO-c-C3H5 | OS02CH3 | OCO-c-C3H5 | H |
| 330 | H | OCO-c-C3H5 | OSO2C2H5 | OCO-c-C3H5 | H |
| 331 | H | OCO-c-C3H5 | OSO2-c-C3H5 | OCO-c-C3H5 | H |
| 332 | H | OCO-c-C3H5 | OCOC6H5 | OCO-c-C3H5 | H |
| 333 | H | OCO-c-C3H5 | OCO-2-pyridyl | OCO-c-C3H5 | H |
| 334 | H | OCO-c-C3H5 | OCO-3-pyridyl | OCO-c-C3H5 | H |
| 335 | H | OCO-c-C3H5 | OCO-4-pyridyl | OCO-c-C3H5 | H |
| 336 | H | OCO-c-C3H5 | OCS-imidazole | OCO-c-C3H5 | H |
| 337 | H | OCO-c-C3H5 | OCOOCH3 | OCO-c-C3H5 | H |
| 338 | H | OCO-c-C3H5 | OCOOC2H5 | OCO-c-C3H5 | H |
| 339 | H | OCO-c-C3H5 | OCOOCH(CH3)2 | OCO-c-C3H5 | H |
| 340 | H | OCO-c-C3H5 | OCOOC(CH3)3 | OCO-c-C3H5 | H |
| 341 | H | OCO-c-C3H5 | OCOO-c-C3H5 | OCO-c-C3H5 | H |
| 342 | H | OCO-c-C3H5 | OCOOC6H5 | OCO-c-C3H5 | H |
| 343 | H | OCO-c-C3H5 | OCOO-3-pyridyl | OCO-c-C3H5 | H |
| 344 | H | OCO-c-C3H5 | OCONHCH3 | OCO-c-C3H5 | H |
| 345 | H | OCO-c-C3H5 | OCONHC2H5 | OCO-c-C3H5 | H |
| 346 | H | OCO-c-C3H5 | OCONHC3H7 | OCO-c-C3H5 | H |
| 347 | H | OCO-c-C3H5 | OCON(CH3)2 | OCO-c-C3H5 | H |
| 348 | H | OCO-c-C3H5 | OCON(C2H5)2 | OCO-c-C3H5 | H |
| 349 | H | OCO-c-C3H5 | OCONH-c-C3H5 | OCO-c-C3H5 | H |
| 350 | H | OCO-c-C3H5 | OCON(CH3)-(c-C3H5) | OCO-c-C3H5 | H |

[Table 15]

| Table A | | | | | |
|---|---|---|---|---|---|
| | R3 | R4 | R5 | R6 | R7 |
| 351 | H | OCO-c-C3H5 | OCONHC6H5 | OCO-c-C3H5 | H |
| 352 | H | OCO-c-C3H5 | OCON(CH3)C6H5 | OCO-c-C3H5 | H |
| 353 | H | OCO-c-C3H5 | OCONH-3-pyridyl | OCO-c-C3H5 | H |
| 354 | H | OCO-c-C3H5 | OCON(CH3)-(3-pyridyl) | OCO-c-C3H5 | H |
| 355 | H | OCO-c-C3H5 | OCO-2-pyridyl | OCO-2-pyridyl | H |
| 356 | H | OCOCH3 | OCO-c-C3H5 | OCOCH3 | H |
| 357 | H | OCOC2H5 | OCO-c-C3H5 | OCOC2H5 | H |
| 358 | H | OCOC3H7 | OCO-c-C3H5 | OCOC3H7 | H |
| 359 | H | OCOCH(CH3)2 | OCO-c-C3H5 | OCOCH(CH3)2 | H |
| 360 | H | OCOC(CH3)3 | OCO-c-C3H5 | OCOC(CH3)3 | H |
| 361 | H | OSO2CH3 | OCO-c-C3H5 | OSO2CH3 | H |
| 362 | H | OSO2C2H5 | OCO-c-C3H5 | OSO2C2H5 | H |
| 363 | H | OSO2-c-C3H5 | OCO-c-C3H5 | OSO2-c-C3H5 | H |
| 364 | H | OCOC6H5 | OCO-c-C3H5 | OCOC6H5 | H |
| 365 | H | OCO-2-pyridyl | OCO-c-C3H5 | OCO-2-pyridyl | H |
| 366 | H | OCO-3-pyridyl | OCO-c-C3H5 | OCO-3-pyridyl | H |
| 367 | H | OCO-4-pyridyl | OCO-c-C3H5 | OCO-4-pyridyl | H |
| 368 | H | OCS-imidazole | OCO-c-C3H5 | OCS-imidazole | H |
| 369 | H | OCOOCH3 | OCO-c-C3H5 | OCOOCH3 | H |
| 370 | H | OCOOC2H5 | OCO-c-C3H5 | OCOOC2H5 | H |
| 371 | H | OCOOCH(CH3)2 | OCO-c-C3H5 | OCOOCH(CH3)2 | H |
| 372 | H | OCOOC(CH3)3 | OCO-c-C3H5 | OCOOC(CH3)3 | H |
| 373 | H | OCOO-c-C3H5 | OCO-c-C3H5 | OCOO-c-C3H5 | H |
| 374 | H | OCOOC6H5 | OCO-c-C3H5 | OCOOC6H5 | H |
| 375 | H | OCOO-3-pyridyl | OCO-c-C3H5 | OCOO-3-pyridyl | H |
| 376 | H | OCONHCH3 | OCO-c-C3H5 | OCONHCH3 | H |
| 377 | H | OCONHC2H5 | OCO-c-C3H5 | OCONHC2H5 | H |
| 378 | H | OCONHC3H7 | OCO-c-C3H5 | OCONHC3H7 | H |
| 379 | H | OCON(CH3)2 | OCO-c-C3H5 | OCON(CH3)2 | H |
| 380 | H | OCON(C2H5)2 | OCO-c-C3H5 | OCON(C2H5)2 | H |
| 381 | H | OCONH-c-C3H5 | OCO-c-C3H5 | OCONH-c-C3H5 | H |
| 382 | H | OCON(CH3)-(c-C3H5) | OCO-c-C3H5 | OCON(CH3)-(c-C3H5) | H |
| 383 | H | OCONHC6H5 | OCO-c-C3H5 | OCONHC6H5 | H |
| 384 | H | OCON(CH3)C6H5 | OCO-c-C3H5 | OCON(CH3)C6H5 | H |
| 385 | H | OCONH-3-pyridyl | OCO-c-C3H5 | OCONH-3-pyridyl | H |
| 386 | H | OCON(CH3)-(3-pyridyl) | OCO-c-C3H5 | OCON(CH3)-(3-pyridyl) | H |
| 387 | H | H | OCO-c-C3H5 | OH | H |

(continued)

Table A

| | R3 | R4 | R5 | R6 | R7 |
|---|---|---|---|---|---|
| 388 | =O | | OCO-c-C3H5 | OH | H |
| 389 | H | OCOCH3 | OCO-c-C3H5 | OH | H |
| 390 | H | OCOC2H5 | OCO-c-C3H5 | OH | H |
| 391 | H | OCOC3H7 | OCO-c-C3H5 | OH | H |
| 392 | H | OCOCH(CH3)2 | OCO-c-C3H5 | OH | H |
| 393 | H | OCOC(CH3)3 | OCO-c-C3H5 | OH | H |
| 394 | H | OS02CH3 | OCO-c-C3H5 | OH | H |
| 395 | H | OSO2C2H5 | OCO-c-C3H5 | OH | H |
| 396 | H | OSO2-c-C3H5 | OCO-c-C3H5 | OH | H |
| 397 | H | OCOC6H5 | OCO-c-C3H5 | OH | H |
| 398 | H | OCO-2-pyridyl | OCO-c-C3H5 | OH | H |
| 399 | H | OCO-3-pyridyl | OCO-c-C3H5 | OH | H |
| 400 | H | OCO-4-pyridyl | OCO-c-C3H5 | OH | H |
| 401 | H | OCS-imidazole | OCO-c-C3H5 | OH | H |
| 402 | H | OCOOCH3 | OCO-c-C3H5 | OH | H |
| 403 | H | OCOOC2H5 | OCO-c-C3H5 | OH | H |
| 404 | H | OCOOCH(CH3)2 | OCO-c-C3H5 | OH | H |
| 405 | H | OCOOC(CH3)3 | OCO-c-C3H5 | OH | H |
| 406 | H | OCOO-c-C3H5 | OCO-c-C3H5 | OH | H |
| 407 | H | OCOOC6H5 | OCO-c-C3H5 | OH | H |
| 408 | H | OCOO-3-pyridyl | OCO-c-C3H5 | OH | H |
| 409 | H | OCONHCH3 | OCO-c-C3H5 | OH | H |
| 410 | H | OCONHC2H5 | OCO-c-C3H5 | OH | H |
| 411 | H | OCONHC3H7 | OCO-c-C3H5 | OH | H |
| 412 | H | OCON(CH3)2 | OCO-c-C3H5 | OH | H |
| 413 | H | OCON(C2H5)2 | OCO-c-C3H5 | OH | H |
| 414 | H | OCONH-c-C3H5 | OCO-c-C3H5 | OH | H |
| 415 | H | OCON(CH3)-(c-C3H5) | OCO-c-C3H5 | OH | H |
| 416 | H | OCONHC6H5 | OCO-c-C3H5 | OH | H |
| 417 | H | OCON(CH3)C6H5 | OCO-c-C3H5 | OH | H |
| 418 | H | OCONH-3-pyridyl | OCO-c-C3H5 | OH | H |
| 419 | H | OCON(CH3)-(3-pyridyl) | OCO-c-C3H5 | OH | H |
| 420 | H | OCO-c-C3H5 | OCOCH3 | OH | H |

[Table 16]

| Table A | | | | | |
|---|---|---|---|---|---|
| | R3 | R4 | R5 | R6 | R7 |
| 421 | H | OCO-c-C3H5 | OCOC2H5 | OH | H |
| 422 | H | OCO-c-C3H5 | OCOC3H7 | OH | H |
| 423 | H | OCO-c-C3H5 | OCOCH(CH3)2 | OH | H |
| 424 | H | OCO-c-C3H5 | OCOC(CH3)3 | OH | H |
| 425 | H | OCO-c-C3H5 | OSO2CH3 | OH | H |
| 426 | H | OCO-c-C3H5 | OSO2C2H5 | OH | H |
| 427 | H | OCO-c-C3H5 | OSO2-c-C3H5 | OH | H |
| 428 | H | OCO-c-C3H5 | OCOC6H5 | OH | H |
| 429 | H | OCO-c-C3H5 | OCO-2-pyridyl | OH | H |
| 430 | H | OCO-c-C3H5 | OCO-3-pyridyl | OH | H |
| 431 | H | OCO-c-C3H5 | OCO-4-pyridyl | OH | H |
| 432 | H | OCO-c-C3H5 | OCS-imidazole | OH | H |
| 433 | H | OCO-c-C3H5 | OCOOCH3 | OH | H |
| 434 | H | OCO-c-C3H5 | OCOOC2H5 | OH | H |
| 435 | H | OCO-c-C3H5 | OCOOCH(CH3)2 | OH | H |
| 436 | H | OCO-c-C3H5 | OCOOC(CH3)3 | OH | H |
| 437 | H | OCO-c-C3H5 | OCOO-C-C3H5 | OH | H |
| 438 | H | OCO-c-C3H5 | OCOOC6H5 | OH | H |
| 439 | H | OCO-c-C3H5 | OCOO-3-pyridyl | OH | H |
| 440 | H | OCO-c-C3H5 | OCONHCH3 | OH | H |
| 441 | H | OCO-c-C3H5 | OCONHC2H5 | OH | H |
| 442 | H | OCO-c-C3H5 | OCONHC3H7 | OH | H |
| 443 | H | OCO-c-C3H5 | OCON(CH3)2 | OH | H |
| 444 | H | OCO-c-C3H5 | OCON(C2H5)2 | OH | H |
| 445 | H | OCO-c-C3H5 | OCONH-c-C3H5 | OH | H |
| 446 | H | OCO-c-C3H5 | OCON(CH3)-(c-C3H5) | OH | H |
| 447 | H | OCO-c-C3H5 | OCONHC6H5 | OH | H |
| 448 | H | OCO-c-C3H5 | OCON(CH3)C6H5 | OH | H |
| 449 | H | OCO-c-C3H5 | OCONH-3-pyridyl | OH | H |
| 450 | H | OCO-c-C3H5 | OCON(CH3)-(3-pyridyl) | OH | H |
| 451 | H | OCOCH3 | OCO-c-C3H5 | H | H |
| 452 | H | OCOC2H5 | OCO-c-C3H5 | H | H |
| 453 | H | OCOC3H7 | OCO-c-C3H5 | H | H |
| 454 | H | OCOCH(CH3)2 | OCO-c-C3H5 | H | H |
| 455 | H | OCOC(CH3)3 | OCO-c-C3H5 | H | H |
| 456 | H | OS02CH3 | OCO-c-C3H5 | H | H |
| 457 | H | OSO2C2H5 | OCO-c-C3H5 | H | H |

(continued)

| Table A | | | | | |
|---|---|---|---|---|---|
| | R3 | R4 | R5 | R6 | R7 |
| 458 | H | OSO2-c-C3H5 | OCO-c-C3H5 | H | H |
| 459 | H | OCOC6H5 | OCO-c-C3H5 | H | H |
| 460 | H | OCO-2-pyridyl | OCO-c-C3H5 | H | H |
| 461 | H | OCO-3-pyridyl | OCO-c-C3H5 | H | H |
| 462 | H | OCO-4-pyridyl | OCO-c-C3H5 | H | H |
| 463 | H | OCS-imidazole | OCO-c-C3H5 | H | H |
| 464 | H | OCOOCH3 | OCO-c-C3H5 | H | H |
| 465 | H | OCOOC2H5 | OCO-c-C3H5 | H | H |
| 466 | H | OCOOCH(CH3)2 | OCO-c-C3H5 | H | H |
| 467 | H | OCOOC(CH3)3 | OCO-c-C3H5 | H | H |
| 468 | H | OCOO-c-C3H5 | OCO-c-C3H5 | H | H |
| 469 | H | OCOOC6H5 | OCO-c-C3H5 | H | H |
| 470 | H | OCOO-3-pyridyl | OCO-c-C3H5 | H | H |
| 471 | H | OCONHCH3 | OCO-c-C3H5 | H | H |
| 472 | H | OCONHC2H5 | OCO-c-C3H5 | H | H |
| 473 | H | OCONHC3H7 | OCO-c-C3H5 | H | H |
| 474 | H | OCON(CH3)2 | OCO-c-C3H5 | H | H |
| 475 | H | OCON(C2H5)2 | OCO-c-C3H5 | H | H |
| 476 | H | OCONH-c-C3H5 | OCO-c-C3H5 | H | H |
| 477 | H | OCON(CH3)-(c-C3H5) | OCO-c-C3H5 | H | H |
| 478 | H | OCONHC6H5 | OCO-c-C3H5 | H | H |
| 479 | H | OCON(CH3)C6H5 | OCO-c-C3H5 | H | H |
| 480 | H | OCONH-3-pyridyl | OCO-c-C3H5 | H | H |
| 481 | H | OCON(CH3)-(3-pyridyl) | OCO-c-C3H5 | H | H |
| 482 | H | OCO-c-C3H5 | OCOCH3 | H | H |
| 483 | H | OCO-c-C3H5 | OCOC2H5 | H | H |
| 484 | H | OCO-c-C3H5 | OCOC3H7 | H | H |
| 485 | H | OCO-c-C3H5 | OCOCH(CH3)2 | H | H |
| 486 | H | OCO-c-C3H5 | OCOC(CH3)3 | H | H |
| 487 | H | OCO-c-C3H5 | OS02CH3 | H | H |
| 488 | H | OCO-c-C3H5 | OSO2C2H5 | H | H |
| 489 | H | OCO-c-C3H5 | OSO2-c-C3H5 | H | H |
| 490 | H | OCO-c-C3H5 | OCOC6H5 | H | H |

[Table 17]

| Table A | | | | | |
|---|---|---|---|---|---|
| | R3 | R4 | R5 | R6 | R7 |
| 491 | H | OCO-c-C3H5 | OCO-2-pyridyl | H | H |
| 492 | H | OCO-c-C3H5 | OCO-3-pyridyl | H | H |
| 493 | H | OCO-c-C3H5 | OCO-4-pyridyl | H | H |
| 494 | H | OCO-c-C3H5 | OCS-imidazole | H | H |
| 495 | H | OCO-c-C3H5 | OCOOCH3 | H | H |
| 496 | H | OCO-c-C3H5 | OCOOC2H5 | H | H |
| 497 | H | OCO-c-C3H5 | OCOOCH(CH3)2 | H | H |
| 498 | H | OCO-c-C3H5 | OCOOC(CH3)3 | H | H |
| 499 | H | OCO-o-C3H5 | OCOO-c-C3H5 | H | H |
| 500 | H | OCO-c-C3H5 | OCOOC6H5 | H | H |
| 501 | H | OCO-c-C3H5 | OCOO-3-pyridyl | H | H |
| 502 | H | OCO-c-C3H5 | OCONHCH3 | H | H |
| 503 | H | OCO-c-C3H5 | OCONHC2H5 | H | H |
| 504 | H | OCO-c-C3H5 | OCONHC3H7 | H | H |
| 505 | H | OCO-c-C3H5 | OCON(CH3)2 | H | H |
| 506 | H | OCO-c-C3H5 | OCON(C2H5)2 | H | H |
| 507 | H | OCO-c-C3H5 | OCONH-c-C3H5 | H | H |
| 508 | H | OCO-c-C3H5 | OCON(CH3)-(c-C3H5) | H | H |
| 509 | H | OCO-c-C3H5 | OCONHC6H5 | H | H |
| 510 | H | OCO-c-C3H5 | OCON(CH3)C6H5 | H | H |
| 511 | H | OCO-c-C3H5 | OCONH-3-pyridyl | H | H |
| 512 | H | OCO-c-C3H5 | OCON(CH3)-(3-pyridyl) | H | H |
| 513 | H | OCOCH3 | OCO-c-C3H5 | =O | |
| 514 | H | OCOC2H5 | OCO-c-C3H5 | =O | |
| 515 | H | OCOC3H7 | OCO-c-C3H5 | =O | |
| 516 | H | OCOCH(CH3)2 | OCO-c-C3H5 | =O | |
| 517 | H | OCOC(CH3)3 | OCO-c-C3H5 | =O | |
| 518 | H | OSO2CH3 | OCO-c-C3H5 | =O | |
| 519 | H | OSO2C2H5 | OCO-c-C3H5 | =O | |
| 520 | H | OSO2-c-C3H5 | OCO-c-C3H5 | =O | |
| 521 | H | OCOC6H5 | OCO-c-C3H5 | =O | |
| 522 | H | OCO-2-pyridyl | OCO-c-C3H5 | =O | |
| 523 | H | OCO-3-pyridyl | OCO-c-C3H5 | =O | |
| 524 | H | OCO-4-pyridyl | OCO-c-C3H5 | =O | |
| 525 | H | OCS-imidazole | OCO-c-C3H5 | =O | |
| 526 | H | OCOOCH3 | OCO-c-C3H5 | =O | |
| 527 | H | OCOOC2H5 | OCO-c-C3H5 | =O | |

(continued)

| Table A | | | | | |
|---|---|---|---|---|---|
| | R3 | R4 | R5 | R6 | R7 |
| 528 | H | OCOOCH(CH3)2 | OCO-c-C3H5 | =O | |
| 529 | H | OCOOC(CH3)3 | OCO-c-C3H5 | =O | |
| 530 | H | OCOO-c-C3H5 | OCO-c-C3H5 | =O | |
| 531 | H | OCOOC6H5 | OCO-c-C3H5 | =O | |
| 532 | H | OCOO-3-pyridyl | OCO-c-C3H5 | =O | |
| 533 | H | OCONHCH3 | OCO-c-C3H5 | =O | |
| 534 | H | OCONHC2H5 | OCO-c-C3H5 | =O | |
| 535 | H | OCONHC3H7 | OCO-c-C3H5 | =O | |
| 536 | H | OCON(CH3)2 | OCO-c-C3H5 | =O | |
| 537 | H | OCON(C2H5)2 | OCO-c-C3H5 | =O | |
| 538 | H | OCONH-c-C3H5 | OCO-c-C3H5 | =O | |
| 539 | H | OCON(CH3)-(c-C3H5) | OCO-c-C3H5 | =O | |
| 540 | H | OCONHC6H5 | OCO-c-C3H5 | =O | |
| 541 | H | OCON(CH3)C6H5 | OCO-c-C3H5 | =O | |
| 542 | H | OCONH-3-pyridyl | OCO-c-C3H5 | =O | |
| 543 | H | OCON(CH3)-(3-pyridyl) | OCO-c-C3H5 | =O | |
| 544 | H | OCO-c-C3H5 | OCOCH3 | =O | |
| 545 | H | OCO-c-C3H5 | OCOC2H5 | =O | |
| 546 | H | OCO-c-C3H5 | OCOC3H7 | =O | |
| 547 | H | OCO-c-C3H5 | OCOCH(CH3)2 | =O | |
| 548 | H | OCO-c-C3H5 | OCOC(CH3)3 | =O | |
| 549 | H | OCO-c-C3H5 | OSO2CH3 | =O | |
| 550 | H | OCO-c-C3H5 | OSO2C2H5 | =O | |
| 551 | H | OCO-c-C3H5 | OSO2-c-C3H5 | =O | |
| 552 | H | OCO-c-C3H5 | OCOC6H5 | =O | |
| 553 | H | OCO-c-C3H5 | OCO-2-pyridyl | =O | |
| 554 | H | OCO-c-C3H5 | OCO-3-pyridyl | =O | |
| 555 | H | OCO-c-C3H5 | OCO-4-pyridyl | =O | |
| 556 | H | OCO-c-C3H5 | OCS-imidazole | =O | |
| 557 | H | OCO-c-C3H5 | OCOOCH3 | =O | |
| 558 | H | OCO-c-C3H5 | OCOOC2H5 | =O | |
| 559 | H | OCO-c-C3H5 | OCOOCH(CH3)2 | =O | |
| 560 | H | OCO-c-C3H5 | OCOOC(CH3)3 | =O | |

[Table 18]

| Table A | | | | | |
|---|---|---|---|---|---|
| | R3 | R4 | R5 | R6 | R7 |
| 561 | H | OCO-c-C3H5 | OCOO-c-C3H5 | =O | |
| 562 | H | OCO-c-C3H5 | OCOOC6H5 | =O | |
| 563 | H | OCO-c-C3H5 | OCOO-3-pyridyl | =O | |
| 564 | H | OCO-c-C3H5 | OCONHCH3 | =O | |
| 565 | H | OCO-c-C3H5 | OCONHC2H5 | =O | |
| 566 | H | OCO-c-C3H5 | OCONHC3H7 | =O | |
| 567 | H | OCO-c-C3H5 | OCON(CH3)2 | =O | |
| 568 | H | OCO-c-C3H5 | OCON(C2H5)2 | =O | |
| 569 | H | OCO-c-C3H5 | OCONH-c-C3H5 | =O | |
| 570 | H | OCO-c-C3H5 | OCON(CH3)-(c-C3H5) | =O | |
| 571 | H | OCO-c-C3H5 | OCONHC6H5 | =O | |
| 572 | H | OCO-c-C3H5 | OCON(CH3)C6H5 | =O | |
| 573 | H | OCO-c-C3H5 | OCONH-3-pyridyl | =O | |
| 574 | H | OCO-c-C3H5 | OCON(CH3)-(3-pyridyl) | =O | |
| 575 | H | OCO-c-C3H5 | OH | OCOCH3 | H |
| 576 | H | OCO-c-C3H5 | OH | OCOC2H5 | H |
| 577 | H | OCO-c-C3H5 | OH | OCOC3H7 | H |
| 578 | H | OCO-c-C3H5 | OH | OCOCH(CH3)2 | H |
| 579 | H | OCO-c-C3H5 | OH | OCOC(CH3)3 | H |
| 580 | H | OCO-c-C3H5 | OH | OSO2CH3 | H |
| 581 | H | OCO-c-C3H5 | OH | OSO2C2H5 | H |
| 582 | H | OCO-c-C3H5 | OH | OSO2-c-C3H5 | H |
| 583 | H | OCO-c-C3H5 | OH | OCOC6H5 | H |
| 584 | H | OCO-c-C3H5 | OH | OCO-2-pyridyl | H |
| 585 | H | OCO-c-C3H5 | OH | OCO-3-pyridyl | H |
| 586 | H | OCO-c-C3H5 | OH | OCO-4-pyridyl | H |
| 587 | H | OCO-c-C3H5 | OH | OCS-imidazole | H |
| 588 | H | OCO-c-C3H5 | OH | OCOOCH3 | H |
| 589 | H | OCO-c-C3H5 | OH | OCOOC2H5 | H |
| 590 | H | OCO-c-C3H5 | OH | OCOOCH(CH3)2 | H |
| 591 | H | OCO-c-C3H5 | OH | OCOOC(CH3)3 | H |
| 592 | H | OCO-c-C3H5 | OH | OCOO-c-C3H5 | H |
| 593 | H | OCO-c-C3H5 | OH | OCOOC6H5 | H |
| 594 | H | OCO-c-C3H5 | OH | OCOO-3-pyridyl | H |
| 595 | H | OCO-c-C3H5 | OH | OCONHCH3 | H |
| 596 | H | OCO-c-C3H5 | OH | OCONHC2H5 | H |
| 597 | H | OCO-c-C3H5 | OH | OCONHC3H7 | H |

(continued)

| Table A | | | | | |
|---|---|---|---|---|---|
| | R3 | R4 | R5 | R6 | R7 |
| 598 | H | OCO-c-C3H5 | OH | OCON(CH3)2 | H |
| 599 | H | OCO-c-C3H5 | OH | OCON(C2H5)2 | H |
| 600 | H | OCO-c-C3H5 | OH | OCONH-c-C3H5 | H |
| 601 | H | OCO-c-C3H5 | OH | OCON(CH3)-(c-C3H5) | H |
| 602 | H | OCO-c-C3H5 | OH | OCONHC6H5 | H |
| 603 | H | OCO-c-C3H5 | OH | OCON(CH3)C6H5 | H |
| 604 | H | OCO-c-C3H5 | OH | OCONH-3-pyridyl | H |
| 605 | H | OCO-c-C3H5 | OH | OCON(CH3)-(3-pyridyl) | H |
| 606 | H | OCOCH3 | OH | OCO-c-C3H5 | H |
| 607 | H | OCOC2H5 | OH | OCO-c-C3H5 | H |
| 608 | H | OCOC3H7 | OH | OCO-c-C3H5 | H |
| 609 | H | OCOCH(CH3)2 | OH | OCO-c-C3H5 | H |
| 610 | H | OCOC(CH3)3 | OH | OCO-c-C3H5 | H |
| 611 | H | OSO2CH3 | OH | OCO-c-C3H5 | H |
| 612 | H | OSO2C2H5 | OH | OCO-c-C3H5 | H |
| 613 | H | OSO2-c-C3H5 | OH | OCO-c-C3H5 | H |
| 614 | H | OCOC6H5 | OH | OCO-c-C3H5 | H |
| 615 | H | OCO-2-pyridyl | OH | OCO-c-C3H5 | H |
| 616 | H | OCO-3-pyridyl | OH | OCO-c-C3H5 | H |
| 617 | H | OCO-4-pyridyl | OH | OCO-c-C3H5 | H |
| 618 | H | OCS-imidazole | OH | OCO-c-C3H5 | H |
| 619 | H | OCOOCH3 | OH | OCO-c-C3H5 | H |
| 620 | H | OCOOC2H5 | OH | OCO-c-C3H5 | H |
| 621 | H | OCOOCH(CH3)2 | OH | OCO-c-C3H5 | H |
| 622 | H | OCOOC(CH3)3 | OH | OCO-c-C3H5 | H |
| 623 | H | OCOO-c-C3H5 | OH | OCO-c-C3H5 | H |
| 624 | H | OCOOC6H5 | OH | OCO-c-C3H5 | H |
| 625 | H | OCOO-3-pyridyl | OH | OCO-c-C3H6 | H |
| 626 | H | OCONHCH3 | OH | OCO-c-C3H5 | H |
| 627 | H | OCONHC2H5 | OH | OCO-c-C3H5 | H |
| 628 | H | OCONHC3H7 | OH | OCO-c-C3H5 | H |
| 629 | H | OCON(CH3)2 | OH | OCO-c-C3H5 | H |
| 630 | H | OCON(C2H5)2 | OH | OCO-c-C3H5 | H |

[Table 19]

| Table A | | | | | |
|---|---|---|---|---|---|
| | R3 | R4 | R5 | R6 | R7 |
| 631 | H | OCONH-c-C3H5 | OH | OCO-c-C3H5 | H |
| 632 | H | OCON(CH3)-(c-C3H5) | OH | OCO-c-C3H5 | H |
| 633 | H | OCONHC6H5 | OH | OCO-c-C3H5 | H |
| 634 | H | OCON(CH3)C6H5 | OH | OCO-c-C3H5 | H |
| 635 | H | OCONH-3-pyridyl | OH | OCO-c-C3H5 | H |
| 636 | H | OCON(CH3)-(3-pyridyl) | OH | OCO-c-C3H5 | H |
| 637 | H | OH | OCO-c-C3H5 | OCOCH3 | H |
| 638 | H | OH | OCO-c-C3H5 | OCOC2H5 | H |
| 639 | H | OH | OCO-c-C3H5 | OCOC3H7 | H |
| 640 | H | OH | OCO-c-C3H5 | OCOCH(CH3)2 | H |
| 641 | H | OH | OCO-c-C3H5 | OCOC(CH3)3 | H |
| 642 | H | OH | OCO-c-C3H5 | OSO2CH3 | H |
| 643 | H | OH | OCO-c-C3H5 | OSO2C2H5 | H |
| 644 | H | OH | OCO-c-C3H5 | OSO2-c-C3H5 | H |
| 645 | H | OH | OCO-c-C3H5 | OCOC6H5 | H |
| 646 | H | OH | OCO-c-C3H5 | OCO-2-pyridyl | H |
| 647 | H | OH | OCO-c-C3H5 | OCO-3-pyridyl | H |
| 648 | H | OH | OCO-c-C3H5 | OCO-4-pyridyl | H |
| 649 | H | OH | OCO-c-C3H5 | OCS-imidazole | H |
| 650 | H | OH | OCO-c-C3H5 | OCOOCH3 | H |
| 651 | H | OH | OCO-c-C3H5 | OCOOC2H5 | H |
| 652 | H | OH | OCO-c-C3H5 | OCOOCH(CH3)2 | H |
| 653 | H | OH | OCO-c-C3H5 | OCOOC(CH3)3 | H |
| 654 | H | OH | OCO-c-C3H5 | OCOO-c-C3H5 | H |
| 655 | H | OH | OCO-c-C3H5 | OCOOC6H5 | H |
| 656 | H | OH | OCO-c-C3H5 | OCOO-3-pyridyl | H |
| 657 | H | OH | OCO-c-C3H5 | OCONHCH3 | H |
| 658 | H | OH | OCO-c-C3H5 | OCONHC2H5 | H |
| 659 | H | OH | OCO-c-C3H5 | OCONHC3H7 | H |
| 660 | H | OH | OCO-c-C3H5 | OCON(CH3)2 | H |
| 661 | H | OH | OCO-c-C3H5 | OCON(C2H5)2 | H |
| 662 | H | OH | OCO-c-C3H5 | OCONH-c-C3H5 | H |
| 663 | H | OH | OCO-c-C3H5 | OCON(CH3)-(c-C3H5) | H |
| 664 | H | OH | OCO-c-C3H5 | OCONHC6H5 | H |
| 665 | H | OH | OCO-c-C3H5 | OCON(CH3)C6H5 | H |
| 666 | H | OH | OCO-c-C3H5 | OCONH-3-pyridyl | H |
| 667 | H | OH | OCO-c-C3H5 | OCON(CH3)-(3-pyridyl) | H |

(continued)

| Table A | | | | | |
|---|---|---|---|---|---|
| | R3 | R4 | R5 | R6 | R7 |
| 668 | H | OH | OCOOH3 | OCO-c-C3H5 | H |
| 669 | H | OH | OCOC2H5 | OCO-c-C3H5 | H |
| 670 | H | OH | OCOC3H7 | OCO-c-C3H5 | H |
| 671 | H | OH | OCOCH(CH3)2 | OCO-c-C3H5 | H |
| 672 | H | OH | OCOC(CH3)3 | OCO-c-C3H5 | H |
| 673 | H | OH | OSO2CH3 | OCO-c-C3H5 | H |
| 674 | H | OH | OSO2C2H5 | OCO-c-C3H5 | H |
| 675 | H | OH | OSO2-c-C3H5 | OCO-c-C3H5 | H |
| 676 | H | OH | OCOC6H5 | OCO-c-C3H5 | H |
| 677 | H | OH | OCO-2-pyridyl | OCO-c-C3H5 | H |
| 678 | H | OH | OCO-3-pyridyl | OCO-c-C3H5 | H |
| 679 | H | OH | OCO-4-pyridyl | OCO-c-C3H5 | H |
| 680 | H | OH | OCS-imidazole | OCO-c-C3H5 | H |
| 681 | H | OH | OCOOCH3 | OCO-c-C3H5 | H |
| 682 | H | OH | OCOOC2H5 | OCO-c-C3H5 | H |
| 683 | H | OH | OCOOCH(CH3)2 | OCO-c-C3H5 | H |
| 684 | H | OH | OCOOC(CH3)3 | OCO-c-C3H5 | H |
| 685 | H | OH | OCOO-c-C3H5 | OCO-c-C3H5 | H |
| 686 | H | OH | OCOOC6H5 | OCO-c-C3H5 | H |
| 687 | H | OH | OCOO-3-pyridyl | OCO-c-C3H6 | H |
| 688 | H | OH | OCONHCH3 | OCO-c-C3H5 | H |
| 689 | H | OH | OCONHC2H5 | OCO-c-C3H5 | H |
| 690 | H | OH | OCONHC3H7 | OCO-c-C3H5 | H |
| 691 | H | OH | OCON(CH3)2 | OCO-c-C3H5 | H |
| 692 | H | OH | OCON(C2H5)2 | OCO-c-C3H5 | H |
| 693 | H | OH | OCONH-c-C3H5 | OCO-c-C3H5 | H |
| 694 | H | OH | OCON(CH3)-(c-C3H5) | OCO-c-C3H5 | H |
| 695 | H | OH | OCONHC6H5 | OCO-c-C3H5 | H |
| 696 | H | OH | OCON(CH3)C6H5 | OCO-c-C3H5 | H |
| 697 | H | OH | OCONH-3-pyridyl | OCO-c-C3H5 | H |
| 698 | H | OH | OCON(CH3)-(3-pyridyl) | OCO-c-C3H5 | H |
| 699 | H | OCO-c-C3H5 | OH | OCO-c-C3H5 | H |
| 700 | H | OH | OCO-c-C3H5 | OCO-c-C3H5 | H |

[Table 20]

| Table A | | | | | |
|---|---|---|---|---|---|
| | R3 | R4 | R5 | R6 | R7 |
| 701 | H | OCO-c-C3H5 | OH | OH | H |
| 702 | H | OH | OCO-c-C3H5 | OH | H |
| 703 | H | OH | OH | OCO-c-C3H5 | H |
| 704 | H | OCO-c-C3H5 | OH | H | H |
| 705 | H | OH | OCO-c-C3H5 | H | H |
| 706 | H | OCO-c-C3H5 | OH | H | H |
| 707 | H | OH | OCO-c-C3H5 | =O | |
| 708 | H | OCO-c-C3H5 | OH | =O | |
| 709 | H | OCO-c-C3H5 | H | OCO-c-C3H5 | H |
| 710 | H | OH | H | OCO-c-C3H5 | H |
| 711 | H | OCO-c-C3H5 | H | OH | H |
| 712 | H | H | OCO-c-C3H5 | OH | H |
| 713 | H | OCO-c-C3H5 | H | H | H |
| 714 | H | H | OCO-c-C3H5 | H | H |
| 715 | H | OCO-c-C4H7 | OCO-c-C4H7 | OCO-c-C4H7 | H |
| 716 | H | OCO-c-C4H7 | OCO-c-C4H7 | OH | H |
| 717 | H | OCO-c-C4H7 | OCO-c-C3H5 | OH | H |
| 718 | H | OCO-c-C3H5 | OCO-c-C4H7 | OH | H |
| 719 | H | OCO-c-C4H7 | OCO-c-C4H7 | H | H |
| 720 | H | OCO-c-C4H7 | OCO-c-C4H7 | =O | |
| 721 | H | OCO-c-C4H7 | OH | H | H |
| 722 | H | OH | OCO-c-C4H7 | H | H |
| 723 | H | OCO-c-C4H7 | H | H | H |
| 724 | H | OCO-c-C5H9 | OCO-c-C5H9 | OCO-c-C5H9 | H |
| 725 | H | OCO-c-C5H9 | OCO-c-C5H9 | OH | H |
| 726 | H | OCO-c-C5H9 | OCO-c-C3H5 | OH | H |
| 727 | H | OCO-c-C3H5 | OCO-c-C5H9 | OH | H |
| 728 | H | OCO-c-C5H9 | OCO-c-C5H9 | H | H |
| 729 | H | OCO-c-C5H9 | OCO-c-C5H9 | =O | |
| 730 | H | OCO-c-C5H9 | OH | H | H |
| 731 | H | OH | OCO-c-C5H9 | H | H |
| 732 | H | OCO-c-C5H9 | H | H | H |
| 733 | H | OCO-c-C6H11 | OCO-c-C6H11 | OCO-c-C6H11 | H |
| 734 | H | OCO-c-C6H11 | OCO-c-C6H11 | OH | H |
| 735 | H | OCO-c-C6H11 | OCO-c-C3H5 | OH | H |
| 736 | H | OCO-c-C3H5 | OCO-c-C6H11 | OH | H |
| 737 | H | OCO-c-C6H11 | OCO-c-C6H11 | =O | |

(continued)

| Table A | | | | | |
|---|---|---|---|---|---|
| | R3 | R4 | R5 | R6 | R7 |
| 738 | H | OCO-c-C6H11 | OCO-c-C6H11 | H | H |
| 739 | H | OCO-c-C6H11 | OH | H | H |
| 740 | H | OH | OCO-c-C6H11 | H | H |
| 741 | H | OCO-c-C6H11 | H | H | H |
| 742 | H | OCO-c-C3H(CH3)4 | OCO-c-C3H(CH3)4 | OCO-c-C3H(CH3)4 | H |
| 743 | H | OH | OCO-c-C3H(CH3)4 | OCO-c-C3H(CH3)4 | H |
| 744 | H | OH | OCOC6H5 | OH | H |
| 745 | H | OCO-(3-pyridyl) | OCO-(3-pyridyl) | OCO-(3-pyridyl) | H |
| 746 | H | OCO-(2-pyridyl) | OCO-(2-pyridyl) | OCO-(2-pyridyl) | H |
| 747 | H | OCO-(4-CF3-3-pyridyl) | OCO-(4-CF3-3-pyridyl) | OCO-(4-CF3-3-pyridyl) | H |
| 748 | H | OCO-(6-CF3-3-pyridyl) | OCO-(6-CF3-3-pyridyl) | OCO-(6-CF3-3-pyridyl) | H |
| 749 | H | OCH2OCH3 | OCH2OCH3 | OCH2OCH3 | H |
| 750 | H | OCH2SCH3 | OCH2SCH3 | OCH2SCH3 | H |
| 751 | H | OCOC2H5 | OCOC2H5 | O-(2-Tetrahydropyranyl) | H |
| 752 | H | OCOC2H5 | OCOC2H5 | OCH2OCH3 | H |
| 753 | H | OH | OCH2C6H5 | OCOCH3 | H |
| 754 | H | OH | OCH2OC2H4OCH3 | OCH2OC2H4OCH3 | H |
| 755 | H | OCH2OC2H4OCH3 | OCH2OC2H4OCH3 | OH | H |
| 756 | H | OH | OCH2OC2H4OCH3 | OH | H |
| 757 | H | OH | OPO(OC6H5)2 | OH | H |
| 758 | H | OPO(OC6H5)2 | OPO(OC6H5)2 | OPO(OC6H5)2 | H |
| 759 | H | OH | OCH2OCH3 | OH | H |
| 760 | H | OCOCH3 | OCOCH3 | O-tetra-O-benzyl-mannose | H |
| 761 | H | O-(2-tetrahydropyranyl) | O-(2-tetrahydropyranyl) | O-(2-tetrahydropyranyl) | H |
| 762 | H | 2-tetrahydropyranyl | 2-tetrahydropyranyl | 2-tetrahydropyranyl | H |
| 763 | H | OCOC2H5 | OCOC2H5 | OCH2SCH3 | H |
| 764 | H | OCOCH3 | OCOCH3 | OCH2OC2H4OCH3 | H |
| 765 | H | OCH2OC2H4OCH3 | OCH2OC2H4OCH3 | OCH2OC2H4OCH3 | H |
| 766 | H | OCOCH3 | OCH2OC2H4OCH3 | OCOCH3 | H |
| 767 | H | OCOCH3 | OCH2OCH3 | OCOCH3 | H |
| 768 | H | OCOCH3 | OCOCH3 | OCH3 | H |
| 769 | H | OCOCH3 | OCOCH3 | OC2H5 | H |
| 770 | H | OCOCH3 | OCOCH3 | OC3H7 | H |

[Table 21]

| Table A | | | | | |
|---|---|---|---|---|---|
| | R3 | R4 | R5 | R6 | R7 |
| 771 | H | OCOCH3 | OCOCH3 | OC4H9 | H |
| 772 | H | OCOCH3 | OCOCH3 | OCH(CH3)2 | H |
| 773 | H | OCOCH3 | OCOCH3 | OC(CH3)3 | H |
| 774 | H | OCOCH3 | OCOCH3 | O-c-C3H5 | H |
| 775 | H | OCOCH3 | OCOCH3 | OCH2-c-C3H5 | H |
| 776 | H | OCOCH3 | OCOCH3 | OCH2C6H5 | H |
| 777 | H | OCH3 | OCOCH3 | OCH3 | H |
| 778 | H | OC2H5 | OCOCH3 | OC2H5 | H |
| 779 | H | OC3H7 | OCOCH3 | OC3H7 | H |
| 780 | H | OC4H9 | OCOCH3 | OC4H9 | H |
| 781 | H | OCH(CH3)2 | OCOCH3 | OCH(CH3)2 | H |
| 782 | H | OC(CH3)3 | OCOCH3 | OC(CH3)3 | H |
| 783 | H | O-c-C3H5 | OCOCH3 | O-c-C3H5 | H |
| 784 | H | OCH2-c-C3H5 | OCOCH3 | OCH2-c-C3H5 | H |
| 785 | H | OCH2C6H5 | OCOCH3 | OCH2C6H5 | H |
| 786 | H | OCH3 | OCOCH3 | OH | H |
| 787 | H | OC2H5 | OCOCH3 | OH | H |
| 788 | H | OC3H7 | OCOCH3 | OH | H |
| 789 | H | OC4H9 | OCOCH3 | OH | H |
| 790 | H | OCH(CH3)2 | OCOCH3 | OH | H |
| 791 | H | OC(CH3)3 | OCOCH3 | OH | H |
| 792 | H | O-c-C3H5 | OCOCH3 | OH | H |
| 793 | H | OCH2-c-C3H5 | OCOCH3 | OH | H |
| 794 | H | OCH2C6H5 | OCOCH3 | OH | H |
| 795 | H | OCOCH3 | OCH3 | OH | H |
| 796 | H | OCOCH3 | OC2H5 | OH | H |
| 797 | H | OCOCH3 | OC3H7 | OH | H |
| 798 | H | OCOCH3 | OC4H9 | OH | H |
| 799 | H | OCOCH3 | OCH(CH3)2 | OH | H |
| 800 | H | OCOCH3 | OC(CH3)3 | OH | H |
| 801 | H | OCOCH3 | O-c-C3H5 | OH | H |
| 802 | H | OCOCH3 | OCH2-c-C3H5 | OH | H |
| 803 | H | OCOCH3 | OCH2C6H5 | OH | H |
| 804 | H | OCOCH3 | OCH3 | H | H |
| 805 | H | OCOCH3 | OC2H5 | H | H |
| 806 | H | OCOCH3 | OC3H7 | H | H |
| 807 | H | OCOCH3 | OC4H9 | H | H |

(continued)

| Table A | | | | | |
|---|---|---|---|---|---|
| | R3 | R4 | R5 | R6 | R7 |
| 808 | H | OCOCH3 | OCH(CH3)2 | H | H |
| 809 | H | OCOCH3 | OC(CH3)3 | H | H |
| 810 | H | OCOCH3 | O-c-C3H5 | H | H |
| 811 | H | OCOCH3 | OCH2-c-C3H5 | H | H |
| 812 | H | OCOCH3 | OCCH2C6H5 | H | H |
| 813 | H | OCOC2H5 | OCOC2H5 | OCH3 | H |
| 814 | H | OCOC2H5 | OCOC2H5 | OC2H5 | H |
| 815 | H | OCOC2H5 | OCOC2H5 | OC3H7 | H |
| 816 | H | OCOC2H5 | OCOC2H5 | OC4H9 | H |
| 817 | H | OCOC2H5 | OCOC2H5 | OCH(CH3)2 | H |
| 818 | H | OCOC2H5 | OCOC2H5 | OC(CH3)3 | H |
| 819 | H | OCOC2H5 | OCOC2H5 | O-c-C3H5 | H |
| 820 | H | OCOC2H5 | OCOC2H5 | OCH2-c-C3H5 | H |
| 821 | H | OCOC2H5 | OCOC2H5 | OCH2C6H5 | H |
| 822 | H | OCH3 | OCOC2H5 | OCH3 | H |
| 823 | H | OC2H5 | OCOC2H5 | OC2H5 | H |
| 824 | H | OC3H7 | OCOC2H5 | OC3H7 | H |
| 825 | H | OC4H9 | OCOC2H5 | OC4H9 | H |
| 826 | H | OCH(CH3)2 | OCOC2H5 | OCH(CH3)2 | H |
| 827 | H | OC(CH3)3 | OCOC2H5 | OC(CH3)3 | H |
| 828 | H | O-c-C3H5 | OCOC2H5 | O-c-C3H5 | H |
| 829 | H | OCH2-c-C3H5 | OCOC2H5 | OCH2-c-C3H5 | H |
| 830 | H | OCH2C6H5 | OCOC2H5 | OCH2C6H5 | H |
| 831 | H | OCH3 | OCOC2H5 | OH | H |
| 832 | H | OC2H5 | OCOC2H5 | OH | H |
| 833 | H | OC3H7 | OCOC2H5 | OH | H |
| 834 | H | OC4H9 | OCOC2H5 | OH | H |
| 835 | H | OCH(CH3)2 | OCOC2H5 | OH | H |
| 836 | H | OC(CH3)3 | OCOC2H5 | OH | H |
| 837 | H | O-c-C3H5 | OCOC2H5 | OH | H |
| 838 | H | OCH2-c-C3H5 | OCOC2H5 | OH | H |
| 839 | H | OCH2C6H5 | OCOC2H5 | OH | H |
| 840 | H | OCOC2H5 | OCH3 | OH | H |

[Table 22]

| Table A | | | | | |
|---|---|---|---|---|---|
| | R3 | R4 | R5 | R6 | R7 |
| 841 | H | OCOC2H5 | OC2H5 | OH | H |
| 842 | H | OCOC2H5 | OC3H7 | OH | H |
| 843 | H | OCOC2H5 | OC4H9 | OH | H |
| 844 | H | OCOC2H5 | OCH(CH3)2 | OH | H |
| 845 | H | OCOC2H5 | OC(CH3)3 | OH | H |
| 846 | H | OCOC2H5 | O-c-C3H5 | OH | H |
| 847 | H | OCOC2H5 | OCH2-c-C3H5 | OH | H |
| 848 | H | OCOC2H5 | OCH2C6H5 | OH | H |
| 849 | H | OCOC2H5 | OCH3 | H | H |
| 850 | H | OCOC2H5 | OC2H5 | H | H |
| 851 | H | OCOC2H5 | OC3H7 | H | H |
| 852 | H | OCOC2H5 | OC4H9 | H | H |
| 853 | H | OCOC2H5 | OCH(CH3)2 | H | H |
| 854 | H | OCOC2H5 | OC(CH3)3 | H | H |
| 855 | H | OCOC2H5 | O-c-C3H5 | H | H |
| 856 | H | OCOC2H5 | OCH2-c-C3H5 | H | H |
| 857 | H | OCOC2H5 | OCCH2C6H5 | H | H |
| 858 | H | OCO-c-C3H5 | OCO-c-C3H5 | OCH3 | H |
| 859 | H | OCO-c-C3H5 | OCO-c-C3H5 | OC2H5 | H |
| 860 | H | OCO-c-C3H5 | OCO-c-C3H5 | OC3H7 | H |
| 861 | H | OCO-c-C3H5 | OCO-c-C3H5 | OC4H9 | H |
| 862 | H | OCO-c-C3H5 | OCO-c-C3H5 | OCH(CH3)2 | H |
| 863 | H | OCO-c-C3H5 | OCO-c-C3H5 | OC(CH3)3 | H |
| 864 | H | OCO-c-C3H5 | OCO-c-C3H5 | O-c-C3H5 | H |
| 865 | H | OCO-c-C3H5 | OCO-c-C3H5 | OCH2-c-C3H5 | H |
| 866 | H | OCO-c-C3H5 | OCO-c-C3H5 | OCH2C6H5 | H |
| 867 | H | OCO-c-C3H5 | OCH3 | OCO-c-C3H5 | H |
| 868 | H | OCO-c-C3H5 | OC2H5 | OCO-c-C3H5 | H |
| 869 | H | OCO-c-C3H5 | OC3H7 | OCO-c-C3H5 | H |
| 870 | H | OCO-c-C3H5 | OC4H9 | OCO-c-C3H5 | H |
| 871 | H | OCO-c-C3H5 | OCH(CH3)2 | OCO-c-C3H5 | H |
| 872 | H | OCO-c-C3H5 | OC(CH3)3 | OCO-c-C3H5 | H |
| 873 | H | OCO-c-C3H5 | O-c-C3H5 | OCO-c-C3H5 | H |
| 874 | H | OCO-c-C3H5 | OCH2-c-C3H5 | OCO-c-C3H5 | H |
| 875 | H | OCO-c-C3H5 | OCH2C6H5 | OCO-c-C3H5 | H |
| 876 | H | OCH3 | OCO-c-C3H5 | OCO-c-C3H5 | H |
| 877 | H | OC2H5 | OCO-c-C3H5 | OCO-c-C3H5 | H |

(continued)

| Table A | | | | | |
|---|---|---|---|---|---|
| | R3 | R4 | R5 | R6 | R7 |
| 878 | H | OC3H7 | OCO-c-C3H5 | OCO-c-C3H5 | H |
| 879 | H | OC4H9 | OCO-c-C3H5 | OCO-c-C3H5 | H |
| 880 | H | OCH(CH3)2 | OCO-c-C3H5 | OCO-c-C3H5 | H |
| 881 | H | OC(CH3)3 | OCO-c-C3H5 | OCO-c-C3H5 | H |
| 882 | H | O-c-C3H5 | OCO-c-C3H5 | OCO-c-C3H5 | H |
| 883 | H | OCH2-c-C3H5 | OCO-c-C3H5 | OCO-c-C3H5 | H |
| 884 | H | OCH2C6H5 | OCO-c-C3H5 | OCO-c-C3H5 | H |
| 885 | H | OCH3 | OCH3 | OCO-c-C3H5 | H |
| 886 | H | OC2H5 | OCH2CH3 | OCO-c-C3H5 | H |
| 887 | H | OC3H7 | OCH2CH2CH3 | OCO-c-C3H5 | H |
| 888 | H | OC4H9 | OCH2CH2CH2CH3 | OCO-c-C3H5 | H |
| 889 | H | OCH(CH3)2 | OCH(CH3)2 | OCO-c-C3H5 | H |
| 890 | H | OC(CH3)3 | OC(CH3)3 | OCO-c-C3H5 | H |
| 891 | H | O-c-C3H5 | O-c-C3H5 | OCO-c-C3H5 | H |
| 892 | H | OCH2-c-C3H5 | OCH2-c-C3H5 | OCO-c-C3H5 | H |
| 893 | H | OCH2C6H5 | OCH2C6H5 | OCO-c-C3H5 | H |
| 894 | H | OCO-c-C3H5 | OCH3 | OH | H |
| 895 | H | OCO-c-C3H5 | OC2H5 | OH | H |
| 896 | H | OCO-c-C3H5 | OC3H7 | OH | H |
| 897 | H | OCO-c-C3H5 | OC4H9 | OH | H |
| 898 | H | OCO-c-C3H5 | OCH(CH3)2 | OH | H |
| 899 | H | OCO-c-C3H5 | OC(CH3)3 | OH | H |
| 900 | H | OCO-c-C3H5 | O-c-C3H5 | OH | H |
| 901 | H | OCO-c-C3H5 | OCH2-c-C3H5 | OH | H |
| 902 | H | OCO-c-C3H5 | OCH2C6H5 | OH | H |
| 903 | H | OCO-c-C3H5 | OCH3 | H | H |
| 904 | H | OCO-c-C3H5 | OC2H5 | H | H |
| 905 | H | OCO-c-C3H5 | OC3H7 | H | H |
| 906 | H | OCO-c-C3H5 | OC4H9 | H | H |
| 907 | H | OCO-c-C3H5 | OCH(CH3)2 | H | H |
| 908 | H | OCO-c-C3H5 | OC(CH3)3 | H | H |
| 909 | H | OCO-c-C3H5 | O-c-C3H5 | H | H |
| 910 | H | OCO-c-C3H5 | OCH2-c-C3H5 | H | H |

[Table 23]

| Table A | | | | | |
|---|---|---|---|---|---|
| | R3 | R4 | R5 | R6 | R7 |
| 911 | H | OCO-c-C3H5 | OCH2C6H5 | H | H |
| 912 | H | OCO-c-C3H5 | OCH3 | =O | |
| 913 | H | OCO-c-C3H5 | OC2H5 | =O | |
| 914 | H | OCO-c-C3H5 | OC3H7 | =O | |
| 915 | H | OCO-c-C3H5 | OC4H9 | =O | |
| 916 | H | OCO-c-C3H5 | OCH(CH3)2 | =O | |
| 917 | H | OCO-c-C3H5 | OC(CH3)3 | =O | |
| 918 | H | OCO-c-C3H5 | O-c-C3H5 | =O | |
| 919 | H | OCO-c-C3H5 | OCH2-c-C3H5 | =O | |
| 920 | H | OCO-c-C3H5 | OCH2C6H5 | =O | |
| 921 | H | OCH3 | OCO-c-C3H5 | OH | H |
| 922 | H | OC2H5 | OCO-c-C3H5 | OH | H |
| 923 | H | OC3H7 | OCO-c-C3H5 | OH | H |
| 924 | H | OC4H9 | OCO-c-C3H5 | OH | H |
| 925 | H | OCH(CH3)2 | OCO-c-C3H5 | OH | H |
| 926 | H | OC(CH3)3 | OCO-c-C3H5 | OH | H |
| 927 | H | O-c-C3H5 | OCO-c-C3H5 | OH | H |
| 928 | H | OCH2-c-C3H5 | OCO-c-C3H5 | OH | H |
| 929 | H | OCH2C6H5 | OCO-c-C3H5 | OH | H |
| 930 | H | OCH3 | OCO-c-C3H5 | H | H |
| 931 | H | OC2H5 | OCO-c-C3H5 | H | H |
| 932 | H | OC3H7 | OCO-c-C3H5 | H | H |
| 933 | H | OC4H9 | OCO-c-C3H5 | H | H |
| 934 | H | OCH(CH3)2 | OCO-c-C3H5 | H | H |
| 935 | H | OC(CH3)3 | OCO-c-C3H5 | H | H |
| 936 | H | O-c-C3H5 | OCO-c-C3H5 | H | H |
| 937 | H | OCH2-c-C3H5 | OCO-c-C3H5 | H | H |
| 938 | H | OCH2C6H5 | OCO-c-C3H5 | H | H |
| 939 | H | OCH3 | OCO-c-C3H5 | =O | |
| 940 | H | OC2H5 | OCO-c-C3H5 | =O | |
| 941 | H | OC3H7 | OCO-c-C3H5 | =O | |
| 942 | H | OC4H9 | OCO-c-C3H5 | =O | |
| 943 | H | OCH(CH3)2 | OCO-c-C3H5 | =O | |
| 944 | H | OC(CH3)3 | OCO-c-C3H5 | =O | |
| 945 | H | O-c-C3H5 | OCO-c-C3H5 | =O | |
| 946 | H | OCH2-c-C3H5 | OCO-c-C3H5 | =O | |
| 947 | H | OCH2C6H5 | OCO-c-C3H5 | =O | |

(continued)

| Table A | | | | | |
|---|---|---|---|---|---|
| | R3 | R4 | R5 | R6 | R7 |
| 948 | H | OH | OCO-c-C3H5 | OH | H |
| 949 | H | OH | OCO-c-C3H5 | H | H |
| 950 | H | OH | OCO-c-C3H5 | =O | |
| 951 | H | OCO-c-C3H5 | OH | OH | H |
| 952 | H | OCO-c-C3H5 | OH | H | H |
| 953 | H | OCO-c-C3H5 | OH | =O | |
| 954 | H | OCH3 | OCH3 | OCH3 | H |
| 955 | H | OC2H5 | OC2H5 | OC2H5 | H |
| 956 | H | OC3H7 | OC3H7 | OC3H7 | H |
| 957 | H | OC4H9 | OC4H9 | OC4H9 | H |
| 958 | H | OCH(CH3)2 | OCH(CH3)2 | OCH(CH3)2 | H |
| 959 | H | OC(CH3)3 | OC(CH3)3 | OC(CH3)3 | H |
| 960 | H | O-c-C3H5 | O-c-C3H5 | O-c-C3H5 | H |
| 961 | H | OCH2-c-C3H5 | OCH2-c-C3H5 | OCH2-c-C3H5 | H |
| 962 | H | OCH2C6H5 | OCH2C6H5 | OCH2C6H5 | H |
| 963 | H | OCH3 | OCH3 | OH | H |
| 964 | H | OC2H5 | OC2H5 | OH | H |
| 965 | H | OC3H7 | OC3H7 | OH | H |
| 966 | H | OC4H9 | OC4H9 | OH | H |
| 967 | H | OCH(CH3)2 | OCH(CH3)2 | OH | H |
| 968 | H | OC(CH3)3 | OC(CH3)3 | OH | H |
| 969 | H | O-c-C3H5 | O-c-C3H5 | OH | H |
| 970 | H | OCH2-c-C3H5 | OCH2-c-C3H5 | OH | H |
| 971 | H | OCH2C6H5 | OCH2C6H5 | OH | H |
| 972 | H | OCH3 | OCH3 | H | H |
| 973 | H | OC2H5 | OC2H5 | H | H |
| 974 | H | OC2H5 | OC2H5 | H | H |
| 975 | H | OC3H7 | OC3H7 | H | H |
| 976 | H | OC4H9 | OC4H9 | H | H |
| 977 | H | OCH(CH3)2 | OCH(CH3)2 | H | H |
| 978 | H | OC(CH3)3 | OC(CH3)3 | H | H |
| 979 | H | O-c-C3H5 | O-c-C3H5 | H | H |
| 980 | H | OCH2-c-C3H5 | OCH2-c-C3H5 | H | H |

[Table 24]

| Table A | | | | | |
|---|---|---|---|---|---|
| | R3 | R4 | R5 | R6 | R7 |
| 981 | H | OCH2C6H5 | OCH2C6H5 | H | H |
| 982 | H | OCH3 | OCH3 | =O | |
| 983 | H | OC2H5 | OC2H5 | =O | |
| 984 | H | OC3H7 | OC3H7 | =O | |
| 985 | H | OC4H9 | OC4H9 | =O | |
| 986 | H | OCH(CH3)2 | OCH(CH3)2 | =O | |
| 987 | H | OC(CH3)3 | OC(CH3)3 | =O | |
| 988 | H | O-c-C3H5 | O-c-C3H5 | =O | |
| 989 | H | OCH2-c-C3H5 | OCH2-c-C3H5 | =O | |
| 990 | H | OCH2C6H5 | OCH2C6H5 | =O | |
| 991 | H | OH | OCH3 | OH | H |
| 992 | H | OH | OC2H5 | OH | H |
| 993 | H | OH | OC3H7 | OH | H |
| 994 | H | OH | OC4H9 | OH | H |
| 995 | H | OH | OCH(CH3)2 | OH | H |
| 996 | H | OH | OC(CH3)3 | OH | H |
| 997 | H | OH | O-c-C3H5 | OH | H |
| 998 | H | OH | OCH2-c-C3H5 | OH | H |
| 999 | H | OH | OCH2C6H5 | OH | H |
| 1000 | H | OCH3 | OH | OH | H |
| 1001 | H | OC2H5 | OH | OH | H |
| 1002 | H | OC3H7 | OH | OH | H |
| 1003 | H | OC4H9 | OH | OH | H |
| 1004 | H | OCH(CH3)2 | OH | OH | H |
| 1005 | H | OC(CH3)3 | OH | OH | H |
| 1006 | H | O-c-C3H5 | OH | OH | H |
| 1007 | H | OCH2-c-C3H5 | OH | OH | H |
| 1008 | H | OCH2C6H5 | OH | OH | H |
| 1009 | H | OH | OCH3 | H | H |
| 1010 | H | OH | OC2H5 | H | H |
| 1011 | H | OH | OC3H7 | H | H |
| 1012 | H | OH | OC4H9 | H | H |
| 1013 | H | OH | OCH(CH3)2 | H | H |
| 1014 | H | OH | OC(CH3)3 | H | H |
| 1015 | H | OH | O-c-C3H5 | H | H |
| 1016 | H | OH | OCH2-c-C3H5 | H | H |
| 1017 | H | OH | OCH2C6H5 | H | H |

(continued)

| Table A | | | | | |
|---|---|---|---|---|---|
| | R3 | R4 | R5 | R6 | R7 |
| 1018 | H | OCH3 | OH | H | H |
| 1019 | H | OC2H5 | OH | H | H |
| 1020 | H | OC3H7 | OH | H | H |
| 1021 | H | OC4H9 | OH | H | H |
| 1022 | H | OCH(CH3)2 | OH | H | H |
| 1023 | H | OC(CH3)3 | OH | H | H |
| 1024 | H | O-c-C3H5 | OH | H | H |
| 1025 | H | OCH2-c-C3H5 | OH | H | H |
| 1026 | H | OCH2C6H5 | OH | H | H |
| 1027 | H | SC6H5 | SC6H5 | SC6H5 | H |
| 1028 | H | S-(3-pyridyl) | S-(3-pyridyl) | S-(3-pyridyl) | H |
| 1029 | H | CH3 | CH3 | CH3 | H |
| 1030 | H | OCH2CHCH2 | OCH2CHCH2 | OCH2CHCH2 | H |
| 1031 | H | OCH2CCH | OCH2CCH | OCH2CCH | H |
| 1032 | H | OCONHCH3 | OCONHCH3 | OCONHCH3 | H |
| 1033 | H | OH | OCONHCH3 | OCONHCH3 | H |
| 1034 | H | OCONHCH3 | OH | OCONHCH3 | H |
| 1035 | H | OCONHCH3 | OCONHCH3 | OH | H |
| 1036 | H | OH | OCONHCH3 | OH | H |
| 1037 | H | OCONHCH3 | OH | OH | H |
| 1038 | H | OCONHCH3 | OCONHCH3 | =O | |
| 1039 | H | OCONHCH3 | OCONHCH3 | H | H |
| 1040 | H | OCONHCH3 | OH | H | H |
| 1041 | H | OH | OCONHCH3 | H | H |
| 1042 | H | OCONHCH3 | H | H | H |
| 1043 | H | OCONHC2H5 | OCONHC2H5 | OCONHC2H5 | H |
| 1044 | H | OH | OCONHC2H5 | OCONHC2H5 | H |
| 1045 | H | OCONHC2H5 | OH | OCONHC2H5 | H |
| 1046 | H | OCONHC2H5 | OCONHC2H5 | OH | H |
| 1047 | H | OH | OCONHC2H5 | OH | H |
| 1048 | H | OCONHC2H5 | OH | OH | H |
| 1049 | H | OCONHC2H5 | OCONHC2H5 | =O | |
| 1050 | H | OCONHC2H5 | OCONHC2H5 | H | H |

[Table 25]

| Table A | | | | | |
|---|---|---|---|---|---|
| | R3 | R4 | R5 | R6 | R7 |
| 1051 | H | OCONHC2H5 | OH | H | H |
| 1052 | H | OH | OCONHC2H5 | H | H |
| 1053 | H | OCONHC2H5 | H | H | H |
| 1054 | H | OCONHC3H7 | OCONHC3H7 | OCONHC3H7 | H |
| 1055 | H | OH | OCONHC3H7 | OCONHC3H7 | H |
| 1056 | H | OCONHC3H7 | OH | OCONHC3H7 | H |
| 1057 | H | OCONHC3H7 | OCONHC3H7 | OH | H |
| 1058 | H | OH | OCONHC3H7 | OH | H |
| 1059 | H | OCONHC3H7 | OH | OH | H |
| 1060 | H | OCONHC3H7 | OCONHC3H7 | =O | |
| 1061 | H | OCONHC3H7 | OCONHC3H7 | H | H |
| 1062 | H | OCONHC3H7 | OH | H | H |
| 1063 | H | OH | OCONHC3H7 | H | H |
| 1064 | H | OCONHC3H7 | H | H | H |
| 1065 | H | OCON(CH3)2 | OCON(CH3)2 | OCON(CH3)2 | H |
| 1066 | H | OH | OCON(CH3)2 | OCON(CH3)2 | H |
| 1067 | H | OCON(CH3)2 | OH | OCON(CH3)2 | H |
| 1068 | H | OCON(CH3)2 | OCON(CH3)2 | OH | H |
| 1069 | H | OH | OCON(CH3)2 | OH | H |
| 1070 | H | OCON(CH3)2 | OH | OH | H |
| 1071 | H | OCON(CH3)2 | OCON(CH3)2 | =O | |
| 1072 | H | OCON(CH3)2 | OCON(CH3)2 | H | H |
| 1073 | H | OCON(CH3)2 | OH | H | H |
| 1074 | H | OH | OCON(CH3)2 | H | H |
| 1075 | H | OCON(CH3)2 | H | H | H |
| 1076 | H | OCON(C2H5)2 | OCON(C2H5)2 | OCON(C2H5)2 | H |
| 1077 | H | OH | OCON(CH3)2 | OCON(C2H5)2 | H |
| 1078 | H | OCON(C2H5)2 | OH | OCON(C2H5)2 | H |
| 1079 | H | OCON(C2H5)2 | OCON(C2H5)2 | OH | H |
| 1080 | H | OH | OCON(C2H5)2 | OH | H |
| 1081 | H | OCON(C2H5)2 | OH | OH | H |
| 1082 | H | OCON(C2H5)2 | OCON(C2H5)2 | =O | |
| 1083 | H | OCON(C2H5)2 | OCON(C2H5)2 | H | H |
| 1084 | H | OCON(C2H5)2 | OH | H | H |
| 1085 | H | OH | OCON(C2H5)2 | H | H |
| 1086 | H | OCON(C2H5)2 | H | H | H |
| 1087 | H | CONHCH3 | CONHCH3 | CONHCH3 | H |

(continued)

| Table A | | | | | |
|---|---|---|---|---|---|
| | R3 | R4 | R5 | R6 | R7 |
| 1088 | H | OCONHC6H5 | OCONHC6H5 | OCONHC6H5 | H |
| 1089 | H | OCOOCH3 | OCOOCH3 | OCOOCH3 | H |
| 1090 | H | OCOCH3 | OCOCH3 | OCOOC6H5 | H |
| 1091 | H | OCOCH3 | OCOCH3 | OCOO(p-Cl-C6H4) | H |
| 1092 | H | OH | OCOOCH3 | OCOOCH3 | H |
| 1093 | H | OCOOCH3 | OH | OCOOCH3 | H |
| 1094 | H | OCOOCH3 | OCOOCH3 | OH | H |
| 1095 | H | OH | OCOOCH3 | OH | H |
| 1096 | H | OCOOCH3 | OH | OH | H |
| 1097 | H | OCOOCH3 | OCOOCH3 | =O | |
| 1098 | H | OCOOCH3 | OCOOCH3 | H | H |
| 1099 | H | OCOOCH3 | OH | H | H |
| 1100 | H | OH | OCOOCH3 | H | H |
| 1101 | H | OCOOCH3 | H | H | H |
| 1102 | H | OCOOC2H5 | OCOOC2H5 | OCOOC2H5 | H |
| 1103 | H | OH | OCOOC2H5 | OCOOC2H5 | H |
| 1104 | H | OCOOC2H5 | OH | OCOOC2H5 | H |
| 1105 | H | OCOOC2H5 | OCOOC2H5 | OH | H |
| 1106 | H | OH | OCOOC2H5 | OH | H |
| 1107 | H | OCOOC2H5 | OH | OH | H |
| 1108 | H | OCOOC2H5 | OCOOC2H5 | =O | |
| 1109 | H | OCOOC2H5 | OCOOC2H5 | H | H |
| 1110 | H | OCOOC2H5 | OH | H | H |
| 1111 | H | OH | OCOOC2H5 | H | H |
| 1112 | H | OCOOC2H5 | H | H | H |
| 1113 | H | OCOOCH(CH3)2 | OCOOCH(CH3)2 | OCOOCH(CH3)2 | H |
| 1114 | H | OH | OCOOCH(CH3)2 | OCOOCH(CH3)2 | H |
| 1115 | H | OCOOCH(CH3)2 | OH | OCOOCH(CH3)2 | H |
| 1116 | H | OCOOCH(CH3)2 | OCOOCH(CH3)2 | OH | H |
| 1117 | H | OH | OCOOCH(CH3)2 | OH | H |
| 1118 | H | OCOOCH(CH3)2 | OH | OH | H |
| 1119 | H | OCOOCH(CH3)2 | OCOOCH(CH3)2 | =O | |
| 1120 | H | OCOOCH(CH3)2 | OCOOCH(CH3)2 | H | H |

[Table 26]

| Table A | | | | | |
|---|---|---|---|---|---|
| | R3 | R4 | R5 | R6 | R7 |
| 1121 | H | OCOOCH(CH3)2 | OH | H | H |
| 1122 | H | OH | OCOOCH(CH3)2 | H | H |
| 1123 | H | OCOOCH(CH3)2 | H | H | H |
| 1124 | H | OCOOC(CH3)3 | OCOOC(CH3)3 | OCOOC(CH3)3 | H |
| 1125 | H | OH | OCOOC(CH3)3 | OCOOC(CH3)3 | H |
| 1126 | H | OCOOC(CH3)3 | OH | OCOOC(CH3)3 | H |
| 1127 | H | OCOOC(CH3)3 | OCOOC(CH3)3 | OH | H |
| 1128 | H | OH | OCOOC(CH3)3 | OH | H |
| 1129 | H | OCOOC(CH3)3 | OH | OH | H |
| 1130 | H | OCOOC(CH3)3 | OCOOCH(CH3)2 | =O | |
| 1131 | H | OCOOC(CH3)3 | OCOOCH(CH3)2 | H | H |
| 1132 | H | OCOOC(CH3)3 | OH | H | H |
| 1133 | H | OH | OCOOC(CH3)3 | H | H |
| 1134 | H | OCOOC(CH3)3 | H | H | H |
| 1135 | H | OCOO-c-C3H5 | OCOO-c-C3H5 | OCOO-c-C3H5 | H |
| 1136 | H | OH | OCOO-c-C3H5 | OCOO-c-C3H5 | H |
| 1137 | H | OCOO-c-C3H5 | OH | OCOO-c-C3H5 | H |
| 1138 | H | OCOO-c-C3H5 | OCOO-c-C3H5 | OH | H |
| 1139 | H | OH | OCOO-c-C3H5 | OH | H |
| 1140 | H | OCOO-c-C3H5 | OH | OH | H |
| 1141 | H | OCOO-c-C3H5 | OCOO-c-C3H5 | =O | |
| 1142 | H | OCOO-c-C3H5 | OCOO-c-C3H5 | H | H |
| 1143 | H | OCOO-c-C3H5 | OH | H | H |
| 1144 | H | OH | OCOO-c-C3H5 | H | H |
| 1145 | H | OCOO-c-C3H5 | H | H | H |
| 1146 | H | OCOOC6H5 | OCOOC6H5 | OCOOC6H5 | H |
| 1147 | H | OH | OCOOC6H5 | OCOOC6H5 | H |
| 1148 | H | OCOOC6H5 | OH | OCOOC6H5 | H |
| 1149 | H | OCOOC6H5 | OCOOC6H5 | OH | H |
| 1150 | H | OH | OCOOC6H5 | OH | H |
| 1151 | H | OCOOC6H5 | OH | OH | H |
| 1152 | H | OCOOC6H5 | OCOOC6H5 | =O | |
| 1153 | H | OCOOC6H5 | OCOOC6H5 | H | H |
| 1154 | H | OCOOC6H5 | OH | H | H |
| 1155 | H | OH | OCOOC6H5 | H | H |
| 1156 | H | OCOOC6H5 | H | H | H |
| 1157 | H | OCOS-(3-pyridyl) | OCOS-(3-pyridyl) | OCOS-(3-pyridyl) | H |

(continued)

| Table A | | | | | |
|---|---|---|---|---|---|
| | R3 | R4 | R5 | R6 | R7 |
| 1158 | H | OSO2C6H5 | OSO2C6H5 | OSO2C6H5 | H |
| 1159 | H | OSO2C6H5 | OSO2C6H5 | OH | H |
| 1180 | H | OSO2C6H5 | OSO2C6H5 | =O | |
| 1161 | H | OSO2C6H5 | OSO2C6H5 | H | H |
| 1162 | H | OH | OSO2C6H5 | OSO2C6H5 | H |
| 1163 | H | OSO2C6H5 | OH | OSO2C6H5 | H |
| 1164 | H | OH | OSO2C6H5 | OH | H |
| 1165 | H | OH | OH | OSO2C6H5 | H |
| 1166 | H | OSO2C6H5 | OH | OH | H |
| 1167 | H | OSO2C6H5 | OH | H | H |
| 1168 | H | OH | OSO2C6H5 | H | H |
| 1169 | H | OSO2C6H5 | H | H | H |
| 1170 | H | OSO2CH3 | OSO2CH3 | OS02CH3 | H |
| 1171 | H | OSO2CH3 | OSO2CH3 | OH | H |
| 1172 | H | OSO2CH3 | OSO2CH3 | =O | |
| 1173 | H | OSO2CH3 | OSO2CH3 | H | H |
| 1174 | H | OH | OSO2CH3 | OSO2CH3 | H |
| 1175 | H | OSO2CH3 | OH | OSO2CH3 | H |
| 1176 | H | OH | OSO2CH3 | OH | H |
| 1177 | H | OH | OH | OSO2CH3 | H |
| 1178 | H | OSO2CH3 | OH | OH | H |
| 1179 | H | OSO2CH3 | OH | H | H |
| 1180 | H | OH | OSO2CH3 | H | H |
| 1181 | H | OSO2CH3 | H | H | H |
| 1182 | H | OSO2C2H5 | OSO2C2H5 | OSO2C2H5 | H |
| 1183 | H | OS02C2H5 | OSO2C2H5 | OH | H |
| 1184 | H | OSO2C2H5 | OSO2C2H5 | =O | |
| 1185 | H | OSO2C2H5 | OSO2C2H5 | H | H |
| 1186 | H | OH | OSO2C2H5 | OSO2C2H5 | H |
| 1187 | H | OSO2C2H5 | OH | OSO2C2H5 | H |
| 1188 | H | OH | OSO2C2H5 | OH | H |
| 1189 | H | OH | OH | OSO2C2H5 | H |
| 1190 | H | OSO2C2H5 | OH | OH | H |

[Table 27]

| Table A | | | | | |
|---|---|---|---|---|---|
| | R3 | R4 | R5 | R6 | R7 |
| 1191 | H | OSO2C2H5 | OH | H | H |
| 1192 | H | OH | OSO2C2H5 | H | H |
| 1193 | H | OSO2C2H5 | H | H | H |
| 1194 | H | OSO2-c-C3H5 | OSO2-c-C3H5 | OSO2-c-C3H5 | H |
| 1195 | H | OSO2-c-C3H5 | OSO2-c-C3H5 | OSO2CH3 | H |
| 1196 | H | OSO2-c-C3H5 | OSO2-c-C3H5 | OSO2C2H5 | H |
| 1197 | H | OSO2-c-C3H5 | OSO2-c-C3H5 | OH | H |
| 1198 | H | OSO2-c-C3H5 | OSO2-c-C3H5 | H | H |
| 1199 | H | OSO2-c-C3H5 | OSO2-c-C3H5 | =O | |
| 1200 | H | OSO2-c-C3H5 | OSO2-c-C3H5 | OCOCH3 | H |
| 1201 | H | OSO2-c-C3H5 | OSO2-c-C3H5 | OCOC2H5 | H |
| 1202 | H | OS02-c-C3H5 | OSO2-c-C3H5 | OCO-c-C3H5 | H |
| 1203 | H | OSO2-c-C3H5 | OSO2CH3 | OH | H |
| 1204 | H | OSO2-c-C3H5 | OSO2C2H5 | OH | H |
| 1205 | H | OSO2-c-C3H5 | OCOCH3 | OH | H |
| 1206 | H | OSO2-c-C3H5 | OCOC2H5 | OH | H |
| 1207 | H | OSO2CH3 | OSO2-c-C3H5 | OH | H |
| 1208 | H | OSO2C2H5 | OSO2-c-C3H5 | OH | H |
| 1209 | H | OCOCH3 | OSO2-c-C3H5 | OH | H |
| 1210 | H | OCOC2H5 | OSO2-c-C3H5 | OH | H |
| 1211 | H | OSO2-c-C3H5 | OSO2CH3 | H | H |
| 1212 | H | OSO2-c-C3H5 | OSO2C2H5 | H | H |
| 1213 | H | OSO2-c-C3H5 | OCOCH3 | H | H |
| 1214 | H | OSO2-c-C3H5 | OCOC2H5 | H | H |
| 1215 | H | OSO2CH3 | OSO2-c-C3H5 | H | H |
| 1216 | H | OS02C2H5 | OSO2-c-C3H5 | H | H |
| 1217 | H | OCOCH3 | OSO2-c-C3H5 | H | H |
| 1218 | H | OCOC2H5 | OSO2-c-C3H5 | H | H |
| 1219 | H | OSO2-c-C3H5 | OSO2CH3 | =O | |
| 1220 | H | OSO2-c-C3H5 | OSO2C2H5 | =O | |
| 1221 | H | OSO2-c-C3H5 | OCOCH3 | =O | |
| 1222 | H | OSO2-c-C3H5 | OCOC2H5 | =O | |
| 1223 | H | OSO2-c-C3H5 | OCO-c-C3H5 | =O | |
| 1224 | H | OSO2CH3 | OSO2-c-C3H5 | =O | |
| 1225 | H | OSO2C2H5 | OSO2-c-C3H5 | =O | |
| 1226 | H | OCOCH3 | OSO2-c-C3H5 | =O | |

(continued)

| Table A | | | | | |
|---|---|---|---|---|---|
| | R3 | R4 | R5 | R6 | R7 |
| 1227 | H | OCOC2H5 | OSO2-c-C3H5 | =O | |
| 1228 | H | OH | OSO2-c-C3H5 | OH | H |
| 1229 | H | OSO2-c-C3H5 | OH | OH | H |
| 1230 | H | OH | OSO2-c-C3H5 | H | H |
| 1231 | H | OSO2-c-C3H5 | OH | H | H |
| 1232 | H | OH | OSO2-c-C3H5 | =O | |
| 1233 | H | OSO2-c-C3H5 | OH | =O | |
| 1234 | H | OSO2-c-C3H5 | H | H | H |
| 1235 | H | S02CH3 | S02CH3 | S02CH3 | H |
| 1236 | H | SO2C6H5 | SO2C6H5 | SO2C6H5 | H |
| 1237 | H | OSi(CH3)3 | OSi(CH3)3 | OSi(CH3)3 | H |
| 1238 | H | OSi(CH3)3 | OSi(CH3)3 | OH | H |
| 1239 | H | OH | OSi(CH3)3 | OH | H |
| 1240 | H | OSi(CH3)3 | OH | OH | H |
| 1241 | H | OSi(CH3)3 | OSi(CH3)3 | H | H |
| 1242 | H | OH | OSi(CH3)3 | H | H |
| 1243 | H | OSi(CH3)3 | OH | H | H |
| 1244 | H | OSiC(CH3)2C(CH3)3 | OSiC(CH3)2C(CH3)3 | OSiC(CH3)2C(CH3)3 | H |
| 1245 | H | OSiC(CH3)2C(CH3)3 | OSiC(CH3)2C(CH3)3 | OH | H |
| 1246 | H | OH | OSiC(CH3)2C(CH3)3 | OH | H |
| 1247 | H | OSiC(CH3)2C(CH3)3 | OH | OH | H |
| 1248 | H | OSiC(CH3)2C(CH3)3 | OSiC(CH3)2C(CH3)3 | H | H |
| 1249 | H | OH | OSiC(CH3)2C(CH3)3 | H | H |
| 1250 | H | OSiC(CH3)2C(CH3)3 | OH | H | H |
| 1251 | H | OCOCH3 | OCOCH3 | H | N3 |
| 1252 | H | OCOCH3 | OCOCH3 | H | NH2 |
| 1253 | H | OCOCH3 | OCOCH3 | H | NHCH3 |
| 1254 | H | OCOCH3 | OCOCH3 | H | OCOCH3 |
| 1255 | H | OCOCH3 | OCOCH3 | H | I |
| 1256 | H | OCOCH3 | OCOCH3 | H | Cl |
| 1257 | H | OCOCH3 | OCOCH3 | H | S-[1-(4-OCH3-C6H4) tetrazolyl) |

(continued)

| Table A | | | | | |
|---|---|---|---|---|---|
| | R3 | R4 | R5 | R6 | R7 |
| 1258 | H | OCO-c-C3H5 | OCO-c-C3H5 | H | OH |
| 1259 | H | OCO-c-C3H5 | OCO-c-C3H5 | H | OCOCH3 |
| 1260 | H | OCO-c-C3H5 | OCO-c-C3H5 | H | NH2 |

[Table 28]

| Table A | | | | | |
|---|---|---|---|---|---|
| | R3 | R4 | R5 | R6 | R7 |
| 1261 | H | OCO-c-C3H5 | OCO-c-C3H5 | H | NHCH3 |
| 1262 | H | OCO-c-C3H5 | OCO-c-C3H5 | H | Cl |
| 1263 | H | OCO-c-C3H5 | OCO-c-C3H5 | H | Br |
| 1264 | H | OCO-c-C3H5 | OCO-c-C3H5 | H | I |
| 1265 | H | OCO-c-C3H5 | OCO-c-C3H5 | H | CN |
| 1266 | H | OCO-c-C3H5 | OCO-c-C3H5 | NH2 | H |
| 1267 | H | OCO-c-C3H5 | OCO-c-C3H5 | NHCH3 | H |
| 1268 | H | OCO-c-C3H5 | OCO-c-C3H5 | Cl | H |
| 1269 | H | OCO-c-C3H5 | OCO-c-C3H5 | Br | H |
| 1270 | H | OCO-c-C3H5 | OCO-c-C3H5 | I | H |
| 1271 | H | OCO-c-C3H5 | OCO-c-C3H5 | CN | H |
| 1272 | N3 | H | OCO-c-C3H5 | OCO-c-C3H5 | H |
| 1273 | NH2 | H | OCO-c-C3H5 | OCO-c-C3H5 | H |
| 1274 | NHCH3 | H | OCO-c-C3H5 | OCO-c-C3H5 | H |
| 1275 | Cl | H | OCO-c-C3H5 | OCO-c-C3H5 | H |
| 1276 | Br | H | OCO-c-C3H5 | OCO-c-C3H5 | H |
| 1277 | I | H | OCO-c-C3H5 | OCO-c-C3H5 | H |
| 1278 | CN | H | OCO-c-C3H5 | OCO-c-C3H5 | H |
| 1279 | H | N3 | OCO-c-C3H5 | OCO-c-C3H5 | H |
| 1280 | H | NH2 | OCO-c-C3H5 | OCO-c-C3H5 | H |
| 1281 | H | NHCH3 | OCO-c-C3H5 | OCO-c-C3H5 | H |
| 1282 | H | Cl | OCO-c-C3H5 | OCO-c-C3H5 | H |
| 1283 | H | Br | OCO-c-C3H5 | OCO-c-C3H5 | H |
| 1284 | H | I | OCO-c-C3H5 | OCO-c-C3H5 | H |
| 1285 | H | CN | OCO-c-C3H5 | OCO-c-C3H5 | H |
| 1286 | H | OCO-c-C3H5 | N3 | OCO-c-C3H5 | H |
| 1287 | H | OCO-c-C3H5 | NH2 | OCO-c-C3H5 | H |
| 1288 | H | OCO-c-C3H5 | NHCH3 | OCO-c-C3H5 | H |
| 1289 | H | OCO-c-C3H5 | Cl | OCO-c-C3H5 | H |

(continued)

| Table A | | | | | |
|---|---|---|---|---|---|
| | R3 | R4 | R5 | R6 | R7 |
| 1290 | H | OCO-c-C3H5 | Br | OCO-c-C3H5 | H |
| 1291 | H | OCO-c-C3H5 | I | OCO-c-C3H5 | H |
| 1292 | H | OCO-c-C3H5 | CN | OCO-c-C3H5 | H |
| 1293 | N3 | H | OCO-c-C3H5 | OH | H |
| 1294 | Cl | H | OCO-c-C3H5 | OH | H |
| 1295 | Br | H | OCO-c-C3H5 | OH | H |
| 1296 | I | H | OCO-c-C3H5 | OH | H |
| 1297 | CN | H | OCO-c-C3H5 | OH | H |
| 1298 | H | N3 | OCO-c-C3H5 | OH | H |
| 1299 | H | NH2 | OCO-c-C3H5 | OH | H |
| 1300 | H | NHCH3 | OCO-c-C3H5 | OH | H |
| 1301 | H | Cl | OCO-c-C3H5 | OH | H |
| 1302 | H | Br | OCO-c-C3H5 | OH | H |
| 1303 | H | I | OCO-c-C3H5 | OH | H |
| 1304 | H | CN | OCO-c-C3H5 | OH | H |
| 1305 | H | OCO-c-C3H5 | N3 | OH | H |
| 1306 | H | OCO-c-C3H5 | NH2 | OH | H |
| 1307 | H | OCO-c-C3H5 | NHCH3 | OH | H |
| 1308 | H | OCO-c-C3H5 | Cl | OH | H |
| 1309 | H | OCO-c-C3H5 | Br | OH | H |
| 1310 | H | OCO-c-C3H5 | I | OH | H |
| 1311 | H | OCO-c-C3H5 | CN | OH | H |
| 1312 | N3 | H | OCO-c-C3H5 | =O | |
| 1313 | NH2 | H | OCO-c-C3H5 | =O | |
| 1314 | NHCH3 | H | OCO-c-C3H5 | =O | |
| 1315 | Cl | H | OCO-c-C3H5 | =O | |
| 1316 | Br | H | OCO-c-C3H5 | =O | |
| 1317 | I | H | OCO-c-C3H5 | =O | |
| 1318 | CN | H | OCO-c-C3H5 | =O | |
| 1319 | H | OCO-c-C3H5 | N3 | =O | |
| 1320 | H | OCO-c-C3H5 | Cl | =O | |
| 1321 | H | OCO-c-C3H5 | Br | =O | |
| 1322 | H | OCO-c-C3H5 | I | =O | |
| 1323 | H | OCO-c-C3H5 | CN | =O | |
| 1324 | N3 | H | OCO-c-C3H5 | H | H |
| 1325 | Cl | H | OCO-c-C3H5 | H | H |
| 1326 | Br | H | OCO-c-C3H5 | H | H |

(continued)

| Table A | | | | | |
|---|---|---|---|---|---|
| | R3 | R4 | R5 | R6 | R7 |
| 1327 | I | H | OCO-c-C3H5 | H | H |
| 1328 | CN | H | OCO-c-C3H5 | H | H |
| 1329 | H | OCO-c-C3H5 | N3 | H | H |
| 1330 | H | OCO-c-C3H5 | Cl | H | H |

[Table 29]

| Table A | | | | | |
|---|---|---|---|---|---|
| | R3 | R4 | R5 | R6 | R7 |
| 1331 | H | OCO-c-C3H5 | Br | H | H |
| 1332 | H | OCO-c-C3H5 | I | H | H |
| 1333 | H | OCO-c-C3H5 | CN | H | H |
| 1334 | H | OH | N3 | OH | H |
| 1335 | H | OH | Cl | OH | H |
| 1336 | H | OH | Br | OH | H |
| 1337 | H | OH | I | OH | H |
| 1338 | H | OH | CN | OH | H |
| 1339 | H | -O-CS-O- | | OH | H |
| 1340 | H | -O-CS-O- | | OCS-imidazole | H |
| 1341 | H | OCH2O | | OH | H |
| 1342 | H | O(C=CH2)O | | OH | H |
| 1343 | H | OC(CH3)2O | | H | H |
| 1344 | H | OC(CH3)2O | | OH | H |
| 1345 | H | OC(CH3)2O | | OCOCH3 | H |
| 1346 | H | OC(CH3)2O | | OCO-c-C3H5 | H |
| 1347 | H | -O-CO-O- | | OCOC4H9 | H |
| 1348 | H | -O-CH(CH3)-O- | | OH | H |
| 1349 | H | -O-CH(CH3)-O- | | OCO-c-C3H5 | H |
| 1350 | H | -O-CH(C6H5)-O- | | OH | H |
| 1351 | H | -O-CH(C6H5)-O- | | =O | |
| 1352 | H | -O-CH(C6H5)-O- | | OCOC2H5 | H |
| 1353 | H | -O-CH(C6H5)-O- | | OCO-c-C3H5 | H |
| 1354 | H | -OCH(C6H5)O- | | OCO-(o-CN-C6H4) | H |
| 1355 | H | -OCH(C6H5)O- | | OCO-(m-CN-C6H4) | H |
| 1356 | H | -OCH(C6H5)O- | | OCO-(p-CN-C6H4) | H |
| 1357 | H | -OCH(C6H5)O- | | OCO-(o-OCH3-C6H4) | H |
| 1358 | H | -OCH(C6H5)O- | | OCO-(m-OCH3-C6H4) | H |
| 1359 | H | -OCH(C6H5)O- | | OCO-(p-OCH3-C6H4) | H |

(continued)

| Table A | | | | | |
|---|---|---|---|---|---|
| | R3 | R4 | R5 | R6 | R7 |
| 1360 | H | -OCH(C6H5)O- | | OCO-(p-NO2-C6H4) | H |
| 1361 | H | -OCH(C6H5)O- | | OCO-(o-F-C6H4) | H |
| 1362 | H | -OCH(C6H5)O- | | OCO-(m-F-C6H4) | H |
| 1363 | H | -OCH(C6H5)O- | | OCO-(p-F-C6H4) | H |
| 1364 | H | -OCH(C6H5)O- | | OCO-(o-Cl-C6H4) | H |
| 1365 | H | -OCH(C6H5)O- | | OCO-(m-Cl-C6H4) | H |
| 1366 | H | -OCH(C6H5)O- | | OCO-(p-Cl-C6H4) | H |
| 1367 | H | -OCH(C6H5)O- | | OCO-(m-Br-C6H4) | H |
| 1368 | H | -OCH(C6H5)O- | | OCO-(p-Br-C6H4) | H |
| 1369 | H | -OCH(C6H5)O- | | OCO-(p-CHO-C6H4) | H |
| 1370 | H | -O-CH(p-CH3-C6H4)-O- | | OH | H |
| 1371 | H | -O-CH(o-F-C6H4)-O- | | OH | H |
| 1372 | H | -O-CH(p-F-C6H4)-O- | | OH | H |
| 1373 | H | -O-CH(o-CH3-C6H4)-O- | | OCOC4H9 | H |
| 1374 | H | -O-CH(m-F-C6H4)-O- | | OCOC4H9 | H |
| 1375 | H | -OCH(2-isopropyl)-O- | | OCOC4H9 | H |
| 1376 | H | -OCH(t-buthyl)-O- | | OH | H |
| 1377 | H | -OCH(OCH3)O- | | OH | H |
| 1378 | H | -OCH(CHCH2)O- | | OH | H |
| 1379 | H | -OCH(CH2C6H5)O- | | OH | H |
| 1380 | H | -O-Si(t-Bu)2-O- | | OCO-(p-CN-C6H4) | H |
| 1381 | H | OH | OSiC(CH3)2C(CH3)3 | OSiC(CH3)2C(CH3)3 | H |
| 1382 | H | OH | OSiC(CH3)2C(CH3)3 | =O | |
| 1383 | H | OH | OSiC(CH3)2C(CH3)3 | H | H |
| 1384 | H | OCO-c-C3H5 | OSiC(CH3)2C(CH3)3 | OSiC(CH3)2C(CH3)3 | H |
| 1385 | H | OCO-c-C3H5 | OSiC(CH3)2C(CH3)3 | OCO-c-C3H5 | H |
| 1386 | H | OCO-c-C3H5 | OSiC(CH3)2C(CH3)3 | =O | |
| 1387 | H | OSO2CH3 | OSO2CH3 | OCO-c-C3H5 | H |
| 1388 | H | OSO2C2H5 | OSO2C2H5 | OCO-c-C3H5 | H |
| 1389 | H | OH | OH | OSiC(CH3)2C(CH3)3 | H |
| 1390 | H | OC(CH3)2O | | OSiC(CH3)2C(CH3)3 | H |
| 1391 | =O | | OCO-c-C3H5 | OCO-c-C3H5 | H |
| 1392 | =O-N-O-CH3 | | OCO-c-O3H5 | OH | H |
| 1393 | =C-CN | | OCO-c-C3H5 | OH | H |
| 1394 | =N-NH-Ph | | OCO-c-C3H5 | OH | H |
| 1395 | =N-NH-CH3 | | OCO-c-C3H5 | OH | H |
| 1396 | =N-N-CS-N-CH3 | | OCO-c-C3H5 | OH | H |

(continued)

| Table A | | | | | |
|---|---|---|---|---|---|
| | R3 | R4 | R5 | R6 | R7 |
| 1397 | =N-N-CO-N-Ph | | OCO-c-C3H5 | OH | H |
| 1398 | -O-CH2-CH2-O- | | OCO-c-C3H5 | OH | H |
| 1399 | H | OCO-c-C3H5 | =O | OCO-c-C3H5 | H |
| 1400 | H | OCO-c-C3H5 | =O | OH | H |

[Table 30]

| Table A | | | | | |
|---|---|---|---|---|---|
| | R3 | R4 | R5 | R6 | R7 |
| 1401 | H | OCOCH3 | =O | OCOCH3 | H |
| 1402 | H | OCOCH3 | =O | OH | H |
| 1403 | H | OCO-c-C3H5 | =O-N-O-CH3 | OCO-c-C3H5 | H |
| 1404 | H | OCO-c-C3H5 | =O-N-O-CH3 | OH | H |
| 1405 | H | OCOCH3 | =O-N-O-CH3 | OCOCH3 | H |
| 1406 | H | OCOCH3 | =O-N-O-CH3 | OH | H |
| 1407 | H | OCO-c-C3H5 | =C-CN | OCO-c-C3H5 | H |
| 1408 | H | OCO-c-C3H5 | =C-CN | OH | H |
| 1409 | H | OCOCH3 | =C-CN | OCOCH3 | H |
| 1410 | H | OCOCH3 | =C-CN | OH | H |
| 1411 | H | OCO-c-C3H5 | OCO-c-C3H5 | =O-N-O-CH3 | |
| 1412 | H | OCO-c-C3H5 | OCO-c-C3H5 | =C-CN | |
| 1413 | H | OCO-c-C3H5 | OCO-c-C3H5 | =H-NH-Ph | |
| 1414 | H | OCO-c-C3H5 | OCO-c-C3H5 | =N-NH-CH3 | |
| 1415 | H | OCO-c-C3H5 | OCO-c-C3H5 | =N-N-CS-N-CH3 | |
| 1416 | H | OCO-c-C3H5 | OCO-c-C3H5 | =N-N-CO-N-Ph | |
| 1417 | H | OCOCH3 | OCOCH3 | =O-N-O-CH3 | |
| 1418 | H | OCOCH3 | OCOCH3 | =C-CN | |
| 1419 | H | OCOCH3 | OCOCH3 | =N-NH-Ph | |
| 1420 | H | OCOCH3 | OCOCH3 | =N-NH-CH3 | |
| 1421 | H | OCOCH3 | OCOCH3 | =N-N-CS-N-CH3 | |
| 1422 | H | OCOCH3 | OCOCH3 | =N-N-CO-N-Ph | |
| 1423 | H | OH | OCO-c-C3H5 | =O-N-O-CH3 | |
| 1424 | H | OH | OCO-c-C3H5 | =C-CN | |
| 1425 | H | OH | OCO-c-C3H5 | =N-NH-Ph | |
| 1426 | H | OH | OCO-c-C3H5 | =N-NH-CH3 | |
| 1427 | H | OH | OCO-c-C3H5 | =N-N-CS-N-CH3 | |
| 1428 | H | OH | OCO-c-C3H5 | =N-N-CO-N-Ph | |
| 1429 | H | OH | OCOCH3 | =ON-OCH3 | |

(continued)

| Table A | | | | | |
|---|---|---|---|---|---|
| | R3 | R4 | R5 | R6 | R7 |
| 1430 | H | OH | OCOCH3 | =C-CN | |
| 1431 | H | OH | OCOCH3 | =N-NH-Ph | |
| 1432 | H | OH | OCOCH3 | =N-NH-CH3 | |
| 1433 | H | OH | OCOCH3 | =N-N-CS-N-CH3 | |
| 1434 | H | OH | OCOCH3 | =N-N-CO-N-Ph | |
| 1435 | H | OCO-(3-pyridyl) | OH | OH | H |
| 1436 | H | OH | OCO-(3-pyridyl) | OH | H |
| 1437 | H | OH | OCO-(3-pyridyl) | OCO-(3-pyridyl) | H |
| 1438 | H | OCO-(3-pyridyl) | OCO-(3-pyridyl) | OH | H |
| 1439 | H | OCO-(2-pyridyl) | OH | OH | H |
| 1440 | H | OH | OCO-(2-pyridyl) | OH | H |
| 1441 | H | OH | OCO-(2-pyridyl) | OCO-(2-pyridyl) | H |
| 1442 | H | OCO-(2-pyridyl) | OCO-(2-pyridyl) | OH | H |

Specific examples of compounds represented by formula (I-b) include compounds shown in Table 31 below.

[Table 31]

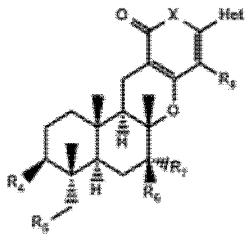

| Compound No. | | R4 | R5 | R6 | R7 | R8 | X | Hetero |
|---|---|---|---|---|---|---|---|---|
| 43- | 1 | OH | OH | OH | H | H | O | 3-pyridyl |
| 43- | 4 | OH | OH | H | H | H | O | 3-pyridyl |
| 43- | 5 | OH | H | H | H | H | O | 3-pyridyl |
| 43- | 260 | OCO-c-C3H5 | OCO-c-C3H5 | OH | H | H | O | 3-pyridyl |
| 43- | 262 | OCO-c-C3H5 | OCO-c-C3H5 | H | H | H | O | 3-pyridyl |
| 43- | 283 | OCO-c-C3H5 | OCO-c-C3H5 | H(=) | | H | O | 3-pyridyl |
| 43- | 259 | OCO-c-C3H5 | OCO-c-C3H5 | OCO-c-C3H5 | H | H | O | 3-pyridyl |
| 43- | 699 | OCO-c-C3H5 | OH | OCO-c-C3H5 | H | H | O | 3-pyridyl |
| 43- | 700 | OH | OCO-c-C3H5 | OCO-c-C3H5 | H | H | O | 3-pyridyl |
| 43- | 701 | OCO-c-C3H5 | OH | OH | H | H | O | 3-pyridyl |
| 43- | 703 | OH | OH | OCO-c-C3H5 | H | H | O | 3-pyridyl |
| 43- | 705 | OH | OCO-c-C3H5 | H | H | H | O | 3-pyridyl |
| 43- | 709 | OCO-c-C3H5 | H | OCO-c-C3H5 | H | H | O | 3-pyridyl |
| 43- | 713 | OCO-c-C3H5 | H | H | H | H | O | 3-pyridyl |
| 43- | 742 | OCO-c-C3H(CH3)4 | OCO-c-C3H(CH3)4 | OCO-c-C3H(CH3)4 | H | H | O | 3-pyridyl |
| 43- | 743 | OH | OCO-c-C3H(CH3)4 | OCO-c-C3H(CH3)4 | H | H | O | 3-pyridyl |
| 44- | 1 | OH | OH | OH | H | H | O | 6-Cl-3-pyridyl |
| 44- | 4 | OH | OH | H | H | H | O | 6-Cl-3-pyridyl |
| 44- | 5 | OH | H | H | H | H | O | 6-Cl-3-pyridyl |

(continued)

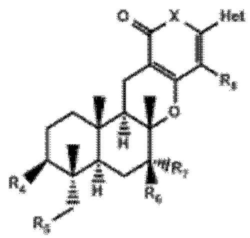

| Compound No. | | R4 | R5 | R6 | R7 | R8 | X | Hetero |
|---|---|---|---|---|---|---|---|---|
| 44- | 341 | OCO-c-C3H5 | OCO-c-C3H5 | OCO-c-C3H5 | H | H | O | 6-Cl-3-pyridyl |
| 44- | 705 | OH | OCO-c-C3H5 | H | H | H | O | 6-Cl-3-pyridyl |
| 44- | 713 | OCO-c-C3H5 | H | H | H | H | O | 6-Cl-3-pyridyl |
| 44- | 260 | OCO-c-C3H5 | OCO-c-C3H5 | OH | H | H | O | 6-Cl-3-pyridyl |
| 44- | 262 | OCO-c-C3H5 | OCO-c-C3H5 | H | H | H | O | 6-Cl-3-pyridyl |
| 45- | 1 | OH | OH | OH | H | H | O | 4-CF3-3-pyridyl |
| 45- | 4 | OH | OH | H | H | H | O | 4-CF3-3-pyridyl |
| 45- | 5 | OH | H | H | H | H | O | 4-CF3-3-pyridyl |
| 45- | 341 | OCO-c-C3H5 | OCO-c-C3H5 | OCO-c-C3H5 | H | H | O | 4-CF3-3-pyridyl |
| 45- | 705 | OH | OCO-c-C3H5 | H | H | H | O | 4-CF3-3-pyridyl |
| 45- | 713 | OCO-c-C3H5 | H | H | H | H | O | 4-CF3-3-pyridyl |
| 45- | 260 | OCO-c-C3H5 | OCO-c-C3H5 | OH | H | H | O | 4-CF3-3-pyridyl |
| 45- | 262 | OCO-c-C3H5 | OCO-c-C3H5 | H | H | H | O | 4-CF3-3-pyridyl |
| 46- | 1 | OH | OH | OH | H | H | O | 2-pyridyl |
| 46- | 4 | OH | OH | H | H | H | O | 2-pyridyl |
| 46- | 5 | OH | H | H | H | H | O | 2-pyridyl |
| 46- | 341 | OCO-c-C3H5 | OCO-c-C3H5 | OCO-c-C3H5 | H | H | O | 2-pyridyl |
| 46- | 705 | OH | OCO-c-C3H5 | H | H | H | O | 2-pyridyl |
| 46- | 713 | OCO-c-C3H5 | H | H | H | H | O | 2-pyridyl |
| 46- | 260 | OCO-c-C3H5 | OCO-c-C3H5 | OH | H | H | O | 2-pyridyl |

(continued)

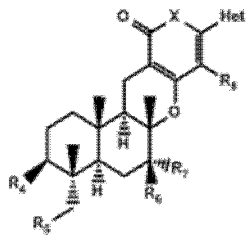

| Compound No. | | R4 | R5 | R6 | R7 | R8 | X | Hetero |
|---|---|---|---|---|---|---|---|---|
| 46- | 262 | OCO-c-C3H5 | OCO-c-C3H5 | H | H | H | O | 2-pyridyl |
| 47- | 1 | OH | OH | OH | H | H | O | 4-pyridyl |
| 47- | 4 | OH | OH | H | H | H | O | 4-pyridyl |
| 47- | 5 | OH | H | H | H | H | O | 4-pyridyl |
| 47- | 341 | OCO-c-C3H5 | OCO-c-C3H5 | OCO-c-C3H5 | H | H | O | 4-pyridyl |
| 47- | 705 | OH | OCO-c-C3H5 | H | H | H | O | 4-pyridyl |
| 47- | 713 | OCO-c-C3H5 | H | H | H | H | O | 4-pyridyl |
| 47- | 260 | OCO-c-C3H5 | OCO-c-C3H5 | OH | H | H | O | 4-pyridyl |
| 47- | 262 | OCO-c-C3H5 | OCO-c-C3H5 | H | H | H | O | 4-pyridyl |

Among compounds represented by formula (I-a') or (I-b), preferred compounds are those wherein Het represents 3-pyridyl,
X represents an oxygen atom,
$R_4$ represents $C_{3-6}$ cycloalkylcarbonyloxy,
$R_5$ represents a hydrogen atom or $C_{3-6}$ cycloalkyl carbonyloxy,
$R_6$ represents a hydrogen atom or hydroxyl, and
$R_7$ and $R_8$ represent a hydrogen atom. Particularly preferred are compounds of Nos. 43-260, 43-262, or 43-713.

Production process

Among compounds represented by formula (I), (1) compounds represented by formula (I-1):

[Chemical formula 6]

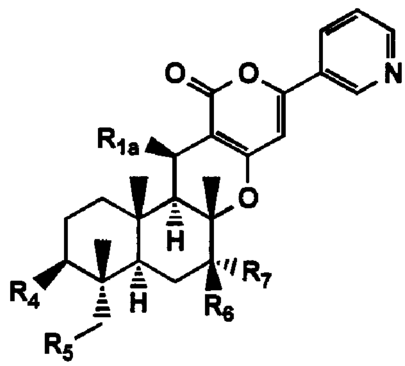

(I-1)

wherein
$R_{1a}$ represents hydroxyl,
optionally substituted $C_{1-18}$ alkylcarbonyloxy, adamantylcarbonyloxy,
optionally substituted aryl $C_{1-6}$ alkylcarbonyloxy,
optionally substituted $C_{2-6}$ alkenylcarbonyloxy,
optionally substituted $C_{2-6}$ alkynylcarbonyloxy,
optionally substituted saturated or unsaturated heterocyclic $C_{1-6}$ alkylcarbonyloxy,
optionally substituted saturated or unsaturated heterocyclic $C_{2-6}$ alkenylcarbonyloxy,
optionally substituted aryl carbonyloxy,
optionally substituted carbamoyloxy,
optionally substituted carbamoyl,
optionally substituted $C_{1-6}$ alkylsulfonyloxy,
optionally substituted $C_{1-6}$ alkylsufonyl,
optionally substituted aryl sufonyloxy,
optionally substituted aryl $C_{1-6}$ alkyloxy,
optionally substituted aryl oxycarbonyloxy,
optionally substituted aryl aminocarbonyloxy,
optionally substituted aryl sulfonyl,
optionally substituted aryl sulfanyl,
optionally substituted saturated or unsaturated heterocyclic sulfanyl,
optionally substituted $C_{1-6}$ alkyloxy,
optionally substituted $C_{2-6}$ alkenyloxy,
optionally substituted $C_{2-6}$ alkynyloxy,
optionally substituted aryl oxy,
$C_{1-6}$ alkyloxy-$C_{1-6}$ alkyloxy,
$C_{1-6}$ alkylthio-$C_{1-6}$ alkyloxy,
$C_{1-6}$ alkyloxy-$C_1$-$_6$ alkyloxy-$C_{1-6}$ alkyloxy,,
optionally substituted $C_{1-6}$ alkyloxycarbonyloxy,
optionally substituted saturated or unsaturated heterocyclic oxy,

optionally substituted saturated or unsaturated heterocyclic thio,
optionally substituted saturated or unsaturated heterocyclic carbonyloxy,
optionally substituted saturated or unsaturated heterocyclic thiocarbonyloxy,
optionally substituted phosphate group,
optionally substituted $C_{1-6}$ alkyl,
tri-$C_{1-6}$ alkylsilyloxy,
optionally substituted saturated or unsaturated heterocyclic group, or
-O-N=C-Y1 wherein Y1 represents a hydrogen atom, optionally substituted $C_{1-6}$ alkyl, optionally substituted $C_{3-7}$ cycloalkyl, optionally substituted $C_{2-6}$ alkenyl, optionally substituted $C_{2-6}$ alkynyl, optionally substituted $C_{1-6}$ alkoxy, optionally substituted phenyl, or optionally substituted heterocyclic group,
$R_4$, $R_5$, $R_6$, and $R_7$ are as defined in formula (I),
can be synthesized by a process described in Japanese Patent Application Laid-Open No. 259569/1996, WO 2009/081957, WO 2006/129714, or WO 2008/066153, using pyripyropene A as a starting material. The pyripyropene A as the starting material can be produced by a process described in Journal of Society of Synthetic Organic Chemistry, Japan (1998), Vol. 56, No. 6, pp. 478-488 or WO 94/09417.

Further, among compounds represented by formula (I), (2) compounds represented by formula (I-2):

[Chemical formula 7]

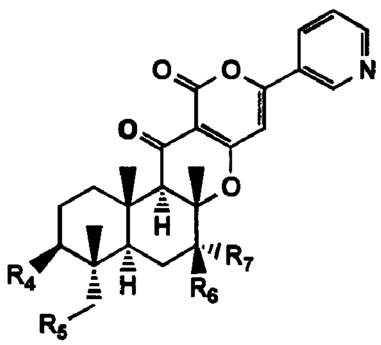

(I-2)

wherein $R_4$, $R_5$, $R_6$, and $R_7$ are as defined in formula (I) can be synthesized by a process described in Japanese Patent Application Laid-Open No. 269062/1996 and Journal of Antibiotics (1997) 50 (3), pp. 229-36, using compounds represented by formula (I-1'):

[Chemical formula 8]

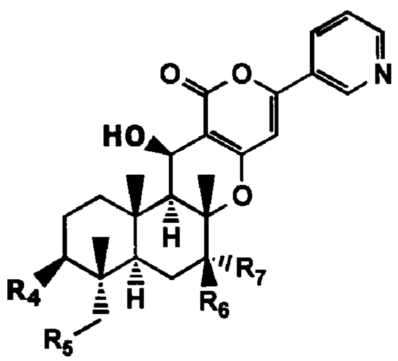

(I-1')

wherein $R_4$, $R_5$, $R_6$, and $R_7$ are as defined in formula (I), which can be synthesized by the process (1), as a starting material.

Furthermore, among compounds represented by formula (I), (3) compounds represented by formula (I-3):

[Chemical formula 9]

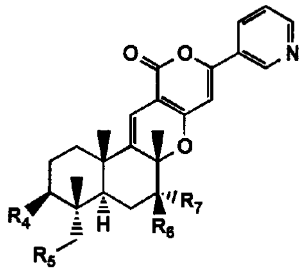

(I-3)

wherein $R_4$, $R_5$, $R_6$, and $R_7$ are as defined in formula (I), can be synthesized by a process described in Japanese Patent Application Laid-Open No. 269063/1996, using compounds represented by (I-1'), which can be synthesized by the process (1), as a starting material.

Among compounds represented by formula (I), (4) compounds represented by formula (I-4):

[Chemical formula 10]

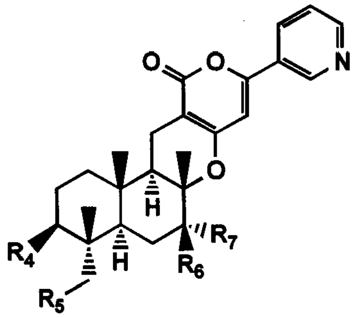

(I-4)

wherein $R_4$, $R_5$, $R_6$, and $R_7$ are as defined in formula (I), can be synthesized by a process described in Japanese Patent Application Laid-Open No. 259569/1996, WO 2009/081957, WO 2006/129714, and WO 2008/066153, using as a staring material compounds of formula (II):

[Chemical formula 11]

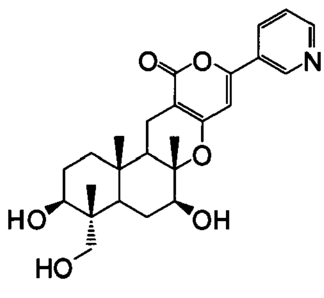

(II)

obtained by treating a cultured product produced by bacteria, which produce pyripyropene compounds, obtained by a method described in Journal of Techniccal Disclosure No. 500997/2008, with a base.

Furthermore, among compounds represented by formula (I), (5) compounds represented by formula (I-5):

[Chemical formula 12]

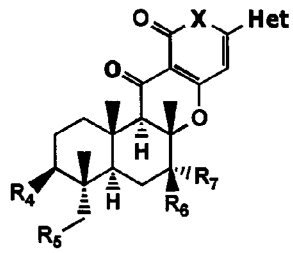

(I-5)

wherein $R_4$, $R_5$, $R_6$, $R_7$, X, and Het are as defined in formula (I), provided that, when X represents an oxygen atom, Het does not represent unsubstituted 3-pyridyl,
can be synthesized by a process described in Japanese Patent Application Laid-Open No. 259569/1996, WO 2009/081957, WO 2006/129714, and WO 2008/066153, using as a starting material compounds represented by formula (I-6):

[Chemical formula 13]

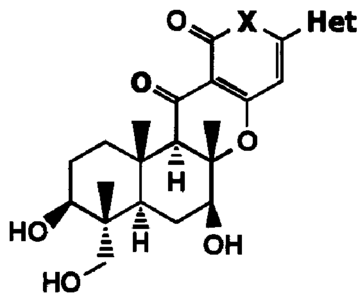

(I-6)

wherein X and Het are as defined in formula (I), provided that, when X represents an oxygen atom, Het does not represent unsubstituted 3-pyridyl,
obtained by a process described in Journal of Antibiotics (1997) 50(3), pp. 229-36, using pyripyropene A as a starting material.

Among compounds represented by formula (I), (6) compounds represented by formula (I-7):

[Chemical formula 14]

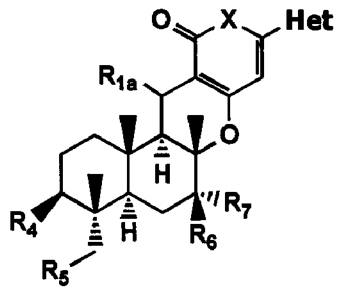

(I-7)

wherein $R_{1a}$ is as defined formula (I-1); and $R_4$, $R_5$, $R_6$, $R_7$, X, and Het are as defined in formula (I), provided that, when X represents an oxygen atom, Het does not represent 3-pyridyl, can be synthesized by obtaining compounds of formula (I-8):

[Chemical formula 15]

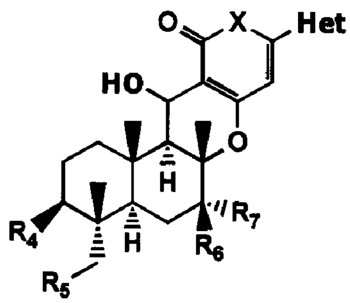

(I-8)

wherein $R_4$, $R_5$, $R_6$, $R_7$, X, and Het are as defined in formula (I), provided that, when X represents an oxygen atom, Het does not represent 3-pyridyl,
by a process described in Journal of Antibiotics (1997) 50(3), 229-36, from compounds represented by formula (I-5) obtained by the process (5), and subjecting the compounds of formula (I-8) as a staring material to treatment by a process described in Japanese Patent Application Laid-Open No. 259569/1996, WO 2009/081957, WO 2006/129714, and WO 2008/066153.

Among compounds represented by formula (I), (7) compounds represented by formula (I-9):

[Chemical formula 16]

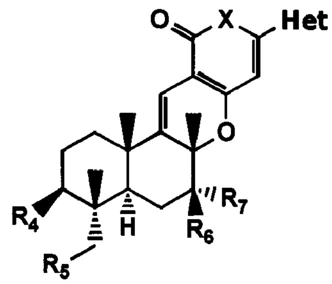

(I-9)

wherein $R_4$, $R_5$, $R_6$, $R_7$, X, and Het are as defined in formula (I), provided that, when X represents an oxygen atom, Het does not represent 3-pyridyl,
can be synthesized by a process described in Japanese Patent Application Laid-Open No. 269063/1996, using compounds represented by formula (I-8) obtained by the process (6).

Among compounds represented by formula (I), (8) compounds represented by formula (I-10):

[Chemical formula 17]

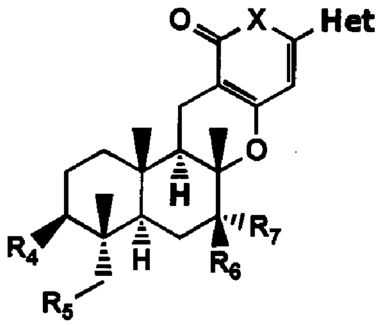

(I-10)

wherein $R_4$, $R_5$, $R_6$, $R_7$, X, and Het are as defined in formula (I), provided that, when X represents an oxygen atom, Het does not represent 3-pyridyl,
can be synthesized by a process described in Journal of Antibiotics (1997) 50(3), pp.229-36, using the compounds represented by formula (I-4) obtained by the process (4) as a staring material.

Among compounds represented by formula (I), (9) compounds represented by formula (I-11):

[Chemical formula 18]

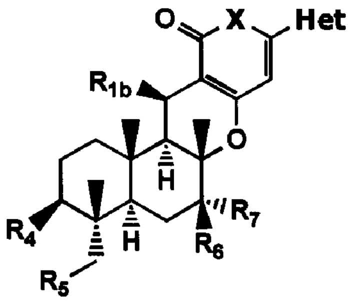

(I-11)

wherein $R_{1b}$ represents azide, optionally substituted amino, optionally substituted imino, optionally substituted hydrazino, cyano, or a halogen atom; and $R_4$, $R_5$, $R_6$, $R_7$, X, and Het are as defined in formula (I), can be synthesized by a conventional method as described, for example, in Jikken Kagaku Koza (Experimental Chemistry) (fourth edition, 1992, Maruzen Company, Limited), using as a starting material compounds represented by formula (I-2) obtained by the process (2) or compounds represented by formula (I-5) obtained by the process (5).

Among compounds represented by formula (I), (10) compounds represented by formula (I-12):

[Chemical formula 19]

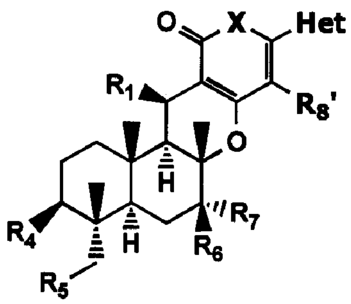

(I-12)

wherein $R_1$, $R_4$, $R_5$, $R_6$, $R_7$, X, and Het are as defined in formula (I); and $R_8$' represents cyano, a halogen atom or benzyl, can be synthesized by a conventional method as described, for example, in Jikken Kagaku Koza (Experimental Chemistry) (fourth edition, 1992, Maruzen Company, Limited), using as a starting material compounds represented by formula (I-13):

[Chemical formula 20]

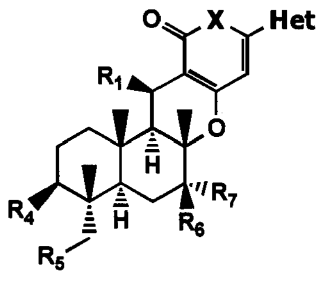

(I-13)

wherein $R_1$, $R_4$, $R_5$, $R_6$, $R_7$, X, and Het are as defined in formula (I),

obtained by the processes (l) to (9).

Among compounds represented by formula (I), (11) compounds represented by formula (I-14):

[Chemical formula 21]

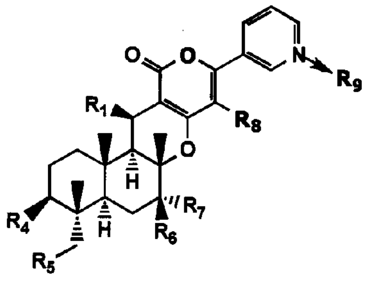

(I-14)

wherein $R_9$ represents an oxygen atom, methyl, or benzyl; and $R_1$, $R_4$, $R_5$, $R_6$, $R_7$, and $R_8$ are as defined in formula (I), can be synthesized by a process described in Japanese Patent Application Laid-Open No. 269064/1996, using compounds represented by formula (I-15) as a starting material:

[Chemical formula 22]

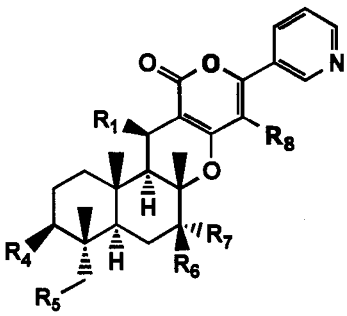

(I-15)

wherein $R_1$, $R_4$, $R_5$, $R_6$, $R_7$, and $R_8$ are as defined in formula (I),
obtained by the processes (1) to (4) and (9) and (10).

Insect species against which the compounds represented by formula (I), (I-a), (I-a') , (I-b) or (I-c) or salts thereof have pesticidal effect as an active ingredient include, for example, lepidopteran insect pests, for example, Spodoptera litura, Mamestra brassicae, Pseudaletia separata, green caterpillar, Plutella xylostella, Spodoptera exigua, Chilo suppressalis, Cnaphalocrocis medinalis, Tortricidae, Carposinidae, Lyonetiidae, Lymantriidae, insect pests belonging to the genus Agrotis spp., insect pests belonging to the genus Helicoverpa spp., or insect pests belonging to the genus Heliothis spp. and the like; hemipteran insect pests, for example, Aphididae, Adelgidae or Phylloxeridae, for example, Myzus percicae, Aphis gossypii Glover, Aphis fabae, Aphis maidis, Acyrthosiphon pisum, Aulacorthum solani, Aphis craccivora, Macrosiphum euphorbiae, Macrosiphum avenae, Methopolophium dirhodum, Rhopalosiphum padi, Schizaphis graminum, Brevicoryne brassicae, Lipaphis erysimi, Aphis citricola, Rosy apple aphid, Eriosoma lanigerum, Toxoptera aurantii, or Toxoptera citricidus, Deltocephalidae such as Nephotettix cincticeps, leafhoppers such as Tea green leafhopper, Delphacidae such as Laodelphax striatellus, Nilaparvata lugens, or Sogatella furcifera, Pentatomidae such as Eysarcoris ventralis, Nezara viridula, or Trigonotylus caelestialium, Aleyrodidae such as Bemisia tabaci or Trialeurodes vaporariorum, Coccoidea such as Pseudococcus comstocki, Planococcus citri Risso, or Aonidiella aurantii (for example, Diaspididae, Margarodidae, Ortheziidae, Aclerdiae, Dactylopiidae, Kerridae, Pseudococcidae, Coccidae, Eriococcidae, Asterolecaniidae, Beesonidae, Lecanodiaspididae, or Cerococcidea), and Psyllidae such as Diaphorina citri; Coleoptera insect pests, for example, Lissorhoptrus oryzophilus, Callosobruchus chinensis, Tenebrio molitor, Diabrotica virgifera

virgifera, Diabrotica undecimpunctata howardi, Anomala cuprea, Anomala rufocuprea, Phyllotreta striolata, Aulacophora femoralis, Leptinotarsa decemlineata, Oulema oryzae, Grapholita molesta, or Cerambycidae; Acari, for example, Tetranychus urticae, Tetranychus kanzawai, or Panonychus citri; Hymenopteran insect pests, for example, Tenthredinoidea; Orthopteran insect pests, for example, Acrididae; Dipteran insect pests, for example, Musca domestica Linnaeus or Agromyzidae; Thysanopteran insect pests, for example, Thrips palmi KARNY or Frankliniella occidentalis; and Plant Parasitic Nematodes, for example, Meloidogyne hapla, Pratylenchus, Aphelenchoides besseyi, or Bursaphelenchus xylophilus. Examples of zoooparasites include Siphonaptera, for example, Ctenocephalides felis or Pulex irritans, Anoplura, for example, Pediculus spp., or Phtirus spp.; Acari, for example, Boophilus spp., Haemaphysalis longicornis, Rhipicephalus sanguineus, Haemaphysalis flava, Sarcoptes spp., Dermanyssus spp., Ornithonyssus sylviarum, Ornithonyssus bacoti, and Leptotrombidium; Tabanidae; flies, for example, Lucilia spp.; mosquitoes, for example, Stegomyia albopicta and Culex pipiens pallens; Simuliidae; Ceratopogonidae; Nematoda, for example, Strongyloides, for example, Strongyloides papillosus or Strongyloides stercoralis, hookworms, for example, A. caninum, Ancylostoma tubaeforme, or Ancylostoma duodenale; Haemonchus spp; Strongylida, for example, mouse Strongyloides; hairworms; Metastrongyloidea, for example, Metastrongylus spp., Angiostrongylus cantonensis, or Aelurostrongylus; Oxyurida; Heterakidae, for example, Heterakis gallinarum; Anisakis simplex; Ascaroidea, for example, Ascaris suum, Parascaris equorum, Toxicara canis, or Toxocara cati; Subuluridae; Spiruroidea, for example, Gnathostoma spinigerum, Physaloptera, Ascarops strongylina, Draschia megastoma, Acuaria, or Ostertagia ostertagi; Filariida, for example, Dirofilaria, or Onchocerca cervicalis; Order Dioctophymatida; Wipeworms and Trichinosis, for example, Trichurisvulpis or Trichinella spiralis; Trematoda, for example, Schistosomatoide, for example, Schistosoma japonicum, or Fasciola hepatica; Acanthocephala, for example, Macracanthorhynchus hirudinaceus, or Moniliformis moniliformis; Cestoda, for example, Bothriocephaloidea, for example, Diphyllobothrium mansoni; Cyclophyllidea, for example, Dipylidium caninum, Hymenolepis diminuta, Echinococcus multilocularis, or Echinococcus granulosus; and protozoa. Preferred insect species include Hemipteran, Dipteran, and Thysanopteran insect pests. Hemipteran insect pests are particularly preferred.

Preferred Hemipteran insect pests include Aphididae, Adelgidae, or Phylloxeridae (preferably Aphididae); Leafhoppers, Aleyrodidae, Pentatomidae, or Coccoidea (Diaspididae, Margarodidae, Ortheziidae, Aclerdiae,Dactylopiidae, Kerridae, Pseudococcidae, Coccidae, Eriococcidae, Asterolecaniidae, Beesonidae, Lecanodiaspididae, or Cerococcidae); and Psyllidae, more preferably myzus persicae, Aphis gossypii Glover, Tea green leafhopper, Bemisia tabaci, Trialeurodes vaporariorum, Trigonotylus caelestialium, or Pseudococcus comstocki, Aonidiella aurantii, and Diaphorina citri.

When compounds represented by formula (I), (I-a), (I-a'), (I-b) or (I-c) are used as harmful organism control agents, the compounds represented by formula (I), (I-a), (I-a'), (I-b) or (I-c) as such may be used. Alternatively, the compounds represented by formula (I), (I-a), (I-a'), (I-b) or (I-c)may be mixed with agriculturally or zootechnically acceptable suitable carriers such as solid carriers, liquid carriers, and gaseous carrier, surfactants, dispersants, or other adjuvants for formulations, to prepare any suitable formulations such as emulsifiable concentrates, EW (emulsion oil in water), liquid formulations, suspensions, wettable powders, water dispersible granules, dusts, DL dusts, grains, granules, tablets, oils, aerosols, floables, dry floables, or microcapsules.

Solid carriers include, for example, talc, bentonite, clay, kaolin, diatomaceous earth, vermiculite, white carbon, or calcium carbonate.

Liquid carriers include, for example, alcohols such as methanol, n-hexanol, or ethylene glycol; ketones such as acetone, methyl ethyl ketone, or cyclohexanone; aliphatic hydrocarbons such as n-hexane, kerosine, or kerosene; aromatic hydrocarbons such as toluene, xylene, or methylnaphthalene; ethers such as diethyl ether, dioxane, or tetrahydrofuran; esters such as ethyl acetate; nitriles such as acetonitrile or isobutyronitrile; acid amides such as dimethylformamide or dimethylacetamide; vegetable oils such as soybean oil or cotton seed oil; dimethylsulfoxide; or water.

Gaseous carriers include, for example, LPG, air, nitrogen, carbon dioxide, and dimethyl ether.

Surfactants or dispersants usable for emulsifying, dispersing, or spreading include, for example, alkylsulfuric esters, alkyl (aryl) sulfonic acid salts, polyoxyalkylene alkyl (aryl) ethers, polyhydric alcohol esters, and lignin sulfonic acid salts. Adjuvants usable for improving the properties of formulations include, for example, carboxymethylcellulose, gum arabic, polyethylene glycol, and calcium stearate.

The above carriers, surfactants, dispersants, and adjuvants may be used either solely or in a combination according to need.

The content of the active ingredient in these formulations is not particularly limited but is preferably 1 to 75% by weight for emulsifiable concentrate, 0.3 to 25% by weight for dust, 1 to 90% by weight for wettable powder, and 0.5 to 10% by weight for granules.

According to another aspect of the present invention, there is provided a method for controlling harmful organisms, the method comprising applying an effective amount of a compound represented by formula (I), (I-a), (I-a'), (I-b), or (I-c) or a salt thereof to an object selected from the group consisting of water surface, soil, nutrient solution in nutriculture, solid medium in nutriculture, and seed, root, tuber, bulb, and rhizome of a plant.

According to one embodiment of the present invention, there is provide a method for controlling a harmful

organism, comprising applying an effective amount of a compound represented by formula (I), (I-a), (I-a') (I-b), or (I-c), or a salt thereof to the harmful organism or a habitat thereof. According to preferred embodiment of the present invention, there is provided a method for controlling a harmful organism, the method comprising applying an effective amount of a compound represented by formula (I), (I-a), (I-a') (I-b), or (I-c) or a salt thereof to a plant or soil.

Compounds represented by formula (I), (I-a), (I-a'), (I-b), or (I-c), or salts thereof as such exert potent control effect against harmful organisms. Further, use of the compounds as a mixture with other harmful organism control agents can be expected to exert higher control effect than the control effect attained when the compounds or other harmful organism control agents are used solely. Thus, according to the present invention, there is provided a harmful organism control composition comprising at least one of compounds represented by formula (I), (I-a), (I-a') (I-b), or (I-c), or salts thereof and at least one other harmful organism control agent. Further, according to another embodiment of the present invention, there is provided use of the harmful organism control composition for the protection of useful plants from harmful organisms. Furthermore, according to another embodiment, there is provided use of the harmful organism control composition in the manufacture of the agent used for the protection of useful plants from harmful organisms.

Compositions or compounds and admixtures thereof with other harmful organism control agents usable as harmful organism control agents according to the present invention are used for the control of many pests for a variety of plants. Object plants include wheat and barley, coarse cereals such as corn, millet, common millet, barnyard millet, and edible sorghum, fruit trees such as oranges, apples, and grapes, vegetables such as cucumbers, pumpkins, melons, cabbages, eggplants, tomatoes, and strawberries, tubers such as potatoes, sweet potatoes, and taros, pulses such as azuki beans, kidney bean, and soybeans, oil crops such as rapeseeds, feed crops such as grazing, sorghum, and corn used for animal feed, ornamental plants, foliage plants, timbers, tea, sugar beet, sugar canes, sunflower, hops, cotton plants, nicotiana, Arabian coffee, lawn grass, and champignon.

Compositions or compounds and admixtures thereof with other harmful organism control agents usable as harmful organism control agents according to the present invention can be applied to harmful insects, plants, and plant propagation materials, specifically, for example, seeds, plant foliages, roots, soil, water surface, culture materials, and room where the entry of pests should be prevented. The treatment by the compounds, admixtures, and composition according to the present invention may be carried out before and after the entry of insect pests.

The present invention encompasses disinfestation of harmful organisms that are parasitic in animals. The disinfestation of harmful organisms can be carried out by application to habitats where zoobiotic harmful organisms grow or would grow, animal farming places, feed, plants, seeds, soil, materials and growth environments, or materials, plants, seeds, soil, and water surface where the entry of zoobiotic harmful organisms should be prevented.

Plant propagation materials as objects to which the present invention is applied mean plants having an ability of reproduction used in plant growth, including, but are not limited to, seeds, slash or lops, a pullout portion of a part of a tuber, specifically seeds, roots, fruits, tubers, bulbs, corms, roots, shoots, and sprouts. Seedling or juvenile plants that have been transplanted after budding or rooting are also included. A plant protecting agent is applied for prevention purposes to these plant propagation materials at the time of settled plating or transplanting.

The term "cultivated plants" is to be understood as including plants which have been modified by breeding, mutagenesis and/or genetic engineering. Genetically modified plants (GMO) are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s) (oligo- or polypeptides) for example by glycosylation or polymer additions such as prenylated, acetylated or farnesylated moieties or PEG moieties(e.g. as disclosed in Biotechnol Prog. 2001 Jul-Aug;17(4):720-8., Protein Eng Des Sel. 2004 Jan;17(1):57-66, Nat Protoc. 2007; 2(5):1225-35., Curr Opin Chem Biol. 2006 Oct;10(5):487-91. Epub 2006 Aug 28., Biomaterials. 2001 Mar;22(5):405-1 7 , Bioconjug Chem. 2 0 0 5 Jan-Feb;16(1):113-21).

The term "cultivated plants" is to be understood also including plants that have been rendered tolerant to applications of specific classes of herbicides, such as hydroxy-phenylpyruvate dioxygenase (HPPD) inhibitors; acetolactate synthase (ALS) inhibitors, such as sulfonyl ureas (see e. g. US 6,222,100, WO 01/82685, WO 00/26390, WO 97/41218, WO 98/02526, WO 98/02527, WO 04/106529, WO 05/20673, WO 03/14357, WO 03/13225, WO 03/14356, WO 04/16073) or imidazolinones (see e. g. US 6,222,100, WO 01/82685, WO 00/26390, WO 97/41218, WO 98/02526, WO 98/02527, WO 04/106529, WO 05/20673, WO 03/14357, WO 03/13225, WO 03/14356, WO 04/16073); enolpyruvylshikimate-3-phosphate synthase (EPSPS) inhibitors, such as glyphosate (see e. g. WO 92/00377); glutamine synthetase (GS) inhibitors, such as glufosinate (see e. g. EP-A-0242236, EP-A-242246) or oxynil herbicides (see e. g. US 5,559,024). Plants resistant to these herbicides can be obtained as a result of conventional methods of breeding or genetic engineering.

Several cultivated plants have been rendered tolerant to herbicides by conventional methods of breeding (mutagenesis), for example Clearfield (registered trademark) summer rape (Canola) being tolerant to imidazolinones, e. g. imazamox. Genetic engineering methods have been used to render cultivated plants, such as soybean, cotton,

corn, beets and rape, tolerant to herbicides, such as glyphosate and glufosinate, some of which are commercially available under the trade names RoundupReady (registered trademark) (glyphosate) and LibertyLink (registered trademark) (glufosinate).

The term "cultivated plants" is to be understood also including plants that are by the use of recombinant DNA techniques capable to synthesize one or more insecticidal proteins, such as δ-endotoxins, e. g. CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, for example Photorhabdus spp. or Xenorhabdus spp.; toxins produced by animals, such as scorpion toxins, arachnid toxins, wasp toxins, or other insect-specific neurotoxins; toxins produced by fungi, such as Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases.

In the context of the present invention these insecticidal proteins or toxins are to be understood expressly also as pre-toxins, hybrid proteins, truncated or otherwise modified proteins.

Hybrid toxins are produced by a recombinant technique using a new combination of protein domains, (see, for example WO 02/015701). Further examples of such toxins or genetically modified plants capable of synthesizing such toxins are disclosed, for example, in EP-A 374 753, WO 93/007278, WO 95/34656, EP-A 427 529, EP-A 451 878, WO 03/018810 and WO 03/052073. The methods for producing such genetically modified plants are generally known to the person skilled in the art and are described, for example, in the publications mentioned above.

These insecticidal proteins contained in the genetically modified plants impart to the plants producing these proteins protection from harmful pests from certain taxonomic groups of arthropods, particularly to beetles (Coleoptera), flies (Diptera), and butterflies and moths (Lepidoptera) and to plant parasitic nematodes (Nematoda).

The term "cultivated plants" is to be understood also including plants that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, for example EP-A 0 392 22), plant disease resistance genes (for example potato cultivars, which express resistance genes acting against Phytophthora infestans derived from the Mexican wild potato Solanum bulbocastanum) or T4-lysozym (e. g. potato cultivars capable of synthesizing the proteins with increased resistance against bacteria such as Erwinia amylvora). The methods for producing such genetically modified plants capable of synthesizing the proteins are generally known to the person skilled in the art and are described, for example, in the publications mentioned above.

The term "cultivated plants" is to be understood also including plants that are by the use of recombinant DNA techniques capable to synthesize one or more proteins to increase the productivity (e. g. bio mass production, grain yield, sugar content, oil content or protein content), tolerance to drought, salinity or other growth-limiting environmental factors or tolerance to pests and fungal, bacterial or viral pathogens of those plants.

The term "cultivated plants" is to be understood also including plants that contain by the use of recombinant DNA techniques a modified amount of substances of content or new substances of content, specifically to improve human or animal nutrition, for example oil crops that produce health-promoting long-chain omega-3 fatty acids or unsaturated omega-9 fatty acids (e. g. Nexera (registered trademark) rape).

The term "cultivated plants" is to be understood also including plants that contain by the use of recombinant DNA techniques a modified amount of substances of content or new substances of content, specifically to improve raw material production, for example, potatoes that produce increased amounts of amylopectin (e. g. Amflora (regsistered trademark) potato).

Preferred methods for applying the compounds of formula (I), (I-a), (I-a') (I-b), or (I-c) or compositions comprising the compounds for use as harmful organism control agents to plants or soil include spreading treatment, soil treatment, surface treatment, or fumigation treatment. Examples of spreading treatment include spreading, spraying, misting, atomizing, granule application, or water surface application. Examples of soil treatment include soil drenching or soil mixing. Examples of surface treatment include coating, dust coating, or covering. Further, examples of fumigation treatment include covering of soil with polyethylene film after soil injection. Accordingly, the control method according to the present invention also includes a method in which a compound represented by formula (I), (I-a), (I-a') (I-b) , or (I-c) or a preparation comprising the compound is applied by fumigation treatment in a closed space.

Other harmful organism control agents admixable into compounds represented by formula (I), (I-a), (I-a') (I-b) , or (I-c) or salts thereof include insecticides, bactericides, miticides or tickicides, herbicides, and plant growth-regulating agents. Specific agents include those described, for example, in The Pesticide Manual, 13th edition, published by The British Crop Protection Council; and SHIBUYA INDEX, the 14th edition, 2009, published by SHIBUYA INDEX RESEARCH GROUP. More specific examples thereof are M.1. to M.27. described below:

M.1. Organophosphate insecticides: acephate, azamethiphos, azinphos-ethyl, azinphos-methyl, chlorethoxyfos, chlorfenvinphos, chlormephos, chlorpyrifos, chlorpyrifos-methyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, flupyrazophos, fosthiazate, heptenophos, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, and vamidothion;

M.2. Carbamate insecticides: aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb, and triazamate;

M.3. Pyrethroid insecticides: acrinathrin, allethrin, d-cis-trans allethrin, d-trans allethrin, bifenthrin, bioallethrin, bioallethrin S-cylclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, imiprothrin, metofluthrin, permethrin, phenothrin, prallethrin, profluthrin, pyrethrin (pyrethrum), resmethrin, silafluofen, tefluthrin, tetramethrin, tralomethrin, and transfluthrin;

M.4. Juvenile hormone mimics: hydroprene, kinoprene, methoprene, fenoxycarb, and pyriproxyfen;

M.S. Nicotinic receptor agonists/antagonists compounds: acetamiprid, bensultap, cartap hydrochloride, clothianidin, dinotefuran, imidacloprid, thiamethoxam, nitenpyram, nicotine, spinosad (allosteric agonist), spinetoram (allosteric agonist), thiacloprid, thiocyclam, thiosultap-sodium and AKD1022;

M.6. GABA gated chloride channel antagonist compounds: chlordane, endosulfan, gamma-HCH (lindane); ethiprole, fipronil, pyrafluprole, and pyriprole;

M.7. Chloride channel activators: abamectin, emamectin benzoate, milbemectin, lepimectin;

M.8. METI I compounds: fenazaquin, fenpyroximate, pyrimidifen, pyridaben, tebufenpyrad, tolfenpyrad, flufenerim, and rotenone;

M.9. METI II compounds: acequinocyl, fluacyprim, and hydramethylnon;

M.10. Uncouplers of oxidative phosphorylation: chlorfenapyr, DNOC;

M.11. Other inhibitors of oxidative phosphorylation: azocyclotin, cyhexatin, diafenthiuron, fenbutatin oxide, propargite, and tetradifon;

M.12. Moulting disruptors: cyromazine, chromafenozide, halofenozide, methoxyfenozide, and tebufenozide;

M.13. Synergists: piperonyl butoxide, tribufos;

M.14. Sodium channel blocker compounds: indoxacarb, and metaflumizone;

M.15. Fumigants: methyl bromide, chloropicrin sulfuryl fluoride;

M.16. Selective feeding blockers: crylotie, pymetrozine, and flonicamid;

M.17. Mite growth inhibitors: clofentezine, hexythiazox, and etoxazole;

M.18. Chitin synthesis inhibitors: buprofezin, bistrifluron, chlorfluazuron, diflubenzuron, flucycloxuron, flufenoxuron, hexaflumuron, lufenuron, novaluron, noviflumuron, teflubenzuron, and triflumuron;

M.19. Lipid biosynthesis inhibitors: spirodiclofen, spiromesifen, and spirotetramat;

M.20. Octapaminergic agonsits: amitraz;

M.21. Ryanodine receptor modulators: flubendiamide and the phtalamid compound (R)-, (S)-3-Chlor-N1-{2-methyl-4-[1,2,2,2-tetrafluor-1-(trifluormethyl)et hyl]phenyl}-N2-(1-methyl-2-methylsulfonylethyl)phthalamid (M21.1) ;

M.22. Isoxazoline compounds: 4-[5-(3,5-Dichloro-phenyl)-5-trifluoromethyl-4,5-dihydro-isoxaz ol-3-yl]-2-methyl-N-pyridin-2-ylmethyl-benzamide (M22.1); 4-[5-(3,5-Dichloro-phenyl)-5-trifluoromethyl-4,5-dihydro-isoxaz ol-3-yl]-2-methyl-N-(2,2,2-trifluoro-ethyl)-benzamide (M22.2), 4-[5-(3,5-Dichloro-phenyl)-5-trifluoromethyl-4,5-dihydro-isoxaz ol-3-yl]-2-methyl-N-[(2,2,2-trifluoro-ethylcarbamoyl)-methyl]-b enzamide (M22.3), 4-[5-(3,5-Dichloro-phenyl)-5-trifluoromethyl-4,5-dihydro-isoxaz ol-3-yl]-naphthalene-1-carboxylic acid [(2,2,2-trifluoro-ethylcarbamoyl)-methyl]-amide (M22.4), 4-[5-(3,5-Dichlorophenyl)-5-trifluoromethyl-4,5-dihydro-isoxazo l-3-yl]-N-[(methoxyimino)methyl]-2-methylbenzamide (M22.5), 4-[5-(3-Chloro-5-trifluoromethyl-phenyl)-5-trifluoromethyl-4,5-dihydro-isoxazol-3-yl]-2-methyl-N-[(2,2,2-trifluoro-ethylcarbam oyl)-methyl]-benzamide (M22.6) , 4-[5-(3-Chloro-5-trifluoromethyl-phenyl)-5-trifluoromethyl-4,5-dihydro-isoxazol-3-yl]-naphthalene-1-carboxylic acid [(2,2,2-trifluoro-ethylcarbamoyl)-methyl]-amide (M22.7) and 5-[5-(3,5-Dichloro-4-fluoro-phenyl)-5-trifluoromethyl-4,5-dihyd ro-isoxazol-3-yl]-2-[1,2,4]triazol-1-yl-benzonitrile (M22.8);

M.23. Anthranilamide compounds: chloranthraniliprole, cyantraniliprole;
5-Bromo-2-(3-chloro-pyridin-2-yl)-2H-pyrazole-3-carboxylic acid
[4-cyano-2-(1-cyclopropyl-ethylcarbamoyl)-6-methyl-phenyl]-a mide (M23.1),
5-Bromo-2-(3-chloro-pyridin-2-yl)-2H-pyrazole-3-carboxylic acid
[2-chloro-4-cyano-6-(1-cyclopropyl-ethylcarbamoyl)-phenyl]-a mide (M23.2),

5-Bromo-2-(3-chloro-pyridin-2-yl)-2H-pyrazole-3-carboxylic acid [2-bromo-4-cyano-6-(1-cyclopropyl-ethylcarbamoyl)-phenyl]-a mide(M23.3),
5-Bromo-2-(3-chloro-pyridin-2-yl)-2H-pyrazole-3-carboxylic acid [2-bromo-4-chloro-6-(1-cyclopropyl-ethylcarbamoyl)-phenyl]-a mide(M23.4),
5-Bromo-2-(3-chloro-pyridin-2-yl)-2H-pyrazole-3-carboxylic acid [2,4-dichloro-6-(1-cyclopropyl-ethylcarbamoyl)-phenyl]-amide (M23.5),
5-Bromo-2-(3-chloro-pyridin-2-yl)-2H-pyrazole-3-carboxylic acid [4-chloro-2-(1-cyclopropyl-ethylcarbamoyl)-6-methyl-phenyl]-a mide (M23.6),
N'-(2-{[5-Bromo-2-(3-chloro-pyridin-2-yl)-2H-pyrazole-3-carbo nyl]-aminol-5-chloro-3-methyl-benzoyl)-hydrazinecarboxylic acid methyl ester (M23.7),
N'-(2-{[5-Bromo-2-(3-chloro-pyridin-2-yl)-2H-pyrazole-3-carbo nyl]-amino}-5-chloro-3-methyl-benzoyl)-N'-methyl-hydrazineca rboxylic acid methyl ester (M23.8),
N'-(2-{[5-Bromo-2-(3-chloro-pyridin-2-yl)-2H-pyrazole-3-carbo nyl]-amino}-5-chloro-3-methyl-benzoyl)-N,N'-dimethyl-hydrazin ecarboxylic acid methyl ester (M23.9),
N'-(3,5-Dibromo-2-{[5-bromo-2-(3-chloro-pyridin-2-yl)-2H-pyr azole-3-carbonyl]-amino}-benzoyl)-hydrazinecarboxylic acid methyl ester (M23.10),
N'-(3,5-Dibromo-2-{[5-bromo-2-(3-chloro-pyridin-2-yl)-2H-pyr azole-3-carbonyl]-amino}-benzoyl)-N'-methyl-hydrazinecarboxy lic acid methyl ester (M23.11) and
N'-(3,5-Dibromo-2-{[5-bromo-2-(3-chloro-pyridin-2-yl)-2H-pyr azole-3-carbonyl]-amino}-benzoyl)-N,N'-dimethyl-hydrazinecar boxylic acid methyl ester (M23.12);
M.24. Malononitrile compounds: 2-(2,2,3,3,4,4,5,5-octafluoropentyl)-2-(3,3,3-trifluoro-propyl)m alononitrile ($CF_2H-CF_2-CF_2-CF_2-CH_2-C(CN)_2-CH_2-CH_2-CF_3$) (M24.1) and 2-(2,2,3,3,4,4,5,5-octafluoropentyl)-2-(3,3,4,4,4-pentafluorobu tyl)-malonodinitrile ($CF_2H-CF_2-CF_2-CF_2-CH_2-C(CN)_2-CH_2-CH_2-CF_2-CF_3$) (M24.2);
M.25. Microbial disruptors: Bacillus thuringiensis subsp. Israelensi, Bacillus sphaericus, Bacillus thuringiensis subsp. Aizawai, Bacillus thuringiensis subsp. Kurstaki, Bacillus thuringiensis subsp. Tenebrionis;
M.26. Aminofuranone compounds:

4-{[(6-Bromopyrid-3-yl)methyl](2-fluoroethyl)amino}furan-2(5 H)-on (M26.1),
4-{[(6-Fluoropyrid-3-yl)methyl](2,2-difluoroethyl)amino}furan-2(5H)-on (M26.2),
4-{[(2-Chloro1,3-thiazolo-5-yl)methyl](2-fluoroethyl)amino}fur an-2(5H)-on (M26.3),
4-{[(6-Chloropyrid-3-yl)methyl](2-fluoroethyl)amino}furan-2(5 H)-on (M26.4),
4-{[(6-Chloropyrid-3-yl)methyl](2,2-difluoroethyl)amino}furan-2(5H)-on (M26.5),
4-{[(6-Chloro-5-fluoropyrid-3-yl)methyl](methyl)amino}furan-2 (5H)-on (M26.6),
4-{[(5,6-Dichloropyrid-3-yl)methyl](2-fluoroethyl)amino}furan-2(5H)-on (M26.7),
4-{[(6-Chloro-5-fluoropyrid-3-yl)methyl](cyclopropyl)amino}fur an-2(5H)-on (M26.8),
4-{[(6-Chloropyrid-3-yl)methyl](cyclopropyl)amino}furan-2(5H) -on (M26.9) and
4-{[(6-Chloropyrid-3-yl)methyl](methyl)amino}furan-2(5H)-on (M26.10);

M.27. Various insecticides: aluminium phosphide, amidoflumet, benclothiaz, benzoximate, bifenazate, borax, bromopropylate, cyanide, cyenopyrafen, cyflumetofen, chinomethionate, dicofol, fluoroacetate, phosphine, pyridalyl, pyrifluquinazon, sulfur, organic sulfur compounds, tartar emetic, sulfoxaflor, N-R'-2,2-dihalo-1-R"cyclo-propanecarboxamide-2-(2,6-dichloro-α,α,α-trifluoro-p-tolyl) hydrazone or N-R'-2,2-di(R"')propionamide-2-(2,6-dichloro-α,α,α-trifluoro-p-t olyl)-hydrazone, wherein R' is methyl, ethyl, or a halogent selected from chloro or bromo, R" is hydrogen atom or methyl and R"' is methyl or ethyl, 4-But-2-ynyloxy-6-(3,5-dimethyl-piperidin-1-yl)-2-fluoro-pyrimi dine (M-27.1), Cyclopropane acetic acid, 1,1'-[(3S,4R,4aR,6S,6aS,12R,12aS,12bS)-4-[[(2-cyclopropylace tyl)oxy]methyl]-1,3,4,4a,5,6,6a,12,12a,12b-decahydro-12-hydr oxy-4,6a,12b-trimethyl-11-oxo-9-(3-pyridinyl)-2H,11H-naphtho [2,1-b]pyrano[3,4-e]pyran-3,6-diyl] ester(M27.2) and 8-(2-Cyclopropylmethoxy-4-trifluoromethyl-phenoxy)-3-(6-triflu oromethyl-pyridazin-3-yl)-3-aza-bicyclo[3.2.1]octane(M27.3).

Paraoxon and their preparation have been described in Farm Chemicals Handbook, Volume 88, Meister Publishing Company, 2001. Flupyrazofos has been described in Pesticide Science 54, 1988, p.237-243 and in US 4822779. AKD 1022 and its preparation have been described in US 6300348. The anthranilamides M23.1 to M23.6 have been described in WO 2008/72743 and WO 200872783, those M23.7 to M23.12 in W02007/043677. The phthalamide M 21.1 is known from WO 2007/101540. The alkynylether compound M27.1 is described e.g. in JP 2006131529. Organic sulfur compounds have been described in WO 2007060839. The isoxazoline compounds M 22.1 to M 22.8 have been described in e.g. WO 2005/085216, WO 2007/079162, WO 2007/026965, WO 2009/126668 and W02009/051956. The aminofuranone compounds M 26.1 to M 26.10 have been described, for example, in WO 2007/115644. The pyripyropene derivative M 27.2 has been described in WO 2008/66153 and WO 2008/108491. The pyridazin compound M 27.3 has

been described in JP 2008/115155. Malononitrile compounds as those (M24.1) and (M24.2) have been described in WO 02/089579, WO 02/090320, WO 02/090321, WO 04/006677, WO 05/068423, WO 05/068432, and WO 05/063694.

According to another aspect of the present invention, there is provided use of the compound represented by formula (I), (1-a), or (I-a') or agricultural or zootechnically acceptable salt thereof as a harmful organism control agent. Further, according to another aspect of the present invention, there is provided use of the compound represented by formula (I-b) , or (I-c) or agricultural or zootechnically acceptable salt thereof as harmful organism control agents. Furthermore, according to still another aspect of the present invention, there is provided use of a compound represented by formula (I), (1-a), (I-a'), (I-b), or (I-c) or agricultural or zootechnically acceptable salt thereof in the manufacture of a harmful organism control agent.

[EXAMPLES]

The present invention is further illustrated by the following Examples that are not intended as a limitation of the invention.

Synthesis Example 1: Compounds 43-4

Pyripyropene O (30 mg) obtained by a method described in J. Antibiot. 1996, 49, 292 was dissolved in methanol-water (19 : 1, 2 mL), and potassium carbonate (20 mg) was added thereto. The mixture was stirred at room temperature for 22.5 hr, acetic acid (0.1 mL) was added thereto, and the mixture was concentrated. Ethyl acetate and water were added to the concentrate, and the mixture was extracted with ethyl acetate.
The ethyl acetate layer was washed with saturated brine and was dried over anhydrous sodium sulfate, and the solvent was removed by evaporaiton under the reduced pressure to give a crude product of 1.11-di-deacetyl pyripyropene O. The crude product was purified by preparative thin layer chromatography (Merck Silica Gel 60F254, 0.5mm, hexane : acetone = 1 : 1) to give compound 43-4 (23 mg). ESI-MS;426 m/z $(M+H)^+$; $^1$H-NMR ($CDCl_3$) $\delta$ 0.89 (3H, s), 0.97 (3H, s), 1.14 (1H, dt, J = 4.2, 12.8 Hz), 1.20-1.25 (1H, m), 1.28 (3H, s), 1.45-1.59 (3H, m), 1.64-1.75 (3H, m), 1.82 (1H, dt, J = 3.5, 9.6 Hz), 2.11-2.14 (1H, m), 2.25 (1H, dd, J = 12.8, 17.1 Hz), 2.54 (1H, dd, J = 4.6, 17.1 Hz), 3.45 (1H, d, J = 10.3 Hz), 3.68 (1H, dd, J = 5.0, 11.2 Hz), 3.75 (1H, d, J = 10.3 Hz), 6.42 (1H, s), 7.39 (1H, dd, J = 4.8, 8.0 Hz), 8.10 (1H, ddd, J = 1.6, 2.0, 8.0 Hz), 8.65 (1H, dd, J = 1.6, 4.8 Hz), 8.99 (1H, d, J = 2.0 Hz)

Synthesis Example 2: Compounds 43-262 and 43-705

Compound 43-4 (22 mg) obtained in Synthesis Example 1 was suspended in ethyl acetate (1 mL), and pyridine (20 mg) and cyclopropane carbonyl chloride (22 mg) were added to the suspension. The mixture was then stirred at room temperature for 4 hr. Water was added thereto, and the mixture was extracted with ethyl acetate. The ethyl acetate layer was washed with saturated brine and was dried over anhydrous sodium sulfate, and the solvent was removed by evaporation under the reduced pressure to gtive crude products of 1,11-di-O-cyclopropanecarbonyl 1.11-di-deacetyl pyripyropene O and 11-O-cyclopropanecarbonyl 1.11-di-deacetyl pyripyropene O. The crude products were purified by preparative thin layer chromatography (Merck Silica Gel 60F254, 0.5 mm, chloroform : methanol = 10 : 1) to give compound 43-262 (17 mg) and compound 43-705 (4 mg).

Compound 43-262

ESI-MS;562 m/z $(M+H)^+$; $^1$H-NMR ($CDCl_3$) $\delta$ 0.88 (3H, s), 0.99 (3H, s), 0.84-1.08 (8H, m), 1.21 (1H, dt, J = 3.6, 13.4 Hz), 1.28 (3H, s), 1.43-1.48 (2H, m), 1.56-1.73 (6H, m), 1.81-1.85 (2H, m), 2.13-2.16 (1H, m), 2.26 (1H, dd, J = 12.8, 17.1 Hz), 2.55 (1H, dd, J = 4.6, 17.1 Hz), 3.71 (1H, d, J = 11.7 Hz), 3.93 (1H, d, J = 11.7 Hz), 4.82 (1H, dd, J = 4.7, 12.0 Hz), 6.44 (1H, s), 7.41 (1H, dd, J = 4.8, 8.0 Hz), 8.12 (1H, ddd, J = 1.4, 2.0, 8.0 Hz), 8.66 (1H, dd, J = 1.4, 4.8 Hz), 9.00 (1H, d, J = 2.0 Hz)

Compound 43-705

ESI-MS;494 m/z $(M+H)^+$; $^1$H-NMR ($CDCl_3$) $\delta$ 0.80 (3H, s), 0.89-0.94 (2H, m), 0.96 (3H, s), 1.00-1.04 (2H, m), 1.13 (1H, dt, J = 4.2, 12.8 Hz), 1.29 (3H, s), 1.30-1.33 (1H, m), 1.42-1.52 (1H, m), 1.57 (1H, dd, J = 4.8, 12.8 Hz), 1.60-1.65 (1H, m), 1.66-1.70 (1H, m), 1.73-1.76 (3H, m), 1.83 (1H, dt, J = 3.2, 13.2 Hz), 2.14-2.18 (1H, m), 2.26 (1H, dd, J = 12.8, 17.2 Hz), 2.56 (1H, dd, J = 4.8, 17.2 Hz), 3.42 (1H, dd, J = 5.6, 10.4 Hz), 3.79 (1H, d, J = 11.2 Hz), 4.28 (1H, d, J = 11.2 Hz), 6.43 (1H, s), 7.40 (1H, dd, J = 5.2, 8.0 Hz), 8.11 (1H, ddd, J = 1.6, 2.0, 8.4 Hz), 8.66 (1H, m), 9.00 (1H, m)

Synthesis Example 3: Compound 43-5

Pyripyropene E (29 mg) obtained by a method described in Japanese Patent Application Laid-Open No. 239385/1996 was dissolved in methanol-water (19 : 1, 1 mL), and potassium carbonate (53 mg) was added to the solution. The mixture was stirred at room temperature for 20.5 hr. Acetic acid (0.1 mL) was then added thereto, and the mixture was concetrated under the reduced pressure. A mixed solvent composed of chloroform-methanol (10 : 1, 1 mL) was added to the concentrate, and insolubles were removed by filtration. The solvent was then removed by evaporation under the reduced pressure to give a crude product of 1-deacetyl pyripyropene E. The crude product was purified by preparative thin layer chromatography (Merck Silica Gel 60F254, 0.5mm, chloroform : methanol = 10 : 1) to give compound 43-5 (18 mg).
ESI-MS;410 m/z $(M+H)^+$; $^1$H-NMR ($CDCl_3$) $\delta$ 0.82 (3H, s), 0.92 (3H, s), 0.99-1.02 (1H, m), 1.03 (3H, s), 1.12 (1H, dt, J = 4.0, 12.8 Hz), 1.27 (3H, s), 1.40-1.46 (1H, m), 1.50 (1H, dd, J = 4.4, 12.8 Hz), 1.62-1.74 (3H, m), 1.79-1.83 (2H, m), 2.14 (1H, dt, J = 3.2, 12.4 Hz), 2.24 (1H, dd, J = 12.4, 16.8 Hz), 2.53 (1H, dd, J = 4.8, 16.8 Hz), 3.25 (1H, dd, J = 4.0, 11.2 Hz), 6.42 (1H, s), 7.38 (1H, dd, J = 4.8, 8.0 Hz), 8.10 (1H, ddd, J = 1.6, 2.0, 8.0 Hz), 8.65 (1H, dd, J = 1.6, 4.8 Hz), 8.99 (1H, d, J = 2.0 Hz)

Synthesis Example 4: Compound 43-713

Compound 43-5 (10 mg) obtained in Synthesis Example 3 was suspended in ethyl acetate (1 mL), and pyridine (10 mg) and cyclopropane carbonyl chloride (10 mg) were added to the suspension. The mixture was stirred at room temperature for 4 hr. Water was then added, and the mixture was extracted with ethyl acetate. The ethyl acetate layer was washed with saturated brine and was dried over anhydrous sodium sulfate. The solvent was then removed by evaporation under the reduced pressure to give a crude product of 1-o-cyclopropanecarbonyl 1-deacetyl pyripyropene E. The crude product was purified by preparative thin layer chromatography (Merck Silica Gel 60F254 0.5 mm, chloroform : methanol = 10 :1 ) to give compound 43-713 (8 mg).
ESI-MS;478 m/z $(M+H)^+$; $^1$H-NMR ($CDCl_3$) $\delta$ 0.84-0.88 (2H, m), 0.91 (3H, s), 0.92 (3H, s), 0.95 (3H, s), 0.98-1.01 (2H, m), 1.07-1.11 (1H, m), 1.18 (1H, dt, J = 3.6, 13.1 Hz), 1.27 (3H, s), 1.40-1.48 (1H, m), 1.52 (1H, dd, J = 4.8, 12.8 Hz), 1.59-1.74 (4H, m), 1.79-1.83 (2H, m), 2.14 (1H, dt, J = 3.1, 12.6 Hz), 2.24 (1H, dd, J = 13.0, 17.2 Hz), 2.52 (1H, dd, J = 4.7, 17.2 Hz), 4.51 (1H, dd, J = 4.8, 11.6 Hz), 6.43 (1H, s), 7.39 (1H, dd, J = 4.8, 8.0 Hz), 8.11 (1H, ddd, J = 1.6, 1.6, 8.0 Hz), 8.66 (1H, dd, J = 1.6, 4.8 Hz), 9.00 (1H, d, J = 1.6 Hz)

Synthesis Example 5: Compound 43-1

An aqueous sodium hydroxide solution was added to an ethyl acetate extract of culture broth, and the mixture was stirred overnight. The insolubles were then separated by filtration. The filtrate was concentrated under reduced pressure to give a crude product (5.0 g) of compound 43-1. The crude product was purified by silica gel column chromatography (Wako Gel C300, dichloromethane : methanol = 90:10 $\rightarrow$ 0:100) and was then purified by preparative HPLC (acetonitrile : water = 20 : 80, L-column 4.6$\times$150 mm) to give compound 43-1(440 mg).
ESI-MS;442 m/z $(M+H)^+$; $^1$H-NMR (DMSO-$d_6$) $\delta$0.55 (3H, s), 0.85 (3H, s), 0.97-1.03 (1H, m), 1.14 (3H, s), 1.26-1.34 (1H, m), 1.37-1.44 (2H, m), 1.53-1.57 (3H, m), 1.72 (1H, m), 2.19 (1H, dd, J = 12.6, 17.0 Hz), 2.31 (1H, dd, J = 4.8, 17.0 Hz), 3.06 (1H, dd, J = 4.8, 10.5 Hz), 3.35-3.38 (1H, m), 3.43-3.47 (1H, m), 3.60 (1H, m), 4.24 (1H, d, J = 5.1 Hz), 4.51 (1H, t, J = 5.0 Hz), 4.99 (1H, d, J = 5.2 Hz), 6.91 (1H, s), 7.51 (1H, dd, J = 4.8, 7.9 Hz), 8.21 (1H, ddd, J = 1.8, 2.0, 7.9 Hz), 8.65 (1H, dd, J = 1.8, 4.8 Hz), 9.03 (1H, d, J = 2.0 Hz)

Synthesis Example 6: Compound 43-259

Compound 43-1 (100 mg) was suspended in ethyl acetate (2 mL), and pyridine (0.4 mL) and cyclopropane carbonyl chloride (237 mg) were added to the suspension. The mixture was stirred at room temperature for 14 hr. Water was then added, and the mixture was extracted with ethyl acetate. The ethyl acetate layer was washed with saturated brine and was dried over anhydrous sodium sulfate. The solvent was then removed by evaporation under the reduced pressure to give a crude product. The crude product was purified by preparative thin layer chromatography (Merck Silica Gel 60F254 0.5 mm, hexane : acetone = 1 : 1) to give compound 43-259 (19 mg).
ESI-MS; 646 m/z $(M+H)^+$; $^1$H-NMR ($CDCl_3$) $\delta$ 0.84-0.89 (4H, m), 0.89 (3H, s), 0.91-0.99 (4H, m), 1.02 (3H, s), 1.03-1.11 (4H, m), 1.16-1.24 (1H, m), 1.34 (3H, s), 1.50-1.77 (7H, m), 1.81-1.91 (3H, m), 2.34 (1H, dd, J = 12.8, 17.2 Hz), 2.58 (1H, dd, J = 4.8, 17.2 Hz), 3. 73 (1H, d, J = 11.6 Hz), 3.84 (1H, d, J = 11.6 Hz), 4.79 (1H, dd, J = 4.8, 11.6 Hz), 5.04 (1H, dd, J = 4.8, 11.6 Hz), 6.43 (1H, s), 7.38 (1H, dd, J = 4.8, 8.0 Hz), 8.10 (1H, ddd, J = 2.0, 2.0, 8.0 Hz), 8.65 (1H, dd, J = 2.0, 4.8 Hz), 9.00 (1H, d, J = 2.0 Hz)

Synthesis Example 7: Compound 43-260

Compound 43-259 (340 mg) was suspended in methanol-water (9:1, 10 mL), and 1,8-diazabicyclo[5.4.0]-7-undecene (40 mg) was added thereto. The mixture was stirred at room temperature for one hr, and 1,8-diazabicyclo [5.4.0]-7-undecene (40 mg) was then added thereto. The mixture was stirred for additional 4.5 hr. Acetic acid (0.1 mL) was then added thereto, and the reaction was stopped. The reaction solution was concentrated under reduced pressure. Water was added to the residue, and the mixture was extracted with ethyl acetate. The ethyl acetate layer was washed with saturated brine and was dried over anhydrous magnesium sulfate. The solvent was then removed by evaporation under the reduced pressure to give a crude product. The crude product was purified by silica gel column chromatography (Mega Bond Elut (Varian), hexane : acetone = 5 : 2 → 1 : 1) and preparative thin layer chromatography (Merck Silica Gel 60F254 0.5 mm, chloroform : methanol = 10 : 1) to give compound 43-260 (57 mg).
ESI-MS; 578 m/z $(M+H)^+$; $^1$H-NMR ($CDCl_3$) δ 0.83-0.90 (4H, m), 0.89 (3H, s), 0.96-1.04 (4H, m), 1.00 (3H, s), 1.15-1.22 (1H, m), 1.29 (3H, s), 1.50-1.62 (5H, m), 1.65-1.76 (1H, m), 1.80-1.89 (3H, m), 2.34 (1H, dd, J = 13.2, 16.8 Hz), 2.56 (1H, dd, J = 4.8, 16.8 Hz), 3.77 (1H, d, J = 11.6 Hz), 3.81-3.84 (1H, m), 3.87 (1H, d, J = 11.6 Hz), 4.81 (1H, dd, J = 4.8, 11.6 Hz), 6.49 (1H, s), 7.40 (1H, dd, J = 4.8, 8.0 Hz), 8.11 (1H, ddd, J = 1.6, 1.6, 8.0 Hz), 8.67 (1H, dd, J = 1.6, 4.8 Hz), 8.99 (1H, d, J = 1.6 Hz)

Synthesis Example 8: Compound 1-6

Compound 1-6 (20 mg) was obtained in the same manner as in Synthesis Example 5.
ESI-MS; 442 m/z $(M+H)^+$; $^1$H-NMR (DMSO-$d_6$) δ0.70 (3H, s), 0.90 (3H, s), 0.97 (1H, m), 1.14-1.19 (1H, m), 1.23 (3H, s), 1.25 (1H, d, J = 3.3 Hz), 1.42-1.49 (1H, m), 1.52 (3H, s), 1.55-1.61 (2H, m), 1.73 (1H, dd, J = 4.2, 13.0 Hz), 1.95 (1H, m), 3.03 (1H, ddd, J = 4.8, 5.7, 11.1 Hz), 3.57 (1H, ddd, J = 5.0, 5.9, 11.6 Hz), 4.38 (1H, d, J = 5.1 Hz), 4.75 (1H, dd, J = 3.4, 5.5 Hz), 4.98 (1H, d, J = 5.2 Hz), 5.26 (1H, d, J = 5.7 Hz), 6.90 (1H, s), 7.53 (1H, dd, J = 4.8, 8.0 Hz), 8.22 (1H, ddd, J = 1.9, 1.9, 8.3 Hz), 8.67 (1H, dd, J = 1.4, 4.8 Hz), 9.05 (1H, d, J = 1.8 Hz)

Synthesis Example 9: Compound 8-260

Compound 8-260 (10 mg) was obtained in the same manner as described in W02009/022702 using as a starting material a crude product containing phenylpropene A described in J. Antibiot. 50(3), 229, 1997.
ESI-MS;615 m/z $(M+Na)^+$; $^1$H-NMR ($CDCl_3$) δ 0.83-0.89 (4H, m), 0.91 (3H, s), 0.96-1.01 (4H, m), 1.35 (1H, dt, J = 4.7, 13.4 Hz), 1.42 (3H, s), 1.45 (1H, m), 1.49 (1H, m), 1.55-1.63 (3H, m), 1.65 (3H, s), 1.81-1.91 (3H, m), 2.16 (1H, dt, J = 3.5, 13.2 Hz), 3.75 (1H, d, J = 11.8 Hz), 3.77-3.81 (1H, m), 3.86 (1H, d, J = 11.8 Hz), 4.82 (1H, dd, J = 5.0, 11.5 Hz), 4.99 (1H, d, J = 4.2 Hz), 6.45 (1H, s), 7.42-7.48 (3H, m), 7.78-7.81 (2H, m)

Synthesis Example 10: Compound 1-709

Compound 1-6 (28 mg) was suspended in N,N-dimethylformamide (1 mL). Pyridine (45 mg) and cyclopropane carbonyl chloride (40 mg) were added to the suspension. The mixture was stirred at 0°C for 2 hr. Water was then added, and the mixture was extracted with ethyl acetate. The ethyl acetate layer was washed with saturated brine and was dried over anhydrous magnesium sulfate. The solvent was removed by evaporation under the reduced pressure, and the residue was dried. The residue was then suspended in ethyl acetate (1 mL), and pyridine(100 mg) and cyclopropane carbonyl chloride (100 mg) were added thereto. The mixture was stirred at room temperature for 19 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product of compound 1-709. The crude product was purified by preparative thin layer chromatography (Merck Silica Gel 60F254 0.5 mm, hexane : acetone = 1 : 1) to give compound 1-709 (12 mg).
ESI-MS;578 m/z $(M+H)^+$; $^1$H-NMR ($CDCl_3$) δ 0.85-0.87 (4H, m), 0.91 (3H, s), 0.93 (3H, s), 0.95-1.00 (4H, m), 1.09-1.19 (1H, m), 1.29-1.38 (1H, m), 1.41 (3H, s), 1.46 (1H, d, J = 4.0 Hz), 1.58-1.67 (3H, m), 1.72 (3H, s), 1.77-1.81 (2H, m), 1.99 (1H, dd, J = 4.0, 13.2 Hz), 2.14 (1H, m), 4.51 (1H, t, J = 8.4 Hz), 4.92-5.03 (2H, m), 6.51 (1H, s), 7.53 (1H, m), 8.23 (1H, m), 8.72 (1H, m), 9.06 (1H, m)

Synthesis Example 11: Compound 1-700

1,7,11-Tri-deacetyl pyripyropene A (20 mg) synthesized by a method described in Japanese Patent Laid-Open No. 259569/1996 and cyclopropanecarboxylic acid (19 mg) were dissolved in N,N-dimethylformamide (1 mL). 1-Ethyl-3-(3-dimethylaminopropyl)carbodiimide hydrochloride (84 mg) and 4-dimethylaminopyridine (5 mg) were added to the solution. The mixture was stirred at room temperature for 6 hr. Water was then added, and the mixture was extracted

with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, chloroform : methanol = 10 : 1) to give compound 1-700 (8 mg).
ESI-MS;594 m/z $(M+H)^+$; $^1H$-NMR ($CDCl_3$) δ 0.82 (3H, s), 0.89-0.98 (4H, m), 1.02-1.13 (4H, m), 1.28 (1H, dt, J = 4.4, 12.0 Hz), 1.39-1.42 (1H, m), 1.42 (3H, s), 1.51 (1H, d, J = 4.0 Hz), 1.61-1.73 (3H, m), 1.72 (3H, s), 1.81-1.84 (2H, m), 1.90 (1H, m), 2.16 (1H, m), 3.37 (1H, dd, J = 5.2, 11.2 Hz), 3.62 (1H, d, J = 12.0 Hz), 4.35 (1H, d, J = 12.0 Hz), 5.00 (1H, d, J = 4.4 Hz), 5.02-5.06 (1H, m), 6.46 (1H, s), 7.42 (1H, m), 8.11 (1H, d, J = 8.0 Hz), 8.70 (1H, m), 9.02 (1H, m)

Synthesis Example 12: Compound 1-948

1,7,11-Tri-deacetyl pyripyropene A (20 mg) synthesized by a method described in Japanese Patent Laid-Open No. 259569/1996 and cyclopropanecarboxylic acid (19 mg) were dissolved in anhydrous N,N-dimethylformamide (1 ml), and 1-ethyl-3-(3-dimethylaminopropyl)-carbodiimide hydrochloride (84 mg) and 4-dimethylaminopyridine (5 mg) were added to the solution. The mixture was stirred at room temperature for 6 hr, the reaction solution was poured into water, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer column chromatography (Merck Silica Gel $60F_{254}$ 0.5 mm, chloroform : methanol = 10 : 1) to give compound 1-948 (9.0 mg).
ESI-MS;m/z 526 $(M+H)^+$; $^1H$-NMR ($CDCL_3$) δ 0.83 (3H, s), 0.88-0.95 (2H, m), 1.00-1.08 (2H, m), 1.26 (1H, m), 1.33 (1H, m), 1.40 (3H, s), 1.43 (1H, m), 1.57-1.74 (2H, m), 1.67 (3H, s), 1.79-1.88 (2H, m), 1.93 (1H, m), 2.15 (1H, m), 2.97 (1H, s), 3.41 (1H, dd, J = 5.2, 11.2 Hz), 3.75 (1H, d, J = 11.6 Hz), 3.82 (1H, dd, J = 5.2, 11.6 Hz), 4.28 (1H, d, J = 11.6 Hz), 5.00 (1H, d, J = 4.0 Hz), 6.53 (1H, s), 7.43 (1H, dd, J = 4.4, 8.0 Hz), 8.12 (1H, dt, J = 8.4 Hz), 8.70 (1H, m), 9.02 (1H, m)

Synthesis Example 13: Compound 1-1346

1,11-O-acetonide-1,7,11-tri-deacetyl pyripyropene A (100 mg) synthesized by a method described in WO 2009/022702 was suspended in ethyl acetate (2 mL), and pyridine (63 mg) and cyclopropane carbonyl chloride (63 mg) were added to the suspension. The mixture was stirred at room temperature for 9 hr. Pyridine (63 mg) and cyclopropane carbonyl chloride (63 mg) were added thereto. The mixture was stirred at room temperature for additional 14 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone =1 : 1) to give compound 1-1346 (10 mg).
ESI-MS;566 m/z $(M+H)^+$; $^1H$-NMR ($CDCl_3$) δ 0.95-0.98 (2H, m), 1.10 (3H, s), 1.04-1.15 (3H, m), 1.37 (1H, dt, J = 3.2, 13.2 Hz), 1.43 (3H, s), 1.44 (6H, s), 1.50 (1H, d, J = 4.0 Hz), 1.58-1.66 (3H, m), 1.71 (3H, s), 1.68-1.74 (1H, m), 1.76-1.83 (1H, m), 2.22 (1H, m), 3.04 (1H, brs), 3.48 (2H, s), 3.54 (1H, dd, J = 3.6, 12.0 Hz), 4.97-5.01 (2H, m), 6.47 (1H, s), 7.42 (1H, dd, J = 4.8, 8.0 Hz), 8.11 (1H, ddd, J = 2.0, 2.0, 8.0 Hz), 8.69 (1H, dd, J = 2.0, 4.8 Hz), 9.02 (1H, d, J = 2.0 Hz)

Synthesis Example 14: Compound 1-703

Compound 1-1346 (42 mg) obtained in Synthesis Example 13 was dissolved in tetrahydrofuran (0.5 mL), water (0.6 mL) and acetic acid (1.0 mL) were added at 0°C, and the mixture was stirred at room temperature for 21 hr, followed by concentration under the reduced pressure. An aqueous sodium hydrogencarbonate solution and chloroform were added to the residue, and the insolubles were collected by filtration to give compound 1-703 (16 mg).
ESI-MS;526 m/z $(M+H)^+$; $^1H$-NMR (DMSO-$d_6$) δ 0.57 (3H, s), 0.89-0.99 (4H, m), 1.14-1.25 (1H, m), 1.30 (3H, s), 1.40-1.70 (6H, m), 1.67 (3H, s), 1.78 (1H, m), 1.94 (1H, m), 2.99 (1H, m), 3.34 (1H, m), 3.46 (1H, m), 4.27 (1H, d, J = 4.8 Hz), 4.51 (1H, t, J = 4.8 Hz), 4.78 (1H, m), 4.88 (1H, dd, J = 5.2, 11.2 Hz), 5.43 (1H, d, J = 5.6 Hz), 6.88 (1H, s), 7.52 (1H, dd, J = 4.8, 8.0 Hz), 8.27 (1H, d, J = 8.0 Hz), 8.66 (1H, d, J = 4.8 Hz), 9.08 (1H, s)

Synthesis Example 15: Compound 43-1343

Compound 43-4 (1.0 g) was suspended in N,N-dimethylformamide (15 mL), and 2,2-dimethoxypropane (1.5 g) and p-toluenesulfonic acid monohydrate (150 mg) were added to the suspension. The mixture was stirred at room temperature for 17 hr. The reaction was stopped by triethylamine, water was added thereto, and the mixture was extracted with ethyl acetate. The ethyl acetate layer was washed with saturated brine and was dried over anhydrous sodium sulfate, and the solvent was then removed by evaporation under the reduced pressure to give a crude product . The crude product was purified by silica gel column chromatography (Mega Bond Elut (Varian), hexane : acetone = 4 : 1 →

1 : 1) to give compound 43-1343 (58 mg).
ESI-MS;466 m/z $(M+H)^+$; $^1$H-NMR ($CDCl_3$) $\delta$ 0.97 (3H, s), 1.08 (3H, s), 1.19 (1H, dt, J = 3.6, 13.2 Hz), 1.27 (3H, s), 1.41-1.46 (2H, m), 1.43 (3H, s), 1.45 (3H, s), 1.49-1.58 (3H, m), 1.64-1.71 (2H, m), 1.87 (1H, dt, J = 3.6, 13.2 Hz), 2.12 (1H, dt, J = 3.2, 12.4 Hz), 2.25 (1H, dd, J = 13.2, 17.2 Hz), 2.53 (1H, dd, J = 4.8, 17.2 Hz), 3.48 (1H, d, J = 10.8 Hz), 3. 51 (1H, m), 3.55 (1H, d, J = 10.8 Hz), 6.42 (1H, s), 7.39 (1H, dd, J = 4.8, 8.0 Hz), 8.10 (1H, ddd, J = 1.6, 2.0, 8.0 Hz), 8.66 (1H, dd, J = 1.6, 4.8 Hz), 8.99 (1H, d, J = 2.0 Hz)

Synthesis Example 16: Compound 134-749

1,7,11-Trideacetyl pyripyropene A (20 mg) synthesized by a method described in Japanese Patent Laid-Open No. 259569/1996 was dissolved in tetrahydrofuran (1 mL), and diisopropylethylamine (56 mg), and methoxymethyl bromide (82 mg) were added to the solution. The mixture was stirred at room temperature for 3 hr. Water was then added, and the mixture was extracted with ethyl acetate. The ethyl acetate layer was washed with saturated brine and was dried over anhydrous sodium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 134-749 (7 mg). ESI-MS;634 m/z $(M+H)^+$

Synthesis Example 17: Compound 135-751

1,7,11-Trideacetyl-1,11-di-O-propionyl pyripyropene A (30 mg) synthesized by a method described in WO 2006/129714 was dissolved in dichloromethane (2 mL), and 3,4-dihydro-2H-pyran (155 mg) and pyridinehydrochloride (18 mg) were added to the solution. The mixture was stirred at room temperature for 26 hr. Water was then added, and the mixture was extracted with chloroform. The chloroform layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 135-751 (9 mg). ESI-MS;738 m/z $(M+H)^+$

Synthesis Example 18: Compound 134-752

1,7,11-Trideacetyl-1,11-di-O-propionyl pyripyropene A (20 mg) synthesized by a method described in WO 2006/129714 was dissolved in tetrahydrofuran (2 mL). Diisopropylethylamine (18 mg) and methoxymethyl bromide (31 mg) were added to the solution. The mixture was stirred at room temperature for 16 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone =1:1) to give compound 134-752 (10 mg).
ESI-MS;658 m/z $(M+H)^+$

Synthesis Example 19: Compound 139-136

1,7,11-Trideacetyl-1,11-di-O-propionyl pyripyropene A (30 mg) synthesized by a method described in WO 2006/129714 was dissolved in N,N-dimethylformamide (1 mL). Triethylamine (64 mg), 4-dimethylaminopyridine(12 mg), and 1-propyl isocyanate (27 mg) were added to the solution. The mixture was stirred at room temperature for 24 hr. Triethylamine (64 mg), N,N-dimethylaminopyridine(12 mg), and 1-propyl isocyanate (27 mg) were then added thereto. The mixture was stirred at room temperature for additional four days. Water was then added, and the mixture was extracted with ethyl acetate. The ethyl acetate layer was washed with saturated brine and was dried over anhydrous sodium sulfate, and the solvent was removed by evaporation under the reduced pressure to give a crude product. The crude product was purified by preparative thin layer chromatography (Merck Silica Gel 60F254 0.5 mm, hexane : acetone = 1 :1) to give compound 139-136 (25 mg).
ESI-MS;740 m/z $(M+H)^+$

Synthesis Example 20: Compound 92-111

Compound 92-1 (18 mg) synthesized by a method described in J. Antibiot., 50 (3), 229, 1997 was dissolved in N,N-dimethylformamide (1 mL). Triethylamine (37 mg), 4-dimethylaminopyridine (0.5 mg), and propionic acid anhydride (16 mg) were added to the solution. The mixture was stirred at room temperature for 6 hr. Water was then added, and the mixture was extracted with ethyl acetate. The ethyl acetate layer was washed with saturated brine and was dried over anhydrous magnesium sulfate, and the solvent was removed by evaporation under the reduced pressure to give

a crude product. The crude product was purified by preparative thin layer chromatography (Merck Silica Gel 60F254 0.5 mm, hexane : acetone = 1 : 1) to give compound 92-111 (7 mg).
ESI-MS;624 m/z $(M+H)^+$

Synthesis Example 21: Compound 4-111

Compound 92-111 (7 mg) was suspended in ethanol (0.5 mL), and cerium chloride heptahydrate (42 mg) was added to the suspension at room temperature. The mixture was cooled at 0°C, and sodium boron hydride (4 mg) was added thereto. The mixture was stirred for 6 hr, water was then added, and the mixture was extracted with ethyl acetate. The ethyl acetate layer was washed with saturated brine and was dried over anhydrous sodium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 4-111 (5 mg).
ESI-MS;626 m/z $(M+H)^+$

Synthesis Example 22: Compound 93-111

Compound 93-1 (28 mg) synthesized by a method described in J. Antibiot., 50(3), 229, 1997 was dissolved in N,N-dimethylformamide (1 mL). Triethylamine (55 mg), 4-dimethylaminopyridine (0.7 mg), and propionic acid anhydride (24 mg) were added to the solution. The mixture was stirred at room temperature for 6 hr, water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 93-111 (7 mg).
ESI-MS;624 m/z $(M+H)^+$

Synthesis Example 23: Compound 5-111

Compound 93-111 (7 mg) was suspended in ethanol (0.5 mL). Cerium chloride heptahydrate (42 mg) was added to the suspension at room temperature. The mixture was cooled to 0°C, and sodium boron hydride (4 mg) was added thereto. The mixture was stirred for 6 hr, water was then added, and the mixture was extracted with ethyl acetate. The ethyl acetate layer was washed with saturated brine and was dried over anhydrous sodium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 5-111 (1 mg).
ESI-MS;626 m/z $(M+H)^+$

Synthesis Example 24: Compound 103-8

Compound 89-8 (20 mg) synthesized by a method described in J. Antibiot., 49(11), 1133, 1996 was suspended in ethanol -water (10 : 1, 2 mL). Benzylamine (184 mg) was added to the suspension at room temperature, the mixture was stirred for 38 hr and was concentrated under the reduced pressure. Chloroform and water were added, and the mixture was extracted with chloroform. The chloroform layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 103-8 (14 mg).
ESI-MS;671 m/z $(M+H)^+$

Synthesis Example 25: Compound 19-8

Compound 103-8 (36 mg) was suspended in methanol (1 mL), and cerium chloride heptahydrate (36 mg) was added at room temperature. The mixture was cooled to 0°C, and sodium boron hydride (20 mg) was then added thereto. The mixture was stirred for one hr and was then concentrated under the reduced pressure. Chloroform and water were added, and the mixture was extracted with chloroform. The chloroform layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 19-8 (3 mg).
ESI-MS;673 m/z $(M+H)^+$

Synthesis Example 26: Compound 107-8

Compound 89-8 (20 mg) synthesized by a method described in J. Antibiot., 49 (11), 1133, 1996 was suspended in N,N-dimethylformamide (1 mL), N-chlorosuccinimide (6 mg) was added to the suspension at room temperature, and the mixture was stirred for 4 days. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 107-8 (3 mg).
ESI-MS; 616 m/z $(M+H)^+$

Synthesis Example 27: Compound 32-8

Pyripyropene A (30 mg) was dissolved in N,N-dimethylformamide (2 mL), N-bromosuccinimide (18 mg) was added to the solution at room temperature, and the mixture was stirred for 14 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone =1 : 1) to give compound 32-8 (18 mg).
ESI-MS;662 m/z $(M+H)^+$

Synthesis Example 28: Compound 109-8

Compound 89-8 (20 mg) synthesized by a method described in J. Antibiot., 49(11), 1133, 1996 was suspended in N,N-dimethylformamide (1 mL), N-bromosuccinimide (12 mg) was added to the suspension at room temperature, and the mixture was stirred for 22 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 109-8 (4 mg).
ESI-MS;660 m/z $(M+H)^+$

Synthesis Example 29: Compound 72-113

1,7,11-Trideacetyl-1,11-di-O-propionyl pyripyropene A (30 mg) synthesized by a method described in WO 2006/129714 was dissolved in dimethylsulfoxide (0.6 mL). Acetic acid anhydride (0.6 mL) and acetic acid (0.6 mL) were added to the solution. The mixture was stirred at room temperature for 24 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 72-113 (5 mg).
ESI-MS;550 m/z $(M+H)^+$

Synthesis Example 30: Compound 1-145

1,7,11-Trideacetyl-1,11-di-O-propionyl pyripyropene A (20 mg) synthesized by a method described in WO 2006/129714 and benzo[b]thiophene-2-carboxylic acid (19 mg) were dissolved in N,N-dimethylformamide (1 mL). 1-Ethyl-3-(3-dimethylaminopropyl)carbodiimide hydrochloride (28 mg) and 4-dimethylaminopyridine (8 mg) were added to the solution, and the mixture was stirred at room temperature for 12 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-145 (24 mg).
ESI-MS;730 m/z $(M+H)^+$

Synthesis Example 31: Compound 1-146

1,7,11-Trideacetyl-1,11-di-O-propionyl pyripyropene A (20 mg) synthesized by a method described in WO

2006/129714 and 3,4- methylenedioxybenzoic acid (35 mg) was dissolved in N,N-dimethylformamide (1 mL). 1-Ethyl-3-(3-dimethylaminopropyl)carbodiimide hydrochloride (28 mg) and 4-dimethylaminopyridine (8 mg) were added to the solution. The mixture was stirred at room temperature for 6 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-146 (8 mg).
ESI-MS; 718 m/z $(M+H)^+$

Synthesis Example 32: Compound 1-3

Compound 1-261 (4.1 g) synthesized by a method described in WO 2008/066153 was suspended in methanol-water (19 : 1, 40 mL), potassium carbonate (5.8 g) was added to the suspension at room temperature, and the mixture was stirred for 24 hr and was concentrated under the reduced pressure. Ethyl acetate and water were added to the concentrate. The insolubles were collected by suction filtration and were washed with ethyl acetate to give compound 1-3 (2.1 g).
ESI-MS;456 m/z $(M+H)^+$ ; $^1$H-NMR (DMSO-$d_6$) $\delta$ 0.61 (3H, s), 1.20-1.26 (1H, m), 1.47 (3H, s), 1.53-1.62 (2H, m), 1.69 (3H, s), 1.72 (2H, m), 2.01-2.05 (1H, m), 2.33 (1H, d, J = 13.2 Hz), 2.82 (1H, dd, J = 4.6, 4.7 Hz), 2.97 (1H, dd, J = 4.9, 10.5 Hz), 3.36 (1H, m), 3.43-3.48 (1H, m), 4.34 (1H, d, J = 5.1 Hz), 4.54 (1H, dd, J = 4.6, 4.7 Hz), 4.82 (1H, dd, J = 3.2, 5.6 Hz), 5.57 (1H, d, J = 5.8 Hz), 7.10 (1H, s), 7.54 (1H, dd, J = 4.9, 8.1 Hz), 8.26 (1H, ddd, J = 1.2, 2.2, 8.0 Hz), 8.67 (1H, dd, J = 1.2, 4.9 Hz), 9.09 (1H, d, J = 2.2 Hz)

Synthesis Example 33: Compound 1-1382

Compound 1-3 (700 mg) obtained in Synthesis Example 32 was dissolved in pyridine (9 mL), and tert-butyldimethylsilyl chloride (700 mg) was added to the solution. The mixture was stirred at room temperature for 16 hr and was then concentrated under the reduced pressure. Water was added, and the mixture was extracted with ethyl acetate. The ethyl acetate was washed with saturated brine and was dried over anhydrous magnesium sulfate, and the solvent was removed by evaporation under the reduced pressure to give a crude product. The crude product was purified by column chromatography on silica gel (Mega Bond Elut (Varian), hexane : acetone =3:1) to give compound 1-1382 (525 mg).
ESI-MS;570 m/z $(M+H)^+$

Synthesis Example 34: Compound 1-1386

Compound 1-1382 (600 mg) obtained in Synthesis Example 33 was dissolved in N,N-dimethylformamide (5 mL). Pyridine (500 mg) and cyclopropane carbonyl chloride (550 mg) were added to the solution at 0°C. The mixture was stirred at that temperature for 4 hr. Pyridine (500 mg) and cyclopropane carbonyl chloride (550 mg) were added. The mixture was stirred for additional 2 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by column chromatography on silica gel (Mega Bond Elut (Varian), hexane : acetone = 10 : 3) to give compound 1-1386 (627 mg).
ESI-MS;638 m/z $(M+H)^+$

Synthesis Example 35: Compound 1-708

Compound 1-1386 (300 mg) obtained in Synthesis Example 34 was dissolved in tetrahydrofuran (1.5 mL). Pyridine (0.45 mL) and hydrogen fluoride-pyridinecomplex (0.54 mL) were added at 0°C. The mixture was stirred at room temperature for 14 hr and was cooled to 0°C. An aqueous sodium hydrogencarbonate solution was added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by column chromatography on silica gel (Mega Bond Elut (Varian), chloroform : methanol = 100 : 1) to give compound 1-708 (140 mg).
ESI-MS;524 m/z $(M+H)^+$ ; $^1$H-NMR ($CDCL_3$) $\delta$ 0.80 (3H, s), 0.87-0.95 (2H, m), 1.00-1.04 (2H, m), 1.41 (1H, m), 1.61 (3H, s), 1.63-1.71 (2H, m), 1.79 (3H, s), 1.80-1.83 (1H, m), 2.00-2.05 (2H, m), 2.28 (1H, m), 2.61-2.73 (2H, m), 2.80 (1H, m), 2.96 (2H, m), 3.22 (1H, m), 4.93 (1H, dd, J = 4.6, 12.2 Hz), 5.04 (1H, m), 6.71 (1H, s), 7.42 (1H, dd, J = 4.9, 8.1 Hz), 8.11 (1H, ddd, J = 1.8, 2.2, 8.1 Hz), 8.69 (1H, dd, J = 1.4, 4.9 Hz), 9.01 (1H, d, J = 2.4 Hz)

Synthesis Example 36: Compound 1-707

Compound 1-3 (500 mg) obtained in Synthesis Example 32 was dissolved in N-methylpyrrolidinone (5 mL), and cyclopropane carbonyl chloride (230 mg) was added to the solution at 0°C. The mixture was stirred at that temperature for 14 hr, an aqueous sodium hydrogencarbonate solution and saturated brine were added thereto, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by column chromatography on silica gel (Mega Bond Elut (Varian), hexane : acetone = 5 : 1 → 2 : 1) to give compound 1-707 (324 mg).
ESI-MS;524 m/z $(M+H)^+$ ; $^1$H-NMR ($CDCL_3$) δ 0.88 (3H, s), 0.88-0.91 (2H, m), 0.98-1.02 (2H, m), 1.35 (1H, m), 1.55-1.60 (1H, m), 1.61 (3H, s), 1.69 (1H, m), 1.75 (1H, d, J = 3.7 Hz), 1.82 (3H, s), 1.85-1.94 (2H, m), 2.26 (1H, m), 2.58-2.66 (2H, m), 2.80 (1H, dd, J = 4.6, 4.7 Hz), 3.04 (1H, d, J = 1.9 Hz), 3.36-3.41 (1H, m), 3.57 (1H, d, J = 1.9 Hz), 4.35 (1H, d, J = 2.0 Hz), 5.07 (1H, m), 6.71 (1H, s), 7.43 (1H, dd, J = 4.8, 8.3 Hz), 8.12 (1H, ddd, J = 1.8, 2.2, 8.1 Hz), 8.70 (1H, dd, J = 1.8, 4.8 Hz), 9.02 (1H, d, J = 2.2 Hz)

Synthesis Example 37: Compound 1-1381

1,7,11-Trideacetyl-7-(tert-butyldimethylsilyl)pyripyr opene A (500 mg) synthesized by a method described in WO 2009/022702 was dissolved in pyridine (7 mL), and tert-butyldimethyl silyl chloride (396 mg) was added to the solution. The mixture was stirred at room temperature for 16 hr and was then concentrated under the reduced pressure. Water was added to the concentrate, and the mixture was extracted wit chloroform. The chloroform layer was washed with saturated brine and was dried over anhydrous magnesium sulfate, and the solvent was removed by evaporation under the reduced pressure to give a crude product. The crude product was purified by column chromatography on silica gel (Mega Bond Elut (Varian), hexane : acetone =10 : 1 → 3 : 1) to give compound 1-1381 (480 mg).
ESI-MS; 686 m/z $(M+H)^+$

Synthesis Example 38: Compound 1-1384

Compound 1-1381 (480 mg) obtained in Synthesis Example 37 was suspended in ethyl acetate (5 mL). Pyridine (277 mg) and cyclopropane carbonyl chloride (293 mg) were added to the suspension at room temperature. The mixture was stirred at that temperature for 4 hr, and pyridine (277 mg) and cyclopropane carbonyl chloride (293 mg) were added thereto. The mixture was stirred for additional 2hr, water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by column chromatography on silica gel (Mega Bond Elut (Varian), hexane : acetone =20 : 1) to give compound 1-1384 (499 mg).
ESI-MS;754 m/z $(M+H)^+$

Synthesis Example 39: Compound 1-701

Compound 1-1384 (350 mg) obtained in Synthesis Example 38 was dissolved in tetrahydrofuran (2 mL), and pyridine (0.5 mL) and hydrogen fluoride·pyridine complex (0.6 mL) were added to the solution at 0°C. The mixture was stirred at room temperature for 22 hr and was cooled to 0°C. An aqueous sodium hydrogencarbonate solution was added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by column chromatography on silica gel (Mega Bond Elut (Varian), chloroform : methanol = 40 : 1) to give compound 1-701 (146 mg).
ESI-MS;m/z 526 $(M+H)^+$; $^1$H-NMR ($CDCL_3$) δ 0.75 (3H, s), 0.87-0.95 (2H, m), 1.01-1.05 (2H, m), 1.24-1.35 (2H, m), 1.41 (3H, s), 1.49 (1H, m), 1.59-1.74 (3H, m), 1.65 (3H, s), 1.95-2.06 (2H, m), 2.18 (1H, m), 2.45 (1H, brs), 2.90 (1H, s), 2.93 (1H, d, J = 12.7 Hz), 3.34 (1H, m), 3.91 (1H, dd, J = 5.2, 11.6 Hz), 4.89 (1H, dd, J = 4.6, 12.2 Hz), 4.97 (1H, d, J = 4.0 Hz), 6.54 (1H, s), 7.41 (1H, dd, J = 4.4, 8.0 Hz), 8.11 (1H, ddd, J = 1.4, 1.6, 8.4 Hz), 8.69 (1H, dd, J = 1.6, 4.6 Hz), 9.01 (1H, d, J = 1.7 Hz)

Synthesis Example 40: Compound 1-1246

1,7,11-Tri-deacetyl pyripyropene A (20 mg) synthesized by a method described in Japanese Patent Laid-Open No. 259569/1996 was suspended in pyridine (0.5 mL). Tert-butyldimethyl silyl chloride (39 mg) was added to the suspension at room temperature. The mixture was stirred for 17 hr, methanol was added thereto, and the mixture was

concentrated under the reduced pressure. Water was added to the residue, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-1246 (16 mg).
ESI-MS;572 m/z $(M+H)^+$

Synthesis Example 41: Compound 1-1385

Compound 1-1246 (12 mg) obtained in Synthesis Example 40 and cyclopropane carboxylic acid (22 mg) were dissolved in N,N-dimethylformamide (1 mL). 1-Ethyl-3-(3-dimethylaminopropyl)carbodiimide hydrochloride (36 mg) and 4-dimethylaminopyridine (4 mg) were added to the solution. The mixture was stirred at room temperature for 15 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, chloroform : methanol = 10 : 1) to give compound 1-1385 (11 mg).
ESI-MS;708 m/z $(M+H)^+$

Synthesis Example 42: Compound 1-699

Compound 1-1385 (11 mg) obtained in Synthesis Example 41 was dissolved in tetrahydrofuran (0.5 mL). Tetra-n-butylammonium fluoride (47 $\mu$L, 1.0 M tetrahydrofuran solution) was added to the solution . The mixture was stirred at room temperature for 17 hr. Water was then added, and the mixture was extracted with ethyl acetate. The ethyl acetate layer was washed with saturated brine and was dried over anhydrous sodium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-699 (1.5 mg).
ESI-MS;594 m/z $(M+H)^+$

Synthesis Example 43: Compound 89-260

Compound 1-260 (20 mg) synthesized by a method described in WO 2006/129714 was dissolved in dichloromethane (1 mL), and dess-martin periodinane (21 mg) was added to the solution at 0°C. The mixture was stirred for 3 hr, an aqueous sodium thiosulfate solution was added thereto, and the mixture was extracted with chloroform. The chloroform layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 89-260 (4 mg).
ESI-MS;592 m/z $(M+H)^+$

Synthesis Example 44: Compound 72-260

Compound 1-260 (100 mg) synthesized by a method described in WO 2006/129714 was dissolved in tetrahydrofuran (1 mL), and p-toluenesulfonic acid monohydrate (64 mg) was added to the solution at room temperature. The mixture was stirred for 24 hr, an aqueous sodium hydrogencarbonate solution was added thereto, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 72-260 (95 mg).
ESI-MS;576 m/z $(M+H)^+$ ; $^1$H-NMR ($CD_3OD$) $\delta$ 0.88-0.92 (8H, m), 0.94 (3H, s), 1.28 (3H, s), 1.30-1.34 (1H, m), 1.54 (3H, s), 1.56-1.70 (4H, m), 1.83-1.89 (3H, m), 2.07 (1H, t, J = 3.4, 13.1 Hz), 3.80 (1H, d, J = 1 : 1.9 Hz), 3.91 (1H, d, J = 1 : 1.9 Hz), 3.95 (1H, dd, J = 4.9, 11.5 Hz), 4.75-4.80 (1H, m), 6.28 (1H, s), 6.95 (1H, s), 7.56 (1H, dd, J = 4.8, 8.1 Hz), 8.27 (1H, ddd, J = 1.6, 2.3, 8.1 Hz), 8.63 (1H, dd, J = 1.6, 4.8 Hz), 9.04 (1H, d, J = 2.3 Hz)

Synthesis Example 45: Compound 1-264

Compound 1-260 (30 mg) synthesized by a method described in WO 2006/129714 was dissolved in N,N-dimethylformamide (1 mL). Triethylamine (46 mg), 4-dimethylaminopyridine (12 mg), and acetic acid anhydride (31 mg)

were added to the solution at room temperature. The mixture was stirred for 30 min, water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-264 (30 mg).
ESI-MS;636 m/z $(M+H)^+$ ; $^1H$-NMR ($CDCl_3$) $\delta$ 0.84-0.89 (4H, m), 0.89 (3H, s), 0.90-1.06 (4H, m), 1.37 (1H, dt, J = 3.8, 13.2 Hz), 1.45 (3H, s), 1.53 (1H, d, J = 4.0 Hz), 1.55-1.67 (4H, m), 1.70 (3H, s), 1.79-1.87 (2H, m), 1.89-1.94 (2H, m), 2.14-2.18 (1H, m), 2.16 (3H, s), 2.97 (1H, d, J = 2.0 Hz), 3.77 (2H, s), 4.81 (1H, dd, J = 4.8, 11.7 Hz), 5.00 (1H, m), 5.02 (1H, dd, J = 5.0, 11.4 Hz), 6.46 (1H, s), 7.40 (1H, dd, J = 4.9, 8.0 Hz), 8.09 (1H, dt, J = 1.9, 8.1 Hz), 8.68 (1H, dd, J = 1.6, 4.8 Hz), 9.00 (1H, d, J = 2.0 Hz)

Synthesis Example 46: Compound 1-265

Compound 1-260 (20 mg) synthesized by a method described in WO 2006/129714 was dissolved in N,N-dimethylformamide (1 mL). Triethylamine (41 mg), 4-dimethylaminopyridine (8 mg), and propionic acid anhydride (26 mg) were added to the solution at room temperature. The mixture was stirred for 4hr, and water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-265 (14 mg).
ESI-MS;650 m/z $(M+H)^+$

Synthesis Example 47: Compound 1-267

Compound 1-260 (20 mg) synthesized by a method described in WO 2006/129714 was dissolved in N,N-dimethylformamide (1 mL). Triethylamine (31 mg), 4-dimethylaminopyridine (8 mg), and isobutyric acid anhydride (32 mg) were added to the solution at room temperature. The mixture was stirred for 6 hr, water was then added, and the mixture was extracted with ethyl acetate. The ethyl acetate layer was washed with saturated brine and was dried over anhydrous sodium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-267 (18 mg).
ESI-MS;664 m/z $(M+H)^+$

Synthesis Example 48: Compound 1-268

Compound 1-260 (20 mg) synthesized by a method described in WO 2006/129714 was dissolved in N,N-dimethylformamide (1 mL). Triethylamine (31 mg), 4-dimethylaminopyridine (8 mg), and pivalic acid anhydride (38 mg) were added to the solution at room temperature. The mixture was stirred for 6 hr, water was then added, and the mixture was extracted with ethyl acetate. The ethyl acetate layer was washed with saturated brine and was dried over anhydrous sodium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-268 (3 mg).
ESI-MS;678 m/z $(M+H)^+$

Synthesis Example 49: Compound 1-269 and compound 72-269

Compound 1-260 (40 mg) synthesized by a method described in WO 2006/129714 was dissolved in pyridine (1 mL), and methanesulfonyl chloride (23 mg) was added at 0°C. The mixture was stirred for one hr and was then concentrated under the reduced pressure to give a crude product. The crude product was purified by preparative thin layer chromatography (Merck Silica Gel 60F254 0.5 mm, hexane : acetone = 1 : 1) to give compound 1-269 (7 mg) and compound 72-269 (2 mg).
Compound 1-269: ESI-MS;672 m/z $(M+H)^+$
Compound 72-269 : ESI-MS;654 m/z $(M+H)^+$

Synthesis Example 50: Compound 1-272

Compound 1-260 (20 mg) synthesized by a method described in WO 2006/129714 and benzoic acid (25 mg) were dissolved in N,N-dimethylformamide (1 mL). 1-Ethyl-3-(3-dimethylaminopropyl)carbodiimide hydrochloride (26 mg)

and 4-dimethylaminopyridine (4 mg) were added to the solution. The mixture was stirred at room temperature for 6 hr. Water was then added, and the mixture was extracted with ethyl acetate. The ethyl acetate layer was washed with saturated brine and was dried over anhydrous sodium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-272 (21 mg).
ESI-MS;698 m/z $(M+H)^+$

Synthesis Example 51: Compound 1-273

Compound 1-260 (20 mg) synthesized by a method described in WO 2006/129714 and picolinic acid (25 mg) were dissolved in N,N-dimethylformamide (1 mL). 1-Ethyl-3-(3-dimethylaminopropyl)carbodiimide hydrochloride (26 mg) and 4-dimethylaminopyridine (4 mg) were added to the solution. The mixture was stirred at room temperature for 6 hr. Water was then added, and the mixture was extracted with ethyl acetate. The ethyl acetate layer was washed with saturated brine and was dried over anhydrous sodium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-273 (22 mg).
ESI-MS;699 m/z $(M+H)^+$

Synthesis Example 52: Compound 1-276

Compound 1-260 (50 mg) synthesized by a method described in WO 2006/129714 was dissolved in toluene (3 mL) and 1,1'-thiocarbonyldiimidazole (90 mg) was added to the solution. The mixture was heated under reflux for 2.5 hr and was allowed to cool to room temperature. Water was added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-276 (41 mg).
ESI-MS;704 m/z $(M+H)^+$

Synthesis Example 53: Compound 1-948

1,7,11-Trideacetyl pyripyropene A (20 mg) (20 mg) synthesized by a method described in Japanese Patent Laid-Open No. 259569/1996 and cyclopropanecarboxylic acid (19 mg) were dissolved in N,N-dimethylformamide (1 mL). 1-Ethyl-3-(3-dimethylaminopropyl)carbodiimide hydrochloride (84 mg) and 4-dimethylaminopyridine (5 mg) were added to the solution. The mixture was stirred at room temperature for 6 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, chloroform : methanol = 10 : 1) to give compound 1-948 (9 mg).
ESI-MS; 526 m/z $(M+H)^+$

Synthesis Example 54: Compound 1-356

Compound 1-948 (30 mg) obtained in Synthesis Example 53 was dissolved in N,N-dimethylformamide (1 mL). Triethylamine (52 mg), 4-dimethylaminopyridine (14 mg), and acetic acid anhydride (70 mg) were added to the solution at room temperature. The mixture was stirred for 20 min. Water was then added, and the mixture was extracted with ethyl acetate. The ethyl acetate layer was washed with saturated brine and was dried over anhydrous sodium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-356 (27 mg).
ESI-MS;610 m/z $(M+H)^+$

Synthesis Example 55: Compound 1-389

Compound 1-356 (27 mg) obtained in Synthesis Example 54 was suspended in methanol-water (9:1, 5 mL), and 1,8-diazabcyclo[5.4.0]-7-undecene (7 mg) was added to the suspension. The mixture was stirred at 0°C for 1.5 hr. The reaction was then stopped by the addition of acetic acid (0.1 mL), followed by concentration under the reduced pressure. Water was added to the residue, and the mixture was extracted with ethyl acetate. The ethyl acetate layer

was washed with saturated brine and was dried over anhydrous sodium sulfate, and the solvent was then removed by evaporation under the reduced pressure to give a crude product. The crude product was purified by preparative thin layer chromatography (Merck Silica Gel 60F254 0.5 mm, hexane : acetone = 1 : 1) to give compound 1-389 (7 mg).
ESI-MS;568 m/z $(M+H)^+$

Synthesis Example 56: Compound 1-357 and compound 114-357

Compound 1-948 (20 mg) obtained in Synthesis Example 53 was dissolved in N,N-dimethylformamide (1 mL). Triethylamine (35 mg), 4-dimethylaminopyridine (9 mg), and acetic acid anhydride (50 mg) were added to the solution at room temperature. The mixture was stirred for 4.5 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-357 (12 mg) and compound 114-357 (6 mg).
Compound 1-357 : ESI-MS;638 m/z $(M+H)^+$
Compound 114-357 : ESI-MS;694 m/z $(M+H)^+$

Synthesis Example 57: Compound 1-390

Compound 1-357 (18 mg) obtained in Synthesis Example 56 was suspended in methanol-water (9 : 1, 3 mL), and 1,8-diazabcyclo[5.4.0]-7-undecene (5 mg) was added to the suspension. The mixture was stirred at 0°C for 1.5 hr. The reaction was stopped by the addition of acetic acid (0.1 mL), followed by concentration under the reduced pressure. Water was added to the residue, and the mixture was extracted with ethyl acetate. The ethyl acetate layer was washed with saturated brine and was dried over anhydrous sodium sulfate, and the solvent was then removed by evaporation under the reduced pressure to give a crude product. The crude product was purified by preparative thin layer chromatography (Merck Silica Gel 60F254 0.5 mm, hexane : acetone = 1 : 1) to give compound 1-390 (6 mg).
ESI-MS; 582 m/z $(M+H)^+$

Synthesis Example 58: Compound 1-359

Compound 1-948 (20 mg) obtained in Synthesis Example 53 was dissolved in N,N-dimethylformamide (1 mL). Triethylamine (35 mg), 4-dimethylaminopyridine (9 mg), and isobutyric acid anhydride (60 mg) were added to the solution at room temperature. The mixture was stirred for 15.5 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-359 (21 mg).
ESI-MS; 666 m/z $(M+H)^+$

Synthesis Example 59: Compound 1-360 and compound 1-641

Compound 1-948 (20 mg) obtained in Synthesis Example 53 was dissolved in N,N-dimethylformamide (1 mL). Triethylamine (35 mg), 4-dimethylaminopyridine (9 mg), and pivalic acid anhydride (71 mg) were added to the solution at room temperature. The mixture was stirred for 15.5 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-360 (3 mg) and compound 1-641 (11 mg).
Compound 1-360: ESI-MS;694 m/z $(M+H)^+$
Compound 1-641: ESI-MS;610 m/z $(M+H)^+$

Synthesis Example 60: Compound 1-364

Compound 1-948 (20 mg) obtained in Synthesis Example 53 and benzoic acid (56 mg) were dissolved in N,N-dimethylformamide (1 mL). 1-Ethyl-3-(3-dimethylaminopropyl)carbodiimide hydrochloride (73 mg) and 4-dimethylaminopyridine (5 mg) were added to the solution. The mixture was stirred at room temperature for 13.5 hr. Water was then added, and the mixture was extracted with ethyl acetate. The ethyl acetate layer was washed with saturated brine and was dried over anhydrous magnesium sulfate, and the solvent was removed by evaporation under the reduced pressure

to give a crude product. The crude product was purified by preparative thin layer chromatography (Merck Silica Gel 60F254 0.5 mm, chloroform : methanol = 10 : 1) to give compound 1-364 (17 mg).
ESI-MS;734 m/z $(M+H)^+$

Synthesis Example 61: Compound 1-361 and compound 1-394

Compound 1-948 (20 mg) obtained in Synthesis Example 53 was dissolved in pyridine (1 mL). Methanesulfonyl chloride (15 mg) was added to the solution at 0°C. The mixture was stirred for one hr and was then concentrated under the reduced pressure to give a crude product. The crude product was purified by preparative thin layer chromatography (Merck Silica Gel 60F254 0.5 mm, hexane : acetone = 1 : 1) to give compound 1-361 (6 mg) and compound 1-394 (17 mg).
Compound 1-361 : ESI-MS;682 m/z $(M+H)^+$
Compound 1-394 : ESI-MS;604 m/z $(M+H)^+$

Synthesis Example 62: Compound 1-744

1,7,11-Trideacetyl pyripyropene A (20 mg) synthesized by a method described in Japanese Patent Laid-Open No. 259569/1996 and benzoic acid (8 mg) were dissolved in N,N-dimethylformamide (1 mL). 1-Ethyl-3-(3-dimethylaminopropyl)carbodiimide hydrochloride (34 mg) and 4-dimethylaminopyridine (1 mg) were added to the solution, and the mixture was stirred at room temperature for 4 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, chloroform : methanol = 10 : 1) to give compound 1-744 (11 mg).
ESI-MS;562 m/z $(M+H)^+$

Synthesis Example 63: Compound 1-342

Compound 1-744 (11 mg) obtained in Synthesis Example 62 and cyclopropanecarboxylic acid (20 mg) were dissolved in N,N-dimethylformamide (1 mL). 1-Ethyl-3-(3-dimethylaminopropyl)carbodiimide hydrochloride (34 mg) and 4-dimethylaminopyridine (4 mg) were added to the solution. The mixture was stirred at room temperature for 7 hr. Water was then added, and the mixture was extracted with ethyl acetate. The ethyl acetate layer was washed with saturated brine and was dried over anhydrous sodium sulfate, and the solvent was then removed by evaporation under the reduced pressure to give a crude product. The crude product was purified by preparative thin layer chromatography (Merck Silica Gel 60F254 0.5 mm, chloroform : methanol = 10 : 1) to give compound 1-342 (8 mg).
ESI-MS;698 m/z $(M+H)^+$

Synthesis Example 64: Compound 1-1176 and compound 1-1171

1,7,11-Trideacetyl pyripyropene A (20 mg) synthesized by a method described in Japanese Patent Laid-Open No. 259569/1996 was dissolved in pyridine (1 mL). Methanesulfonyl chloride (5 mg) was added to the solution at 0°C. The mixture was stirred for 30 min and was then concentrated under the reduced pressure to give a crude product. The crude product was purified by preparative thin layer chromatography (Merck Silica Gel 60F254 0.5 mm, chloroform : methanol = 10 : 1) to give compound 1-1176 (6 mg) and compound 1-1171 (6 mg).
Compound 1-1176: ESI-MS;536 m/z $(M+H)^+$
Compound 1-1171: ESI-MS;614 m/z $(M+H)^+$

Synthesis Example 65: Compound 1-329 and compound 1-673

Compound 1-1176 (6 mg) obtained in Synthesis Example 64 and cyclopropanecarboxylic acid (12 mg) were dissolved in N,N-dimethylformamide (1 mL). 1-Ethyl-3-(3-dimethylaminopropyl)carbodiimide hydrochloride (21 mg) and 4-dimethylaminopyridine (2 mg) were added to the solution. The mixture was stirred at room temperature for 4 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, chloroform : methanol = 10 : 1) to give compound 1-329 (1 mg) and compound 1-673 (2 mg).
Compound 1-329: ESI-MS;672 m/z $(M+H)^+$
Compound 1-673: ESI-MS;604 m/z $(M+H)^+$

Synthesis Example 66: Compound 1-1387

Compound 1-1171 (6 mg) obtained in Synthesis Example 64 and cyclopropanecarboxylic acid (6 mg) were dissolved in N,N-dimethylformamide (1 mL). 1-Ethyl-3-(3-dimethylaminopropyl)carbodiimide hydrochloride (9 mg) and 4-dimethylaminopyridine (1 mg) were added to the solution. The mixture was stirred at room temperature for 4 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, chloroform : methanol = 10 : 1) to give compound 1-1387 (3 mg).
ESI-MS;682 m/z $(M+H)^+$

Synthesis Example 67: Compound 1-1188 and compound 1-1183

1,7,11-Trideacetyl pyripyropene A (30 mg) synthesized by a method described in Japanese Patent Laid-Open No. 259569/1996 was dissolved in pyridine (1 mL), and ethanesulfonyl chloride (5 mg) was added to the solution at 0°C. The mixture was stirred for 4 hr and was then concentrated under the reduced pressure to give a crude product. The crude product was purified by preparative thin layer chromatography (Merck Silica Gel 60F254 0.5 mm, chloroform : methanol = 10 : 1) to give compound 1-1188 (15 mg) and compound 1-1183 (11 mg).
Compound 1-1188: ESI-MS;550 m/z $(M+H)^+$
Compound 1-1183: ESI-MS;642 m/z $(M+H)^+$

Synthesis Example 68: Compound 1-330

Compound 1-1188 (15 mg) obtained in Synthesis Example 67 and cyclopropanecarboxylic acid (28 mg) were dissolved in N,N-dimethylformamide (1 mL). 1-Ethyl-3-(3-dimethylaminopropyl)carbodiimide hydrochloride (47 mg) and 4-dimethylaminopyridine (5 mg) were added to the solution. The mixture was stirred at room temperature for 7 hr. Water was then added, and the mixture was extracted with ethyl acetate. The ethyl acetate layer was washed with saturated brine and was dried over anhydrous sodium sulfate, and the solvent was then removed by evaporation under the reduced pressure to give a crude product. The crude product was purified by preparative thin layer chromatography (Merck Silica Gel 60F254 0.5 mm, chloroform : methanol = 10 : 1) to give compound 1-330 (12 mg).
ESI-MS;686 m/z $(M+H)^+$

Synthesis Example 69: Compound 1-1388

Compound 1-1183 (11 mg) obtained in Synthesis Example 67 and cyclopropanecarboxylic acid (9 mg) were dissolved in N,N-dimethylformamide (1 mL). 1-Ethyl-3-(3-dimethylaminopropyl)carbodiimide hydrochloride (13 mg) and 4-dimethylaminopyridine (2 mg) were added to the solution. The mixture was stirred at room temperature for 7 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, chloroform : methanol = 10 : 1) to give compound 1-1388 (7 mg).
ESI-MS;710 m/z $(M+H)^+$

Synthesis Example 70: Compound 1-365

Compound 1-744 (20 mg) obtained in Synthesis Example 62 and picolinic acid (56 mg) were dissolved in N,N-dimethylformamide (1 mL). 1-Ethyl-3-(3-dimethylaminopropyl)carbodiimide hydrochloride (73 mg) and 4-dimethylaminopyridine (5 mg) were added to the solution. The mixture was stirred at room temperature for 13.5 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, chloroform : methanol = 10 : 1) to give compound 1-365 (14 mg).
ESI-MS; 736 m/z $(M+H)^+$

Synthesis Example 71: Compound 1-1440 and compound 1-1441

1,7,11-Trideacetyl pyripyropene A (20 mg) synthesized by a method described in Japanese Patent Laid-Open No. 259569/1996 and picolinic acid (8 mg) were dissolved in N,N-dimethylformamide (1 mL). 1-Ethyl-3-(3-dimethylaminopropyl)carbodiimide hydrochloride (34 mg) and 4-dimethylaminopyridine (1 mg) were added to the solution. The mixture was stirred at room temperature for 4 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, chloroform : methanol = 10 : 1) to give compound 1-1440 (10 mg) and compound 1-1441 (6 mg).
Compound 1-1440: ESI-MS; 563 m/z $(M+H)^+$
Compound 1-1441: ESI-MS; 668 m/z $(M+H)^+$

Synthesis Example 72: Compound 1-355

Compound 1-1441(6 mg) obtained in Synthesis Example 71 and cyclopropanecarboxylic acid (5 mg) were dissolved in N,N-dimethylformamide (1 mL). 1-Ethyl-3-(3-dimethylaminopropyl)carbodiimide hydrochloride (6 mg) and 4-dimethylaminopyridine (1 mg) were added to the solution. The mixture was stirred at room temperature for 7 hr. Water was then added, and the mixture was extracted with ethyl acetate. The ethyl acetate layer was washed with saturated brine and was dried over anhydrous sodium sulfate, and the solvent was then removed by evaporation under the reduced pressure to give a crude product. The crude product was purified by preparative thin layer chromatography (Merck Silica Gel 60F254 0.5 mm, chloroform : methanol = 10 : 1) to give compound 1-355 (2 mg).
ESI-MS; 736 m/z $(M+H)^+$

Synthesis Example 73: Compound 1-333

Compound 1-1440 (10 mg) obtained in Synthesis Example 71 and cyclopropanecarboxylic acid (18 mg) were dissolved in N,N-dimethylformamide (1 mL). 1-Ethyl-3-(3-dimethylaminopropyl)carbodiimide hydrochloride (31 mg) and 4-dimethylaminopyridine (3 mg) were added to the solution. The mixture was stirred at room temperature for 7 hr. Water was then added, and the mixture was extracted with ethyl acetate. The ethyl acetate layer was washed with saturated brine and was dried over anhydrous sodium sulfate, and the solvent was then removed by evaporation under the reduced pressure to give a crude product. The crude product was purified by preparative thin layer chromatography (Merck Silica Gel 60F254 0.5 mm, chloroform : methanol = 10 : 1) to give compound 1-333 (4 mg).
ESI-MS;699 m/z $(M+H)^+$

Synthesis Example 74: Compound 1-716

Compound 1-715 (44 mg) obtained by a synthesis method described in WO 2006/129714 was suspended in methanol-water (9:1, 1 mL), and 1,8-diazabcyclo[5.4.0]-7-undecene (10 mg) was added to the suspension. The mixture was stirred at room temperature for 15 hr. The reaction was then stopped by the addition of acetic acid (0.1 mL), followed by concentration under the reduced pressure. Water was added to the residue, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-716 (15 mg).
ESI-MS;622 m/z $(M+H)^+$

Synthesis Example 75: Compound 1-742 and compound 1-743

1,7,11-Trideacetyl pyripyropene A (20 mg) synthesized by a method described in Japanese Patent Laid-Open No. 259569/1996 and 2,2,3,3-tetramethylcyclopropanecarboxylic acid (124 mg) were dissolved in N,N-dimethylformamide (1 mL). 1-Ethyl-3-(3-dimethylaminopropyl)carbodiimide hydrochloride (84 mg) and 4-dimethylaminopyridine (5 mg) were added to the solution. The mixture was stirred at room temperature for 5 hr. Water was then added, and the mixture was extracted with ethyl acetate. The ethyl acetate layer was washed with saturated brine and was dried over anhydrous sodium sulfate, and the solvent was then removed by evaporation under the reduced pressure to give a crude product. The crude product was purified by preparative thin layer chromatography (Merck Silica Gel 60F254 0.5 mm, acetone : hexane = 1 : 1) to give compound 1-742 (17 mg) and compound 1-743 (1 mg).
Compound 1-742 : ESI-MS;830 m/z $(M+H)^+$
Compound 1-743 : ESI-MS;706 m/z $(M+H)^+$

Synthesis Example 76: Compound 1-745

1,7,11-Trideacetyl pyripyropene A (30 mg) synthesized by a method described in Japanese Patent Laid-Open No. 259569/1996 and nicotinic acid (161 mg) were dissolved in N,N-dimethylformamide (2 mL). 1-Ethyl-3-(3-dimethylaminopropyl)carbodiimide hydrochloride (126 mg) and 4-dimethylaminopyridine (80 mg) were added to the solution. The mixture was stirred at room temperature for 14 hr. Water was then added, and the mixture was extracted with ethyl acetate. The ethyl acetate layer was washed with saturated brine and was dried over anhydrous sodium sulfate, and the solvent was then removed by evaporation under the reduced pressure to give a crude product. The crude product was purified by preparative thin layer chromatography (Merck Silica Gel 60F254 0.5 mm, chloroform : methanol = 10 : 1) to give compound 1-745 (34 mg).
ESI-MS;773 m/z $(M+H)^+$

Synthesis Example 77: Compound 1-746

1,7,11-Trideacetyl pyripyropene A (20 mg) synthesized by a method described in Japanese Patent Laid-Open No. 259569/1996 and picolinic acid (32 mg) were dissolved in N,N-dimethylformamide (1 mL). 1-Ethyl-3-(3-dimethylaminopropyl)carbodiimide hydrochloride (84 mg) and 4-dimethylaminopyridine (5 mg) were added to the solution. The mixture was stirred at room temperature for 5 hr. Water was then added, and the mixture was extracted with chloroform. The chloroform layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, chloroform : methanol = 10 : 1) to give compound 1-746 (28 mg).
ESI-MS; 773 m/z $(M+H)^+$

Synthesis Example 78: Compound 1-748

1,7,11-Trideacetyl pyripyropene A (30 mg) synthesied by a method described in Japanese Patent Laid-Open No. 259569/1996 and 6-trifluoromethylnicotinic acid (250 mg) were dissolved in N,N-dimethylformamide (2 mL). 1-Ethyl-3-(3-dimethylaminopropyl)carbodiimide hydrochloride (126 mg) and 4-dimethylaminopyridine (80 mg) were added to the solution. The mixture was stirred at room temperature for 17.5 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, chloroform : methanol = 10 : 1) to give compound 1-748 (31 mg).
ESI-MS;977 m/z $(M+H)^+$

Synthesis Example 79: Compound 1-747

1,7,11-Trideacetyl pyripyropene A (30 mg) synthesized by a method described in Japanese Patent Laid-Open No. 259569/1996 and 4-trifluoromethylnicotinic acid (250 mg) were dissolved in N,N-dimethylformamide (2 mL). 1-Ethyl-3-(3-dimethylaminopropyl)carbodiimide hydrochloride (126 mg) and 4-dimethylaminopyridine (80 mg) were added to the solution. The mixture was stirred at room temperature for 17.5 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, chloroform : methanol = 10 : 1) to give compound 1-747 (36 mg).
ESI-MS;977 m/z $(M+H)^+$

Synthesis Example 80: Compound 72-763

Compound 1-112 (30 mg) synthesized by a method described in WO 2006/129714 was dissolved in dimethylsulfoxide (0.6 mL). Acetic acid anhydride (0.6 mL) and acetic acid (0.6 mL) were added to the solution. The mixture was stirred at room temperature for 24 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 72-763 (13 mg).
ESI-MS; 612 m/z $(M+H)^+$

Synthesis Example 81: Compound 1-1043

1,7,11-Trideacetyl pyripyropene A (20 mg) synthesized by a method described in Japanese Patent Laid-Open No. 259569/1996 was dissolved in N,N-dimethylformamide (1 mL). Triethylamine (132 mg), 4-dimethylaminopyridine (27 mg), and ethyl isocyanate (62 mg) were added to the solution at room temperature. The mixture was stirred for 12.5 hr, and ethyl isocyanate (62 mg) was then added. The mixture was stirred for additional 24 hr. Water was then added, and the mixture was extracted with ethyl acetate. The ethyl acetate layer was washed with saturated brine and was dried over anhydrous sodium sulfate, and the solvent was then removed by evaporation under the reduced pressure to give a crude product. The crude product was purified by preparative thin layer chromatography (Merck Silica Gel 60F254 0.5 mm, chloroform : methanol = 10 : 1) to give compound 1-1043 (7 mg).
ESI-MS;671 m/z $(M+H)^+$

Synthesis Example 82: Compound 89-261

Compound 1-260 (20 mg) synthesized by a method described in WO 2006/129714 was dissolved in dichloromethane (1 mL). Dess-martin periodinane (57 mg) was added to the solution at 0°C. The mixture was stirred for 1.5 hr, an aqueous sodium thiosulfate solution was added to the solution, and the mixture was extracted with chloroform. The chloroform layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 89-261 (18 mg). ESI-MS; 590 m/z $(M+H)^+$

Synthesis Example 83: Compound 1-1182

1,7,11-Trideacetyl pyripyropene A (20 mg) synthesized by a method described in Japanese Patent Laid-Open No. 259569/1996 was dissolved in pyridine (1 mL). Ethanesulfonyl chloride (10 mg) was added to the solution at 0°C. The mixture was stirred for 9 hr and was then concentrated under the reduced pressure to give a crude product. The crude product was purified by preparative thin layer chromatography (Merck Silica Gel 60F254 0.5 mm, chloroform : methanol = 10 : 1) to give compound 1-1182 (7 mg).
ESI-MS;734 m/z $(M+H)^+$

Synthesis Example 84: Compound 72-1228 and compound 1-1228

1,7,11-Trideacetyl pyripyropene A (20 mg) synthesized by a method described in Japanese Patent Laid-Open No. 259569/1996 was dissolved in pyridine (1 mL), and cyclopropanesulfonyl chloride (10 mg) was added at 0°C. The mixture was stirred for 30 min and was then concentrated under the reduced pressure to give a crude product. The crude product was purified by preparative thin layer chromatography (Merck Silica Gel 60F254 0.5 mm, chloroform : methanol = 10 : 1) to give compound 72-1228 (5 mg) and compound 1-1228 (4 mg).
Compound 72-1228 : ESI-MS;544 m/z $(M+H)^+$
Compound 1-1228 : ESI-MS;562 m/z $(M+H)^+$

Synthesis Example 85: Compound 1-1259

Compound 1-260 (300 mg) synthesized by a method described in WO 2006/129714 was dissolved in dichloromethane (5 mL). 4-Dimethylaminopyridine (246 mg) was added to the solution at room temperature. The mixture was cooled to 0°C, and trifluoromethanesulfonic acid anhydride (285 mg) was added then thereto, and the mixture was stirred for 2.5 hr. Water was then added, and the mixture was extracted with chloroform. The chloroform layer was washed with saturated brine and was dried over anhydrous magnesium sulfate, and the solvent was removed by evaporation under the reduced pressure. The residue was dissolved in N,N-dimethylformamide (3 mL) and hexamethylphosphoric triamide (3 mL). Lithium acetate (334 mg) was added to the solution. The mixture was stirred at 6°C for 14.5 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by column chromatography on silica gel (Mega Bond Elut (Varian), hexane : acetone = 9 : 1 $\rightarrow$ 2 : 1) to give compound 1-1259 (274 mg).
ESI-MS;636 m/z $(M+H)^+$

Synthesis Example 86: Compound 1-1262

Compound 1-260 (200 mg) synthesized by a method described in WO 2006/129714 was dissolved in dichloromethane (3 mL). 4-Dimethylaminopyridine (123 mg) was added to the solution at room temperature, and the mixture was cooled to 0°C. Trifluoromethanesulfonic acid anhydride (142 mg) was then added thereto. The mixture was stirred for 3 hr. Water was then added, and the mixture was extracted with chloroform. The chloroform layer was washed with saturated brine and was dried over anhydrous magnesium sulfate, and the solvent was removed by evaporation under the reduced pressure to give a residue (329 mg). The residue (82 mg) was dissolved in N,N-dimethylformamide (1 mL), and lithium chloride (130 mg) was added to the solution. The mixture was stirred at room temperature for 20 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-1262 (38 mg).
ESI-MS;612 m/z $(M+H)^+$ ; $^1$H-NMR ($CDCL_3$) $\delta$ 0.81-0.87 (4H, m), 0.90 (3H, s), 0.98-1.03 (4H, m), 1.45 (3H, s), 1.45-1.54 (1H, m), 1.56-1.64 (2H, m), 1.78 (3H, s), 1.80-1.88 (1H, m), 1.92-1.96 (1H, m), 1.99-2.00 (2H, m), 2.10-2.22 (3H, m), 2.88 (1H, d, J = 3.5 Hz), 3.61 (1H, d, J = 1 : 1.9 Hz), 3.95 (1H, d, J = 1 : 1.9 Hz), 4.43 (1H, t, J = 2.7 Hz), 4.93 (1H, dd, J = 4.8, 11.7 Hz), 5.05 (1H, dd, J = 3.2, 3.4 Hz), 6.53 (1H, s), 7.41 (1H, dd, J = 4.8, 8.0 Hz), 8.10 (1H, ddd, J = 1.2, 1.8, 8.0 Hz), 8.69 (1H, dd, J = 1.2, 4.8 Hz), 9.02 (1H, d, J = 1.8 Hz)

Synthesis Example 87: Compound 1-1263

Compound 1-260 (200 mg) synthesized by a method described in WO 2006/129714 was dissolved in dichloromethane (3 mL). 4-Dimethylaminopyridine (123 mg) was added to the solution at room temperature. The mixture was cooled to 0°C. Trifluoromethanesulfonic acid anhydride (142 mg) was added thereto. The mixture was stirred for 3 hr. Water was then added, and the mixture was extracted with chloroform. The chloroform layer was washed with saturated brine and was dried over anhydrous magnesium sulfate, and the solvent was removed by evaporation under the reduced pressure to give a residue (329 mg). The residue (80 mg) was dissolved in N,N-dimethylformamide (1 mL), and lithium bromide (170 mg) was added to the solution. The mixture was stirred at room temperature for 20 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-1263 (26 mg). ESI-MS;656 m/z $(M+H)^+$ ; $^1$H-NMR ($CDCL_3$) $\delta$ 0.80-0.88 (4H, m), 0.91 (3H, s), 0.98-1.03 (4H, m), 1.46 (3H, s), 1.49 (1H, m), 1.56-1.64 (2H, m), 1.81 (3H, s), 1.84-1.96 (2H, m), 2.03 (1H, d, J = 4.0 Hz), 2.07 (1H, m), 2.13-2.18 (2H, m), 2.27 (1H, m), 2.91 (1H, d, J = 2.0 Hz), 3.64 (1H, d, J = 12.0 Hz), 3.91 (1H, d, J = 12.0 Hz), 4.62 (1H, t, J = 2.4 Hz), 4.94 (1H, dd, J = 4.8, 12.0 Hz), 5.05 (1H, dd, J = 3.2, 3.6 Hz), 6.52 (1H, s), 7.41 (1H, dd, J = 4.8, 8.0 Hz), 8.11 (1H, ddd, J = 1.2, 2.0, 8.0 Hz), 8.69 (1H, dd, J = 1.2, 4.8 Hz), 9.02 (1H, d, J = 2.0 Hz)

Synthesis Example 88: Compound 1-1264

Compound 1-260 (200 mg) synthesized by a method described in WO 2006/129714 was dissolved in dichloromethane (3 mL). 4-Dimethylaminopyridine (123 mg) was added to the solution at room temperature. The mixture was cooled to 0°C, and trifluoromethanesulfonic acid anhydride (142 mg) was added thereto. The mixture was stirred for 3 hr. Water was then added, and the mixture was extracted with chloroform. The chloroform layer was washed with saturated brine and was dried over anhydrous magnesium sulfate, and the solvent was removed by evaporation under the reduced pressure to give a residue (329 mg). The residue (80 mg) was dissolved in N,N-dimethylformamide (1 mL). Sodium iodide (180 mg) was added to the solution. The mixture was stirred at room temperature for 20 hr. Water was then added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-1264 (16 mg).
ESI-MS;704 m/z $(M+H)^+$; $^1$H-NMR ($CDCL_3$) $\delta$ 0.81-0.90 (4H, m), 0.92 (3H, s), 0.97-1.06 (4H, m), 1.47 (3H, s), 1.51-1.60 (3H, m), 1.83 (3H, s), 1.85-1.99 (3H, m), 2.05-2.10 (1H, m), 2.13-2.17 (2H, m), 2.22-2.25 (1H, m), 2.84 (1H, d, J = 2.0 Hz), 3.71 (1H, d, J = 12.0 Hz), 3.84 (1H, d, J = 12.0 Hz), 4.84 (1H, t, J = 2.1 Hz), 4.96 (1H, dd, J = 4.8, 12.0 Hz), 5.03 (1H, dd, J = 2.4, 4.0 Hz), 6.51 (1H, s), 7.41 (1H, dd, J = 4.8, 8.0 Hz), 8.11 (1H, ddd, J = 1.2, 1.6, 8.0 Hz), 8.69 (1H, dd, J = 1.2, 4.8 Hz), 9.03 (1H, d, J = 1.6 Hz)

Synthesis Example 89: Compound 1-263

Compound 1-1264 (50 mg) obtained in Synthesis Example 88 was dissolved in N,N-dimethylformamide (1 mL). Sodium azide (70 mg) and 15-crown-5 (10 mg) were added to the solution. The mixture was stirred at 90°C for 16 hr and was then allowed to cool. Water was added, and the mixture was extracted with ethyl acetate. The ethyl acetate layer was washed with saturated brine and was dried over anhydrous magnesium sulfate, and the solvent was removed by evaporation under the reduced pressure to give a crude product. The crude product was purified by preparative thin layer chromatography (Merck Silica Gel 60F254 0.5 mm, hexane : acetone = 1 : 1) to give compound 1-263 (37 mg).
ESI-MS;576 m/z $(M+H)^+$ ; $^1$H-NMR ($CDCL_3$) $\delta$ 0.82-0.88 (4H, m), 0.93 (3H, s), 0.95-1.02 (4H, m), 1.33 (3H, s), 1.49-1.53 (1H, m), 1.55-1.62 (2H, m), 1.70 (3H, s), 1.83-1.91 (1H, m), 1.95 (1H, d, J = 3.8 Hz), 1.96-1.98 (1H, m), 2.11 (1H, dt, J = 3.3, 11.9 Hz), 2.34 (1H, s), 2.82 (1H, brs), 3.79 (1H, d, J = 1 : 1.9 Hz), 3.93 (1H, d, J = 1 : 1.9 Hz), 4.88 (1H, dd, J = 5.0, 11.7 Hz), 4.99 (1H, d, J = 3.4 Hz), 5.85 (1H, dd, J = 2.6, 10.4 Hz), 5.88 (1H, d, J = 1 : 1.4 Hz), 6.48 (1H, s), 7.41 (1H, dd, J = 4.8, 8.0 Hz), 8.11 (1H, ddd, J = 1.6, 2.3, 8.0 Hz), 8.69 (1H, dd, J = 1.6, 4.8 Hz), 9.03 (1H, d, J = 2.3 Hz)

Synthesis Example 90: Compound 1-1258

Compound 1-1259 (270 mg) obtained in Synthesis Example 85 was suspended in methanol-water (9:1, 5 mL). Potassium carbonate (29 mg) was added to the suspension at 0°C. The mixture was stirred at 0°C for one hr. Potassium carbonate (29 mg) was added thereto. The mixture was stirred for 3.5 hr. The reaction was stopped by the addition of acetic acid (0.1 mL), followed by concentraiton under the reduced pressure. Water was added to the residue, and the mixture was extracted with chloroform. The chloroform layer was washed with saturated brine and was dried over anhydrous sodium sulfate, and the solvent was then removed by evaporation under the reduced pressure to give a crude product. The crude product was purified by preparative thin layer chromatography (Merck Silica Gel 60F254 0.5 mm, hexane : acetone = 1 : 1) to give compound 1-1258 (51 mg).
ESI-MS;594 m/z $(M+H)^+$

Synthesis Example 91: Compound 1-1390

1,11-O-Acetonide-1,7,11-tri-deacetyl pyripyropene A (168 mg) synthesized by a method described in WO 2009/022702 was dissolved in N,N-dimethylformamide (2 mL). Imidazole (92 mg) and tert-butyldimethylchlorosilane (204 mg) were added to the solution. The mixture was stirred at room temperture for 22 hr. Water was poured into the reaction soluiton, and the mixture was extracted with chloroform. The chloroform layer was washed with saturated brine and was dried over anhydrous sodium sulfate, and the solvent was then removed by evaporation under the reduced pressure to give a crude product. The crude product was purified by preparative thin layer chromatography (Merck Silica Gel $60F_{254}$ 0.5 mm, chloroform : methanol = 20 : 1) to give compound 1-1390 (187 mg).
ESI-MS;m/z 612 $(M+H)^+$; $^1$H-NMR ($CDCl_3$) $\delta$ 0.11 (3H, s), 0.16 (3H, s), 0.96 (9H, s), 1.03 (1H, m), 1.10 (3H, s), 1.33 (1H, dt, J = 3.6, 12.8 Hz), 1.40 (3H, s), 1.43 (3H, s), 1.44 (3H, s), 1.39-1.44 (1H, m), 1.55-1.58 (2H, m), 1.58 (3H, s), 1.64 (1H, q, J = 12.0 Hz), 1.81 (1H, dq, J = 3.6, 12.8 Hz), 2.20 (1H, dt, J = 3.2, 12.8 Hz), 2.81 (1H, d, J = 1.6 Hz), 3.42 (1H, d, J = 10.8 Hz), 3.51 (1H, d, J = 10.4 Hz), 3.50-3.53 (1H, m), 3.72 (1H, dd, J = 4.8, 11.2 Hz), 4.97 (1H, m), 6.35 (1H, s), 7.41 (1H, dd, J = 4.8, 8.0 Hz), 8.10 (1H, dt, J = 1.6, 8.0 Hz), 8.69 (1H, dd, J = 1.6, 4.8 Hz), 9.00 (1H, d, J = 2.0 Hz)

Synthesis Example 92: Compound 1-1389

Compound 1-1390 (116 mg) obtained in Synthesis Example 91 was dissolved in tetrahydrofuran (1 mL), and 63% acetic acid (4 mL) was added to the solution at 0°C. The mixture was stirred at room temperature for 24 hr. An aqueous sodium hydrogencarbonate solution was then added thereto, and the mixture was extraced with chloroform. The chloroform layer was washed with a saturated aqueous sodium hydrogencarbonate solution and saturated brine and was dried over anhydrous sodium sulfate, and the solvent was then removed by evaporation under the reduced pressure to give a crude product. The crude product was purified by preparative thin layer chromatography (Merck Silica Gel $60F_{254}$ 0.5 mm, chloroform : methanol = 10 : 1) to give compound 1-1389 (91 mg).
ESI-MS;m/z 572 $(M+H)^+$; $^1$H-NMR ($CD_3OD$) $\delta$ 0.08 (3H, s), 0.13 (3H, s), 0.64 (3H, s), 0.90 (9H, s), 1.19 (1H, dt, J = 3.6, 12.8 Hz), 1.31 (3H, s), 1.33-1.36 (2H, m), 1.48 (1H, t, J = 12.0 Hz), 1.53 (3H, s), 1.62-1.80 (3H, m), 1.99-2.03 (1H, m), 3.16 (1H, d, J = 10.8 Hz), 3.44 (1H, d, J = 10.8 Hz), 3.56 (1H, dd, J = 4.8, 11.6 Hz), 3.76 (1H, dd, J = 5.2, 11.2 Hz), 4.86 (1H, d, J = 3.2 Hz), 6.47 (1H, s), 7.47 (1H, ddd, J = 0.8, 4.8, 8.0 Hz), 8.17 (1H, dt, J = 2.0, 8.4 Hz), 8.55 (1H, dd, J = 2.0, 4.8 Hz), 8.91 (1H, dd, J = 0.8, 2.4 Hz)

Synthesis Example 93: Compound 1-1244

1,7,11-Trideacetyl pyripyropene A (20 mg) synthesized by a method described in Japanese Patent Laid-Open No. 259569/1996 was dissolved in dichloromethane (1 mL). 2,6-Lutidine (28 mg) and trifluoromethanesulfonic acid tert-butyldimethylsilyl (28 mg) were added to the solution at 0°C. The mixture was stirred for 4.5 hr, water was added to thereto, and the mixture was exctracted with dichloromethane. The dichloromethane layer was washed with saturated brine and was dried over anhydrous magnesium sulfate, and the solvent was then removed by evaporation under the reduced pressure to give a crude product. The crude product was purified by preparative thin layer chromatography (Merck Silica Gel 60F254 0.5 mm, ethyl acetate : hexane = 1 : 2) to give compound 1-1244 (32 mg).
ESI-MS;800 m/z $(M+H)^+$

Synthesis Example 94: Compound 226-264

Compound 1-264(1.0 g) synthesized by a method described in W02009/022702 was dissolved in N,N-dimethylformamide (10 mL). triethylamine(1.6 g) and N-dimethylaminopyridine (191 mg) were added to the solution. Further, acetic anhydride (1.6 g)was added to the solution at 0°C. The mixture was stirred for 4.5 hr, water was added thereto, and the mixture was exctracted with ethyl acetate. The ethyl acetate layer was washed with saturated brine and was dried over anhydrous magnesium sulfate, and the solvent was then removed by evaporation under the reduced pressure to give a crude product of compound 226-264. The crude product was purified by Silica Gel chromatography (Mega Bond Elut (Varian), hexane : acetone = 3 : 1) to give compound 226-264 (877 mg).
ESI-MS;678 m/z $(M+H)^+$; $^1$H-NMR (CDCl3) δ 0.83-0.90 (4H, m), 0.87 (3H, s), 0.96-1.06 (4H, m), 1.13 (3H, s), 1.25-1.32 (1H, m), 1.54-1.66 (4H, m), 1.74 (3H, s), 1.68-1.90 (4H, m), 2.11 (3H, s), 2.18 (3H, s), 2.40-2.44 (1H, m), 3.74 (1H, d, J = 12.0 Hz), 3.78 (1H, d, J = 12.0 Hz), 4.82 (1H, dd, J = 4.8, 11.6 Hz), 5.02-5.06 (1H, m), 6.38 (1H, d, J = 3.6 Hz), 6.42 (1H, s), 7.40 (1H, dd, J = 4.8, 8.0 Hz), 8.11 (1H, ddd, J = 1.6, 1.6, 8.0 Hz), 8.69 (1H, dd, J = 1.6, 4.8 Hz), 9.00 (1H, d, J = 1.6 Hz)

Synthesis Example 95: Compound 121-264

Compound 226-264 (100 mg) was dissolved in methanol (1 mL). 5% HCl(82 mg) was added to the solution. The mixture was stirred at room temperature for 47 hrs, triethylamine was added to thereto, and the solvent was then removed by evaporation under the reduced pressure to give a crude product of comoppund 121-264. The crude product was purified by preparative thin layer chromatography (Merck Silica Gel 60F254 0.5 mm, hexane:acetone = 2:1) to give compound 121-264.
ESI-MS; 650 m/z $(M+H)^+$; $^1$H-NMR (CDCl3) δ 0.85-0.88 (4H, m), 0.90 (3H, s), 0.96-1.05 (4H, m), 1.38 (3H, s), 1.31-1.40 (1H, m), 1.47 (1H, d, J = 3.2 Hz), 1.54-1.64 (4H, m), 1.71 (3H, s), 1.79 (1H, dd, J = 3.6, 12.6 Hz), 1.83-1.95 (2H, m), 2.01-2.05 (1H, m), 2.17 (3H, s), 3.59 (3H, s), 3.76 (2H, s), 4.68 (1H, d, J = 2.8 Hz), 4.81 (1H, dd, J = 4.8, 12.0 Hz), 4.97 (1H, dd, J = 4.8, 12.0 Hz), 6.39 (1H, s), 7.40 (1H, dd, J = 4.8, 8.0 Hz), 8.10 (1H, ddd, J = 1.6, 1.6, 8.0 Hz), 8.68 (1H, dd, J = 1.6, 4.8 Hz), 9.01 (1H, d, J = 1.6 Hz)

Synthesis Example 96: Compound 121-260

Compound 121-264 (10 mg) was dissolved in methanol-water (10 : 1, 1.1 mL). Pottasium carbonate(6 mg) was added to the solution. The solution wa stireed at 0°C for 9 hours, acetic acid (0.1 mL) was added thereto, and the mixture was then concentrated under the reduced pressure. Water was added to the residue, and the solution was exctracted with ethyl acetate. The ethyl acetate layer was washed with saturated brine and was dried over anhydrous magnesium sulfate, and the solvent was then removed by evaporation under the reduced pressure to give a crude product of compound 121-260. The crude product was purified by preparative thin layer chromatography ( Merck Silica Gel $60F_{254}$ 0.5 mm , Chloroform:acetone=2:1) to give compound 121-260 (4.5 mg) .
ESI-MS; 608 m/z $(M+H)^+$; $^1$H-NMR (CDCl3) δ 0.85-0.87 (4H, m), 0.91 (3H, s), 0.97-0.98 (4H, m), 1.36 (3H, s), 1.31-1.45 (3H, m), 1.55-1.64 (3H, m), 1.69 (3H, s), 1.79-1.93 (3H, m), 2.01-2.04 (1H, m), 2.73 (1H, brs), 3.61 (3H, s), 3.73-3.76 (1H, m), 3.74 (1H, d, J = 11.6 Hz), 3.85 (1H, d, J = 11.6 Hz), 4.67 (1H, d, J = 2.8 Hz), 4.81 (1H, dd, J = 4.8, 11.6 Hz), 6.47 (1H, s), 7.42 (1H, dd, J = 4.8, 8.0 Hz), 8.11 (1H, d, J = 8.0 Hz), 8.68 (1H, d, J = 4.0 Hz), 8.99 (1H, s)

Synthesis Example 97: Compound 227-264

Compound 1-264(1.0 g) synthesized by a method described in W02009/022702 was dissolved in ethyl acetate(10 mL). Pridine(3.7 g)and cyclopropane carbonyl chloride (4.9 g)was added to the solution. The mixture was stirred at 40°C for 8 hrs, methanol was added to thereto, and the solution was concentrated under the reduced pressure. Water

was added to the residue, and the solution was exctracted with ethyl acetate. The ethyl acetate layer was washed with saturated brine and was dried over anhydrous magnesium sulfate, and the solvent was then removed by evaporation under the reduced pressure to give a crude product of compound 227-264. The crude product was purified by preparative thin layer chromatography ( Mega Bond Elut (Varian) , hexane:acetone=5:1 ) to give compound 227-264 (220 mg) .
ESI-MS; 704 m/z $(M+H)^+$; $^1$H-NMR (CDCl3) δ 0.84-0.90 (6H, m), 0.87 (3H, s), 0.95-1.06 (6H, m), 1.14 (3H, s), 1.23-1.33 (1H, m), 1.54-1.65 (5H, m), 1.73 (3H, s), 1.69-1.90 (4H, m), 2.18 (3H, s), 2.42-2.45 (1H, m), 3.74 (1H, d, J = 12.0 Hz), 3.78 (1H, d, J = 12.0 Hz), 4.82 (1H, dd, J = 4.4, 12.0 Hz), 5.02-5.08 (1H, m), 6.39 (1H, d, J = 3.2 Hz), 6.42 (1H, s), 7.40 (1H, dd, J = 4.8, 8.0 Hz), 8.10 (1H, ddd, J = 2.0, 2.0, 8.0 Hz), 8.68 (1H, dd, J = 2.0, 4.8 Hz), 9.00 (1H, d, J = 2.0 Hz)

Synthesis Example 98 : Compound 227-260

Compound 227-264 (220 mg) was dissolved in methanol-water (10 : 1, 2.2 mL). Pottasium carbonate (18 mg) was added to the solution. The solution wa stireed at 0°C for 4 hours, acetic acid was added thereto, and the mixture was then concentrated under the reduced pressure. Water was added to the residue, and the solution was exctracted with ethyl acetate. The ethyl acetate layer was washed with saturated brine and was dried over anhydrous magnesium sulfate, and the solvent was then removed by evaporation under the reduced pressure to give a crude product of compound 227-260. The crude product was purified by preparative thin layer chromatography ( Mega Bond Elut (Varian), hexane:acetone =3:1 ) to give compound 227-260 (100 mg) .
ESI-MS;662 m/z $(M+H)^+$;$^1$H-NMR (CDCl3) δ 0.81-0.92 (6H, m), 0.89 (3H, s), 0.93-1.07 (6H, m), 1.13 (3H, s), 1.22-1.26 (1H, m), 1.47 (1H, d, J = 12.0 Hz), 1.57-1.66 (6H, m), 1.70 (3H, s), 1.84-1.87 (2H, m), 2.42 (1H, d, J = 13.2 Hz), 2.84 (1H, brs), 3.71 (1H, d, J = 11.6 Hz), 3.80-3.82 (1H, m), 3.87 (1H, d, J = 11.6 Hz), 4.82 (1H, dd, J = 4.0, 11.6 Hz), 6.38 (1H, s), 6.50 (1H, s), 7.42 (1H, dd, J = 5.2, 7.2 Hz), 8.12 (1H, d, J = 7.6 Hz), 8.68 (1H, d, J = 4.0 Hz), 9.00 (1H, s)

Synthesis Example 99 : Compound 72-263

Compound 1-260(20 mg) synthesized by a method described in W02006/129714 was dissolved in Toluene (2 ml) . 1, 1'-thiocarbonylimidazol (36 mg) was added thereto at room temperature. The solution was refluxed under heating for 14 hours, and then the reaction mixture was cooled to room temperature. Water was added thereto and the mixture was exctracted with ethyl acetate. The ethyl acetate layer was washed with saturated brine and was dried over anhydrous magnesium sulfate, and the solvent was then removed by evaporation under the reduced pressure to give a crude product of compound 72-263. The crude product was purified by preparative thin layer chromatography (Merck Silica Gel $60F_{254}$ 0.5 mm, acetone : hexane=1 : 1) to give compound 72-263 (1.4 mg) .
ESI-MS ; 558 m/z $(M+H)^+$

Synthesis Example 100 : Compound 97-8

Compound 89-8(20 mg) synthesized by a method described in J. Antibiot., 49(11), 1133, 1996 was dissolved in ethanol (2 mL). 28%aquerous ammonia(114 mg) was added to the solution at room temperature. The solution was stirred for 39hrs, and then concentrated under the reduced pressure. Chloroform and water were added to the residue. Further, the mixture was exctracted with Chloroform. The chlorofor layer was washed with saturated brine and was dried over anhydrous magnesium sulfate, and the solvent was then removed by evaporation under the reduced pressure to give a crude product of compound 97-8. The crude product was purified by preparative thin layer chromatography (Merck Silica Gel 60F254 0.5 mm, hexane: acetone =1:1) to give compound 97-8 (11 mg).
ESI-MS ; 581 m/z $(M+H)^+$

Synthesis Example101 : Compound 227-259

Compound 227-260 (30 mg) was dissolved in N,N-dimethylformamide (1 mL). Pyridine(21 mg) and cyclopropane carbonyl chloride (14 mg) was added to the solution. The solution was stirred at 0°C for 4 hours, water was added therto, and the mixture was extracted with ethyl acetate. The ethyl acetate layer was washed with saturated brine and was dried over anhydrous magnesium sulfate, and the solvent was then removed by evaporation under the reduced pressure to give a crude product of compound 227-259. The crude product was purified by preparative thin layer chromatography (Merck Silica Gel 60F254 0.5 mm, hexane: acetone =1:1) to give compound 227-259 (9.8 mg) .
ESI-MS; 730 m/z $(M+H)^+$; $^1$H-NMR (CDCl3) δ 0.83-0.91 (8H, m), 0.87 (3H, s), 0.95-1.12 (8H, m), 1.14 (3H, s), 1.21-1.26 (1H, m), 1.54-1.64 (5H, m), 1.75 (3H, s), 1.67-1.89 (5H, m), 2.42-2.45 (1H, m), 3.72 (1H, d, J = 11.6 Hz), 3.79 (1H, d, J = 11.6 Hz), 4.80 (1H, dd, J = 4.8, 11.6 Hz), 5.01-5.07 (1H, m), 6.38 (1H, d, J = 3.2 Hz), 6.41 (1H, s), 7.40 (1H, dd, J = 4.8, 8.0 Hz), 8.11 (1H, ddd, J = 1.6, 2.0, 8.0 Hz), 8.68 (1H, dd, J = 1.6, 4.8 Hz), 9.01 (1H, d, J = 2.0 Hz)

Synthesis Example102 : Compound 121-259

Compound 121-260 (20 mg) was dissolved in N,N-dimethylformamide (1 mL). Pyridine (16 mg) and cyclopropane carbonyl chloride (10 mg) was added to the solution. The solution was stirred at 0°C for 2 hours, water was added therto, and the mixture was extracted with ethyl acetate. The ethyl acetate layer was washed with saturated brine and was dried over anhydrous magnesium sulfate, and the solvent was then removed by evaporation under the reduced pressure to give a crude product of compound 121-259. The crude product was purified by preparative thin layer chromatography (Merck Silica Gel 60F254 0.5 mm, hexane: acetone =1:1) to give compound 121-259 (9.1 mg) .
ESI-MS ; 676 m/z $(M+H)^+$ ; $^1$H-NMR (CDCl3) $\delta$ 0.84-1.11 (12H, m), 0.90 (3H, s), 1.31-1.40 (1H, m), 1.38 (3H, s), 1.46 (1H, d, J = 3.2 Hz), 1.55-1.64 (4H, m), 1.68-1.73 (1H, m), 1.73 (3H, s), 1.79-1.95 (3H, m), 2.01-2.05 (1H, m), 3.61 (3H, s), 3.71 (1H, d, J = 11.6 Hz), 3.81 (1H, d, J = 11.6 Hz), 4.67 (1H, d, J = 2.8 Hz), 4.79 (1H, dd, J = 4.8, 11.6 Hz), 4.96 (1H, dd, J = 4.8, 11.6 Hz), 6.39 (1H, s), 7.41 (1H, dd, J = 4.8, 8.0 Hz), 8.11 (1H, d, J = 8.0 Hz), 8.68 (1H, d, J = 4.8 Hz), 9.01 (1H, s)

Synthesis Example 103 : Compound 226-259

Compound 1-259(30 mg) synthesized by a method described in W02006/12971 was dissolved in dichloromethane (1 mL). Triethylamine (46 mg), N-dimethylaminopyridine (5 mg) and acetic anhydride were added to the solution. The solution was stirred at room temperature for 13 hours, , water was added thereto, and the mixture was exctracted with ethyl acetate. The ethyl acetate layer was washed with saturated brine and was dried over anhydrous magnesium sulfate, and the solvent was then removed by evaporation under the reduced pressure to give a crude product of compound 226-259. The crude product was purified by Silica Gel chromatography (Merck Silica Gel 60F254 0.5 mm, hexane:acetone=1:1) to give compound 226-259 (23 mg) .
ESI-MS;704 m/z $(M+H)^+$;$^1$H-NMR (CDCl3) $\delta$ 0.83-0.89 (6H, m), 0.87 (3H, s), 0.95-1.10 (6H, m), 1.13 (3H, s), 1.25-1.32 (1H, m), 1.54-1.64 (5H, m), 1.72 (3H, s), 1.67-1.90 (4H, m), 2.11 (3H, s), 2.40-2.43 (1H, m), 3.72 (1H, d, J = 11.6 Hz), 3.79 (1H, d, J = 11.6 Hz), 4.80 (1H, dd, J = 4.8, 11.6 Hz), 5.01-5.07 (1H, m), 6.37 (1H, d, J = 3.2 Hz), 6.41 (1H, s), 7.40 (1H, dd, J = 4.8, 8.0 Hz), 8.11 (1H, ddd, J = 1.6, 2.0, 8.0 Hz), 8.68 (1H, dd, J = 1.6, 4.8 Hz), 9.00 (1H, d, J = 2.0 Hz)

Preparation Example 1: Wettable powder

| | |
|---|---|
| Compound 43-260 | 30 wt% |
| Clay | 30 wt% |
| Diatomaceous earth | 35 wt% |
| Calcium lignin sulfonate | 4 wt% |
| Sodium lauryl sulfate | 1 wt% |

The above ingredients were intimately mixed together, and the mixture was ground to prepare wettable powder.

Preparation Example 2: Dust

| | |
|---|---|
| Compound 43-260 | 2 wt% |
| Clay | 60 wt% |
| Talc | 37 wt% |
| Calcium stearate | 1 wt% |

The above ingredients were intimately mixed together to prepare dust.

Preparation Example 3: Emulsifiable concentrate

| | |
|---|---|
| Compound 72-260 | 20 wt% |

(continued)

| | |
|---|---|
| N,N-Dimethylformamide | 20 wt% |
| Solvesso 150 (Exxon Mobil Corporation) | 50 wt% |
| Polyoxyethylene alkyl aryl ether | 10 wt% |

The above ingredients were intimately mixed together and dissolved to prepare an emulsifiable concentrate.

Preparation Example 4: Granules

| | |
|---|---|
| Compound 1-1258 | 5 wt% |
| Bentonite | 40 wt% |
| Talc | 10 wt% |
| Clay | 43 wt% |
| Calcium lignin sulfonate | 2 wt% |

The above ingredients were homogeneously ground and intimately mixed together. Water was added to the mixture, followed by thorough kneading. Thereafter, the kneaded product was granulated and dried to prepare granules.

Preparation Example 5: Floables

| | |
|---|---|
| Compound 1-1264 | 25 wt% |
| POE polystyrylphenyl ether sulfate | 5 wt% |
| Propylene glycol | 6 wt% |
| Bentonite | 1 wt% |
| 1% aqueous xanthan gum solution | 3 wt% |
| PRONAL EX-300 (Toho Chemical Industry Co., Ltd.) | 0.05 wt% |
| ADDAC 827 (K.I. Chemical Industry Co., Ltd.) | 0.02 wt% |
| Water | To 100 wt% |

All the above ingredients except for the 1% aqueous xanthan gum solution and a suitable amount of water were premixed together, and the mixture was then ground by a wet grinding mill. Thereafter, the 1% aqueous xanthan gum solution and the remaining water were added to the ground product to prepare 100 wt% floables.

Test Example 1: Pesticidal effect against Myzus persicae

A leaf disk having a diameter of 2.8 cmϕ was cut out from a cabbage grown in a pot and was placed in a 5.0 cm-Schale. Four adult aphids of Myzus persicae were released in the Schale. One day after the release of the adult aphids, the adult aphids were removed. The number of larvae at the first instar born in the leaf disk was adjusted to 10, and a test solution, which had been adjusted to a concentration of 20 ppm by the addition of a 50% aqueous acetone solution (0.05% Tween 20 added) was spread over the cabbage leaf disk. The cabbage leaf disk was then air dried. Thereafter, the Schale was lidded and was allowed to stand in a humidistat chamber (light period 16 hr - dark period 8 hr) (25°C). Three days after the initiation of standing of the Schale, the larvae were observed for survival or death, and the death rate of larvae was calculated by the following equation.

$$\text{Death rate (\%)} = \{\text{number of dead larvae}/(\text{number of survived larvae} + \text{number of dead larvae})\} \times 100$$

As result, it was found that the insecticidal activity was not less than 80% for compounds of Nos. 1-145, 1-146, 1-264, 1-265, 1-267, 1-268, 1-269, 1-272, 1-273, 1-276, 1-329, 1-330, 1-333, 1-342, 1-355, 1-356, 1-357, 1-359, 1-360, 1-361,

1-364, 1-365, 1-389, 1-390, 1-394, 1-641, 1-700, 1-716, 1-745, 1-746, 1-748, 1-1262, 1-1263, 1-1264, 72-763, 72-113, 72-260, 89-260, 114-357, 135-751, and 139-136.

Test Example 2: Pesticidal effect against Myzus persicae

A leaf disk having a diameter of 2.8 cm was cut out from a cabbage grown in a pot and was placed in a 5.0 cm-Schale. Four adult aphids of Myzus persicae were released in the Schale. One day after the release of the adult aphids, the adult aphids were removed. The number of larvae at the first instar born in the leaf disk was adjusted to 10, and a test solution, which had been adjusted to a concentration of 0.156 ppm by the addition of a 50% aqueous acetone solution (0.05% Tween 20 added) was spread over the cabbage leaf disk. The cabbage leaf disk was then air dried. Thereafter, the Schale was lidded and was allowed to stand in a humidistat chamber (light period 16 hr - dark period 8 hr) (25°C). Three days after the initiation of standing, the larvae were observed for survival or death, and the death rate of larvae was calculated by the following equation.

Death rate (%) = {number of dead larvae/(number of survived larvae + number of dead larvae)} x 100

As result, it was found that the insecticidal activity was not less than 80% for compounds of Nos. 1-264, 1-265, 1-267, 1-268, 1-269, 1-272, 1-273, 1-276, 1-330, 1-357, 1-359, 1-360, 1-389, 1-390, 1-394, 1-641, 1-716, and 1-1262.

Test Example 3: Pesticidal effect against Aphis gossypii

A leaf disk having a diameter of 2.0 cmϕ was cut out from a cucumber grown in a pot and was placed in a 5.0 cm-Schale. Test solutions adjusted to a 5 ppm concentration (50% aqueous acetone solution; 0.05% Tween 20 added) were applied to the leaf disk. The leaf disk was then air dried, and ten larvae at the first instar born of Aphis gossypii were released. Thereafter, the Schale was lidded and was then allowed to stand in a humidistat chamber (light period 16 hr - dark period 8 hr) (25°C). Three days after the release, the larvae were observed for survival or death, and the death rate of larvae was calculated by the following equation.

Death rate (%) = {number of dead larvae/(number of survived larvae + number of dead larvae)} x 100

As result, it was found that the insecticidal activity was not less than 80% for compounds of Nos. 1-3, 1-357, 1-361, 1-389, 1-394, 1-701, 1-707, 1-708, 1-709, 1-1258, 43-260, 43-262, 43-713, 72-260, 121-259, 121-260, 121-264, 226-259, 226-264, 227-259, 227-260, 227-264.

Test Example 4: Pesticidal effect against Trialeurodes vaporariorum

A haricot leaf disk having a diameter of 1.6 cmϕ was put on an absorbent cotton placed in a plastic Schale. Test solutions adjusted to a 5 ppm concentration (50% aqueous acetone solution; 0.05% Tween 20 added) were applied to the leaf disk. The leaf disk was then air dried, and five adults of Trialeurodes vaporariorum were released and were then allowed to stand in a humidistat chamber (light period 16 hr - dark period 8 hr) (25°C). Six days after the release, the larvae were observed for survival or death, and the death rate of larvae was calculated by the following equation.

Death rate (%) = {number of dead larvae/(number of survived larvae + number of dead larvae)} x 100

As result, it was found that the insecticidal activity was not less than 80% for compounds of Nos. 1-269, 1-389, 1-701, 1-708, and 1-1264.

## Claims

1. A composition for controlling harmful organisms, comprising as an active ingredient one or more of compounds represented by formula (I) or salts thereof, and an agriculturally or zootechnically acceptable carrier:

[Chemical formula 1]

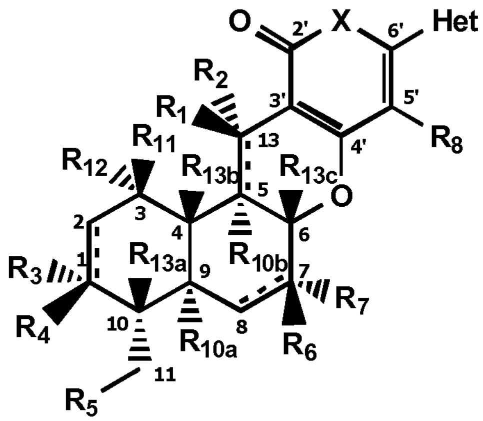

(I)

wherein

Het represents optionally substituted heterocyclic group, or

optionally substituted phenyl,
optionally substituted $C_{1-18}$ alkyl, or
optionally substituted $C_{2-18}$ alkenyl,

X represents an oxygen atom or $NR_9$ wherein $R_9$ represents a hydrogen atom, $C_{1-6}$ alkyl, or aryl $C_{1-6}$ alkyl, $R_1$, $R_2$, $R_3$, $R_4$, $R_5$, $R_6$, $R_7$, $R_{10a}$, $R_{10b}$, $R_{11}$, and $R_{12}$, which may be the same or different, each independently represent

a hydrogen atom,
hydroxyl,
**optionally substituted** $C_{1-18}$ alkylaminocarbonyloxy,
**optionally substituted** $C_{1-18}$ alkylcarbonyloxy,
adamantylcarbonyloxy,
**optionally substituted aryl C**$_{1-6}$ alkylcarbonyloxy,
**optionally substituted** $C_{2-6}$ alkenylcarbonyloxy,
**optionally substituted** $C_{2-6}$ alkynylcarbonyloxy,

optionally substituted saturated or **unsaturated heterocyclic** $C_{1-6}$ alkylcarbonyloxy,
optionally substituted saturated or **unsaturated heterocyclic** $C_{2-6}$ alkenylcarbonyloxy,

optionally substituted arylcarbonyloxy,
optionally substituted carbamoyloxy,
optionally substituted carbamoyl,
optionally substituted $C_{1-6}$ alkylsulfonyloxy,
optionally substituted $C_{1-6}$ alkylsufonyl,
optionally substituted arylsufonyloxy,
optionally substituted aryl $C_{1-6}$ alkyloxy,
optionally substituted aryloxycarbonyloxy,
optionally substituted arylaminocarbonyloxy,
optionally substituted arylsulfonyl,
optionally substituted arylsulfanyl,
optionally substituted saturated or unsaturated heterocyclic sulfanyl,
optionally substituted $C_{1-6}$ alkyloxy,
optionally substituted $C_{2-6}$ alkenyloxy,
optionally substituted $C_{2-6}$ alkynyloxy,

optionally substituted aryloxy,
$C_{1-6}$ alkyloxy-$C_{1-6}$alkyloxy,
$C_{1-6}$ alkylthio-$C_{1-6}$alkyloxy,
$C_{1-6}$ alkyloxy-$C_{1-6}$ alkyloxy-$C_{1-6}$alkyloxy,
**optionally substituted** $C_{1-6}$ alkyloxycarbonyloxy,
optionally substituted saturated or unsaturated heterocyclic oxy,
optionally substituted saturated or unsaturated heterocyclic thio,
optionally substituted saturated or unsaturated heterocyclic carbonyloxy,
optionally substituted saturated or unsaturated heterocyclic thiocarbonyloxy,
optionally substituted phosphate group,
optionally substituted $C_{1-6}$ alkyl,
tri-$C_{1-6}$ alkylsilyloxy,
optionally substituted saturated or unsaturated heterocyclic ring,
azide,
optionally substituted imino,
optionally substituted amino,
optionally substituted hydrazino, cyano,
a halogen atom,
-O-N=C-Y1

wherein Y1 represents a hydrogen atom, optionally substituted $C_{1-6}$ alkyl, optionally substituted $C_{3-7}$ cycloalkyl, optionally substituted $C_{2-6}$ alkenyl, optionally substituted $C_{2-6}$ alkynyl, optionally substituted $C_{1-6}$ alkoxy, optionally substituted phenyl, or optionally substituted heterocyclic ring or
either $R_1$ and $R_2$, $R_3$ and $R_4$, $R_6$ and $R_7$, and $R_{11}$ and $R_{12}$ each independently together, or one of hydrogen atoms substituted at the carbon atom of the 11-position and $R_5$ together represent
oxo,

=C-Y2 wherein Y2 represents nitro, cyano, optionally substituted imino, hydroxymethyl, hydroxycarbonyl, optionally substituted $C_{1-6}$ alkoxycarbonyl, optionally substituted phenyl, optionally substituted benzyl, optionally substituted phenoxymethyl, optionally substituted aryl oxymethyl, optionally substituted pyridyloxymethyl, optionally substituted pyrimidinyloxymethyl, optionally substituted $C_{1-6}$ alkylcarbonyl, optionally substituted $C_{1-6}$ alkyloxy carbonyl, optionally substituted $C_{1-4}$ alkylaminocarbonyl, optionally substituted phenylaminocarbonyloxy, optionally substituted benzylaminocarbonyloxy, or optionally substituted heterocyclic aminocarbonyloxy, or
=N-Q-Y3 wherein Y3 represents $R_1$', -Z-$R_1$', -Z-O -$R_1$', or -Z-N($R_1$') ($R_1$"), Z represents a bond, -C(=O)-, -C(=S)-, -C(=O)-N-, -C(=S)-N-, or -$SO_2$-, Q represents O or -N-$R_5$', $R_1$' and $R_1$", which may be the same or different, each independently represent a hydrogen atom, optionally substituted $C_{1-}C_{12}$ alkyl, optionally substituted $C_2$-$C_{12}$ alkenyl, optionally substituted $C_2$-$C_{12}$ alkynyl, optionally substituted $C_3$-$C_{12}$-cycloalkyl, optionally substituted $C_5$-$C_{12}$-cycloalkenyl, optionally substituted aryl, or optionally substituted heterocyclic ring, or $R_1$' and $R_1$" together may form an optionally substituted three- to seven-membered saturated or unsaturated cycloalkyl, or a three- to seven-membered heterocyclic ring comprising one or two atoms or groups selected from oxygen, nitrogen, and sulfur atoms and sulfoxide and sulfone groups, the carbon and nitrogen atoms optionally comprised in the ring are optionally substituted with $C_1$-$C_8$ alkyl, hydroxy-$C_1$-$C_8$ alkyl, $C_3$-$C_8$ cycloalkyl, $C_2$-$C_8$ alkenyl, $C_2$-$C_8$ alkynyl, phenyl, benzyl, $C_{1-6}$ alkyl carbonyl, $C_{2-6}$ alkenyl carbonyl, $C_{1-6}$ alkyl carbonylmethyl, or $C_{2-6}$ alkenyl carbonylmethyl, $R_5$' represents a hydrogen atom, $C_{1-8}$ alkyl, hydroxy-$C_{1-8}$ alkyl, $C_{3-8}$ cycloalkyl, $C_{2-8}$ alkenyl, $C_{2-8}$ alkynyl, phenyl, benzyl, $C_{1-6}$ alkylcarbonyl, $C_{2-6}$ alkenylcarbonyl, $C_{1-6}$ alkylcarbonylmethyl, or $C_{2-6}$ alkenylcarbonylmethyl,

when Y3 represents $R_1$' while Q represents -N-$R_5$', $R_1$' and $R_5$' together may form an optionally substituted three- to seven-membered saturated or unsaturated cycloalkyl, or a three- to seven-membered heterocyclic ring comprising one or two atoms or groups selected from oxygen, nitrogen, and sulfur atoms, sulfoxide and sulfone groups, the carbon and nitrogen atoms comprised in the ring being optionally substituted by a group selected from the group consisting of $C_{1-8}$ alkyl, hydroxy-$C_{1-8}$ alkyl, $C_{3-8}$cycloalkyl, $C_{2-8}$ alkenyl, $C_{2-8}$ alkynyl, phenyl, benzyl, $C_{1-6}$ alkyl carbonyl, $C_{2-6}$ alkenyl carbonyl, $C_{1-6}$ alkyl carbonylmethyl, or $C_{2-6}$ alkenyl carbonylmethyl, wherein the substituent optionally substituted at each of $R_1$', $R_1$", and $R_5$' represents a halogen, cyano, nitro, hydroxyl, $C_{1-4}$ alkyl optionally substituted by a halogen, $C_{1-4}$ alkyloxy optionally substituted by a halogen, $C_{1-4}$ alkylthio optionally substituted by a halogen, $C_{1-4}$ alkylsulfinyl optionally substituted by a halogen, $C_{1-4}$ alkylsufonyl optionally substituted

by a halogen, $C_{1-4}$ alkyl carbonyl optionally substituted by a halogen, $C_{1-4}$ alkoxycarbonyl optionally substituted by a halogen, and $C_{3-6}$ trialkylsilyl, or

one of hydrogen atoms substituted at the carbon atom of 11-position and $R_5$ together may further represent formyl,

carboxyl,
aryl, or $C_{1-6}$ alkyloxy carbonyl optionally

substituted by a saturated or unsaturated heterocyclic ring,

**aryl** $C_{1-6}$ alkylaminocarbonyl optionally substituted by $C_{1-6}$ alkyloxy,
$C_{1-6}$ alkylaminocarbonyl,
saturated or unsaturated heterocyclic aminocarbonyl,
hydroxy $C_{1-6}$ alkylaminocarbonyl or
$C_{1-6}$ alkylaminocarbonyl optionally substituted by $C_{1-6}$ alkyloxycarbonyl and/or aryl, or

$R_1$ and $R_2$, $R_3$ and $R_4$, $R_6$ and $R_7$, and $R_{11}$ and $R_{12}$ each independently together represent

optionally substituted three- to seven-membered saturated or unsaturated cycloalkyl,

or may form a three- to seven-membered heterocyclic ring comprising one or two atoms or groups selected from oxygen, nitrogen, sulfur atoms and sulfoxide and sulfone groups, the carbon and nitrogen atoms comprised in the ring being optionally substituted by $C_{1-8}$ alkyl, hydroxy-$C_{1-8}$ alkyl, $C_{3-8}$cycloalkyl, $C_{2-8}$ alkenyl, $C_{2-8}$ alkynyl, phenyl, benzyl, $C_{1-6}$ alkyl carbonyl, $C_{2-6}$ alkenyl carbonyl, $C_{1-6}$ alkyl carbonylmethyl, or $C_{2-6}$ alkenyl carbonylmethyl, or
$R_1$ or r $R_2$ is absent, and a hydrogen atom substituted at the carbon atom of the 5-position is lost to represent a double bond between the 5-position and the 13-position, or
$R_6$ or $R_7$ is absent, and one of hydrogen atoms substituted at the carbon atom of the 8-position is lost to represent a double bond between the 7-position and the 8-position, or
$R_3$ or $R_4$ is absent, and a hydrogen atom substituted at the carbon atom of the 2-position is lost to represent a double bond between the 1-position and the 2-position, or
$R_3$ or $R_4$ and $R_5$ together represent -O-$CR_3$'($R_4$')-O-wherein $R_3$' and $R_4$', which may be the same or different, represent a hydrogen atom, $C_{1-6}$ alkyl, $C_{1-6}$alkyloxy, $C_{2-6}$ alkenyl, optionally substituted aryl, or optionally substituted aryl $C_{1-6}$ alkyl, or $R_3$' and $R_4$' together represent oxo, thioxo, or $C_{2-6}$ alkylene; or -O-$SiR_3$'($R_4$')-O- wherein $R_3$' and $R_4$' are as defined above,
$R_8$ represents a hydrogen atom, cyano, a halogen atom, or benzyl,
$R_{13a}$, $R_{13b}$, and $R_{13c}$, which may be the same or different, each independently represents

$C_{1-6}$ alkyl optionally substituted by a group selected from the group consisting of hydroxyl, halogen atoms, and cyano or
$C_{2-6}$ alkenyl optionally substituted by a group selected from the group consisting of hydroxyl, halogen atoms, and cyano.

**2.** The composition according to claim 1, which comprises one or more of compounds of formula (I) or salts thereof, wherein
Het represents optionally substituted pyridyl or phenyl,
X represents an oxygen atom or $NR_9$ wherein $R_9$ represents a hydrogen atom, $C_{1-6}$ alkyl, or aryl $C_{1-6}$ alkyl,
$R_2$, $R_3$, $R_{10a}$, $R_{10b}$, $R_{11}$, and $R_{12}$ represent a hydrogen atom,
$R_1$ represents a hydrogen atom, hydroxyl, optionally substituted $C_{1-18}$ alkylcarbonyloxy, optionally substituted carbamoyloxy, $C_{1-6}$ alkyloxy-$C_{1-6}$ alkyloxy, or optionally substituted saturated or unsaturated heterocyclic oxy,
$R_4$ represents hydroxyl, optionally substituted $C_{1-18}$ alkylcarbonyloxy, optionally substituted arylcarbonyloxy, optionally substituted $C_{1-18}$ alkylsulfonyloxy, optionally substituted aryl sufonyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, or optionally substituted saturated or unsaturated heterocyclic oxy,
$R_5$ represents a hydrogen atom, hydroxyl, optionally substituted $C_{1-18}$ alkylcarbonyloxy, optionally substituted arylcarbonyloxy, optionally substituted $C_{1-18}$ alkylsulfonyloxy, optionally substituted arylsufonyloxy, optionally substituted aryl $C_{1-6}$ alkyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, or optionally substituted phosphate group,

$R_6$ represents a hydrogen atom, hydroxyl, optionally substituted $C_{1-18}$ alkylcarbonyloxy, optionally substituted arylcarbonyloxy, optionally substituted aryl thiocarbonyloxy, optionally substituted $C_{1-18}$ alkylsulfonyloxy, optionally substituted carbamoyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, optionally substituted saturated or unsaturated heterocyclic oxy, or optionally substituted saturated or unsaturated heterocyclic thiocarbonyloxy,
$R_7$ represents a hydrogen atom, hydroxyl, optionally substituted $C_{1-18}$ alkylcarbonyloxy, or a halogen atom, or
$R_1$ and $R_2$ each independently together represent oxo, or
$R_1$ or $R_2$ is absent and a hydrogen atom substituted at the carbon atom of the 5-position is lost to form a double bond between the 5-position and the13-position, or
$R_6$ or $R_7$ is absent and one of hydrogen atoms substituted at the carbon atom of the 8-position is lost to form a double bond between the 7-position and the 8-position or
$R_4$ and $R_5$ together represent -O-$CR_3$'($R_4$')-O- wherein $R_3$' and $R_4$', which may be the same or different, represent a hydrogen atom or $C_{1-6}$ alkyl; or $R_3$' and $R_4$' together represent thioxo, or
$R_6$ and $R_7$ each independently together represent oxo,
$R_8$ represents a hydrogen atom or a halogen atom, and
$R_{13a}$, $R_{13b}$, and $R_{13c}$ represent methyl.

**3.** The composition according to claim 1 or 2, which comprises one or more of compounds of formula (I) or salts thereof, wherein
$R_1$, $R_2$, $R_3$, $R_7$, $R_{10a}$, $R_{10b}$, $R_{11}$, and $R_{12}$ represent a hydrogen atom, and
$R_{13a}$, $R_{13b}$, and $R_{13c}$ represent methyl.

**4.** The composition according to claim 1, which comprises one or more of compounds of formula (I) or salts thereof, wherein
Het represents optionally substituted pyridyl,
X represents an oxygen atom or $NR_9$ wherein $R_9$ represents a hydrogen atom, $C_{1-6}$ alkyl, or aryl $C_{1-6}$ alkyl,
$R_1$, $R_2$, $R_3$, $R_7$, $R_{10a}$, $R_{10b}$, $R_{11}$, and $R_{12}$ represent a hydrogen atom,
$R_4$ represents hydroxyl, optionally substituted $C_{1-18}$ alkylcarbonyloxy, optionally substituted aryl sufonyloxy, $C_{1-6}$ alkyloxy-$C_{1-6}$ alkyloxy, $C_{1-6}$ alkyloxy-$C_{1-6}$ alkyloxy-$C_{1-6}$alkyloxy, or optionally substituted saturated or unsaturated heterocyclic oxy,
$R_5$ represents a hydrogen atom, hydroxyl, optionally substituted $C_{1-18}$ alkylcarbonyloxy, optionally substituted aryl sufonyloxy, optionally substituted aryl $C_{1-6}$ alkyloxy, $C_{1-6}$ alkyloxy-$C_{1-6}$ alkyloxy, $C_{1-6}$ alkyloxy-$C_{1-6}$ alkyloxy-$C_{1-6}$ alkyloxy, or optionally substituted phosphate group, or
$R_4$ and $R_5$ together represent -O-$CR_3$'($R_4$')-O- wherein $R_3$' and $R_4$', which may be the same or different, represent a hydrogen atom or $C_{1-6}$ alkyl, or $R_3$' and $R_4$' together represent thioxo,
$R_6$ represents a hydrogen atom, hydroxyl, optionally substituted $C_{1-18}$ alkylcarbonyloxy, optionally substituted carbamoyloxy, $C_{1-6}$ alkyloxy-$C_{1-6}$alkyloxy, $C_{1-6}$ alkyloxy-$C_{1-6}$ alkyloxy-$C_{1-6}$alkyloxy, optionally substituted saturated or unsaturated heterocyclic oxy, or optionally substituted saturated or unsaturated heterocyclic thiocarbonyloxy, or
$R_6$ or $R_7$ is absent and one of hydrogen atoms substituted at the carbon atom of the 8-position is lost to form a double bond between the 7-position and the 8-position,
$R_8$ represents a hydrogen atom or a halogen atom, and
$R_{13a}$, $R_{13b}$, and $R_{13c}$ represent methyl.

**5.** The composition according to claim 1, which comprises one or more of compounds of formula (I) or salts thereof, wherein
Het represents optionally substituted pyridyl,
X represents an oxygen atom,
$R_1$, $R_2$, $R_3$, $R_7$, $R_8$, $R_{10a}$, $R_{10b}$, $R_{11}$, and $R_{12}$ represent a hydrogen atom,
$R_4$ represents hydroxyl or optionally substituted $C_{3-6}$ cycloalkylcarbonyloxy,
$R_5$ represents a hydrogen atom or optionally substituted $C_{3-6}$ cycloalkylcarbonyloxy.
$R_6$ represents a hydrogen atom, hydroxyl, or optionally substituted $C_{3-6}$ cycloalkylcarbonyloxy, or
$R_6$ or $R_7$ is absent and one of hydrogen atoms substituted at the carbon atom of the 8-position is lost to form a double bond between the 7-position and the 8-position, and
$R_{13a}$, $R_{13b}$, and $R_{13c}$ represent methyl.

**6.** A compound represented by formula (I-a') or a salt thereof:

[Chemical formula 2]

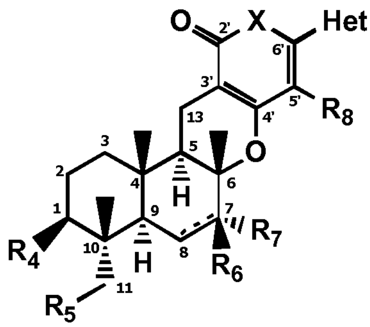

(I-a')

wherein

Het represents optionally substituted pyridyl,
X represents an oxygen atom,
$R_4$ represents hydroxyl or optionally substituted $C_{1-18}$ alkylcarbonyloxy,
$R_5$ represents a hydrogen atom, hydroxyl, or optionally substituted $C_{1-18}$ alkylcarbonyloxy or
$R_4$ and $R_5$ together represent -O-$CR_3$'($R_4$')-O-wherein $R_3$' and $R_4$', which may be the same or different, represent a hydrogen atom or $C_{1-6}$ alkyl, or $R_3$' and $R_4$' together represent thioxo,
$R_6$ represents a hydrogen atom, hydroxyl, or optionally substituted $C_{1-18}$ alkylcarbonyloxy,
$R_7$ and $R_8$ represent a hydrogen atom,
$R_6$ or $R_7$ is absent and one of hydrogen atoms substituted at the carbon atom of the 8-position is lost to form a double bond between the 7-position and the 8-position, provided that
the following compounds are excluded:

the compound wherein $R_5$, $R_6$, and $R_7$ simultaneously represent hydrogen atoms, and $R_4$ represents hydroxyl, acetyloxy, or propionyloxy,
the compound wherein $R_6$ and $R_7$ represent a hydrogen atom, and $R_4$ represents acetyloxy and $R_5$ represents propionyloxy,
the compound wherein $R_6$ and $R_7$ represent a hydrogen atom, and $R_4$ and $R_5$ represents acetyloxy,
the compound wherein $R_6$ and $R_7$ represent a hydrogen atom, and $R_4$ represents propionyloxy and $R_5$ represents acetyloxy, and
the compound wherein $R_4$ and $R_5$ represent acetyloxy, $R_6$ represents propionyloxy and $R_7$ represents a hydrogen atom.

**7.** A compound represented by formula (I-b) or a salt thereof:

[Chemical formula 3]

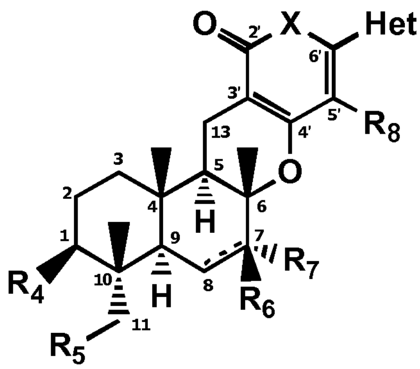

(I-b)

wherein

Het represents optionally substituted pyridyl,
X represents an oxygen atom,
$R_4$ represents hydroxyl or optionally substituted $C_{3-6}$ cycloalkylcarbonyloxy,
$R_5$ represents a hydrogen atom, hydroxyl, or optionally substituted $C_{3-6}$ cycloalkylcarbonyloxy,
$R_6$ represents a hydrogen atom, hydroxyl, or optionally substituted $C_{3-6}$ cycloalkylcarbonyloxy,
$R_7$ and $R_8$ represent a hydrogen atom, or
$R_6$ or $R_7$ is absent and one of hydrogen atoms substituted at the carbon atom of the 8-position is lost to form a double bond between the 7-position and the 8-position, provided that, when $R_5$ and $R_6$ simulatneously represent a hydrogen atom, $R_4$ does not represent hydroxyl.

**8.** A compound represented by formula (I-c) or a salt thereof:

[Chemical formula 4]

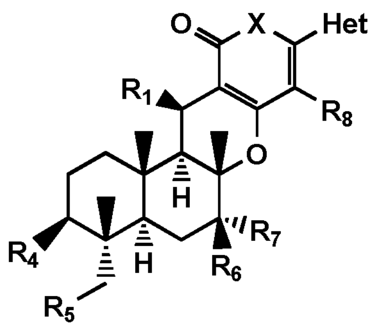

(I-c)

wherein

Het represents 3-pyridyl,
X represents an oxygen atom,
$R_1$ represents liner, branched or cyclic $C_{1-6}$ alkylcarbonyloxy, or $C_{1-6}$ alkyloxy
$R_4$ represents $C_{3-6}$ cycloalkylcarbonyloxy,
$R_5$ represents $C_{3-6}$ cycloalkylcarbonyloxy,
$R_6$ represent hydroxyl, acetyloxy or $C_{3-6}$ cycloalkylcarbonyloxy,
$R_7$ or $R_8$ is a hydrogen atom.

**9.** A composition for use as a harmful organism control agent, the composition comprising as an active ingredient a compound represented by formula (1-a') according to claim 6 or a salt thereof and an agriculturally or zootechnically acceptable carrier.

**10.** A composition for use as a harmful organism control agent, the composition comprising as an active ingredient a compound represented by formula (1-b) according to claim 7 or a salt thereof and an agriculturally or zootechnically acceptable carrier.

**11.** A composition for use as a harmful organism control agent, the composition comprising as an active ingredient a compound represented by formula (1-c) according to claim 8 or a salt thereof and an agriculturally or zootechnically acceptable carrier.

**12.** A method for controlling a harmful organism, comprising applying an effective amunt of a compound represented by formula (I) according to claim 1 or a salt thereof to the harmful organism or a habitat thereof.

**13.** A method for controlling harmful organisms, comprising applying an effective amount of a compound represented by formula (I-a') according to claim 6 or a salt thereof to a plant or soil.

**14.** A method for controlling a harmful organism, comprising applying an effective amount of a compound represented by formula (I-b) according to claim 7 or a salt thereof to the harmful organism or a habitat thereof.

**15.** A method for controlling a harmful organism, comprising applying an effective amount of a compound represented by formula (I-c) according to claim 8 or a salt thereof to the harmful organism or a habitat thereof.

**16.** A harmful organism control composition comprising as an active ingredient at least one of compounds represented by formula (I) according to claim 1 or salts thereof and other harmful organism control agent.

**17.** A harmful organism control composition comprising as an active ingredient at least one of compounds represented by formula (I-a') according to claim 6 or salts thereof and other harmful organism control agent.

**18.** A harmful organism control composition comprising as an active ingredient at least one of compounds represented by formula (I-b) according to claim 7 or salts thereof and other harmful organism control agent.

**19.** A harmful organism control composition comprising as an active ingredient at least one of compounds represented by formula (I-c) according to claim 8 or salts thereof and other harmful organism control agent.

**20.** Use of a harmful organism control composition according to any one of claims 16 to 19 for the protection of useful plants from harmful organisms.

**INTERNATIONAL SEARCH REPORT**

International application No.
PCT/JP2011/061730

A. CLASSIFICATION OF SUBJECT MATTER
*A01N43/90*(2006.01)i, *A01N47/18*(2006.01)i, *A01N55/00*(2006.01)i, *A01P7/04* (2006.01)i, *C07D491/052*(2006.01)i, *C07D493/04*(2006.01)i, *C07F7/18* (2006.01)i

According to International Patent Classification (IPC) or to both national classification and IPC

B. FIELDS SEARCHED

Minimum documentation searched (classification system followed by classification symbols)
A01N43/90, A01N47/18, A01N55/00, A01P7/04, C07D491/052, C07D493/04, C07F7/18

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2006-513233 A (Korea Research Institute of Bioscience and Biotechnology),<br>20 April 2006 (20.04.2006),<br>claims<br>& US 2006/0135564 A1 & US 2009/0182014 A1<br>& EP 1589816 A1 & WO 2004/060065 A1<br>& KR 10-2004-0063416 A & BR 200317269 A<br>& CA 2512728 A & CN 1744817 A<br>& RU 2305403 C2 & AU 2003303489 A1 | 1,2,12,16,20<br>3-7,9,10,13,14,17,18 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 July, 2011 (01.07.11) | 12 July, 2011 (12.07.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2011/061730

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y | WO 2006/129714 A1 (Meiji Seika Kaisha, Ltd.), 07 December 2006 (07.12.2006), claims; paragraphs [0011], [0096]<br>& JP 2007-211015 A & US 2006/0281780 A1<br>& US 2009/0137634 A1 & EP 1889540 A1<br>& EP 2111756 A1 & CA 2609527 A1<br>& NZ 563781 A & KR 10-2008-0012969 A<br>& CN 101188937 A & AR 53882 A<br>& ZA 200710207 A & IL 187411 D<br>& AU 2006253364 A1 & IN 200708915 P1<br>& TW 200721978 A | 1,2,8,11,12,15,16,19,20<br><br>3-7,9,10,13,14,17,18 |
| X | WO 2008/066153 A1 (Meiji Seika Kaisha, Ltd.), 05 June 2008 (05.06.2008), claims<br>& EP 2107060 A1 | 1,2,12,16,20 |
| X | WO 2009/081851 A1 (Meiji Seika Kaisha, Ltd.), 02 July 2009 (02.07.2009), claims<br>& US 2010/0281584 A1 & EP 2223599 A1<br>& AU 2008342070 A & CA 2709466 A<br>& CN 101902916 A & KR 10-2010-0094550 A<br>& TW 200936047 A | 1,2,12,16,20 |
| A | WO 2008/108491 A1 (Meiji Seika Kaisha, Ltd.), 12 September 2008 (12.09.2008), claims<br>& US 2010/0113525 A & EP 2119361 A1<br>& CA 2678542 A1 & CN 101626687 A<br>& KR 10-2009-0119915 A & IL 200477 D<br>& AU 2008221838 A1 | 1 |

Form PCT/ISA/210 (continuation of second sheet) (July 2009)

**INTERNATIONAL SEARCH REPORT**

International application No.
PCT/JP2011/061730

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

Since the invention set forth in claim 1 is not novel as described in WO 2006/129714 A1 (Meiji Seika Kaisha, Ltd.), 7 December 2006 (07.12.2006), the matter common to the inventions set forth in claims 1, 6 - 8 are also not novel. Consequently, the matter common to the inventions set forth in claims 1, 6 - 8 cannot be considered to be a special technical feature, and therefore, this international application involves two or more inventions which do not comply the requirement of unity.

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2010118397 A
- JP 4360895 A
- WO 9409417 A
- JP 8259569 A
- JP 8269062 A
- WO 2009081957 A
- WO 2004060065 A
- WO 2006129714 A
- WO 2008066153 A
- WO 2009081851 A
- JP 8269063 A
- JP 8269064 A
- US 6222100 B
- WO 0182685 A
- WO 0026390 A
- WO 9741218 A
- WO 9802526 A
- WO 9802527 A
- WO 04106529 A
- WO 0520673 A
- WO 0314357 A
- WO 0313225 A
- WO 0314356 A
- WO 0416073 A
- WO 9200377 A
- EP 0242236 A
- EP 242246 A
- US 5559024 A
- WO 02015701 A
- EP 374753 A
- WO 93007278 A
- WO 9534656 A
- EP 427529 A
- EP 451878 A
- WO 03018810 A
- WO 03052073 A
- EP 039222 A
- US 4822779 A
- US 6300348 B
- WO 200872743 A
- WO 200872783 A
- WO 2007043677 A
- WO 2007101540 A
- JP 2006131529 B
- WO 2007060839 A
- WO 2005085216 A
- WO 2007079162 A
- WO 2007026965 A
- WO 2009126668 A
- WO 2009051956 A
- WO 2007115644 A
- WO 200866153 A
- WO 2008108491 A
- JP 2008115155 A
- WO 02089579 A
- WO 02090320 A
- WO 02090321 A
- WO 04006677 A
- WO 05068423 A
- WO 05068432 A
- WO 05063694 A
- JP 8239385 A
- WO 2009022702 A
- WO 200612971 A


### Non-patent literature cited in the description


- *Journal of Antibiotics,* 1993, vol. 46 (7), 1168-9
- *Journal of Society of Synthetic Organic Chemistry,* 1998, vol. 56 (6), 478-488
- *Applied and Environmental Microbiology,* 1995, vol. 61 (12), 4429-35
- *Journal of Antibiotics,* 1997, vol. 50 (3), 229-36
- Jikken Kagaku Koza. Maruzen Company, Limited, 1992
- *Biotechnol Prog.,* July 2001, vol. 17 (4), 720-8
- *Protein Eng Des Sel.,* January 2004, vol. 17 (1), 57-66
- *Nat Protoc.,* 2007, vol. 2 (5), 1225-35
- *Curr Opin Chem Biol.,* 28 August 2006, vol. 10 (5), 487-91

- *Biomaterials,* March 2001, vol. 22 (5), 405-1 7
- *Bioconjug Chem.,* January 2005, vol. 16 (1), 113-21
- The Pesticide Manual. The British Crop Protection Council
- SHIBUYA INDEX. SHIBUYA INDEX RESEARCH GROUP, 2009
- Farm Chemicals Handbook. Meister Publishing Company, 2001, vol. 88
- *Pesticide Science,* 1988, vol. 54, 237-243
- *J. Antibiot.,* 1996, vol. 49, 292
- *J. Antibiot.,* 1997, vol. 50 (3), 229
- *J. Antibiot.,* 1996, vol. 49 (11), 1133